(19) Europäisches Patentamt
European Patent Office
Office européen des brevets

(11) EP 1 405 921 B1

(12) EUROPEAN PATENT SPECIFICATION

(45) Date of publication and mention of the grant of the patent:
**17.01.2007 Bulletin 2007/03**

(51) Int Cl.:
***C12Q 1/68*** *(2006.01)*

(21) Application number: **03022242.6**

(22) Date of filing: **01.10.2003**

# (54) Detection of susceptibility to autoimmune diseases, especially type 1 diabetes

Bestimmung der Empfindlichkeit zu Autoimmunerkrankungen, ins besondere Typ 1 Diabetes

Détection de la susceptibilité à des maladies auto-immunes, spécifiquement le diabète de type 1

(84) Designated Contracting States:
**AT BE BG CH CY CZ DE DK EE ES FI FR GB GR HU IE IT LI LU MC NL PT RO SE SI SK TR**

(30) Priority: **04.10.2002 US 264965**
**08.10.2002 US 267844**

(43) Date of publication of application:
**07.04.2004 Bulletin 2004/15**

(73) Proprietors:
- **Roche Diagnostics GmbH**
  **68305 Mannheim (DE)**
  Designated Contracting States:
  **DE**
- **F. HOFFMANN-LA ROCHE AG**
  **4070 Basel (CH)**
  Designated Contracting States:
  **AT BE BG CH CY CZ DK EE ES FI FR GB GR HU IE IT LI LU MC NL PT RO SE SI SK TR**

(72) Inventors:
- **Mirel, Daniel B.**
  **Oakland, CA 94610 (US)**
- **Erlich, Henry A.**
  **Oakland, CA 94602 (US)**
- **Bugawan, Teodorica L.**
  **Castro Valley, CA 94552 (US)**
- **Noble, Janelle A.**
  **Berkeley, CA 94702 (US)**
- **Valdes, Ana Maria**
  **40069 Zola Predosa (BO) (IT)**

(56) References cited:
**WO-A-01/23404** **WO-A-01/23410**

- **OHKUBO T ET AL: "A novel polymorphism in the promoter region of the IL-4 gene is associated with type 1 diabetes in Japanese" DIABETOLOGIA, vol. 44, no. Supplement 1, August 2001 (2001-08), page A 80 XP002266508 37th Annual Meeting of the European Association for the Study of Diabetes;Glasgow, Scotland, UK; September 09-13, 2001 ISSN: 0012-186X**
- **BUGAWAN T L ET AL: "Association of specific IL4 receptor SNPs with type I diabetes in Filipinos" AMERICAN JOURNAL OF HUMAN GENETICS, vol. 69, no. 4 Supplement, October 2001 (2001-10), page 403 XP002266510 51st Annual Meeting of the American Society of Human Genetics;San Diego, California, USA; October 12-16, 2001 ISSN: 0002-9297**
- **BUGAWAN TEODORICA L ET AL: "Association and interaction of the IL4R, IL4, and IL13 loci with type 1 diabetes among Filipinos." AMERICAN JOURNAL OF HUMAN GENETICS, vol. 72, no. 6, June 2003 (2003-06), pages 1505-1514, XP002266511 ISSN: 0002-9297**
- **HACKSTEIN H ET AL: "Definition of human interleukin-4 receptor alpha chain haplotypes and allelic association with atopy markers" HUMAN IMMUNOLOGY, NEW YORK, NY, US, vol. 60, no. 11, November 1999 (1999-11), pages 1119-1127, XP002243327 ISSN: 0198-8859**
- **EMIKO NOGUCHI ET AL: "Haplotypes of the 5' region of the IL-4 gene and SNPs in the intergene sequence between the IL-4 and IL-13 genes are associated with atopic asthma" HUMAN IMMUNOLOGY, NEW YORK, NY, US, vol. 62, 2001, pages 1251-1257, XP002957169 ISSN: 0198-8859**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- JAHROMI MOHAMMED ET AL: "A CA repeat polymorphism of the IFN-gamma gene is associated with susceptibility to type 1 diabetes" JOURNAL OF INTERFERON AND CYTOKINE RESEARCH, vol. 20, no. 2, February 2000 (2000-02), pages 187-190, XP008026906 ISSN: 1079-9907
- OLAVESEN ET AL: "Analysis of single nucleotide polymorphisms in the interleukin-4 receptor gene for association wih inflammatory bowel disease" IMMUNOGENETICS, vol. 51, 2000, pages 1-7,
- DEICHMANN ET AL: "Common polymorphisms in the coding part of the IL-4 receptor gene" BIOCHEMICAL AND BIOPHYSICAL RESEARCH COMMUNICATIONS, vol. 231, 1997, pages 696-697,

SEQUENCE LISTING

<110> F. Hoffmann-La Roche AG

<120> DETECTION OF SUSCEPTIBILITY TO AUTOIMMUNE DISEASES

<130> case 21907

<150> US 10/267,844
<151> 2002-10-08

<150> US 10/264,965
<151> 2002-10-04

<160> 90

<170> PatentIn version 3.2

<210> 1
<211> 3597
<212> DNA
<213> Homo sapiens

<400> 1

ggcgaatgga gcaggggcgc gcagataatt aaagatttac acacagctgg aagaaatcat 60
agagaagccg ggcgtggtgg ctcatgccta taatcccagc acttttggag gctgaggcgg 120
gcagatcact tgagatcagg agttcgagac cagcctggtg ccttggcatc tcccaatggg 180
gtggctttgc tctgggctcc tgttccctgt gagctgcctg gtcctgctgc aggtggcaag 240
ctctgggaac atgaaggtct tgcaggagcc cacctgcgtc tccgactaca tgagcatctc 300
tacttgcgag tggaagatga atggtcccac caattgcagc accgagctcc gcctgttgta 360
ccagctggtt tttctgctct ccgaagccca cacgtgtatc cctgagaaca acggaggcgc 420
ggggtgcgtg tgccacctgc tcatggatga cgtggtcagt gcggataact atacactgga 480
cctgtgggct gggcagcagc tgctgtggaa gggctccttc aagcccagcg agcatgtgaa 540
acccagggcc ccaggaaacc tgacagttca caccaatgtc tccgacactc tgctgctgac 600
ctggagcaac ccgtatcccc ctgacaatta cctgtataat catctcacct atgcagtcaa 660
catttggagt gaaaacgacc cggcagattt cagaatctat aacgtgacct acctagaacc 720
ctccctccgc atcgcagcca gcaccctgaa gtctgggatt tcctacaggg cacgggtgag 780
ggcctgggct cagtgctata acaccacctg gagtgagtgg agccccagca ccaagtggca 840
caactcctac agggagccct tcgagcagca cctcctgctg ggcgtcagcg tttcctgcat 900
tgtcatcctg gccgtctgcc tgttgtgcta tgtcagcatc accaagatta agaaagaatg 960
gtgggatcag attcccaacc cagcccgcag ccgcctcgtg gctataataa tccaggatgc 1020
tcaggggtca cagtgggaga agcggtcccg aggccaggaa ccagccaagt gcccacactg 1080

gaagaattgt cttaccaagc tcttgccctg ttttctggag cacaacatga aaagggatga 1140

agatcctcac aaggctgcca aagagatgcc tttccagggc tctggaaaat cagcatggtg 1200

cccagtggag atcagcaaga cagtcctctg gccagagagc atcagcgtgg tgcgatgtgt 1260

ggagttgttt gaggccccgg tggagtgtga ggaggaggag gaggtagagg aagaaaaagg 1320

gagcttctgt gcatcgcctg agagcagcag ggatgacttc caggagggaa gggagggcat 1380

tgtggcccgg ctaacagaga gcctgttcct ggacctgctc ggagaggaga atgggggctt 1440

ttgccagcag gacatggggg agtcatgcct tcttccacct tcgggaagta cgagtgctca 1500

catgccctgg gatgagttcc caagtgcagg gcccaaggag gcacctccct ggggcaagga 1560

gcagcctctc cacctggagc caagtcctcc tgccagcccg acccagagtc cagacaacct 1620

gacttgcaca gagacgcccc tcgtcatcgc aggcaaccct gcttaccgca gcttcagcaa 1680

ctccctgagc cagtcaccgt gtcccagaga gctgggtcca gacccactgc tggccagaca 1740

cctggaggaa gtagaacccg agatgccctg tgtcccccag ctctctgagc caaccactgt 1800

gccccaacct gagccagaaa cctgggagca gatcctccgc cgaaatgtcc tccagcatgg 1860

ggcagctgca gcccccgtct cggcccccac cagtggctat caggagtttg tacatgcggt 1920

ggagcagggt ggcacccagg ccagtgcggt ggtgggcttg ggtcccccag gagaggctgg 1980

ttacaaggcc ttctcaagcc tgcttgccag cagtgctgtg tccccagaga aatgtgggtt 2040

tggggctagc agtggggaag aggggtataa gcctttccaa gacctcattc ctggctgccc 2100

tggggaccct gccccagtcc ctgtcccctt gttcaccttt ggactggaca gggagccacc 2160

tcgcagtccg cagagctcac atctcccaag cagctcccca gagcacctgg gtctggagcc 2220

gggggaaaag gtagaggaca tgccaaagcc cccacttccc caggagcagg ccacagaccc 2280

ccttgtggac agcctgggca gtggcattgt ctactcagcc cttacctgcc acctgtgcgg 2340

ccacctgaaa cagtgtcatg gccaggagga tggtggccag acccctgtca tggccagtcc 2400

ttgctgtggc tgctgctgtg gagacaggtc ctcgccccct acaacccccc tgagggcccc 2460

agacccctct ccaggtgggg ttccactgga ggccagtctg tgtccggcct ccctggcacc 2520

ctcgggcatc tcagagaaga gtaaatcctc atcatccttc catcctgccc ctggcaatgc 2580

tcagagctca agccagaccc ccaaaatcgt gaactttgtc tccgtgggac ccacatacat 2640

gagggtctct taggtgcatg tcctcttgtt gctgagtctg cagatgagga ctagggctta 2700

tccatgcctg ggaaatgcca cctcctggaa ggcagccagg ctggcagatt tccaaaagac 2760

ttgaagaacc atggtatgaa ggtgattggc cccactgacg ttggcctaac actgggctgc 2820

agagactgga ccccgcccag cattgggctg ggctcgccac atcccatgag agtagagggc 2880

```
actgggtcgc cgtgccccac ggcaggcccc tgcaggaaaa ctgaggccct tgggcacctc   2940
gacttgtgaa cgagttgttg gctgctccct ccacagcttc tgcagcagac tgtccctgtt   3000
gtaactgccc aaggcatgtt ttgcccacca gatcatggcc cacgtggagg cccacctgcc   3060
tctgtctcac tgaactagaa gccgagccta gaaactaaca cagccatcaa gggaatgact   3120
tgggcggcct tgggaaatcg atgagaaatt gaacttcagg gagggtggtc attgcctaga   3180
ggtgctcatt catttaacag agcttcctta ggttgatgct ggaggcagaa tcccggctgt   3240
caaggggtgt tcagttaagg ggagcaacag aggacatgaa aaattgctat gactaaagca   3300
gggacaattt gctgccaaac acccatgccc agctgtatgg ctgggggctc ctcgtatgca   3360
tggaaccccc agaataaata tgctcagcca ccctgtgggc cgggcaatcc agacagcagg   3420
cataaggcac cagttaccct gcatgttggc ccagacctca ggtgctaggg aaggcgggaa   3480
ccttgggttg agtaatgctc gtctgtgtgt tttagtttca tcacctgtta tctgtgtttg   3540
ctgaggagag tggaacagaa ggggtggagt tttgtataaa taaagtttct ttgtctc      3597
```

<210> 2
<211> 2478
<212> DNA
<213> Homo sapiens

<400> 2

atggggtggc tttgctctgg gctcctgttc cctgtgagct gcctggtcct gctgcaggtg 60
gcaagctctg ggaacatgaa ggtcttgcag gagcccacct gcgtctccga ctacatgagc 120
atctctactt gcgagtggaa gatgaatggt cccaccaatt gcagcaccga gctccgcctg 180
ttgtaccagc tggtttttct gctctccgaa gcccacacgt gtatccctga gaacaacgga 240
ggcgcggggt gcgtgtgcca cctgctcatg gatgacgtgg tcagtgcgga taactataca 300
ctggacctgt gggctgggca gcagctgctg tggaagggct ccttcaagcc cagcgagcat 360
gtgaaaccca gggccccagg aaacctgaca gttcacacca atgtctccga cactctgctg 420
ctgacctgga gcaacccgta tccccctgac aattacctgt ataatcatct cacctatgca 480
gtcaacattt ggagtgaaaa cgacccggca gatttcagaa tctataacgt gacctaccta 540
gaaccctccc tccgcatcgc agccagcacc ctgaagtctg ggatttccta cagggcacgg 600
gtgagggcct gggctcagtg ctataacacc acctggagtg agtggagccc cagcaccaag 660
tggcacaact cctacaggga gccccttcgag cagcacctcc tgctgggcgt cagcgtttcc 720
tgcattgtca tcctggccgt ctgcctgttg tgctatgtca gcatcaccaa gattaagaaa 780
gaatggtggg atcagattcc caacccagcc cgcagccgcc tcgtggctat aataatccag 840
gatgctcagg ggtcacagtg ggagaagcgg tcccgaggcc aggaaccagc caagtgccca 900

cactggaaga attgtcttac caagctcttg ccctgttttc tggagcacaa catgaaaagg 960

gatgaagatc ctcacaaggc tgccaaagag atgcctttcc agggctctgg aaaatcagca 1020

tggtgcccag tggagatcag caagacagtc ctctggccag agagcatcag cgtggtgcga 1080

tgtgtggagt tgtttgaggc cccggtggag tgtgaggagg aggaggaggt agaggaagaa 1140

aaagggagct tctgtgcatc gcctgagagc agcagggatg acttccagga gggaagggag 1200

ggcattgtgg cccggctaac agagagcctg ttcctggacc tgctcggaga ggagaatggg 1260

ggcttttgcc agcaggacat gggggagtca tgccttcttc caccttcggg aagtacgagt 1320

gctcacatgc cctgggatga gttcccaagt gcagggccca aggaggcacc tccctggggc 1380

aaggagcagc ctctccacct ggagccaagt cctcctgcca gcccgaccca gagtccagac 1440

aacctgactt gcacagagac gcccctcgtc atcgcaggca accctgctta ccgcagcttc 1500

agcaactccc tgagccagtc accgtgtccc agagagctgg gtccagaccc actgctggcc 1560

agacacctgg aggaagtaga acccgagatg ccctgtgtcc cccagctctc tgagccaacc 1620

actgtgcccc aacctgagcc agaaacctgg gagcagatcc tccgccgaaa tgtcctccag 1680

catggggcag ctgcagcccc cgtctcggcc cccaccagtg gctatcagga gtttgtacat 1740

gcggtggagc agggtggcac ccaggccagt gcggtggtgg gcttgggtcc cccaggagag 1800

gctggttaca aggccttctc aagcctgctt gccagcagtg ctgtgtcccc agagaaatgt 1860

gggtttgggg ctagcagtgg ggaagagggg tataagcctt tccaagacct cattcctggc 1920

tgccctgggg accctgcccc agtccctgtc cccttgttca cctttggact ggacagggag 1980

ccacctcgca gtccgcagag ctcacatctc ccaagcagct ccccagagca cctgggtctg 2040

gagccggggg aaaaggtaga ggacatgcca aagcccccac ttccccagga gcaggccaca 2100

gacccccttg tggacagcct gggcagtggc attgtctact cagcccttac ctgccacctg 2160

tgcggccacc tgaaacagtg tcatggccag gaggatggtg gccagacccc tgtcatggcc 2220

agtccttgct gtggctgctg ctgtggagac aggtcctcgc cccctacaac ccccctgagg 2280

gccccagacc cctctccagg tggggttcca ctggaggcca gtctgtgtcc ggcctccctg 2340

gcaccctcgg gcatctcaga gaagagtaaa tcctcatcat ccttccatcc tgcccctggc 2400

aatgctcaga gctcaagcca gacccccaaa atcgtgaact ttgtctccgt gggacccaca 2460

tacatgaggg tctcttag 2478

<210> 3
<211> 21
<212> DNA
<213> Artificial sequence

<220>
<223> Description of artificial sequence: Probe used to identify IL4R polymorphisms

<400> 3
ccacacgtgt atccctgaga a 21

<210> 4
<211> 19
<212> DNA
<213> Artificial sequence

<220>
<223> Description of artificial sequence: Probe used to identify IL4R polymorphisms

<400> 4
tctcagggac acacgtgtg 19

<210> 5
<211> 22
<212> DNA
<213> Artificial sequence

<220>
<223> Description of artificial sequence: Probe used to identify IL4R polymorphisms

<400> 5
tggagtgaaa acgacccggc ag 22

<210> 6
<211> 21
<212> DNA
<213> Artificial sequence

<220>
<223> Description of artificial sequence: Probe used to identify IL4R polymorphisms

<400> 6
ctgccgggtc attttcgctc c 21

<210> 7
<211> 21
<212> DNA
<213> Artificial sequence

<220>
<223> Description of artificial sequence: Probe used to identify IL4R polymorphisms

<400> 7
gagggaaggg agggcattgt g 21

<210> 8
<211> 19
<212> DNA
<213> Artificial sequence

<220>
<223> Description of artificial sequence: Probe used to identify IL4R polymorphisms

<400> 8
agggaagggc gggcattgt 19

<210> 9
<211> 19
<212> DNA
<213> Artificial sequence

<220>
<223> Description of artificial sequence: Probe used to identify IL4R polymorphisms

<400> 9
ctctccgagc aggtccagg 19

<210> 10
<211> 21
<212> DNA
<213> Artificial sequence

<220>
<223> Description of artificial sequence: Probe used to identify IL4R polymorphisms

<400> 10
tcctggacct tctcggagag g 21

<210> 11
<211> 23
<212> DNA
<213> Artificial sequence

<220>
<223> Description of artificial sequence: Probe used to identify IL4R polymorphisms

<400> 11
aaggtggaag aaggcatgac tcc 23

<210> 12
<211> 23
<212> DNA
<213> Artificial sequence

<220>
<223> Description of artificial sequence: Probe used to identify IL4R polymorphisms

<400> 12
aaggtgggag aagacatgac tcc 23

<210> 13
<211> 23
<212> DNA
<213> Artificial sequence

<220>
<223> Description of artificial sequence: Probe used to identify IL4R polymorphisms

<400> 13
ggagtcacgt cttctcctac ctt 23

<210> 14
<211> 22
<212> DNA
<213> Artificial sequence

<220>
<223> Description of artificial sequence: Probe used to identify IL4R polymorphisms

<400> 14
tggctcagag agttgctgaa gc 22

<210> 15
<211> 19
<212> DNA
<213> Artificial sequence

<220>
<223> Description of artificial sequence: Probe used to identify IL4R polymorphisms

<400> 15
ttcagcaacc ccctgagcc 19

<210> 16
<211> 20
<212> DNA
<213> Artificial sequence

<220>
<223> Description of artificial sequence: Probe used to identify IL4R polymorphisms

<400> 16
agtggctatc aggagtttgt 20

<210> 17
<211> 20
<212> DNA
<213> Artificial sequence

<220>
<223> Description of artificial sequence: Probe used to identify IL4R polymorphisms

<400> 17
agtggctatc gggagtttgt 20

<210> 18
<211> 21
<212> DNA
<213> Artificial sequence

<220>
<223> Description of artificial sequence: Probe used to identify IL4R polymorphisms

<400> 18
ctcttctctg agatgcccga g 21

<210> 19
<211> 21
<212> DNA

<213> Artificial sequence

<220>
<223> Description of artificial sequence: Probe used to identify IL4R polymorphisms

<400> 19
ctcgggcatc ccagagaaga g 21

<210> 20
<211> 8
<212> DNA
<213> Artificial sequence

<220>
<223> Description of artificial sequence: Sequences used to determine computationally estimated haplotype frequencies compared between Filipino controls and diabetics

<400> 20
acatttat 8

<210> 21
<211> 8
<212> DNA
<213> Artificial sequence

<220>
<223> Description of artificial sequence: Sequences used to determine computationally estimated haplotype frequencies compared between Filipino controls and diabetics

<400> 21
acagttgt 8

<210> 22
<211> 8
<212> DNA
<213> Artificial sequence

<220>
<223> Description of artificial sequence: Sequences used to determine computationally estimated haplotype frequencies compared between Filipino controls and diabetics

<400> 22
acctccgt 8

<210> 23
<211> 8
<212> DNA
<213> Artificial sequence

<220>
<223> Description of artificial sequence: Sequences used to determine computationally estimated haplotype frequencies compared between Filipino controls and diabetics

<400> 23
gcagttat 8

<210> 24
<211> 8

<212> DNA
<213> Artificial sequence

<220>
<223> Description of artificial sequence: Sequences used to determine computationally estimated haplotype frequencies compared between Filipino controls and diabetics

<400> 24
gccgccgt 8

<210> 25
<211> 19
<212> DNA
<213> Artificial sequence

<220>
<223> Description of artificial sequence: Amplicon primer

<400> 25
cagcccctgt gtctgcaga 19

<210> 26
<211> 20
<212> DNA
<213> Artificial sequence

<220>
<223> Description of artificial sequence: Amplicon primer

<400> 26
ctgacctgga gcaacccgta 20

<210> 27
<211> 25
<212> DNA
<213> Artificial sequence

<220>
<223> Description of artificial sequence: Amplicon primer

<400> 27
attgtgtgag gaggaggagg aggta 25

<210> 28
<211> 17
<212> DNA
<213> Artificial sequence

<220>
<223> Description of artificial sequence: Amplicon primer

<400> 28
ctcgtcatcg caggcaa 17

<210> 29
<211> 19
<212> DNA
<213> Artificial sequence

<220>
<223> Description of artificial sequence: Amplicon primer

<400> 29
gccgaaatgt cctccagca 19

<210> 30
<211> 15
<212> DNA
<213> Artificial sequence

<220>
<223> Description of artificial sequence: Amplicon primer

<400> 30
ccggcctccc tggca 15

<210> 31
<211> 26
<212> DNA
<213> Artificial sequence

<220>
<223> Description of artificial sequence: Amplicon primer

<400> 31
gtccagtgta tagttatccg cactga 26

<210> 32
<211> 19
<212> DNA
<213> Artificial sequence

<220>
<223> Description of artificial sequence: Amplicon primer

<400> 32
actgggcctc tgctggtca 19

<210> 33
<211> 22
<212> DNA
<213> Artificial sequence

<220>
<223> Description of artificial sequence: Amplicon primer

<400> 33
gttgggcatg tgagcactcg ta 22

<210> 34
<211> 18
<212> DNA
<213> Artificial sequence

<220>
<223> Description of artificial sequence: Amplicon primer

<400> 34
agggcatctc gggttcta 18

<210> 35
<211> 21
<212> DNA
<213> Artificial sequence

<220>
<223> Description of artificial sequence: Amplicon primer

<400> 35
ccacatttct ctggggacac a 21

<210> 36
<211> 20
<212> DNA
<213> Artificial sequence

<220>
<223> Description of artificial sequence: Amplicon primer

<400> 36
gcagactcag caacaagagg 20

<210> 37
<211> 21
<212> DNA
<213> Artificial sequence

<220>
<223> Description of artificial sequence: Hybridization probe

<400> 37
ccacacgtgt atccctgaga a 21

<210> 38
<211> 22
<212> DNA
<213> Artificial sequence

<220>
<223> Description of artificial sequence: Hybridization probe

<400> 38
tggagtgaaa acgacccggc ag 22

<210> 39
<211> 21
<212> DNA
<213> Artificial sequence

<220>
<223> Description of artificial sequence: Hybridization probe

<400> 39
gagggaaggg agggcattgt g 21

<210> 40
<211> 19
<212> DNA
<213> Artificial sequence

<220>
<223> Description of artificial sequence: Hybridization probe

<400> 40
ctctccgagc aggtccagg 19

<210> 41
<211> 23
<212> DNA
<213> Artificial sequence

<220>
<223> Description of artificial sequence: Hybridization probe

<400> 41
aaggtggaag aaggcatgac tcc 23

<210> 42
<211> 23
<212> DNA
<213> Artificial sequence

<220>
<223> Description of artificial sequence: Hybridization probe

<400> 42
aaggtgggag aagacatgac tcc 23

<210> 43
<211> 22
<212> DNA
<213> Artificial sequence

<220>
<223> Description of artificial sequence: Hybridization probe

<400> 43
tggctcagag agttgctgaa gc 22

<210> 44
<211> 20
<212> DNA
<213> Artificial sequence

<220>
<223> Description of artificial sequence: Hybridization probe

<400> 44
agtggctatc aggagtttgt 20

<210> 45
<211> 21
<212> DNA

<213> Artificial sequence

<220>
<223> Description of artificial sequence: Hybridization probe

<400> 45
ctcttctctg agatgcccga g 21

<210> 46
<211> 19
<212> DNA
<213> Artificial sequence

<220>
<223> Description of artificial sequence: Hybridization probe

<400> 46
tctcagggac acacgtgtg 19

<210> 47
<211> 21
<212> DNA
<213> Artificial sequence

<220>
<223> Description of artificial sequence: Hybridization probe

<400> 47
ctgccgggtc attttcgctc c 21

<210> 48
<211> 19
<212> DNA
<213> Artificial sequence

<220>
<223> Description of artificial sequence: Hybridization probe

<400> 48
agggaagggc gggcattgt 19

<210> 49
<211> 21
<212> DNA
<213> Artificial sequence

<220>
<223> Description of artificial sequence: Hybridization probe

<400> 49
tcctggacct tctcggagag g 21

<210> 50
<211> 23
<212> DNA
<213> Artificial sequence

<220>

<223> Description of artificial sequence: Hybridization probe

<400> 50
ggagtcacgt cttctcctac ctt 23

<210> 51
<211> 19
<212> DNA
<213> Artificial sequence

<220>
<223> Description of artificial sequence: Hybridization probe

<400> 51
ttcagcaacc ccctgagcc 19

<210> 52
<211> 20
<212> DNA
<213> Artificial sequence

<220>
<223> Description of artificial sequence: Hybridization probe

<400> 52
agtggctatc gggagtttgt 20

<210> 53
<211> 21
<212> DNA
<213> Artificial sequence

<220>
<223> Description of artificial sequence: Hybridization probe

<400> 53
ctcgggcatc ccagagaaga g 21

<210> 54
<211> 21
<212> DNA
<213> Artificial sequence

<220>
<223> Description of artificial sequence: allele-specific PCR primer

<400> 54
ccacatttct ctggggacac a 21

<210> 55
<211> 25
<212> DNA
<213> Artificial sequence

<220>
<223> Description of artificial sequence: allele-specific PCR primer

<400> 55

attgtgtgag gaggaggagg aggta 25

<210> 56
<211> 25
<212> DNA
<213> Artificial sequence

<220>
<223> Description of artificial sequence: allele-specific PCR primer

<400> 56
attgtgtgag gaggaggagg aggta 25

<210> 57
<211> 17
<212> DNA
<213> Artificial sequence

<220>
<223> Description of artificial sequence: allele-specific PCR primer

<400> 57
ttccaggagg gaaggga 17

<210> 58
<211> 20
<212> DNA
<213> Artificial sequence

<220>
<223> Description of artificial sequence: allele-specific PCR primer

<400> 58
caccgcatgt acaaactcct 20

<210> 59
<211> 17
<212> DNA
<213> Artificial sequence

<220>
<223> Description of artificial sequence: allele-specific PCR primer

<400> 59
ggtgactggc tcaggga 17

<210> 60
<211> 17
<212> DNA
<213> Artificial sequence

<220>
<223> Description of artificial sequence: allele-specific PCR primer

<400> 60
ttccaggagg gaagggc 17

<210> 61

<211> 20
<212> DNA
<213> Artificial sequence

<220>
<223> Description of artificial sequence: allele-specific PCR primer

<400> 61
caccgcatgt acaaactccc 20

<210> 62
<211> 17
<212> DNA
<213> Artificial sequence

<220>
<223> Description of artificial sequence: allele-specific PCR primer

<400> 62
ggtgactggc tcagggg 17

<210> 63
<211> 18
<212> DNA
<213> Artificial sequence

<220>
<223> Description of artificial sequence: Probe used to identify IL4 and IL13 polymorphisms

<400> 63
aacattgtcc cccagtgc 18

<210> 64
<211> 20
<212> DNA
<213> Artificial sequence

<220>
<223> Description of artificial sequence: Probe used to identify IL4 and IL13 polymorphisms

<400> 64
agcactgggg aacaatgttc 20

<210> 65
<211> 20
<212> DNA
<213> Artificial sequence

<220>
<223> Description of artificial sequence: Probe used to identify IL4 and IL13 polymorphisms

<400> 65
ttctactcac gtgctgacct 20

<210> 66
<211> 21
<212> DNA
<213> Artificial sequence

<220>
<223> Description of artificial sequence: Probe used to identify IL4 and IL13 polymorphisms

<400> 66
ggtcagcaca tgagtagaac g 21

<210> 67
<211> 19
<212> DNA
<213> Artificial sequence

<220>
<223> Description of artificial sequence: Probe used to identify IL4 and IL13 polymorphisms

<400> 67
tcagttgaac cgtccctcg 19

<210> 68
<211> 21
<212> DNA
<213> Artificial sequence

<220>
<223> Description of artificial sequence: Probe used to identify IL4 and IL13 polymorphisms

<400> 68
gagggacagt tcaactgaaa c 21

<210> 69
<211> 21
<212> DNA
<213> Artificial sequence

<220>
<223> Description of artificial sequence: Amplicon primer

<400> 69
actaggcctc acctgatacg a 21

<210> 70
<211> 20
<212> DNA
<213> Artificial sequence

<220>
<223> Description of artificial sequence: Amplicon primer

<400> 70
ctcggacatg caagctggaa 20

<210> 71
<211> 22
<212> DNA
<213> Artificial sequence

<220>
<223> Description of artificial sequence: Amplicon primer

<400> 71
aatcgaggtg gcccagtttg ta 22

<210> 72
<211> 24
<212> DNA
<213> Artificial sequence

<220>
<223> Description of artificial sequence: Amplicon primer

<400> 72
catagaggca gaataacagg caga 24

<210> 73
<211> 22
<212> DNA
<213> Artificial sequence

<220>
<223> Description of artificial sequence: Amplicon primer

<400> 73
actgaatgag acagtccctg ga 22

<210> 74
<211> 21
<212> DNA
<213> Artificial sequence

<220>
<223> Description of artificial sequence: Amplicon primer

<400> 74
cctaaccctc cttcccgcct a 21

<210> 75
<211> 26
<212> DNA
<213> Artificial sequence

<220>
<223> Description of artificial sequence: Amplicon primer

<400> 75
actgacttat ctttactgtc acttct 26

<210> 76
<211> 19
<212> DNA
<213> Artificial sequence

<220>
<223> Description of artificial sequence: Amplicon primer

<400> 76
gcaagacagc caccaaccc 19

<210> 77
<211> 24
<212> DNA
<213> Artificial sequence

<220>
<223> Description of artificial sequence: Amplicon primer

<400> 77
tgacttatct ttactgtcac ttcc 24

<210> 78
<211> 0
<212> PRT
<213> Homo sapiens

<400> 78
000

<210> 79
<211> 18
<212> DNA
<213> Artificial sequence

<220>
<223> Description of artificial sequence: Amplicon primer

<400> 79
cctgctccca ggactgac 18

<210> 80
<211> 24
<212> DNA
<213> Artificial sequence

<220>
<223> Description of artificial sequence: Amplicon primer

<400> 80
cccagacttt atctgtgact gctc 24

<210> 81
<211> 18
<212> DNA
<213> Artificial sequence

<220>
<223> Description of artificial sequence: Amplicon primer

<400> 81
cctgctccca ggactgat 18

<210> 82
<211> 17
<212> DNA
<213> Artificial sequence

<220>

<223> Description of artificial sequence: Amplicon primer

<400> 82
ggaaacaggc ccgtaga 17

<210> 83
<211> 18
<212> DNA
<213> Artificial sequence

<220>
<223> Description of artificial sequence: Amplicon primer

<400> 83
gagtgccgct acttggcc 18

<210> 84
<211> 16
<212> DNA
<213> Artificial sequence

<220>
<223> Description of artificial sequence: Amplicon primer

<400> 84
gaaacaggcc cgtagc 16

<210> 85
<211> 22
<212> DNA
<213> Artificial sequence

<220>
<223> Description of artificial sequence: Amplicon primer

<400> 85
tctggaggac ttctaggaaa ac 22

<210> 86
<211> 18
<212> DNA
<213> Artificial sequence

<220>
<223> Description of artificial sequence: Amplicon primer

<400> 86
tgcagccatg tcgccttt 18

<210> 87
<211> 22
<212> DNA
<213> Artificial sequence

<220>
<223> Description of artificial sequence: Amplicon primer

<400> 87

tctggaggac ttctaggaaa at 22

<210> 88
<211> 130020
<212> DNA
<213> Homo sapiens

<400> 88

```
aagcttcact ctcacgctct gtggtgtgac atccactcag caaagaggtg caaacctgga     60
cccccagagt gtgaatcaca gacacctctt ataccccagc ccctctgctg ggaatctagc    120
ggcagaaact cccgagctca tgggcggggc cttcctgagg gcaggctccc tttgcagcct    180
gtgtttgcct ctggggccgc tttgggtaca cacatacaca cacacacaca cacacacaca    240
ccatgccaaa gccacctccc aggcccttcc atgccctgcc tagttctgtt ttgttttgtt    300
tgagacggag tctcactctg acgcccaggc tggagtgcgg tggcatgatc ttggctcatt    360
gcaacctctg tctcccaggt tcaaacaatt ctcctgcctc agcctcccga gtagccggga    420
ttacgggcgt gtgccaccac gcccggcaaa tttttttgta tttttagtag agatggggtt    480
tcaccatgtt gcccaggctg gtctcgaaca cctgacctca aacgatccac ctgcctcagc    540
ttcccaaagt gctgggatta gaggcgtgag ccacagtgcc tggcccctgc cctggttctt    600
gttttgtgtt ttgagatgag gtctcgctat attgcccagg ttggtcttaa agccccaggt    660
tcaagcaatc ctcctgcctc agccttccaa agtgttggaa ttacaggcat gagccacctc    720
gccctgccac ctgccctaat tctgaactca cagataaaac tgctcccacc aaccaggccc    780
tcctgcccct gggacccccc tctacctcca gcctggaggg aaattccagg cctccctgga    840
```

gcttatgaag gggaaaaaac ccaagagggt ttttttttc tgtaactcag tgtcgtccat 900

tcgcctcatt atggggtcag gatcccagaa agtcagggtt aaaaggacat ttgctctaca 960

aatagggaag ctgtcccaga ggtaggtatg cctcatccat ggtcacacag atgggcagag 1020

ctgagccgga acacgaaccc agaagtcccg ggtcccagcc tagctccttc aatacctctc 1080

aaacctatta tcaacctttc acactttagt aaaaaatagc gcaactcaaa acagaactct 1140

aaggtgtagt gaggacatag gccaggtcag atctgatgga aggaggagag ctggcccaga 1200

gggcagggag taggggaccc agcccctacc tataatccta gcactttggg aggccaaggc 1260

aagtggatcg ctggagccca ggagttcgag actggcctgg gcaacatggc aagatccccg 1320

tctctactaa aaatacaaaa attagctggg tgtagtggtg tacacctgtt gtcccagcta 1380

cacgggaggc tgaggtggga ggatcacctg agcccagaga ggtcagggct gcggtgagct 1440

aagattgagt gccaccctaa gcctggggtc ccaggatact cactgctctc agccaggaga 1500

aagctgccac actcctggga gtagttgcca gactggtctg tgatgttgac actgaaaatg 1560

tcgtcggcca tgaagtggaa tacatccatg tggcaggtgt aggtggcatg cgtggcattg 1620

tgggccgacc tgtggaggct gcaggaggtg gcctcgtcct tcagctcttc atactggtct 1680

tgccttcaag gacaaagcag ccggtcatgg ctggaagcca ggggcagggg ggatggtggt 1740

gggctcagtg ctcagggcca gggcagaagc ccagactctc agggctggga gggatttgag 1800

gcagggctgg ggggtggtca cctggtccaa ccaacctttt cccagggaag gtatcacctc 1860

ccctacagca tcctcaatga attgtgtccg ggctcagctt gcacacctcc caggggccag 1920

gaactcacta cctcatttag ggcagcagct ctcaattttg gaaatttggg ccattcttgc 1980

ttggactaag cctaaatctg tccctgttgg tcccaacaac cagatggctg tcctgcagga 2040

agggcgctag gcattttggg tttccttctc tcctttctgt ccaacatcca ttttttgag 2100

acagggtctc actctggccc caggctggag tatagtagtg tgatcatggc ttactgcagc 2160

ctcgaccttc ccaggctcag ctgatcctcc cacctcagcc tcctcagtac ctgggactac 2220

atgcatgcac caccatgccc agctaacttt ttgtattttt tgtagagatg gggtcccacc 2280

atgttgctta ggctggtctc aaactcctgg gctcaagtga tctgccagcc ttggcctccc 2340

aaagtgctgg gattacagga ctgagccacc tcgcccagtc cccaaatcca ctgaatatct 2400

aataatagcc tgtgctgagg gccaggaaaa gagctaaatg aacctcatct cctcctctgg 2460

gtggacttct gggaaagttg gtgggatggg attagggaaa atgctgtggc tttttaactc 2520

ccactggaca aaaatgatgg gttttgaagt atgtatagta gtttaccgga tattaatttc 2580

cctacctaga tgttcaggat ctgaacatga ccactctcta ggcccagtag ggatgtgtgg 2640

actgagttct ctgccaaatt tgctggaata caagttcctg ctgaataaat gctctccaag 2700
cctcccagcc tgaacttgct ccttattccc cctcacctgc ctcactccac tccagccaca 2760
ctgacctcct ccctattcct caaacgcacc agctcttctc ccacagggcc tttgcactgg 2820
cagtgccctc tgcccagctc cctggcttcc tccttctcac tagcagttgt ggtcccttta 2880
cctgaggtcc agtgttctgt ccttgatata gcacccagta ttattagaaa gtatcctgtt 2940
tggggcagaa cacggtggct tatgcctgta atcccaacac tttgggaggc tggccatgcg 3000
gcagaccggc catcccgggg tctcaaaacc atgggcgacc tgtgtgggcg agagagtggt 3060
ctttgaagct ggtggataga ccaaccctgc gggctcctga cttttcaagt cctgtgagcc 3120
ctgagtggtt gtctgaaagg cgggtacagt gggcggatca cttgaggcca ggagttcgag 3180
accaggtggg ctaacatgac aaaaccctgt ctctacttaa aatacaaaaa ttagccaggc 3240
atggtggtgc acacctggat gcaatcccag atacttggga ggttgaggca ggagaattgc 3300
ttgaacccag gaggtggagg ttgcagtgag ccgagatcat gccaccgcac tccagcctgg 3360
gcaacagagt gagactccgt ctccaaaaaa aagaaagaaa gaaagtgtcc tgtttgggcc 3420
tggcatggtg gctcacacct gtaatcccag cactttggga ggctgaggtg ggcagattgc 3480
ttgagcccag gagtttgaat ccagcctggg taacatggca aaaccctgtc tttacaaaaa 3540
ctacaaaaat tatccaggta tggtgatgca tgcctgtagt cccagctact caggaggctt 3600
aggtggaggg attgcttgag cccaggaggt caaggctaca gtgagttctg atcccaccac 3660
tgcactccag cctgggcaac agagcaagac cctgtctcaa aaaaataaaa taaaataaaa 3720
taaaataaaa taaaaaagag aaagcacctt gttggttgtt ttgtgtgtct gtctccccca 3780
gcctccgccc caccgtggta cgtcagttcc aaggcatctg gggcctggtc agtcttgctc 3840
tgctgaatcc ccagtgcctg caacagtgtc tggcaggaac ttaatacatg ttttgttttg 3900
ttttgttttg tttttgagtt gggatcttgc ttttttgccc aggctggagt gcagtggcgt 3960
gatcatagct cactgcagcc tcgacctcct gagcttggtc cccctgcctt agactctcaa 4020
gtagctggga ttacaggatt gtgccaccac gcccgactga tttatttttt gtagagacag 4080
ggttttgtga tgttgcccag gctggtctca aactcctggg ctcaagtgat cctccttcct 4140
tggcctccca aagtgctggg gttagaaaca tgagccactg cacctggcct ataaatggtt 4200
ttgaatgaat gaatgagctg atggataaca gaagctcttc tttatcctga gctgcatagg 4260
gtctaaaagg gacccatgga ctttggagtt gggcggtctg aatttgaatc ccagctccag 4320
cattcactgg ctgtgtgacc ttaggcaagt tgcttaacct ctctgaacct caatgtcctc 4380
atctgtaaaa tgggagtaat cattgtgagg gttgggggtg accattcatg ggaagcgtgt 4440

agttgggggc ctggtaccta agtgcttgaa aaatcatgcg ggaaaccagc catcccgggg 4500
tctcaaaacc atggccgacc tgtgtgggtg agagagtggc ctttgaagct ggtggataga 4560
ccaaccctgc gggctcctga cttttcaagt cctgtgagcc ctgagtggtt gtctgaaagg 4620
cggacacagt ggtcctcagt gcacagtgtc ctcctggggg aatcactgag caggcaccct 4680
gaattgcatc cccggggact ggtgaggccc ggctacttac caggtaaggg tgagcgtgct 4740
ggggtggagg ttccacattt ccaggatgca gatgaccgtc tggaggtaat cggtgtagca 4800
gacgaggtcg gggcagcccc agcctggagg gtcagtgggg agagaggcct tggtgggggg 4860
tggcttggga ctgcagagct tcctctccat cccctcgagg gctggaggaa gaatgggtgc 4920
aggggtcccc aggccgggac aaatatgagg actcaggagc tgcttagatg taggcctggc 4980
acccaggggg cctcaccacc tggaaattcc ttgtttgagc accaatgttt caaggctggc 5040
tatgaagaca ggatgcatgt gatggcaaat aatataaaac tagctggtcc ccagctcagc 5100
accctcctcc agagccctgc gcccggccac caggtctgtg tccctcctgg ccaagctccc 5160
actcagaatg gcaatatcct aaatgcccca gatgtggtag gaacccagcc agagcacacg 5220
cacacacccc aagagggtca ggacctcata gagtcaagga cagctatgct gaaaatgtcc 5280
caaaaccctg acaaggaaga ggactcggct ctacagaagt tggatccaca ggacacccac 5340
tctcggagat tcaggaagtg cttcaaggag taggaaaccg tgagagaatt aaaaagccct 5400
cggacacttt tgcataaagg tctgcgtgat cctgcggcca aaaatgccac cgccaccaag 5460
aagtcttcta ggtattgccc tatgaggcaa ttttgttgga agatttaaca cctttccatc 5520
ctgctcccca aacattaatt gtttcgctgt gttctaaaac aggggtgcaa tcatcatcta 5580
cattttgtta aatgtggaca aaatcaatgg aataatttgt agctttgttt tgtatattcg 5640
agtgccaatt ccactatttt ttgctgctat ttactagaaa cttgtaccgc tcacctcttt 5700
ttgagatagg atctcttgct ctgttgccca ggctggaatg cagtggtgcg atctcagctc 5760
actgcagcct ccacctcctg ggctcaagca gtcctcccac ttcaacttcc tgagtagctg 5820
agactacagt tgtgcaccac catgcctgga taatttttgt gtttttgtag agacagggtt 5880
tcaccatgtt gcccaggctg gtctcaatct cctgagctca agcgatccac ctgcctcagc 5940
ctcccaaagt gctgggattg taagaatgag ccatcgtgcc tgaccttgtt ccccttttta 6000
agtaggttca tttttggtgg ctttttccta ttttccctgg actaatgaag acccctgagg 6060
tggtggtggt agattccctt cctgggtctc actctaggac agaacctata tgcggagcca 6120
agaatactgt gtctggaaat atcctttagt tcattgtaga gatgagaaca ctgaggctcc 6180
aagactagac catgcctctt gtctctcttg gtctgggctg ctgagacact cacccaggga 6240

aggaaatacc tagtctgtgc tagtttttca tagccgcaca catgacagat attgctaatg 6300
gatcacagca ctccctcccc taggtgtgac ctcggaatgc ttctcaactt ctctcgaggc 6360
agtggctacc aagggatctg agtcatggaa gtaaatcttg ctctaggagg gggctttcct 6420
cagggagaca cacagaactt gtcggtggag tgaaaaggag ctagttccag cacttggtgg 6480
agaaaacagg actgattttg gcaaaacctc aacacagagc ctctaagtag ctgtgtcatc 6540
tctgagttcg tatcctcgtg gttctttctc agagctctgg gaatttggag aggagctacc 6600
attcctagcc aggctcaaag ctaccaaggg acagggtttg tctccaaact tggggaggca 6660
acggcagccc ctgagtggca gggaggtggc accagggatg agagggtggg aaaatgccct 6720
gtcagtaccc cagccacagg gcagagagcc ccagggccat cccatgtctg gggttatgaa 6780
agcgtgcagg aggcagtgag aggtgcacaa agtagctctg tgactgcaaa catgtgactc 6840
aaaactgctt ctcctcccca tgtgtaagcc ggagacactg cttgccctag aggctgttga 6900
ggtgttgaga tgagatcatg cctgtgcctg catgctgccc gcacatggct atcactccac 6960
agatagcagc acagcatggt gaaggccgtg ctacggtttg acgtctgtgt ctccccagaa 7020
actcagttgc caatgtgatc atattaaaag gtggggactc taggagatga ttaagtcatg 7080
ggggcagagc ccccctgaat gaattattga tgctataaga gggctggacg aacccaacat 7140
tcagacccctt tttgcccttc tgccatgtga ggatgcagcg tgtgtcccct ctgggggtgg 7200
tgcagcgttc aatgtggcat cttaaaggca aagatggggg ctgggtgcgg taatcccagc 7260
acttttggga ggccgaggcg ggtggatcat ttgaggtcag gagttcaaga ccatcctggc 7320
caacatggtg aaaccccaac tctactaaaa atacgaaaat tagccatggc atgtgcctgt 7380
tatcccagct gctcgggagg ctgaggcatg agaattgctt gaacctggga ggcagaggtt 7440
gcagtgagcc aagattgcac cattgcactc cagcctgtgt gagaaagcaa ggctcagtct 7500
caaaaaaaaa aagcaaagat gggcccctca ccggaatcaa agctactgct ggtgccttga 7560
tcttggactt cccagcctcc agaactgcga gcagtaaatg tctattattt ataaattacc 7620
cagtctcacg tattcagtta cagcagcaca gacagactaa gacagacccc aagagcaaac 7680
tgatcccagc tctgcctctt ccgggtagtg tggccatgca cctgtctctt agcctctctg 7740
agcctcctca gccaaaaaac tgggcgtcat tacaacatgc gccttgtggg gctgttggga 7800
cagtggaatg ggatgacgct ccgagctttg catgcagcca gggcttgtta tctgcacccg 7860
ccagatgtgg agccatggtg ggtgacccaa aggcttttta gaaccaagat gaggatgctt 7920
gccactcaga aagggaaaat caaagcttga catctgtttc ctgaaggagc tatttgaggt 7980
cctgggtctt ctggtaaaaa ccagagaaaa atgcccatga gtcagcagag cagctgttga 8040

gttcctcccc tgcccctagg ccggcctcca cacaagtctg tgctggatcc cgcctcctca 8100
tggccccctc ctccaggaag ccctccagga tctcttagtc ctaggcagtg ccctttcagt 8160
cggactcctg gtcttcccag ttctgctttc taacgtcacc ccctctaacc acaacatctg 8220
gtcctctggg ggctgcctac caaggttcag tctccttttg gctcttcctc ctgtccccag 8280
cccagggacc accagtgcct gctaagaatc tagcgatttt tttgtttgtt tattgttgtt 8340
gttgttgctg ttgttttgag acagggtctc gctctgttgc ccaggctgga gtacagttgt 8400
caccttgact cactgtaacc tccacctccc aggctcaagc aatcctcccg cctcagcccc 8460
acaaaaagct gagactacaa gcatgcgctc ctatgcctgg ctaatttttt ttcttttgta 8520
gagacagggc ttcaccatgt tgcccaggct ggtcttgaga tcctgagctc aagcgaccta 8580
ccctcctcag cctcccaaag ggctgggatt ccagtaggag ccaccgcacc cggtgcagca 8640
attgttgaaa aacatttcca aggagagtca ttacataagg agaaactgag gttagatgga 8700
aggcagaact tcctcactca gagagttggg agccagccaa ggcagaggtc aggggaaatg 8760
aaggacgaag cctttcatcc ccccagtgac acccgaagac acaagcctgt gggctgggcc 8820
acctgagact catgtctacc gcttctgttt catggagccc tttggtggat ggattctgat 8880
tagtggagag ccggctactg gactcgtgtc agcctcaagg gcatggatgt catcttttct 8940
gaatctgtgg actctagccg tgtttttgag cccagaaagc cagcccggcc ctggctcagc 9000
tctgtgatgg gggccctccc cacccgcaga ccacggggca gccacttacc tccctggagc 9060
agcagcagga gcaagggggc ggcccagcca cgcggcatgc tgactcccac gggcctgcaa 9120
gagacgtaca gtggagggtg agccaggcgg gctcagggct cccccggcct ccccctcatc 9180
ctgtctcttc ccaggcagac ttgcccccga ttcttggaac tgtcttaaga aaaatgcaag 9240
atcccgaggg gcctgtggat gaccccaaga actcccacct cttagaaatg gcagattcac 9300
cccaagaatc ccttctccca ccttcttcct gcccccgccc catcacgtca caaatgcaaa 9360
gtgaatttgt tttattattt tattttaata tttttagaga tggggtcatg ttctgttgcc 9420
caggctggag tgcaatggcg caatcacggc tcactgcagc ctcaacctcc tgggctcaag 9480
cgatcctcct gcatcagcct cccgagtagc tgggaccaaa ggcacgtgcc accacgcctg 9540
gctaattttt aaaacatttt tgtagaggcg gcatcttgct atgttgccca ggctggtctc 9600
aaactcctgg gatcacatga tcctcctacc tcggcctctc aaagtgctgg gatgacaggc 9660
atgagtcacc acgcccagca caaggtgaat ttaaagaggg aggagactgc tgactatttt 9720
gcccttgctg agcctattga tgtagcattg tgtgtgaaat taaacttgtg tatttatcta 9780
tctaacaaag aaagagggag ggaaaaagag aggaagggag agagaaaagg agaaaaaact 9840

agtttggaaa gggggatctg ctgaggagca attttggatc ttccttccta taaacatcca 9900
tgttgtaaaa aaaagccagt aaattggctt ttttatgttt attttttaga caaagtctca 9960
ctctatcgcc caggctggag tgcagtggcc cgatcttggc tcactgcaac ctctacctcc 10020
caggttcaag cgattctcct gcctcagctt cccaagtagc tgggaataca ggtggccgcc 10080
accacgcctg gctaattttt gtatttttag tagagacagg gttttgccat gtggactagg 10140
ctggtctcga actcctggcc acaggtgatc cacccacctc agcctctcaa agtgctggaa 10200
ttacaggcat gagccactgc acccagccat caataaattg gcttcttact gaataataaa 10260
tccaaagtta acccacaaag ttaaccactg ctgaatactt aagataaggt tagcccaata 10320
atgaggctcc ttactccatc acaaagccca gattagccca ctcgggagca tgctaggaaa 10380
tccaggcctg accaaacaga gacaacctga cctcctaatt tgggccagga tggagaagga 10440
acctctttgg ggagcagagt gagtttatcc agaaagagtg ccctctactg tcttgggcag 10500
aggaatgccc caccagtctg gcgatgccag gcttctcctg ggacttgtct gtttcaccca 10560
ccagctctgt gtctgtttga ggaagtgcca gccagagggc agtggtccac ctgcccagac 10620
tcaggagccc ctcccagacc agcccctata gacaagaatg gagctaaagc taaacctaag 10680
agggatgccc catgcaggac ccaagtcaca ggcatggctt tctccagacg ccatccctga 10740
ctccacaagg ggaagatcct cctcctctgt gccggggttc ccaaagatcc tactctggcc 10800
ccatccatag ttccctctga cctccctcac agagccatca aagaaccctg gggcagggca 10860
caggctgggg ggccaggaag gttcagatga ccagctgtgt ggctccgggc cagtcccttg 10920
ccctctctga gccttgtaaa gtggaaacag caaggcctgc ttccaggctt gtgagaattc 10980
aaggaggtga tttgcataaa accctgcaga tggcccccat cacataaaca ctcaaacaac 11040
agctgtaatt gatggagaag gtagagcctc agaaacagag ggggtctcag aatgatctag 11100
ccaagagttg ccaaaatatc tcatatccaa aattttggcc atatttgtat attgtgtgca 11160
ctgttgttta ttttaataca tattttaata tattttctat agataagcac acttaaaaat 11220
ttaactccag catcatccta agcaataaca tcactactca aataaaataa tgttgaggta 11280
gatgcagttt caacctgctt ttaggaatat gaatcctgtg tgattccaac ccctgcagag 11340
gtcctgttat cacactttga agatgtggcc tctgatcgca ggagggggag tctgtgtctg 11400
ccccctcact attctgtcca catccatcac cccagccacg gtgcgagtcc tgacatagct 11460
ctgtccccgt cactccctga tggttttatc ttgaccacag cacagcaaaa agtgcaaact 11520
cctttgccca gattcaatat tcctcatctc cagcttgacc aacatagcaa gaccttgtct 11580
ctaccaaaaa taaaaataga agtaaaaata agttagccgg gtgctgtgtg cctgtggtcc 11640

cagctattcg ggagactgag gaaggaggat cacttgagtg ccggaggtcc aggctgcagt 11700
gagccatgat tgcactattg cactccagcc tgaatgaaag agtgagaccc tgtctttaaa 11760
aaaacaaaca gacaaacaaa aaactcctca tccctcctaa tttcttacct ctgcctacat 11820
cacagagccc tagggatgtg gagggggtac ctgctgcttc tgcctgcccg gcgtctcttc 11880
ccaaagagct cctctatctg agctgcctct agtggtttgc ctgaggctga ctccacccca 11940
tgctccacaa agtgggctcc tgacccaggc ttggccaatc aaaacacccc atctgtctga 12000
ccacagtgat tgcatcaggg atggatgtca acccaggcaa ggccagtgaa agcagtcctg 12060
agattttttt tctagaactc tagaaaactg acactttttt tggtatcttc tcagagcttg 12120
tagccatctt tgtcacctct tagagacagc ctccttgaaa atgacagcca tactgagaaa 12180
tacaggtcag gagtccccat ggcatcatgg gaggtcctgg atccagccat atctgatgtc 12240
caccctgtgg atttctcatt cccatgagcc aaaaaccacc tatgttgctt aatcatgttg 12300
aattctattt ctgtcacttg ccaccaaaga tccctgacac agtggagttg agaaaagggc 12360
tcagaatttg aagccagaag atcttgaagc aaatagtagc tcttctctgg ctcttttatt 12420
ctctgaactc agttaccatc tctgaaatgg ggacaatagt gctcactgca cctggcagtg 12480
aagaagaggc tgtgagaaag cagatgtggt agtgcttcat ggactgtgaa gtgctgtacc 12540
agtgcacggg tttcctgcca ttactggctg actgccctga attactcatc gtctccccgg 12600
cagcccaggc ttcgtcatgc atctttgcct ctgctggagc agactcctgc cctccaacac 12660
ctttcccctc ccatcctctc aactcaactt tcatggtcca tgtgctgtat ccctgcagcc 12720
acactggagt gttccagaac tcctgccact agcctattgc gtttgaggtt ttcactggtg 12780
ctactattaa ttatccactt actattgatc tttaccttgc ctcctatttt ttactccaat 12840
aaacatgtct caaaaatatt atatcatgac catcagcaga taactggcat gaaataggca 12900
gtgtgcgcca tgctggtcaa gacacagact atggagctgg ctggtctcag ttcaaatccc 12960
agctctgcca cttataggct ttgtggcctt gggcagtttg cttaaccttt ctgggcctca 13020
tctgtaaaat gggagtaatg ataatcacag cacctgcctc ataggtcttt gggagaatta 13080
aattagtcaa catttataaa gcatttagaa cagtgcctgg catatagcag atattatata 13140
agtgttactt aaacaaaaca atcaacttac cataaaacta aatacaatac aacataaaaa 13200
ctaaatacaa gaaaaacaaa ggcagtaatt gaattttagt gacatgctac tgcctgcctg 13260
agagtcctag aaactggaga tgtggtttgt ctttgtttaa agttaagaaa gggaggccgg 13320
gcacactggg tcatgcctgt aatcccagca ctttgggagg ccgaggtggg aggatcattt 13380
gaggtcagga gttcgagacc agcctggcca acacggcgaa accccatctc tactaaaaat 13440

acaaaaatta gccgggtgtg atggtgtgtg cctataatcc cagctactca ggaggctgag 13500
acaggagaat tgcttgaacc caggaagagg cggaggttgc agtgagcaga gattacacca 13560
ctgtactcca gcctgggcaa cagagcaaga cactctgtct caaaaaaaaa caaaacaaaa 13620
cacaaaaaaa gagagagtag tgtttaagat gcgatagcac caaattgaga atcttgcctc 13680
ctccatcagc agggcaagtg atgaaagagc ctacgctttc actaggggat ccagggatat 13740
ttaacactgt ttaaaataaa ctgctcactt ctgggaaaca tcagcttaaa aacacagact 13800
ttctccatca ctccctcctc atgctcccac agcaccaaat ggggcctacc ctaattccac 13860
tgccttctct gccccgaatc catccccttt tgggtctttg cacgcaggct gacccctgag 13920
ggcaacagca ccttatcctc agatacagat cgggccacca tgtgtgtttc ctacaaggac 13980
agaaggtgtc ttaggaggct tcctgctaca ggagcccctt gatatggttt ggatatttgt 14040
cccacccaaa tctcatgttg aaatgtcatc cccaatgttg gaggtggggc ctggtgggag 14100
gtgattggat catgggggcg gatttctcat gaatggctta gtgccatccg cttggtgatg 14160
agtgagttca cctgagatct aattgttcag aagtgcgtgg cgcctccccc ttttgctcca 14220
gctgtcgcta tgtgatgtgt ctgcttctgc ttcaccttca gccttgagta aaggctccct 14280
gagggcctcc ttaccagaag ccgagaagat gctggcagta tgcttgtaca gcctgcagaa 14340
ccatgagcca attaaacctc ttttctttat aaattaccca gtctcaggta ttgctttatg 14400
gcaatgcaag aatggcctga cacaccgctg cagcacccaa cctctgcagt ctcctacgga 14460
ggagtgaagt gcacctgact tggagtgtgc caggtcccag ctgtgcgtct ttgggcaagt 14520
tctctaaccc ctctgattct ctgttttctc atccataaaa tggaggtcat caatctttgc 14580
tgcaggtttg tcatgagcat taaaagtgtc atcgaatttg aagtcctgta ggtgcattta 14640
tccaccagtt tcagtgctga gaaatgtttc ctggagttgc tgtaattcac cctaaaagtt 14700
gaccaaattt caagagattc tggggaaatg gagaagcttg gggcaggaaa ttagattgaa 14760
gtgagtgcta tggagaatat aaggtgctta acttcatggt caacgtggtg gctcatgcct 14820
gtaatcccaa tactttggga ggccgaggtg ggtggatcac ctgaggtcag gagttcgaga 14880
ccagcctggc caacatggtg aaacccatct ctactaaaaa tacaaaaatt agccaggtgt 14940
ggtggtgggc acctgtaatc ccagctactc tggaggctga ggcaggagaa ttgcttgaac 15000
ccgggaggtg gaggttgcag tgagccgaga ttgcaccact gcactccagc ctgggtgaca 15060
gagcgagact ccatctcaaa aaaaagaaaa agaagagaaa agaaatacaa agaaaaacca 15120
caatgacata ccattttaat tttgtcggat tggcaaaaac tgaaagtctg atgacatcac 15180
atattgctca ggatatgata taataggaac tctcatttat agccagagga aatgtaaatt 15240

attacaactg gtctggtaaa aagtttggca tgacctaata aaagcgaaca catgccctac 15300
ctaaaaccca gtaacttcac tcttatgtaa actggtaaag aaattcttcc tgcatgtcta 15360
tagcagcatt gattttaata gtgaaaaatt ggaaacaatc taaatagcca tcatcaggag 15420
agtgaaaaaa taaatcgcaa tactgtcact cagtgtaaac atgaatcatt tacagccaca 15480
cacaacaaag tgaatgactc tcacaaacaa aatttggagt gaaaaaagga agttagagaa 15540
cagcataagc ataataacac cattgacacc acactgaaaa ccatacaaaa tcatcctata 15600
tattgtttag gtaagcacac ccatcataaa aatatttaaa aattcatggg gataataaca 15660
tcaaattttg aatagtgtgg aaaaggaggg ggtgtgagaa ggaggaatgt tcaagggtct 15720
caacagtttg gtaaagcttg gtgaagctac catgggtgtt gattatattt tatgcccttt 15780
tgtatgttca aactatttca taccatttta aatgtaagtg aggcttagtt ttgtaaaata 15840
aaatatggat gagggacttc cacttctggc tataatgaag tagctgttgt cacactaatc 15900
ctcccactga gggcaactac taagcagaaa aaaaaaaaac tgcacaagag agtgccaact 15960
tatatagtac ttaagagagg aaaaatgcat tgaaattctg catgcaattt cccctcaaat 16020
catgtgcaaa gtcattgaaa ataagtagca tgccaagaaa cttagcaaaa aaacacctgc 16080
taataggtgg gaaaactaag aaggaatttt cacagtttta caagctgagg gaagaattag 16140
agttcagggc ctggcaaaaa aaagatgggc cctggtgagt acccagctat ttagaaaata 16200
ctattccttg ggaataagga aaaattagaa atagttcatt cttgactgca tcaaggtgta 16260
atctgcttat atgctatatg tctgctagaa gaaaatttaa attgatctag aaaaagttag 16320
cttcatccaa agcctctata ctttttatac ataatgtcca actttcagtt aaaattacaa 16380
ggtaaaccaa caagcagtat caaatgatca acaatgaaga taaaatagac aatagaaaag 16440
ttacttaggt gaattggaaa atggcattat cagatatgat ttaaaataac tatgattaat 16500
gtgttcaaaa attctatcag aaatttggaa cctatttaaa aagtagaaat cctggaactg 16560
aaaaatataa taactgaagt ttataaggac tgaagagatg gatttggcaa catactagta 16620
acatcagaag agaggattag taaactggaa gattggtcaa tagaaaatac caagattgaa 16680
gcatgcagag aaaaaaagtt caaaaataca gaaaagagca aaagagacat agaagacatt 16740
gtgaaatgat ctaacataca tgcatttgca attaagaagc agaagagaga gaaaacaagt 16800
aagaagaaat agcagtgatc ttaaaatttt tttccaaact aacaaaagac attaagccac 16860
agatttaaat agctctacaa accacaaata ggataaatac aaagaaaacc acatctaggc 16920
atatcataga aaaactattt taaaaaataa aagacaaaga tatgcagtat gcagaaaaaa 16980
aagacacttc accttcaagg aagaaacaat aagactggca ggggactttt taacaaaatg 17040

atggaagaca gcagacaatg gaacaacttc tttcaatgct gaaatttcaa aagccaattt 17100
agaattttat atccagaaaa aaatctctta aaaaataaaa gtaaggtcaa tatgatacag 17160
acagctataa aaatattaca gttaacatca tacttaatgg taaagaaatg aaagctttgc 17220
tcctaagatc atgaacaaga caagtatgtt cactcttgtg acttttataa aacattttac 17280
tagaggttct agccgaggca attaggtaag gaaaagaaat aaaagacttt cagattggaa 17340
aggaagaagt aaaccatttc tctttgcaga taatatgatc ttgtatatac aaaactaaag 17400
gaatttacac ctaaaacgaa caaaaaccaa ttagagctaa taaacaaatt tagcctagtt 17460
gtagaatata agattaatag acacaaataa tgatatttta tacacactgg caataaataa 17520
tctgaagatg aaattaagaa agtgatttca tttacaataa tacaaaacag aacaaaatgg 17580
ttaaagtaaa tttaacaaaa ttcattagtt atgttaatat gagatgaatt tgtttattag 17640
tgtgagactt gtgcaataaa acctacaaat gttattgaaa taaattatag aaaacctaaa 17700
taaatggagg atataccatg ttcatagatt ggaaaactta atgttatcaa gatgacgcct 17760
caaattgatc tacagattca aagtaatctc tataaaaaat cccagctggc tctttttttt 17820
tttttttttt ttttttgcag aaattgacaa actagtccta aaatttatag ggaaatgtaa 17880
gggactagaa tagccaaaaa ggttttgaga aagaacacag ttggaggagt caaacttcct 17940
tatttcaaag cttactacaa agctacactt atcaacactg tggtactgac acatagatgt 18000
acatatagat caatgggatt gaactgagag ttcagaaaga agtgcatata tttatgttta 18060
accgattctt gatgagggtg ccaaaacaat tcaatggggg aaagaatagt ctttaaaaca 18120
aatggtacca cacaaatgga tatccacaat gcaatataat gaagctagat ttctacttca 18180
caccgtatac aaaaatttac tcaaaataga ttaaagacta gatgtaaaaa gtaaaactat 18240
taaactctta gaataaacct aggtgtaaat cttcatgacc ttgtatgaga caataatagt 18300
tttttaaata tgacacaaaa gcataagtaa acaaagaaaa aatagatcta ttaaactgta 18360
tcaaaattta aaatgtttgt gcttcaaaaa atactatcaa gaaagtaaaa ctataacata 18420
cagcatggga aaaaaagcat ttgcaaatca cgtatttgtt aaaggtctgg tatttagaat 18480
atgcaaagaa ttcttgcaac tcaagatcta aaaggcaaga attaaaattt aaaatgggca 18540
aaagatttgg atagacattt ctccaaagaa gatatccaaa caacaaacac atgaaaaggt 18600
ggccattgac attactctct agagagatgc aaatcaagaa cataatgaga taccacttca 18660
catgcattag gatggtaata ataataatca atcacaagtg tgaatgaggg ggcggagggt 18720
gagaatgtaa aatggtgcag ccactgtgga aaacagtttt gcagttcttc aaaatgttaa 18780
atacagtcac gtgctgcata atgatatttc attcaatgac agactgcatg tacataggcg 18840

ggcccatagg actataatga aggtgaataa ttcctattgt ctagtgacgt catgacacaa 18900

cactttactc atgggcttgt gatgatgctg gtggaaacaa atttactgca ctgccagtca 18960

tataaaagta tagcatatgc agggctaggt gcggtggctc acgcctgtaa tcccagcact 19020

ttgggaggcc aaggcaggca gatcatttga ggtcaggact tcaagaccag tgtgacaaac 19080

atggtgaaac cccgtttcaa ctaaaaacac aaaaaactta gccaggcgtg gtgacacatg 19140

cctgtagtcc cagctactca ggaggccgag gcaggagaat cacttgaatg gggaggcaga 19200

ggtagcagtg agccgagatt gcgccactgc actccagcct ggacaacaga gtgagactcc 19260

gtctcaaaaa aaaaaaaaaa agatagcata tacaattatg tattgtacat aatgcttgat 19320

aataataata ataataataa taaactatgt tactggtgta tgtatttact atactatatc 19380

ttaaatcact attttagggt gtgctccttg tacttactta aaaaaaaatg ttggaggggc 19440

acagtggctt acgcctgtaa tcccagcact ttgggaggct gaggcaggca gatcacttga 19500

ggtcaggagt tggaaaccag cctggccaac atggtgaaac cccatctcta ctaaaaatac 19560

aaaaattagc caggcacggt ggtgtgtgct tgtaattcca gctattcgtg agactgaggc 19620

acaagaatcg catgaaccca ggaggtggag gctgcagtga gccaagattg tgccactgca 19680

ctccagcctg ggtgacagag caagactctt aaaaacaaac aaaaaatgtt aacttgtaaa 19740

acagcctcag gcaggtcctt caggatggat tccggaagaa ggcatcatta tcataggaga 19800

tgccagctcc atgtgtgttt ttgccactga agaccttcca gtgggacaaa atgtggaggt 19860

ggaagacagt catattggtg atcttgaccc tgtgtaggcc taggctaatg tgtctgcctg 19920

tgtcttagat tttttgtttt ttgttttctg ggtttttttg agatggagtt ttgctcttgt 19980

tgcctaggct ggagtgcaat ggcgtggtgt tggttttccc gggttcaagt gattctcctg 20040

tctcagcctc ccaagtagct gggattacag gcgcccgcca ccatgcttgg ccaattttgt 20100

atttttagta gagatggggc tttcaccatg ttggccaggc tggtctcgaa ttcctgattg 20160

caggcgatcc gccctccttg gcctcccaaa gtgctgggat tacgggcatg agccaccatg 20220

ccccgcgtgt cttaggtttt aacaaaaaaa attttttttaa gtaaaaaaat aaaaaataaa 20280

aaattttaaa atataatata taaaaaggct tttagaataa gaatataaag aaaaatattt 20340

tgtatagctg tacaatgtgt ttgtgtctta agtgttagta caagagtgca cgaaaacact 20400

taagtgttat tacaataaga taactgttat tacaaaagtg ttattcaaaa agttttaaaa 20460

agttaaaatg tttataaaga aaaaatgttg gcggggcgca gcggctcatg cctgtaatcc 20520

cagcactttg ggaggccaag gcgggcagat cacgaggtca ggtgatcaag actatcctgg 20580

ctaacatggt gaaaccccgt ctctactaaa aataccaaaa aaattagccg agcgtggtgg 20640

cgcgcacctg tagtcccagc tactctggag gctgagacag gagaattgcc tgaacctggg 20700
aggcggaggt tgcagtgagc tgagatcgcg acactgcact ccagcctggg tgatagagtg 20760
agactctgtc tcaaaaaatg aaataaaata gggccgggcg cggtagctta cgcctgtaat 20820
cccagcgctt tggaagggcg agacgggcag atcacaaggt caggagatcg agaccatcct 20880
ggttagcacg tgaaacccca tctctactaa aaatacaaaa aattagccgg gcgcagtggc 20940
aggcgcctgt agtcccagct acttgggagg ctgaggcagg agaatggcgt gaacccggga 21000
ggcggagctt gcagtgagcc gagatcacgc cactgcactc cagcctgggc gatagagcga 21060
acctccgtct taaataaaat aaaataacat aaataaaata aaataaaata aaataaaata 21120
aaataaaata aataggaaaa atgttacagt aagccaaagt taatttatta ttttaaaaat 21180
tcaaataaat ttagagtagc ctaagagttc agtgttgata aagtttacag gagtgtacag 21240
caatgtcctg ggccttcaca ttccctcagc acttaatcac tgacacccag agcaacttcg 21300
aatcctgcaa actgcattca tggtaagtgc cctacacagg tgtgccgttt tttcatattt 21360
tatattatgt tttcactgta ccttttctat gtttagatac acaaatactt acaattgtgt 21420
tacagttgcc tacagtattc agtaccgtat catgctgtgc aggtttgtag ccaattagca 21480
ataggctgta agatacagcc taggtgtgta gtaggctata ccacctaggt ttgtgtaagt 21540
gcactctatg acgttcacac aatgacaaaa ttacctaatg acacatttct cagaatgtat 21600
cctcgtcatt aagcaatgta tgactgtttc catatgacca agaaatttca cacctagtta 21660
tatagtgaaa agaattgacc agatgcagta gctcatgcct gtaatcccag catactggga 21720
ggccaaggcg ggagggttgc ttgagcccag gagtttgaga ctagcctggg taacatggca 21780
agacctcatc tctacaaaaa ttaaaatgaa aacattttct gggcatggtg gtatgtacct 21840
gtggccccag ctactcagga ggctaaggtg ggaggatcac atgagcccaa gagtttgagg 21900
ctacagtgag ctgttttcac accattgcag tccagcctgg gtgacagagt gtgaccctgt 21960
ctcaaaaaaa aaaaaaaaaa agaaaagaaa aaagaaagaa aaaggaaaga aaacaattgc 22020
ggatatatgt ccagaaagaa atgtacaaac cattgtagac aaatattcac agcaacatta 22080
ttcataatac caaaaaactg gaaacaagca aaatttctat cgactggtga atggataaag 22140
aaatgtagta tatccatgca gtagaatgtt atttagccat ataagggaat gaactactga 22200
tacctgctac aaaatggatg aactttaaaa attcatgcta aagtaaagac accagacaca 22260
aaaggcctca tattgtatga ctccatatgt atgaaatgtc cagcattggc agatccatag 22320
agacagaaag taggttagtg gttgccagga gctgaggctg aggggagggg ggatagagag 22380
taacagcaat ggatgtaggt ttgtttttgg agtgatgaaa atatagagtt agtggtgatg 22440

actgcacaac tttgttgaat tgcacatctt aagagtacat tttcagtttt gtgaattaca 22500
tctgaaatac agatttttga aaatgaaggc aaaataagga tttttttca gacaaaaata 22560
agtaagagat tttgtcactg atagacctgc atcaagagat atacaacagt gagtttttca 22620
ggtaaaagaa aaataattcc agatgggaag caagttactg tagggtgaaa tgaagagcaa 22680
cagaaagtac tctaaatata tgaattaggc tgggtgcggt ggttcacgcc tgtaatccca 22740
gcactctggg aggcctaggt gggtggatga cctgaggtca ggagttcgag atcagcctgg 22800
acaacatggc aagacccagt ctctactaaa aacacaaaaa attagccagg tgtggtggcg 22860
caaacctgta gtgtaggcta ctagggaggc agaggcagga gaatcgcttg aatccggaag 22920
gtggagcttg cagtgagctg caactccagc ctgggtgaga aagtgagact ccagcctggg 22980
tgagaaagtg agactctgta tcaaaataaa taaataaata agtgaattac tatgattttt 23040
aatttcttaa catactaatg atgtaaacta tatgcagaat taaaatacac gaccatgaaa 23100
ccccaaaggt aagaagaggt gaataaagtt aaagaggact ttgtacaaga ggacttcaaa 23160
ttgtacaaga ggacttcaaa aagctcatgg aaacatggaa ttaaagataa caattaaaaa 23220
ataaactcta tttctcaaca taaattcagt caaggtcaag gcacttttgt aagtgatgat 23280
accaggcaca tagtccatct ccaaagaact gggggtcct gggaatatag ccatgtcaat 23340
gcaggctttt ttacattatt aactaaagaa aaatgggtga tcgttaaaga ttgcttaaga 23400
ttaggagaca aaaagaagtc agaaggagcc aaacaggact ttaaggtgga tgcctaattt 23460
ctcatggaag ctgtaaaatt gcccttattt gatgagaaga atgagcagga gcattgctat 23520
ggtggaggac tctctggtga agctttcctg cgcgtttttc tgctaaagct ttggctaatt 23580
tccccaaaac actctcatga taagcagatg ttatcattct ttggctgtcc agaaagtcaa 23640
caagcaaaat gccttgagca tcccaaaaac ctgttgccat gacctttgct tttcttgacc 23700
ggtctgcttt tgtcttgact gaacctcgtc tacctctttg gagccactgc tttgtcttca 23760
ggatgaactg gtaaagccac gtttaatctc ctgttacaat tatgtgagga aatgcttctg 23820
aatcttgatc ctattagttt aaaatttgca tggaagtttc tgctgttgtc tgcagctgat 23880
ctgggcacag tggtttggca cccagtaagt gggaagtttg cccaacttta atttttttggt 23940
cagaattgtg taacctgaac taattgagat gtctatggtg ttggctattg tttgggctgt 24000
taatagttgg tcctcttcaa ttagggcatg aacaagatga atttttcct aacaaattga 24060
tatggatgat cggccacgga gggctttatc tttatcattg atttcgccat ccttccaccc 24120
tagttgcacc ataaatttga tgtttgttcc tgcttcaatt ttagcagaat tcatgttgca 24180
atgatagggg ctttttgca aacagacgcc ttatccttct tagtgcctca aactagatcc 24240

tgttcagaca tgttatagca agttagtaca agtttatttt ggtgcagaaa aaagtttgaa 24300

ctttatgcat agtttttttca taatttgaat tttccatgaa cttttggaag aattcatatt 24360

aggtagatcc taataataat aagtatgcat gttgtaatct ctaagataag cacaaaaaga 24420

atataaaaga aagtacaatg aacaaactaa taggaaaaat ggaataataa tttttaaaac 24480

ttgattttct aaaaactaga acaaagaagg aagaatagat aggtcaaaaa agaaaacaaa 24540

tagcaagatg gtagacataa tcccagacac atcagtgctt tcattgatgt ctgaaataaa 24600

aggcagtctt aataccaaaa cttgacatta aaggaaagta gaattacagg tcaatctttc 24660

ccataaataa tctaaaaaat atctgaaaca aaatattaac aagtcaaatc taataatatt 24720

tataaaaata gataacatat tattatcaca ttggtttatt tcaggaatgt aaggttagta 24780

tgacatttag aaaccataat caatttcacc acattaactc aataaacaag aaaatttata 24840

ttatcattta ataagttcag aaagcacatt taataaattg caacacccat tcatgatttt 24900

taaaacctgt caggaaagca gaaatagagg agtcttcttt aatctaatta cgatcacaca 24960

cacacacaca cacacacaca cacacacaca cactatacat agctaacatt atccttaatt 25020

gtgaaatatt gaacacttcc ttactgaggt cagggcaaaa acaaaaacat cttatactag 25080

aggtgctaat taacataata aggcaatgat aagaaacttt taatttataa agattagaaa 25140

actatgtgtt taagttatat aagaattgag gaaccagaaa agtccacgat actcaacaaa 25200

taaactatta gaataataat tgttattatt attcaaagaa gctgaagatg agatcaatat 25260

aaaaatctat tgtgtttcta acaatcagca aaattgacaa acccttagtt aaactaagaa 25320

aaaaagagag aagactcaaa ttaactaaaa tcagaaatga aagaggagac attgtaacta 25380

tgtcacagag ataaaaagga ttctaagaga ctactatgaa caattatatg ccaacgaatt 25440

ggatacttca aataaattag taaattccta gacatataca acctacccat actgatcatg 25500

acaaaataga aaatctaagc agacctaaac tagaaaggag attgaatcag taatcgaaaa 25560

cctcctagaa aaaaaaaaaa aaaggccaag accaaatggc ttcactagag aatgctacca 25620

aatacttaaa gaatcaacac caattcatcc ctaacacttc caaaaattga acagtaggga 25680

acacttccaa actcatttta tgatgcccca gtaccaaagc cagacaaaga tgcaagaaaa 25740

caaaaccata gaccagtact cttgatgaat actgatgcaa aaatcctcaa caaaatgcta 25800

gcaaaccaaa tttaacaaca cattaaaagg ctatacacca tgaccaagta ggatttatct 25860

gcagaattca aggatagtta caaaaatcag ataaatgtaa tacatgaaat taacacaaag 25920

aaggacaaaa atcacatggt catctcacct gatgcagaaa aagtatttga aaaaattcaa 25980

tactctttca tgataaagaa aacattcaag gaactagaaa tagaaataaa ttacctcaac 26040

ataacaaagt ttatatataa aaagcccaca gtgaatatat tcaacagtaa aaaactgaaa 26100

cctttttcctc taagatcagg aacaaggcat ggatgcccac acttgccatt tctattcaac 26160

tggaagttct agccagagca attaggtaag aaaaagaaat aaaaggtatc taaattggaa 26220

aggaagaaat gcaattatct ctgttcacag attacgtgat tttatatgta ggaaacccta 26280

aatattaaaa aaatggttag aactaatacg tgaattaagc aaagttgcag gatacaaagt 26340

caacactcaa aactcagttg catttttac attaacaatg gacaatccaa aaagaaactt 26400

aagcaaaaaa tttcatttac aatagcatca aaaagagaaa agtacttagg aataaactta 26460

gccaaggagg agaaagactt gtataaaaca tttttgaagg aaattaaaga acacagataa 26520

gtgaaaagac atcccatgat catggattgg aagacttaat attgttaaga tgacaatact 26580

actcacagaa aactgaattt aatataagcc ctatcaaaat ctcaatgaca gtttttgcag 26640

aaataaaaaa agaattcatc ctaaaattca tatggaactt caagggaccc taaatagcca 26700

aaataatctt gagaaagaag aacactctaa aaaaaaaaag aagaagaaga aggagaagga 26760

gaaggagaag gagaagaaag ctggaggcct cacatttcct gatttcaaaa tgtattaaaa 26820

agctacagta atcaatatag tatggtactg gcatacagat agatatacag accaatggaa 26880

aagaatagag agcccagaaa taaacccttg tgtatatagt caaatgatct acaaaggcac 26940

taagtctata caagagggaa aaggaagttt gttcaaggag atagtgctgg aagaaccaaa 27000

tatccatatg caaaagaatg atgttggatc cttattttac tccatatgca aacattaact 27060

caaaatgggt taaagaccca aacataagac ctgaaactat aaaactccta gaagaaaaca 27120

tatgggaaaa tcttcgtgaa attggaatgg gcaatgattt cttgtgtgtg ataacaaaag 27180

cacaggcaac aaaagtaaaa atagacatat gagactacat caaactttaa aacttctgtt 27240

cagcagagga aataatcaac ggaatcaaaa ggcaacctat ggaatgggac aaaatatttg 27300

caaaccatac aagagatatg gggttaatat ctagaatata caaagaactc ctacaactca 27360

ataacaacac aaaaacaaat aactcaattt aaaaatggcc gggtgtggtg gctcacacct 27420

gtaatcccag cattttggga ggccgaggcg ggcgaatcac ctgaggtcag gagttcaaga 27480

ccagcctggc caagatggcg aaatcccatc tctactaaca aatacaaaaa ttagccaggc 27540

atggtggcag gcacctgtaa ttccagttac tcaggaggct gaggcaggag aattgcttga 27600

acctgggagg cggaggttgc agtgagccaa gatcacacca ctgcactcca gcctgggcaa 27660

cagagcaaga ctctgtctca aaaaaaaaaa aaaaaaaaaa aaaaggacaa aggacatgaa 27720

tagacatttc cccaaagaag atcagcaaat gcccattaag catacgaaaa gatgctcaat 27780

atcactaatc atcagggaaa tacaaattaa aactacaatg agatatcatc ttgcacttgt 27840

taggatggtt attataaaaa aaaagaaaag aacaaatggt ggtgaagatg tagagaattg 27900
aaacccttgt gcacttttag tgggattgta aaatgatgcg aaactttaaa aaattttttt 27960
aattaaaaaa atagaaatac cagacatata tccaaaagaa ataaaaacag catctcaaag 28020
agatatttgc acacctgttt ctgcagcatt atttacaata gctaagatga gcaagtaacc 28080
taaatttatt gataggtgaa tggataaaga aaatgtagta catacataat ggaatattat 28140
tcagacttta aaaaggagga agtcctgtca tatgttacga catggatgct tcttgaggac 28200
attctgcaaa gtgaaataaa ccaattgcaa aaaaacaaat actgcattgt atcacttata 28260
tgatgtatgt aaagtaatca atctcataga aacagaaagt ggaatgctga ttgccaggga 28320
ctgggggagg gagaaatgaa aagttgtttt tcaatgggta tagagtttca cccatgcaat 28380
aggaaaaagt tctagagatc tcttgtaaac caatgtgcat acagtttaca atattgtact 28440
gcacatctaa aaattgttaa gggtaaaaaa gatctactgg ttaataaaat ccaaacccgg 28500
gacactatac tttgcactac aataccctct cttgttagat tagcaaagga aaggatccat 28560
aaacattaca aaagcaaaga aaggcaggaa gccctcaact ggttgtcagt tgaagtgggc 28620
tgggtttgat agctatttct tttaacatct ggaactttag ctttaaaaag tgttaataaa 28680
gttgtaaagt ataaaaactc attatatttc tgtacactac aaacagtcaa gaaattttaa 28740
aattatgcca tttacatcag catcaaaaaa tcaaatatcc gagaagaaat ctaatgaaag 28800
atgtacaaga cttttttttt ttttttgaga tggagtcttg ctctgtcact cagctctgtc 28860
actagaggca cagtctctgc tcactgcaac ctccgcctcc tgggttcaag caattctgcc 28920
tcagcctcct gagtagctgg gattacaagc atgcaccacc acactcagct aatttttgta 28980
tttttagtgg agacagggtt tcaccacatt ggccaggctg gtctcgaact cctgacctca 29040
aaagatccac ccaccttggc ctcccaaagt gctgggatta caggtgtcag ccactgcacc 29100
tagttgatgt acaagacttc tacacagaaa cctacaagac attactaaga aaaattagag 29160
aaaacataaa taagtggtgg tacatactat gttcatggat tggaaaattt aatactttaa 29220
ggatgctagt ccccccaaa ttgaagtaaa gtttcagtaa aatccaaagt ttcaagaaaa 29280
aaatctccag caagattttc tttggtggaa attgacaaga tagttctaaa atttatatag 29340
aaatacaaag gactgaaaat agtttagaca attttgaaga agaatgtcaa gatttataaa 29400
gtaatttaga aagtatggca ttgttgcaag gaaaaacaaa ctaaccaatg gaaaagaaca 29460
gaaacagtag aaataaactc atgtattcaa ccacttgatt ttcagtaagt caccagtgca 29520
aggcagtgga gagaaaacat gatgttttct taaatgatgg tatattaatt gaatattcat 29580
atggaaaaag taaaccttga cccctacctt acaccataca gaaaaataaa ctacagatag 29640

actgtagacc taaatgtgaa aggtaaaaca gtaaatcgtc tagaagaaaa cacagaataa 29700
tatttttatc actttattgc aggcaacact tcttaaacag cacatagaaa gttctggtca 29760
taaagaaaga tttataaata aacttcatga aaattaagat tttttattga tcaaaagttt 29820
ccattaagag aatggaaaag aaactgaaag aggaagaggg aaagaattat atatatgtaa 29880
atttataata aaatataaat ataaaaatat ttatttataa atattatata tatttataaa 29940
atataaatta tatataataa acatatataa tttattttta taaatatata tatggagaga 30000
gagagaaata aacacaattc caagtgctga aaaggataca gagtaactaa actctctgtc 30060
tctactcaat attctggtaa gagtgtaaat cgacacaacc actttggaaa acttctgctg 30120
agcagagact caacatttcc acttccaagt tatataacca aagagaaatg caagcatatg 30180
tgcccaagca gacatgtacc agcatgttca tagcagccat tatttacaac ctccccacac 30240
tggaaagaac ccaagttctt ggtttccctg tatctttttt ctgtcacacg attgaggaat 30300
gggatgcgtc tagttcctca agtcggccat tctctgcaat gtgacccagg tagggacatg 30360
gggctgaaag tgaaaggtgg gtagtgtggt aaaagtaaaa tggccctatt tcctataggg 30420
atcacaaaga aatgaggtgg gcgaagaggg actccccaga agtgccagga acaggagcca 30480
acaggtcacc agaagaggaa gtgttaaggc ggagggactt cccgagaggg aacacagaag 30540
aaagaactcc taaagttatc cagggtcttc aaggacttct gcccacctgc tctcctgctg 30600
gtcctcctct ctgaccttcc tgctatagct acagagctgt ggttcctcca actccattct 30660
tactccaacc gtgtcattcc atggctctct gttatccaca aaagaaaaac cccaaattcc 30720
ttgtctggta ttcaaggcat ttcgggatct agcccccagt attcatactg gacttcaggc 30780
tcatcagacc actgacagcc tcaccaacat gctccattca cctggcttgc acacctgtgc 30840
atgtgctgtt ttagccacgg aaactatctg cccttttga ctgactgtta gcctcctact 30900
cagtcttcaa aatcccattc aaatggcagc tcctctgtca atctttgacc tctccaaaga 30960
gcatgagtca ctccttcctc tggggtccac cctccacata gtacatagct catggtgttt 31020
tcatctatat tctgggcacc atctaggcag ggacagtgtg tttctttgtt actgctgaat 31080
ccccagtaac taaccagtgc ctacagtggg tgctcaaaac atttgctaca tgagtgacta 31140
aatgaaagag aatgctttct atggaaacga attgtgaaca aacattatcc tcaatgttaa 31200
gacctatgta gaccgtacac tccaaagtta aatcttaccc tttttgaccc atttttctgg 31260
agcttagggg attgagagcc ttatgtattt ggtgaggata cgttttttgt tgttgtttgt 31320
tttgttttgt ttttgagatg gagtcttgct ctgttgccca tgctggagtg cagtggcacg 31380
atcttggctc actgcaacct ccgcctcccg ggttcaagtg agtctcctgc ctcagcctcc 31440

caagtagctg ggattacagg catgggctac caagcttggc taactttgta tttttagtag 31500
agacagggtt tcaccatgtt ggtcaggctg atctcaaact cctgacctca ggtgatccac 31560
cctccttggc ctcccaaaat actgggatta caggcatgag ccaccgcacc tggccgagga 31620
taaaagcttt aatgcttcaa tctttttttc ctgcatcagg gcctttggac atgcctttcc 31680
ctctgcctag aatgcttttc ccccagcttc tttcctttct catcctcagg tttcaagttc 31740
agtgttgcct cttcaaagag gcaaccctat ctgaactggg tctacgccac agatctctgt 31800
agcccacatc ccctagtttg tctccttcat agcaggtgta agatgaggaa tcgttttgta 31860
aatgtctctg tgtatgtgtt tgtaacttgt ttctgtactg aagcatgagc tctcagaggc 31920
tgggtaattg acctattttg ttcagcacag tatccctagt gcagagaagg ggacctgtag 31980
gtgccccata agtagctgaa gaagaatgtt aagtgaatga atgaatgagg ctaaaaatgg 32040
agacagcccc taagaggtcc tgctttcctc attagcttcc tcactgtccc cagaaactcc 32100
ccccagaccc acctcaggca acatcttctg cttccagagt gtgaataatc atgactaatg 32160
tcatcagatt agtaacaagg aactttcaga gaaggacagt gggctgccca gggtcacaca 32220
gcaagttctc atctcagtgg gttctcaggc tggggaagaa ctagcccagt ggccctgggt 32280
cagaaagcag actctgccat tcactcacag tgggaacttg gccgtatctc tgcctttctt 32340
tgagcctcag ctttcctcat ctgtggaatg gggcagcag gcttagcctg gatgcggagt 32400
gtgaggcagg ttagtgtggt tagtattagg tgtgtggtta gtgttagttg cttttcatct 32460
tttttttttt cgccagtggc aaggggtggg ttatgggtgt ctgaaggcag gtatgttggg 32520
gacctcaggc ttacagttaa tcagtagaag acgaaggtga gaggaagggc cttctcctcc 32580
ccagctcgtg caacctctga ggctgtgcga cctctcagcc tatctctctc tcctagccag 32640
gcagcaaatc tctattagct ccagcaatct ttccctcctg gcacttcttc cagcctgtgc 32700
aacctctcct agctgtggag catgaggtcc cagtcgcagc acccgcaccc cccactcccc 32760
acccccgctg cctgtgcaat ctctatctcc ctgcaagccc tgccatcctc tgcagccttg 32820
cccaaacctt gcaacctctc agcctctgca gccagatctc actccagctc catttcctcc 32880
agcccaggag gggccgcttg tagagcagtg aaaggaggct ttgaactatg ccacgagatg 32940
gagcggagct ggccacccac cgtctctctc ccctcagttc ccagcagccc ccatcagccc 33000
gccctggccc tggccaaagt ggactggtgt tactgattca ttctggggaa agttggcaag 33060
ccctcgtcct agtcaagccc gcttggcaga tggggaaagg aggcccagag aggcccaggg 33120
ccttgtcctg ttcacacagt gggggatggc aggggcctgg gctaggaccc gggctggtgc 33180
acttgcccca actccccggg gacaattcag ggcaggggtc ggggtggcca gatccttcct 33240

gtgggctgca gagatcagca ggctgggttc ctgatgcaga tgtcctgggc agtcgggggc 33300
cctccttgtg gcccctgccc cccagcctgg ggaatattct caggtcgttt cagaagttcc 33360
aagtttactc agaggtctca tttgagctgt catgaaaagt ccctttggcc tggggccagg 33420
gcagggagtg gggcggtgg cacctgtgag ccaggggtgg gggtgggttc ctcccattgc 33480
cccctctccc tccccttcac gtgtccgggg gctccggagg gcagggaagg agggcatgtg 33540
aggaaggggc tgcgagggca agaagaccct cttcctggag gaagcatctg ttcccctctt 33600
ttcactgctc ccttctagac ggttccctga gtgaccctag aaaaaccccc accctgctct 33660
gaacttcagt ttccccatct gtaaaagggg ggaagcagaa tcatcataag agctcttatt 33720
tactgaggtc tcatatatgc caatatgacc aataatattt attattttt ttctgtattt 33780
tcatagctaa aagcaaagag ctcttacata tttaaagtaa aacataggcc ggccacggtg 33840
gctcatgtct ataatcccag cactttggga gactgagaca ggcagatcac ttgagcccag 33900
gagttcaaga ccagcctggc caacatggtg aaaccccgcc tccactaaaa atacaaaaat 33960
tagccaggtg tggtggcagg tgcctgtagt cccagctact cgggaggctg aggcaggaga 34020
atcgcttgaa cctgggagat gaaggttgca gtgagttgag attgcgccac cgcactccag 34080
cctgggcaac agagcaagac tctgtctcaa aaaaaaatta aaaaaaaaaa aaagtaaaac 34140
atgatagcgg gcacttctca tgagccatct gtgcccagct cttttgcctt ccccatcaca 34200
ctggctctct ctgtgtaccc cagagcttca ggacctaggg aggtgcctgg gccatggtag 34260
atgttccata aagaataggt tgagtaaatc ctcaccacag acctaagaca ggcattctta 34320
ttcctatttc cactcttaca gatgcagcaa ctgagctgca gaaaggttag gtggcttcct 34380
caaggtcaca caggtactaa gcggtggact tgaacccagg tctggccagt tccacagctc 34440
aaagtcacaa ggggtggtgt gctgggaagc cagttccagt tcccccatct aaccgcaggc 34500
accccttcct agttgtgtgg agaaggcccc tgtgcctgcc tgtgaggatg ctgaaggcgc 34560
tagcttccct ctctggctgt ggaaggaacg gataccccgt tcctttcatt catttattta 34620
tcaggcacct gggttgtcct gatgctacaa agaggtgggt tcctcccaga acttctggtg 34680
gctggctttc tcttgaatgg gcctcctcct ctggtatcca ccccctactc tactgcccac 34740
aagcccctta ctggctctaa tccccacctc ccagtttctt ctcctagcct gaaaccagga 34800
acgctgggca gtgggagcct gcagtctgag ctcagagctg tctttgcctt gctctcccca 34860
cctctcctgg tcctggattc tgcctcctcc tatcacaagg gcttttagct acctccactg 34920
tgggactgag taaggatccg gacaccctcc tggcagagga gcgggcagga caggggcatc 34980
gctgagccca cggtggggag caggtgagca gggcctctcc tctgggggtt gcctgtcctc 35040

gtgggaatga atactccgcc atccccatcc aatgctgctt cccagaagct acccaagccc 35100
atgagtcttg ttttcatgtt attctatcta gcaggtggtg gccttgtcct acagactggc 35160
atccaatccc agctccatgt catctggtca cagttaacct ggggcaaggt tgtccccact 35220
aagcctcact cttgtcatct gtgaaatggg ctgaatgagg ctgcccgctt cacctgttga 35280
ggactatggg agacgcttaa gtgcctacaa catggtgagt tggcactggg ggggccagag 35340
ccaggtggct ggccaggcag gaccacagca gcctgagtgt gtcgctcccc ctctgtcact 35400
cttggagtcg ggcccgtctc catgggctca aagcttctca tgccacaaac tctcttcctc 35460
aaaggaaaag ggccattagg agcacagctt tcaccctgtt cggccctgag gtccccacaa 35520
ctagctccac caggatgggg gtaccttggt ccccatttca tagatgagaa aaccatggct 35580
tctcaaggct agaagcagag agacttgccc aagttcaccg agtgaggaag gcgcgtggcc 35640
gggttttgac ctggcaggtt ttccccgact ccaccatgtg cttctgaaag ccccactctc 35700
cccccaggga atcacagatg tcctcctcgc agcagcctcc tggctttgaa tgtcccgcac 35760
ctggtcttct gccttcactg agcagagacg tcattcctgc ggcaagtcct aagttcccgc 35820
tccccagggc tcctccacca ggaatgttct tcctttccca tccttcaagg ctcagcctcc 35880
actgacctcc ccaccctcct ctcctccaca tgcacaggga actgggttgg cctcttacca 35940
tccgacccgt tccccgtgct tcatgagaaa gatgcagcga cagctcatgg ggtcacctgg 36000
cttgactgcc cgacctgcta cgtcccagtt gagtcaccga gggaaaatca ctctacctct 36060
cgcagcctca ttgcctcatc tgcgaaatgg gcacaccagc cattgttttg aatgactggg 36120
tggaatcaag gtggtaagac atagaaactg acacagaatg ggtcccatca ggctctacac 36180
ccatgcccgt cacctcccga ctcagccgcc cagaatcggg tgattgacat gttatgtttg 36240
gcttgaaact ggctttcctt ccccagcccg ctacattttg gaggctggtt ttcaagatag 36300
agggtcgtct cagccacatc cctgctgctg ggcccctaag ctgtgctgaa caagagaccc 36360
caacctcatt tctgcccagc ctcactgcat gaatcttctt tgcctcagtg tccccaattg 36420
tgacttgggc acagtcctcc gagccctccc actgtgacac tagatgcagc aggcagggaa 36480
cagtccctcc tgggcagaga aggcaccctg gtcccagcag aaaaccaggg ctggaattac 36540
cttctgtttc ggcttctttc tcagaaatcc attccgtgtg ctgggggcag tctgatgggc 36600
ttctgggggt ggcagctggg ttcagcgtgg gggtgacaga tgagggtctg agaaagcagg 36660
cagaggcctg gggtgagaat ccacacccag cccgagtcac caatcaggct gtcataagac 36720
ctgtcactct gatgacgggc agctgggcct ccaggcaggt gtcccacaga gcagcagaca 36780
gaggaaagag acggcaagga aggtaccagg cggccgcagc agggatgcac gggccgcaga 36840

cagacagaca cagctgcgtg gctgtcagcc acactcaggg agaggcaggc agagggccag 36900
acgccgagct tacggtcact cagcagagag acgccagtgg gtctgtctga ggccgctggc 36960
tgccacccag ccgcatgtgc agagtgggga ggaagaggag gatgtggtga gccgctcccc 37020
gccagccccg cccacccgca gctgggctcc tcctgggagg gagtgagaaa gaggtgggag 37080
ggggcctggc cccacagcag gcacccactg tcaccaaagg tcacctgcaa gtaagacatt 37140
tggggccatc ggggtgacac ttccctctta gggacctgtg acatcatccc cacaggcctc 37200
tcagtcataa cccatgaggc aggcagtgcc tcctggagcc accacattgc acagatatgg 37260
aaactgaggc cagaggtgtg aagatgtggc acagtttcac ccgtggaccc agagtcctca 37320
gtggccgact ccagcccctc agcacagaga gaaggaccag aaccttgccc aaggtcccac 37380
ggaagcccct ttttcagact ttgcaaacca gcctgtttct tattcctgag ccttacctgc 37440
ctaatagggt ctgagcttgg gaccctctgc tctggcttga cccccatgcc tctctgcctc 37500
catttccttt ggatccagtc tcctgctgca gagctccagg gagcaggacc ccttgggcac 37560
gtcacatcac ctctctgaat gcctcacttt gcccatctgt caaatggggg cacctagcta 37620
tgaggaagag ggcagacatt aacacatgag aattcaagaa gaaaatagag aaaagaaatt 37680
ctcaagagag ttttcatctt gctaacatgc cagaggccca tgagagggtc agatgtttct 37740
ggaagacatt caagaaacca ggagacaaaa tgtaacaagc ttaatgccta gtgcagtctc 37800
caaaggagag caagagaatt gctaccacca ccaccgctac caccaccacc actaccactg 37860
ccatcaccac cacctcgaat tactttggtg cctacatgat ctcactttat cccacaaagt 37920
gtgaggcaga caagattagt cccattttac agatggagag actgaggccc agaggagtga 37980
aacgtcacac aagccaagaa gaattagagc tgggatttga acctgggtgt gtttgattcc 38040
taagcttata ccaggctgtg aaaacggggc cataccagga tgaaatgtcc cacagttggg 38100
ggaggctatt ttatgctgtt ttgataacaa gatgcaattt gtagctctcc ctgctgactc 38160
atttttggga atcaacttgc tgttgacttt cgtgaccact ccagagaaaa aagagaaagc 38220
gtcttgctat tctttctttc tttttttttc tagctgtttt tgaggaactt ggtacaaatt 38280
cccctgtggt tgatttgagg gatcctggcc ctgtagagct cttcccagct accgaagtgt 38340
gtcgtgttct ctggagaacg aaccttggga agacaccagc ttttgttttt ctttctcttg 38400
atcttcgggg tgaattctct gataacatgc actgaaattt gtagtggttg aaagatcagg 38460
agaagagaca gtaaaaagag ctaatatttg ctgaaagctc attgtgtgcc agggttttaa 38520
ggactttaaa gatgcaccct ctcatttaaa cctcagaaat actcgagaag gctgtgtgca 38580
gcggtgcaca cctataattc catcattttg ggaggctgag gtgggaggat cgattgaggc 38640

taggagttca agaccagtct gggcaacata gcaagaccct gtctctataa aaaaatttaa 38700

aaattagaca gtcatggtgg tgcacgcctg tagtcccagc tactctggag gctgaggtgg 38760

gaggaccact tgagccaagg agctgaaggc tgcagtgagc tatgactgta gtgctgcact 38820

caagcctggg caacagagca agactctgtc ttcaaaaata aaatccccaa aatgaaaaag 38880

aatgagtcta gaagatagtt atcatcatca tcatcatcgt ccctgtttta cagatgagga 38940

aactgaggct cagagaggtt aagtgacttg tccaaggtca cacagctgat gagtgccaga 39000

gttgggattt gaactcagtc atttgggctc cagagcccgc actcttaact cactacactg 39060

ctctggggtg tgtatgaaaa caaggatatt caatttctcc tccctccctc ctgcctgcct 39120

tctttatcta cctgctttct acccacccac ctacctatcc acccattcat tcttctctgc 39180

acccacccac ccagccattc cttgcttcct ccccctaata tttattgagc aggctcagag 39240

aggttaagtg acttgtccaa aatcacgcag cttggctaca tcctggggct agcccacaga 39300

aatatacaca cagtgacagc ccccatggag cctatattct agtagggaga gttagacaaa 39360

aagaagtaac agacaaactc aatgtaaact gtactaaatg tcatagaaaa ccaatgagag 39420

atcaaggttg tgacatttgg gggtacacta ggtgctaaga agtgcccagc catgggaaga 39480

acattatcgg cagaaggatc tgcaagtgca aaggcactga ggtaggaaag aatgtggctg 39540

gctccaggaa cttgagaaca gcatggcgta atacagtgag caaatggagg aatagtagaa 39600

ggtgaaggca cggaaatcac acatctacac cacatgggac ctcacggttt tgggtgagga 39660

aggtagattg ttccaaagga aatgggaagt catcattgga gggtttcatc ttaagagtgg 39720

cttaatccag tttacatttt cagttcaggc caggagttgg ggaatgggtg ggatcagcag 39780

gggtcacagt cccttggtgc ttaaaaagta gatgcatttg gagctgccag gcccatgtcc 39840

actggggtaa acatgagtcc cctcagaaat agacatgtgg tcccttgagg ggttcctctg 39900

ggtctgtaaa tgtctcataa gtatggcctc tatacccttg gaaggaaggt gccataggat 39960

ctcagcggcc ctccaaccat gcctcagtgc tgatagctgg tcagcgggga ggaagtggtc 40020

acatttgcat ttcagccaag ggcctcctg ggaaaaaata attgacacac cccctccctc 40080

aacccatttc ctcttttta tttttatttt aagagcttta tttagctatg ttttacataa 40140

cataaaatca agccactgta aatgtacagt tcaatgattt ttagtgaatt tatcgaattg 40200

tgcaattata accacgatcc aattttaaaa caatcccatc actcctgaaa ggttccttgt 40260

gcctgcttat agtcaatctc cattcccatt cccagggcca ctcgtctact ttatgcctct 40320

actgattttg cttttttttt ttcctaggca tctcatatca atatgttgtc ttttgtgttt 40380

agtttctttc agcatcgcgt gtttggggtt caaccacgtt gtagcgtgtg ttggcaccac 40440

atatctaaaa atccattttt aggccaggcg cggtggctca tgcctgtaat ctcaacactt 40500
tgggaggcca aggtgggcgg atcacttgag atcaggagtt cgagaccagc ttggacaaca 40560
tggtaaaacc ccgcctctac taaaaaatac aataattagc ctggtgtggt tgcacacgcc 40620
gggagattga gaccagcctg gccaacatgg tgaaaccctg ttctactaaa aatacaaaaa 40680
ttagttaggt atggtgacgt gcacctgtag ccctagctac tcaggaggct gaggcaggag 40740
aattgcttga acacgggaag tggaggttgc agtgagcaga gctagtgcca ctgccctcca 40800
gcctgggaga cagagcgaaa ctccatctca aaaaaataaa aaataaaaat tgatttttat 40860
tgctgaagaa tattacattg catagatatg ccacattcat ttatcctttc atgagatgat 40920
ggacatttgg agtatgtcca ctttttcctt tactacaaac aagcatgcta tgaacacttg 40980
catgcgagtc tttgtgtaga catgcatttt caaatctttt ggatatatac ccagaaggag 41040
acttgttgag ttaaaggtag tttaacttat taacttttaa ataagtttaa cttattaaga 41100
aactgacaaa ctgtttttca cagcagctgc cctgtattat attcccacca gcaatgtctg 41160
aaggttccca tttctctacg tccttgacaa tatgtatttt cttttttttt tttttctttt 41220
tttttttttt tttgagatgg agtctcggtc tgtcgcccag gctggagtgc agtggcgcga 41280
tctcggctca ctgcaagctc cgcctcccgg gttcacgcca ttctcctgcc tcagcttccc 41340
gagtagctgg gactacaggt gcctgccacc acgcctggct aatttttgt attttctttt 41400
gtagagacgg ggtttcaccg tgttagccag gatggtctcg atctcctgac ctcgtgatgc 41460
gcccgcctcg gcctcccaaa gtgctgggat tacaggcgtg agccaccgca cccagcagac 41520
aatatgtatt ttctgttggt ttttattata gccatcctag cggatgtgaa gtggtatctc 41580
attgtggttt tgatttgcat ttccctaatg gctaatgatg gtaagcatct tttcatgtgc 41640
ttggtagtca tttgcatatc ttgtttggag taatgtctat tgaagtccct tgccaaattt 41700
tatttatttt attttttttt tagacagagt cttgatctgt ctcccacact ggagtgcagt 41760
ggtgtgatct tggctcactg aaacctctac ctcctgggtt caagcgattc ttgtgcctcg 41820
gcctcccgag tagctgggct tacaaacgtg tgccaccata tccagctaat ttttgtgttt 41880
ttagtagaga cgggttttca ccatgttgac caggctggtc tcgaaccctt aacctcaggt 41940
gatccgccca cctcagtctc ccaaagtgct gggattacag gcataagcca ccgtgcccag 42000
ctccttgcca atttttaaat ttgattgttc atcatttgtt gttgaatttt aagagttctt 42060
tgtatattct ggaaactcac ctttatcaga tatatgaatt acaaatattt ttcccattct 42120
gtggctgtct tttcactttg ttgatagtgt gctttaatgc aaaaaaaatg aaaaaaaatt 42180
gactatagat tcatgggttt atttctgaac tctaagttca attccattga tctatatgtc 42240

tagccttgtg ccaggattat cacactgttt tgattactgt agctctgcag taagttttgt 42300
tttgttttgt ttttgttttt gtgttgtttt gagacagagt ttcgctcttg ttgcccaggc 42360
tggaatgcaa tggcacaatc ttggctctgc agtaaatttt aaaaatggga atttgagtcc 42420
tccaacttta ttgttttggc ttttctgggt cccttgaaat ttcacatgat ttttaggatc 42480
agtttttcca ttttttaaaa aaggccattg ggacttttat aggaattgca ttaaatctat 42540
aaattacttt gaggagtgca atgttaaatc ttccattaca ttaacataga atttaaaaaa 42600
ctttatttag gtcttttcta attttttcag caatatttta tagtgttcag tgtacaaatc 42660
ttgcacctcc ttggttaaat ttattcttaa gtattttatt atttttgatg ctattataaa 42720
tagaatttat ttaatttcct tttggggttg tttattgcta gtatatacaa acacaattta 42780
tttttgtatg ttgatcttat accctgtcac ttggctgaat ttattagctc taataatttc 42840
tttgtgcgtt ctttagaatt ttccatatat atgatcatgt catctgcgaa taacagtagt 42900
ttttactctt tccttttccc atttgaatgc cttttatttc tttttcttgc tgaattgttc 42960
tggttaaaac tttcagtgca atgttgaata ccagtggtga aagtgggcat cctctttcct 43020
gatcttggtg gaaaagtttt cagtctttca ctgatttagt gtgatgttag cttgttagct 43080
gcgggttttt ctttctttat ttctctttct ttctttctct ctttccttct ttctctgtct 43140
ctctcttttt tatttgagat ggagtctcac tctggtgcag gttggagtgc agtggcatga 43200
tcttagctca ttgcaacctc atgggttcaa gtgattctcc tgcctcagcc tcccaagtag 43260
ctgggattac aggtgtgtgc caccacatcc aactaatttt tgtattttta gtagagacag 43320
catttcacca tgttggccag gctggtctcg aactcctgat ctcaggtgat ctgcctgtct 43380
cggcctccca aagtgctggt attacaggca tgagccactg tgcccagcca ggtttttcat 43440
atatggtctt tatcatgttg agggagttct ctcttattcc tactttactg agtgttttta 43500
tcatgaaagg gtgttaaatt ttgtcaaatg cttttctgca tcaatttgga tgatcgtgtg 43560
ggttttctta ttctattaat gttatgtatt gtattgatta attttcttat gttgaaccac 43620
ccttgcattc ctgggataaa tcctttttgg tcacactgta taatcctttt aatgtgctgt 43680
agaattagtt tgcttgtatt tttgtgaaat tttatatcta tattcataag gaatattagt 43740
ctgtagtttt gttttcttgt ggttctcttg tctggatttc tctcctgttt tgtggaggag 43800
tttgagaaga actgatgtta attcttcttc caatgtttag tggaattcac cagtgtagcc 43860
atctgactct ggacttttct tcattgggag gtttttgatt caactcttta cttgttgtat 43920
gtttattaag attttctatt tcttcttgag tcagttttgg cagtttgtgt taccaaactt 43980
taatatttaa gttgacaact attattatct aatttgttgg catacagtca ttcacagtac 44040

tcccttatga tcctttttat ttctgtatga tttgtagtaa tattcccact tccatttctg 44100
attttagtga tttgtattgt ttcttttgct tagtccaact aaaggcttgt caattttgtc 44160
aatctttttg aagaaacgac tcttggtttc tttcatctct attgattttg tattctttat 44220
tttgcttgtt tgcactctaa tctttattat ttccttcctt ctgctacctt tgggtttcat 44280
ttgtccttct tttttagtt cctttaaaag taaagttagg ttatgatttg tgatcttatt 44340
tgttgttttt ttttaatgaa ggcatgtagg gctacacatt tccctgttag cactgctttt 44400
gctacatccc ataattctgg gtgtgttttt ggtgctttca ttttcatttg tctctaagta 44460
ttttctaatt tccttgtgat ttcttcattg acatgatgac tgtttaaaaa tatgctattt 44520
aatttccaca taattgtaaa ttttccagtt ttccttctgc tattgatacc taacttcatt 44580
gtgatcagag aagacacttc atatgatttc aatcttttaa aatttatcaa gacttgtttt 44640
gtggcttaac atatagtcag tcctggagaa tgttccatgt gcactttgtg gtcactgtta 44700
ttgttgggtg gagtgttcta tgtgtgtcta ttagctctgg ttggtttata gttgtacaag 44760
tcccctactt tcttagtctt ctgtctagat ggtctccaac tattattgta gaactgtcta 44820
ttcctctatt cagttctgtc aatatttgct tcatatattt ttgggctctg atgtttagca 44880
catgtatggt cataattatt atgtcttctt ttttttgaga cagagtgttg ctctgtcaca 44940
caggctggag tgcaatggtg caatctcagc tcactgcaac ctccgcctcc caagttcaag 45000
caattctctt gcctcagcct ctcgagtagc tgggactaca ggtgtgcacc accgtgccct 45060
gctaattttt gtatttttag tagagacagg gtttcgccat gttggccagg ctggtctcaa 45120
actcctgacc tcaggtgatc cactcatctt ggcctcccaa agtgctggga tgacaggcat 45180
gagccaccac ctggccaatc ttcttgacaa attaacacat ttatcaacat gtaatgtcct 45240
tgtttgtctc ttgtaacaat tttttactta agactcattt tatctgacat tagtatagcc 45300
acctcagctc tctcttggtt tctattttca tagaatatct ttttccatcc tttcactttt 45360
aacttatttg tgtctttgga tataaagtga gtctcttgta gacagcatgt agttggatca 45420
tgttttttaa ttccttctgt caatccattc tgtcttctaa tcagtgaatt taatctactt 45480
acatttaaag tgattcatga taaaaggctt accactgcat tttgctattc atttcattta 45540
tatcaaattt tcttttgtta ttcaattccc tcattacctc cttctcttgt attgattgat 45600
tttcttctag tgcaccattt tgatagcctc ctcatttctt ttctgtatat ttttagttat 45660
tttcttagtg gttaccttga ggtttacact tcacaggaaa tttataacaa tatagtatga 45720
attgataaca acttggttta aatagcaata caaaaactct actcctatac agctccctct 45780
actctattct actcctatcc ctccccctct tttgttgtca ttatcacaaa ctacatcttt 45840

atgtggcatg tgctcattag catagattta taattatttt tatgtatgta tcttttaaat 45900
aatataggaa acaaaaagag gagtcataag ccaaaaatat aattatacta gtttttatat 45960
ttacctttac tagtgttctg tatttattca tatggctttg atttactgcc tagtgtcctt 46020
tcatctcagc ctgaaagact ccctttagtg tttttcatag ggaaagcctc ctagtgatga 46080
aatacttcac cttttttttt tgcaatcttt gaatgtttta atttcttaat cattttggaa 46140
agatagttgt gccagataaa gaattctcag ttggcagggt tttttccttc agcactttaa 46200
atatatcatc cttctgtctt ctggccttgt ggcttctgat gagactttgg tcattaatct 46260
tgttgaggac cccttgtatg tgacaagtta cttctctctt gctagtctcc agattctcga 46320
tttgtctttg tctttcaaca gtttgattat aatgtgtctt ggtgtagatt cctttggttt 46380
tatcctagtt ggagttcact gagcttcttg gatgtatata ctcatacctt tcatcaaatt 46440
ttggaaatgt ttggccttta ttcaaatatt ctttctggtc ctttctttct cctccttctg 46500
ggattcacat aatgcatatg tttatacctt tttttttttt ttgagatgga gtctcgctct 46560
gttgtctcac aggtctctca ggctctgttt atttttattc ctttttactt tatgctcctc 46620
agactggatt atttcaattt acctagcttc aagcttattg attctttctt ctgcctactc 46680
aaatctggtg ttggagctct ctaataaatt tttcattttg gctattatat ttttcaggtc 46740
caaaatttct atttggttat tttaaataat gtctagctct tattgatatt atctacttgt 46800
tcacacgtag ttctggttta acttagttct tcactcatgg tttcctttag ctttttgagc 46860
atatttaaga caattgattt aatgtctttg tctagcagct ccaatgtctg gacttttta 46920
gggacatttt ctatccattt ttcccccgtg aatgggctgg gtgttcctgt ttctttgtat 46980
acgttatagg tttttgttgg aaacttggca ttttgaatgt tacaatgtga taattctaga 47040
aatcacattc tcccttcccc tgtccacaag gcttgctttt gtcatttgct gtgagcttca 47100
gccattcatt tacttagtga cttttccaaa ctatttttgc aaagactata tcccttttg 47160
tgtatgttca ctgaaatctc cattctgtta tctcagcagt tggtcagtga tctgtaagag 47220
gtttccttat atacctgcag ccaaaaagaa aaacaaaata aaagatactc ccctagtctt 47280
tgtaaattgg ctctgagctg ggatactcct ttacacttag ccaggcagac tacaattctg 47340
tcttagcctt cacctctgct tgtgcagagc ccaattagcc agaggtgcaa gcctaggatc 47400
atctcatatc atttctgtgc atgacttcag ccctgggcat atgtgttata ttctagattc 47460
cctggtatac acggaagctt ttcaaagccc ttattccccc atatacattc ctccttagtc 47520
tttttcttct ccagcttttt ggtcggtctc tgctttttgc tttgtctgtc atcctttgcc 47580
ccacatgggt agtgtgtgtc tttaaagttt gtcaacagac attgcctggg aagctgctcc 47640

agcctagagt tcaggggaag tgggagggca aaacaaaggc aagcacctga gtggatcctt 47700

tagggagatg ccaaacagat caaaatgtag aaacacaatt ctttgagacc aaggcttgtg 47760

ttgttctttc tggtaccagc aagccatgcc aagaatgcag gctactgtac tcatagccac 47820

gactaagctg atgaatgtga gatggtaggc aagtaagcaa aaatgctaca gtattttctt 47880

accaagaatt agcagccttt tctggttgct aaagggtttt gattagattt caggttccaa 47940

aaaagtttct tttgacactt tcttccagct taatgtttgc tttagtggag gaactgatcc 48000

ttggagttcc ttactctgcc atttttaaat aatgtactta atggtgtttt ttttcttttg 48060

aagcataacc cttttaaatt ttattgaaac ccaatttatc aatttttttc ttttgtggat 48120

tggactttgg gtgccatatc taagaaatct tcacctatac caaagtagca aaaatctcct 48180

ctcatatttt cttctaaaag tttaatagtt ttagctcttg catttttatt ttttattatt 48240

tgtttatttt ctgagacagg gttttgctct gttgcccaga ctggagtcca gtggctcact 48300

gcaatctcag gtcactgcaa ccttggcctc ttgggttcaa gcgattctcc tacctcagcc 48360

tcctgagtag ctgggactat aggtgcatac caccatgtcc gcccaatttt tgtatttttg 48420

gtagatatgg ggtttctcca tgttgctcag gctggtcttg aactccgggc tcaagtgatc 48480

catctgcctt gacctcccaa agtgagccat tgagcccagc cttacctttt taaaattatg 48540

attttgactt aattttgtga atgctgtgag gtaatgatct aaatttatct tttttcatat 48600

aaatatctaa atgtttcagc acaatattta gaaaagattg ccctctccct tttgaattgc 48660

cctgacacct attttgataa ttgactataa atatatgggt taatttctgg accctgtatt 48720

ctgattcatt gctttatatg tctatccatt tactagcacc ttactgcctt gattgctgta 48780

gctttgtagt aagttttgaa atcaggaagt agaagtcttc caaatttgtt ctgctttttc 48840

agaattgttt tggtcattct gagtcctttg catttccatt ataaatatta aattccatat 48900

aagtttcagc ttgctaattt ctgcaaaaac tcctactgga attttgacaa ggattgtctt 48960

gaatcaatag atcagtttga ggaaatctac caccttaaaa acttggagac tttcaatcct 49020

ggatataggc actctctcca tttatttaga tctttgaaaa ttcttctcag acaaccgctt 49080

cctcatccat tcattttttt aaattaattt tttattgtaa taaatacaca tcacatgaca 49140

tgtatgatat ggtttggctg tgtccccacc caaatctcat ctgggattgt agctcccata 49200

gttcccacgt gttgtgggag ggagcctgtg ggagataact gaatcatggg ggtggttttc 49260

cctatactgt tcttgtggta gtgaatgagt ctcacgagat ctgatggttt tataaggaga 49320

aacccctttc acttggttct cattgtcttt tgtctgccgc catgtaagat gtgccctttg 49380

ctttctgcct ttcaccatga ttgtgaggcc tccccagcca catcaaactg tgagtccatt 49440

aaacctcttt ttctttataa attacccagt cttgggtatg tctttatcag cagcgtgaaa 49500
acagactaat acgatgtacc atcttatcca attttaagtg taagttcagt ggtattaact 49560
gatgcattcc tttctgaggt ccaacattgt ggggttcctg taatggacca gatggtctgc 49620
tgagagccac agccaagaaa gggcagggga tgtttcatgg ggagcaaacc acaaaggaag 49680
gagacgtcta gagggctgga ttgtgcgcaa catgtggctg aaactgggaa ccccctttc 49740
actcagttac cctgagacag caggtttatt ttaagagcga tggctgaata taaagaaaaa 49800
actaataaat agtgccctca gttatgagta acacagaaag aatatgttta tcctgcctta 49860
aaagctcatc ttggtttgta ttaggctctg gactaatagg ggtatcattt aagcagatga 49920
taagactaag aattttgtaa atttctttcc aaaattagga tctctaatgg gctgatcaga 49980
ctgccctagc cattgatcac atcacttgct gtttggcaca tgagttgtgt cttggttggg 50040
ttcccccaga ggcaaattct aagacaagga tttaaggata agtcatagat ttgggaaata 50100
atcctaggaa acacagggga atggagaagt ggggcaggca aagcgaggaa gccagtaaag 50160
ggcgctttaa ggagaaggcc actgctgttg gcaactggag ctgaatccca ctgggtacct 50220
ctctgggaga caatatagaa catgtctcca acttgtccca aaggagaaac aagaaatctg 50280
ggatatttct tttccaggac ttcatttgtt gttagttgag ggctgcttcc agggccacga 50340
ctctctgaca cttccagctt gccgattgca tgcacctgtt gccaaaaatg aaccctcacg 50400
cagggagttg caggtgtttg cagtacaaat gctttggtgt ataaagctga gaagttaagg 50460
gtaaggcttc aacatctgct acagggacca tgatagggac aattaattgt gtcccagctt 50520
ccatcttgtc cttctttctc atatcaggac catagtgtaa ttctggatgg caacagaccc 50580
agctaataga ccactttcca gtttcccttg cagctagtga tgggagtgtt cacatggacc 50640
ttctggaagc actctgtggc ccttccagta ttccccttcc tcctttctcc tgcctaaagt 50700
gtacacgtga tggctggagc tcaagcagcc atttagaact aaagaaaaag aataggataa 50760
tcacagagaa cttggccctg atatcatggg tcatgaacaa atgtcagcaa tcatttgtct 50820
ctagacttct cgttccataa aggaattagc tcttaagtgt ttaagccact gtggttggtc 50880
tctattagaa gttggaactt ctaaccatac aaggggattg agtgactatc aaaaggatga 50940
agaggcttat ctgtgtaccc aaactggtga agcaagaaat aaccaggtat gctgtgtgta 51000
taaagtactg actaggttga aataaatcat gaagagaggg ttggatgaat aatttataaa 51060
acatctgtga aacagaatac tctgtagcta tgccatgtga ggacataaca agaaggtggc 51120
tgtctgcaag ctagaaaggt tgttcagaag acaaccatca accaccttga cttagactac 51180
cagcctccag aagtgtggga aaataaactg ttgcctaagc cacacagtct atggtatttt 51240

gctatggcag cctgagctga ctaaaacaga tgtatctgta ttagtcagtg ttctccggag 51300
aaacagaact gtaaccaaca caattattaa taacaataat acaaaattgt ttacagttgt 51360
aagggacttg ccatctgcaa taagctatca aaaccacctt ttattttaat tttgtatttt 51420
tgagacaggg tctcactctg tcgcccaaac tggaatgcaa tggtgtgatc atggtgtgat 51480
cactgcagct taaacctctc aaactcaagc cattctccct cttcactctc ccgagtagct 51540
gggaccacag gtgtgcacca ccgcgcctgg ttaagtttta aaaagctttt tgtaggaatg 51600
aagtctcatt atattgccag ggctggtctc aaactcctgg gttcaagtgg tcctcctgcc 51660
tcagcctcct aaagtgttgg gattacaagc gtgagctgct gtgcccagcc ccaaactacc 51720
ttttaatatc cccattttag acataagaaa acaggtgcag gagactaagc aactggtcca 51780
caggcacata gccagtaagt ggcggggtca agatttgaac ctgggccgtc tagcctcaag 51840
ggttaatagg cccatttctg ggtggtggga atttttattt ttaatttttt atgttattct 51900
gtattatctg atttttaaaa tgatatctta cttctggaat ctgggaaaaa taaccaagct 51960
atttccattt tgaagagaaa aaaaaaagga catcacaaaa catcagtatg gtgggaaaag 52020
ggtgcagaga agggaaggta taaaaaaatc tatagagatg tgagccatgg tccccctgaa 52080
gcatgtgacg tcctttactt caagccagat attcaattac atgtatatga agattagaac 52140
cacagaacca acatgtcaca tggaaaatgc ttgcgagatt ttcccaaagt caagagtgtg 52200
tcacagccaa ggggaacctg aagcacccta gggttgcatt aatagaggta tggtgtccag 52260
aacaagggag gctattctca ctgcactggc cacaccttca gtctacgtcc agttctggac 52320
cttcattttt aaagaacatt ggcaaattga tgtatattca gaacaggcaa ccgagaaggt 52380
agagcagctg gtgtggagac agtaaaaaaa accatgagac agcagaagta tttggaaaga 52440
gaagactcag ggtgggggtc atgagcaggt ccagaccagc atagtcagag ttgatgaact 52500
ggagaagaca gtgagctccc tgtccctggt atgtaaacaa ctcctcactg tgggggtatg 52560
gttggattaa ctgagtgtgt ggttggacca agggcaatga agtctccttt catgctgagt 52620
gtctgtgtgt ctctgattct tagaaagaca taggagggct taccatggag ggagataatt 52680
atggaccgaa tgtgctgctt tcaacctctg ccaaattcat atgctgaagg cttaacccac 52740
aatgtggctg tatttggaga tgaggccgct aaggaagtaa ttaaggtgaa atgagctcag 52800
aagggtggga ccctgatctg ataatgttag tgcccttata agaagagaca ccagggagtt 52860
tgccctcaat ctccctcaca gcaagaaggc agctgtctgc cagccaggaa gagagccttc 52920
atcaggcatg gattcagctg cctttctttt ctgactgcag agctggggag atgggagttt 52980
accaaagttt tccagatgct gcacagagaa ttcaggtggt gcactgaaaa tgtgcctacc 53040

agagactggc acaaatgcgc gaaggaattt cctttgcatt ctgatcccta atgttattcc 53100
tggaccaact tgttgatcat ggactttcca gcctccagaa ctgtgagaaa taaacgtctg 53160
ttgtgcaaac cacccatcca ttgtgttata gcatcctgag cagctaagac agagggttac 53220
agcaaccact gagctgattg ggcacctctg caagctttct cttaggggac cctggggctg 53280
agccccagcc tccaagttca ggggtggggg tgcggtgggg tgagctgatt acagtcttag 53340
ggcagaggag gaattgccca tcttctccct cccctgccac ctccaaaatt ttcacagtta 53400
acaaaacaga tcttcttttc actgtcaaag gttttaaatt caatggaaag gcggatacac 53460
aggtctaaaa gcaaagcact tgagttagtg gcatgtacta agtataagag gtactgccgg 53520
gcatggtggc tcacgcctgt aatcccagca ctttggaagg ccgaggcctt aagctcttaa 53580
acacttaaga gctaattccc ttatggaatg agaagtctag agacaaatga ttgcttaggc 53640
cgagacctaa gatcaggaat ttgagaccat cctggccaac atggtgaaac cccatctgta 53700
ctaaaaatac aaaaattagc cgggcgtggt ggcacatgcc tgtattccca gctcctgggg 53760
aggctgaggc aggacaattg cttgaacccg ggaggtggag gttgcagtga tctgaaatcg 53820
tgccactaca ctccagcctg ggtgataaga gcaaaagtcc atctaaaaaa taaaggtact 53880
tataattgtt gctgtttact agactagtat gattattatt aacagcagta gcatattgtc 53940
tttgtttttg tttttgtttt tgttttgttt tgttttgttt tctagtcctg atttactctt 54000
ctcctggtca ctgtatcccg gctgtttctg agatatcatg ccccctccat tctcagttcc 54060
tttccctagg agcgtgcatg actctagcct aaaccaatcc actatattcc ctaagccaca 54120
ttaacagatc cagtcagagt ctaacagatc ccacgagact ctagagggat tgatcagggc 54180
aaagactccg cacttggagc cgggagaacg tattcatgtg ttccctgcag ctgctgtatg 54240
tctctgtggc cacacgggag aaactgcctc aggatggggt cgacctggaa gaagtgactc 54300
aagagatgaa ctgggccatc aggaatcagg atcccttgat ccagctatgc ctgaaagcca 54360
agacatttta gttaaatagg acaataaatt ccctcttggc ttcagccagt ttggattaag 54420
taagtttctt tcacttgaaa ctgaaagaat cttgacagat acattactgt gtgaccttaa 54480
agtgctataa tggtggttgg tacaaagtgc tatgacagca gacacgagcg acccctgatg 54540
atactggcca cagtgtggag ggagtgtcag ggaaggctgc atggaggagg ggtcacttgt 54600
tctggccttg gaaggatgaa gaacagttca gcgtgttgaa aatataggag agtgcatttc 54660
aaaacgttct gaaaatgtgg acaggaaacc aagaaatacg cttctaaatc acccaagaga 54720
caatgagaaa aattgcaatg gaaaattaga aaacattttg aactgaatgg ttttaaaaaa 54780
acaaccacat gtaaacttgt gggttgcagc taaaaaagta ctcagaggga aatttgtagc 54840

cttaacagtc acagattagc aaagaagaaa agctaaaaat taatgttcca aacgtctctc 54900
tcgagaagtt agaaaatgac agtgaaataa acccaaaaga aaggaaagga aaattataaa 54960
gatcagaatt ggctgaaaga ggaaggaagc acagcagagg gccaaccaag cctggagtcg 55020
gttctctggt aagccaaaga catgagggcg tgacttcccc gcctgttcgg ggaatgatga 55080
gggccctggt gtgggtgggg agggcagagg gggacaccag agcaaggagg aagtgtcgag 55140
gccatagcct gctggcgaag acagtgccag agcctggaga agtttccctg tagagcagcg 55200
ggagcagaac cccaccccat cgacggccag agccatgaga ggatctcagg aggggagtga 55260
tgggagcaag ctacctttgg tccctatcca actgggctgg gaacttgtag ggtcctggga 55320
gactgtctgc agatgagggt atgtggcccg tgtgcctagg tctgattgga aaggagaaga 55380
taagccacag aggataccaa gtttagtttt ccaggaaccg gcctctgtcg ctggggaaac 55440
tgccaccaca gctgctggct gtccagtgcg accttggcac aggctggctt ccctttcagg 55500
gaggggcacc cagacccatt gagtggtgtc tgagaaggcc ctggagggct gggggaggc 55560
tgccgagcta cggcatctcc agcccttcaa tagaatattt ggcagggttt ctccaccttg 55620
atagtgttga catttgggct ggatcattta cggtgggggc catcctgtgc atggtgggat 55680
ggtgaccagc atccctggcc tcttcccact agatgccttt cccccagttg tgaccaccaa 55740
aggtgtcccc tggagggcaa aattgccccc aggttgagaa cctctgattt catgttgtag 55800
ggggaggagc actggaccca gagtcctaca gaagatgtcg gcagtgccaa actcatccgc 55860
tagggctgca tgaccccagg caagatcctt cctctctctg agcctctaca accccctacct 55920
gccaattgtg gtgagaatac ttacccctgc cccaggata attaggagga tgctgtgcct 55980
ggaagagcag gctgccacaa acagcagagg ttaggtggga atcattgccc ctgtacctgg 56040
agcgaggcta aagagctgaa cggcgtgtgg ggcggggtcc ccatggtgac aatagcatga 56100
tctttcttgc ttccttgctt tctctcacca ccccaatccc acccgttggg aattcctgtt 56160
ggttccactt tcaaaacatg cccagaatcc acagctgtgg tcgccgcctg ctctggacat 56220
tggaaaatta gggcctcccc taccaaaaac atccccttct aactgcagcc agaatgatca 56280
ttttgagaca atcatctgat caagtcactt ccacatccaa agccttccta tggttggtca 56340
aacaaaccct aggtcctgtg tgtggccagt aaggccctcc gtggctgatc cccattgcct 56400
ctgaatttgt ctctttcctc tcccccagtt ttctgactct ggcctcctgg ctgcaccccc 56460
ttcacgccat tcccttccca gggccttcgc acagcccgct ccctcctcct agaacaatct 56520
ctttctccct gtccctccag atatctgcgt ggctcattcc cctagttcac tcaagcctct 56580
attcagacct ccttggtgag gtctttcctg gcacctccta aacagctctc cacccgctaa 56640

tttcccctgg cccctcccct gcctgtctgt tctgcatagc actcaagccc tctgacgtca 56700
cactgtgggc tcacctgcac atcttttggc tctctgtcct cactaggtgt cacttcatga 56760
gagcagggac tctgttctgc tcactgcaga accttcctca gtccctttag ggcagtgcct 56820
tgcacacagt aggtgctcac taaaaagtca gccctgcttc agccccactt aggattcact 56880
ctgggaaaaa gaatcacccc tgccccctg agtcaggttg gcctacaaat ggcaaggcct 56940
cttcagtctc aaatctcaca tgtggctcac acagctctgc ctttccctcc ggctgaatta 57000
gcctctcacc tcaccctccc ttcttgcatg atgtgcttca gccggaatgg gttgtttcct 57060
cctggctccc acatgggccg tgtcctctac ctggtgcatt ctttctgttg ccatctgttg 57120
gaaaactagt tccagtcctt cagattttaa cttagatgtc atctcctcca ggaagctttc 57180
cctgattgcc caagtctggg tcaattgtcc ctcctctgag cccaagcttc cgagtcttgt 57240
tatgaacaca ctgcattgtt gttgcttatg cacttgatcc caatcccatg caggtagggg 57300
ccagtcttgt atcccccagg gcctaggaca gtgctgggac tcaagaaacg tgattggagt 57360
gaatcagtgg atgaatgaat gctctgaatg acaccacctc aaaggacctg gagcattctc 57420
cttaatcact cttgtgggtg ccaatctcct tgggcgcctt tagaagcact atattttctt 57480
cctgagtact gcagctcttt ttaggcccct gaaaggcacc tggctctttc agttttatgg 57540
gcatttgtga aagaactcag cccaacagac ctctgatggg cacacctaga accacctcca 57600
aatggattca ccatggcaca tgctgggacc accttctacc ttggagagag cttcccaccc 57660
attctctcat ttctgcctcc cctaaacagt gagctggcct ggaaaagtct ggcctctcca 57720
ggtggggagg atggagtcag gtgactatgt cctctttgat gagtgggcag tgaggtcccc 57780
caagaagaag gggcaatgct gagtagagac cccttcccca ccctccagtc atctgagacc 57840
atgacttggt tatcttcttg gaacccacat ggtaatcttg gcacccatca gagactgtcc 57900
ttgtctctct agggctggga tgggggacat gctggaggac ccagatgtgc acaccacagg 57960
tgcccacaac acacaaatga aaacagcagc agtagcaggt gttgagtact tactcggtgc 58020
cagatgctag tatcttattt attccactaa acaactccaa gtctgtgttg ttgctggagg 58080
cttagggtcc ttgggccaca tgtggggctg aaaataatcc tgatctctgt ccgtctgtga 58140
taagagctag taataataat aatgtctgcc ttctgttgtc tccctgtatt cctgtattag 58200
tctgttttca cgctgctggt aaagacatac ctgagactgg gcaatttaca aaagaaaggt 58260
ttaattagac ttaacagttc aatatggctg gggaggcctc ataatcatgg ccaaaggcaa 58320
ggaggagcaa gttacatctt atgtaaatgg aggcaggcag agagcttgtg caggcaaact 58380
cccgttttta aagccatcag atgtcatgag acttattcac tatcatgaga acagcacagg 58440

aaagacctgt ccccatgatt caattatctc ccactgggtc cctcccacaa cacatgggaa 58500
ttatgggagc tacaagatga gatttgggtg gggacacaga gccaaaccat atcacccccc 58560
aacaagagaa ggcaggcatt attactagcg catatcacag atggggaaac caagccacag 58620
ggaatttaag taacttgttc attgtcacac agataagaag acatgaacct gggattcaaa 58680
cctggacagt tcggctgcag agcttgagct gttaacgatg acactctcat ctttttcaca 58740
aatgcccaca caagtcccct tctgtacata tcaacatatc tcacttacat atatgtgcaa 58800
atcagcatat gaaatcccgc tcttgctcat gcactcatgg acacatacaa cccaatcaca 58860
aagacataga ttctcctgta gcatgaaatt catcatacat cgtcaatagg cagcgagaat 58920
tcacccgagt cacgcacatt tacacacagg tgtgcatgtc cctgttaata tgcctgcgtg 58980
aaaagcatgc agaaccgcca ctcacaagtt cacacatgtg caagtacaca cacattgtca 59040
cataggcaca gacatatata tgtgtgcaca cttgcatata aacatttctt gagcacctac 59100
tatgtgccag gcactgtgcc ggttctggga atataaaagt gaagaaaacc gagtttctac 59160
tctctcagag tgtgtaatcc aggggagggg gaggcatgcg tgagcaaatc agatagaaag 59220
tgcagtggct catgcctgta atcccaacac ttttagaggc cgaggcaggc acatcacctg 59280
agttcaggag ttcaagacca gcctggccaa catagtgaag ccacgtctct actaaaaata 59340
caaaaattag ctgggcaagg tggcgcatgc tgtaatctca actacttggg aggctgaggc 59400
gggagaatca tctaagcccg ggaggcagag gatgttgcag tgagccaaga tcgtgccact 59460
gcactcaagc ctgggtgaca cagcaagact ccatctcaaa aaaaaaaaaa aaaagaaagc 59520
tcatagttat ggagtgctta ttatgcataa gttgcagagc tgaatctgcc tcatgcatta 59580
ggggtccgta aacaccacag cccccatgct tacatgcaca catatgaata taaacctccc 59640
tccctgcaga agccacatgg cacaacctgg gcccttgaca ggagtggatg ggtgtagggg 59700
acggggatca gcattatttt cagccccccct cgtggcccaa ggacctcaag cctccagcaa 59760
cttccctgag atgagaccat ggcctgtgcg acaaaatgca catgcaattt gcagacatgt 59820
gtacatctct ccacgtgctt gtatgtgtgc aggtccctca gcagtccccc attctcctca 59880
ggacctcccg ccctatcaag agcccctgtc cctaagtcac caacctcctc cccctaccct 59940
catcttgcac cttagggacc tgtgggacgg agacatttgg atcttccaag acgctgcaca 60000
cacaggcgga ggatgggctg caatgactcg gcttccaggc agctgtggtg agtttctgca 60060
aagactccct tcctcaaaga ggctctgctt tactgaaagt tcaggttgcc gtggtttccc 60120
cgcattcaca tgggggaaag gtcagactct cacaaaaata tttaccaaga actgtctgtc 60180
agtcaatcaa caaacattta cagacagctg ctgctcacat gctggggaga gaggggggat 60240

gaataagcac aggccttgtc ctacataaaa gctcaggggc catgagggag ctgtgattga 60300
cccagcaagg gacacagtgg ccaggaagag ggaactgctt gtccaaaacc tggcatcaaa 60360
agagtgtgcc ctggtgagac caaaacaacc ttttttttt ttttttaatt gagacggagt 60420
cttgctctgt cgcccaggct ggagtgcagg ggcgcgatct cggctcactg caagctccgc 60480
ctcccaggtt cactccattc tcttgcctca gcctcctgag tagctgggac tacaggcacc 60540
cgccaccatg cccggctaat ttttttgtat ttttagtaga gacggggttt caccgtgtta 60600
gccaggatgg tcacgatctc ccgactttgt gatctgcctg cctcggcctc ccaaagtgct 60660
ggaattacag gagtgagcca ctgtacccag ctcaaaacaa ccatttttta tggcccctaa 60720
aatcacaacc agtggtgtgc tggtaagcca acaaaaacaa caggaaacaa accaaaaacc 60780
caatatgtgt gtttgctgca aaatcctgaa acacggtgtt tgctgctttg catggtgtag 60840
atagtctcat tgtggccaat ttcaggctac caaggtgatg tcaggggctg cggaggcaaa 60900
gatgaccaca ggagctggtg caagctggac tcaacacact gtttcttccc acagacacca 60960
ggaggacaga gccagagctt tggtgcctgg aacagagtaa ggagtctcag ttagaagtgc 61020
cagggacaag acccaaacct cttgttcatc ttttgatgca tattccgacc tgttctttca 61080
ttcactcagc acttaccctg tgccaggtac tgtgcacctg aaggtgctga gaccattgct 61140
gcctgtgctg atggggaagt actggtgtta aggagcccag aagagaggca ttaaccaatg 61200
cctggggagc tagaggactt cctggaggag gtagtatcta agctgaaatg gaagggagta 61260
aagcagcaag tgaggggtag aaaagagtta tttgcaagca gaggaaatag gatgtgcaac 61320
agcttagtga tctcatgcac cactgggttc ccccagcaaa agcatcctgt tgagcagatt 61380
ttgtaaatga accagcacca tcctaggtgc tttacaagta ttcactcatt taatcctcag 61440
acgatcctat ggggtattta atattcttag ctcctttgta cagatgggac cactgggaca 61500
gatggaagtt atgaataagt caatatgccc aagatcactg gtctgccctt cctctccacg 61560
atgctacctt gaagcatctg aagtaggtgc agcttgggga aattttaggg ggtacccaga 61620
tatgggaaac aactgtaaat ttaattctgg gtttaatgaa atgtcagagt gtgggaagtg 61680
atgggttcag tccctcccct gctccctgcc agcccttcac acacaaagac aagagtattg 61740
tccatcatgg acacattgac cgaggctgtc ggtcagccag tcaacagaga tttgtcagaa 61800
tctttatgtc ttggtctggc ctaggactgg gatgtttgca gtgaacctct gctcacattt 61860
ttgttttgtt tttccttttt tttttttttt tttgagacag agtcttgccc tgttgcccag 61920
gctggagtgc aatggcacaa tctcggctaa ctgcaacctc cgcctcccgg gttcaagcga 61980
ttctcctccc tcagcctcct gtgtagctgg gattacaggg gcatgccacc acgcccagct 62040

aatttttgt gtctttagta gagacggggt ttcacaatgt taaccaggct ggtcttgaac 62100

tcctgacctt gtgatctgcc caccttggcc tcccaaagtg ctgggattac aggcgtgagc 62160

caccccatct ggccttgttt tgtttttcta ctgaacactt aatctgggct gggctctagg 62220

ctgaggattg gggcacagaa gaagtaatga atctgtgtcc ctgtcatcaa ggggcatgat 62280

aggagacata caggagacaa ggttttctgt atgtttgaaa tattccatga tttaatataa 62340

aaaataattt ttatattaaa ttttagacac aaggtcttgc tctgttgccc aggctggagt 62400

gcagtggcac gattatgact cactgcagcc tcaaactcct gggcttaaac gatcctcctg 62460

cctcagcctc ctgagtagct gggactacag gcgcaagtag tcaattgcaa taaatgacga 62520

ttacaataag tgatcgaaga ggcccaagga caaaggagag aacaattctg ccccccatcg 62580

aggggaggac tcacggggaa aggcctgctg ccactgggcc tgagaagatg atattttaca 62640

gcagttgcca gggaatcagg ggaggacgat ttggacctac agatatggat aaggattttg 62700

gttttttttt agagaagagg caatggtgga aatgttctgg aattacatag tggagatgtg 62760

tgtactacaa cattgtgaat acactgaaac ccactgagtt atatattttt aagtggttga 62820

aatagtgaat tttacgtcat ataaatttta tctcaataaa aaataagctt atgactgggt 62880

gcagtggctc gcacctataa tcccagcact ttgggaggcc aaggcgagtg ggtcacctga 62940

ggtcaggagt ttgagaccaa cctggacaac atggcaaaat gccatctcta ctaaaaaata 63000

caaaaattag ctgagcgtgg tggtgggtgc cagtaatccc agctactaag aaagctgagg 63060

caggagaatt ccttgaacct gggaagcaga ggttgcagtg agccaagata gtgccattgc 63120

actccagccc aggcaacaag aatgaaactc catctcaaat aaatacataa ataaataagc 63180

ttatgtttca accacaggga gctttctggt tgtaagtgac aaaaatgcct gtttaaaaac 63240

aaagtttagg ccgggcatgg tggctcacac ctgtaatccc agagctttgg gagactgagg 63300

caggagaatc acttgagact aggagtttga aatcagcctg ggcaacattc agcctgggca 63360

acatagcgag acaccatttc tacaaaaaat acaagtatta gccaggcgtg gtcacttgtg 63420

cctgtagtct cagctactca ggaggctgag gcaggaggat cgtttaagcc caggagtttg 63480

aggctgcagt gagccataat cgtgccactg cactccagcc tgggcaacag agcaagacct 63540

tgtgtctaaa ataataataa tacattttaa aaataaataa gaagtttatg aatatagttt 63600

caggcatgga tagatcaagg ttctcaaata atttcttcag gttggaaggt tctactctgg 63660

gatctgcttt cttttgtatt ggctttagtc tcattctcaa gcaagctgag gaagcctcat 63720

tggtggccaa agaaaagcag ggacctcaga agccaagacc taccacctac cagctttcaa 63780

agcccaaagg aaagaaggca tgttttccta atggttccaa tgatgctacc aggaaaagct 63840

ctcattggtc agttttaatc atatgcctgt ccctgaacca atcttcagat tgggaaagga 63900
atgctctgat tggccaagcc tgagtcatgt gccctgttgg aaagctagga gttagatcag 63960
ttctctggca gttagatcag ttctctggaa ctgcgagtgg gaagacgctg tcccggcaag 64020
gaccaccagg tggtgatgtc agaagaaggg agagtagata cagggcaggc aggatagccg 64080
ggttcgtccg gaagagtggt agggaggggc gtgcctgcca gagggaaagg catgagaaaa 64140
ggcttggagg ttgaaattgc atggtgtggg ctgggaactg tggctgggtg gtggcgtgta 64200
gtcagatgat ggtgtctaga ggggtcaggt catgtgtctg aagacatgag aagcctcact 64260
tctcactttg aaccagggcc aggtacaaag aggtttggac agggcctgta cgccagtgtt 64320
gcccaaactc tcatccctat gcacacaagt catttctgcc gtatttacat tttttctcca 64380
tcattacatt ttcttcatat tggcctcctc gctttgttta aatcacttta ttgtcaaatg 64440
aaacattgtc accatcacac atgaaagatc aggatccttt actgtaaata gaagggaact 64500
gtaaaaacaa gcccattgaa accaaaaccg agtgattaac ttccagctag atgctgcagc 64560
ctgtggtagg ctaatctgga gaagagaaag agaactgaaa aaggaattgt tttctcagct 64620
cactatgccc acatgccacc taaaatcatc ttagggaccc cagggagcag ggaaggacgc 64680
agtcacaaag atggtcctgg tcctggcatg ggggagtgtt tgataaagac actaccggca 64740
taaacccagg ggttacaata gggaggctga catggctgaa cagaggagct ggagagcagg 64800
gtcccaggga cagactcaca gtaccggaag ttctgcaggg aggaaggagg tagtcaattg 64860
aggagcaccc gctctcttgc tttagcaatc aaaaccagga accagaagga ggaggtttgg 64920
cgaggatgag aaagagctcg tttggatata ctgaatctga gatgtgggga tgtctctgtg 64980
gagatccatt catctgttca agaagtattt atttatttac ttatttttgt gtatttattt 65040
agagacaggg tctctgtcgc ccaggctgga gtgcagtggc gtgatcgtgg ctcactccaa 65100
cctcgacctc ctgggctcaa gcgatcctct cacctaagcc tccccagtag atgagactac 65160
agttatgcac cgccatgcct ggctactttt tgtatttttt gtagagatgg agttttgcca 65220
tgttgcccag gctggtctca aactcctggg ctcaagcagt ccacctgcct tggcctccca 65280
aagtgctggg atgacaggcg tgagccatgg tgcccagcca agaagtattt attgagcacc 65340
tactatgtgc caggcactgt tctaggccct gggataaggc aatgagccaa ccaaactgaa 65400
atctctgccc accatgagtc tactttcttt gggggagata tgggagacct ggtgagaaat 65460
aagaaataaa agacttaata atatatacaa tatataataa catatgtcac acgagatagg 65520
aatggctact agctaggaca tgcaaaatgt ttgggtggga gatgtggaaa ctgagacata 65580
cccaactgca gttgaatgta taactctgga gttcaggcaa gagatctcag ctggggagat 65640

atatttagaa gacatcattc attcattcat tcattcactt agtaaatgtt tattgagtat 65700
gctctatgtg ccaagcacag tgctgggctg tggaccacac agttcctgat ccatgcgact 65760
ccagtacaga tttagtgaag gttgtaatta actctgtgga aagaggagag gagatgggga 65820
gggccccaga tgggccctgc ggacccctaa cgtgtaagag agggcagagg agaagacctt 65880
ggaaaaagag actggagcca ggtgcggtgg ctcatgcctg taatcccagc actttgggag 65940
accgaggcag gcgaatcaca agatcaagag ctcaagacca gcatggccaa catggtgaaa 66000
ccccgtctct actaaaaata caaaaaaatt atccaggtgt ggtggtggtg ggtgcctgta 66060
atcccagcta cttgggaggc tgaggcagga gaatcgtttg aacctgggag atggaggttg 66120
tagtgagctg agactgcgcc actgcactcc agcctgggtg atagagcaag actctgcctc 66180
agaaaaaaag aaaaagagac tggacaggaa caaacagcca gtgtcagaga accaagaggg 66240
gccgagtttc aggagtgagg gagatgctag tgccacagag tagtagggat tgaaactgag 66300
acttggcttg ggtggtggag ctggcttcag tgaaagtagt ttagtagcag agtcaggccg 66360
actctgcaca gagagggtag aggagtgggt gcagaggaag gaagtgaagc ccaagagagg 66420
agacagcttt tgagcacaag cactgtggct gggggagaga aggcgagaga gaaataggag 66480
ccggggtgga gggaagcttg ggctgtttga agatggaaag acttgagtag atatgtgtgg 66540
caatgaggaa aggccagcag agggaggcag gtgaagacac aggggagtga tggtgtttta 66600
gggtccagag ggagaagaga gctcagcctg ggagagaggg aatgaccccg catcaggaat 66660
ggctgcatca ttggaggggc actgtgcaaa ataaaaatgc aggtcttctt attcaaaaag 66720
ggctgataat ttggccaggc gcggtggttc acgcttgtaa tcccagcact ttgggaggcc 66780
aaggcgggcg gatcacttga gtccagcagt ttgagaccag cctggccaac atggtgaaac 66840
cccgtctcta ctaaaaataa aaaattagct gggcatggtg gctcatgcct gcaatcccag 66900
ctacttggga ggctgaggca ggagaattgc ttgtactcgg gaggcagagg ttgcagtgag 66960
ccgagattga gccactgcac tccagcctag ggacaagag cgagactccg tctcaaaaag 67020
aaaaaacaaa agggctgaga aatcaagatg gtcactcttg gttcctggat tagaagtggg 67080
ctgagttgtg gcttctggct gcccagcaga tacctctggg gtaggaatgg ctgtcaccat 67140
gctccacccc aaggtcccgt gaggtgagct actgaccttg atgatccttt gcacctgtgc 67200
ccaggcctcc ttcaagggca gaaggcagca atggtcatgg atggtggtgg aatggggagg 67260
atgagtaggg cctggtgtgc tggggacaga gcagcaggtg gccaggagcc tgtaccaggg 67320
aagcaggcag gtggcagatg tcccagggaa gcaggcagga ggtgggaccc tgtgtgagct 67380
gaggctccaa gccctcaatg ggtgctcctt ggtcccatta aacctcccta taaaacacaa 67440

cttcagagat aaaattttta agaatttctt tttctttttt tatttttttt tgaaacaaag 67500
tctcgctctg ttgctcaggc tggagtgcag tggcacagtc ttggctcact gcaacctcca 67560
cctcccgggt tcaagtgatt ctcctgcctc agcctccctg agtagctggg attacaggca 67620
tgcaccacca tgcctggcta atttttgtat ttttagtaga gacggggttt caccatattg 67680
gccaggctgg tctcaaactc ttgacttcga gtgatccgcc caccttggcc tcccaaagtg 67740
ctgggattac aggtgtgggc cactgcgcct ggccaagaat ttcaaaactg ggacttcaga 67800
gcatgacact gaagagtagg ggtcctccga gcaggggggt acgggctgct gcctggcgca 67860
cagctgtgga gccagcctgc cctacgtcca ctctaacaag aaagggtgga tgccgatatg 67920
gaatgggcag gaatttggca ggatcctctg gaagtgaaga ttgagtgcca ataaagaggg 67980
agaggatggg gattggggaa ggagcttgag gaagagtcca gagcctaaag ttggatgcgt 68040
gaatgcacct gcacgttcat ttcccattgc ttctctgaca aatttccaca agtttagtgg 68100
cctaaaactg aaatttatga tcttgcaatt ccagaggtca gaagtctgaa actggtctca 68160
ctgagctcaa aatcagggtg ttaacaaggc tgtgttcatt ccagaaactc tagggaagtc 68220
tgttccttga cttttccagt ttcaagtggt cacctgcatt ccttagccca tggcccttcc 68280
ctccatcttc aaagccagca gcataatatt ttgaaatctc tctctgactt ggcttctcct 68340
gcatctctca cttatgaaga tccttgggat tatattgggc ctgtctggat aattaaggat 68400
aatctccctc tacatctcag gttcagttga ttagcaaccc tgcttccatc tgtaacctta 68460
atcccccccct tgccatgtaa tgtaacatgt tcacaggttc cagggattgg gacatggaca 68520
tcttgggggg ttcattattg tacctactac aatttttaag gttgctttat gactgcaaaa 68580
gaaaactcac agtggtttaa agaaaagcac acttacagtt tatttaagaa tttaaggttg 68640
gtttgatcac ttggtgatgt aatcaaagcc ccaggcaggc cttcttcttc ttccactcca 68700
ccatattcag aatgcctcct ttcatctcca ggcttgtctc ctcatgattg caaaagcacc 68760
atccaaagca gaaaggaagg tggcaaagga gccttctcct ccatgctgct ttttgattag 68820
gaaggagaac tgctgacttg ctctgtggtt ccatggcaga gaggaaggtc aggcagccca 68880
gctatgttga ccaactcaag ctaaatttgc tgctagaaat caggaaatca tggatggtgg 68940
tggaaggggg aggatgcgta gggcctggtg tgcccagggc agagcagcag gtggccaaga 69000
gcctgtccca ggaaatgcca gttcctgggt gggatcacgc taaggtgggt catcagtata 69060
taggagtggt ggttcagggc agccagaatc agagatcagc ctatagctgg gatcaaagat 69120
ccatctgtgg tgagggtcag ggtcagcat gtggctgggg ccagcctgca accaggatca 69180
tgagtaagtc agtaacatgg gtcgggactc agttcctggc ttgagtcaga gctgttctgt 69240

ggctgaggac aggaggtgga ctgtggccag gatcagaagt cagtctgtgg ctggatcagg 69300
gtcattctgt ggttggagtc agatatcata atggagttgg gatcaatcag tttagtcttc 69360
tctgtctgtc tttctctctg catctctgct tctctctgtc tcattctcct ttgctgtaaa 69420
cacctgttgg aagctcagca acttgggtcc atggggaaga gggaagttga gtctgggcaa 69480
ggtcctataa gtcccggtat ggaagaacat aagggaaaaa gagaaaaatg tcaggaatat 69540
aaagatattc cctagaaaaa tgtcaggaat ataaatatat gtatgtatga tgagaaatga 69600
atgatataaa accgcaaata cgcaaacact gactctgcat gtaaacaaca cacagaagtg 69660
gcacaaacag atacaaacac aagtgggttg aatgatggga ttgggacaat tttttgtcct 69720
ggattactgt tgctccattc tgcccttttt gtaataaagt caaattaaat ttagaatgtc 69780
attatttcct ctaattatgt tttataccct cacctcccac aaaatccaca gacatattca 69840
gtgtaatatt aaggataatg ttagcagact aggcatggtg gctcacatct gtaatcctag 69900
cactttggga ggccgaggtg gatggattgc ttgaacccta ggagtctggg actagcctag 69960
gcaacatagt gattccctgt ctctacaaaa aattaaaaaa caaattagcc aggcttagtg 70020
gtgtgtgcac ctgtggtcac agctacttga gaggctgaag tgggagaatc acatgagccc 70080
aggtgttcaa ggctgcagtg agccgggatt gggccactgc gctccagcct gggtgacaga 70140
gaaagaccct gtctcaaaaa aaaaaaaaaa agaagaagaa gaagaatgtt agcaaatact 70200
tttaaggcat taactatttg caggcatagt tctttttttt tgagacagag tcccacttgt 70260
tgcccaggct ggagtgcagt ggcacaatct ccgcttgctg caacctctgc ctcctgggtt 70320
caggcaattc tcctgcctca tcctcccgag tagctgggat tacaggcacc cgccaccatg 70380
tctggctgat ttttgtattt ttaatagaga cagggtttca ccatgttggc caggctggtc 70440
tcaaacgcct acctcaggca atctgcccac cttggcctcc catagtgctg ggattacagg 70500
cgtgagccac tgtgcccagc cgcaggcata gttctaagga cctcacaggt actaacatat 70560
ttaattctca caatagctac atgaggcaga tattataatg attcccattt tacagataac 70620
gaatcaagca cagagaggtt acacgctggc ctggcgccac acagcatatc taatggacat 70680
taggctcttt tgtctggtgc tgggggaatt tctggaaaat gcagagatgg aagagaggtg 70740
ggatcggggc agggcgggtg gggaatctcc caggccaagt tttctgggca cggcctggac 70800
ctggagctgc tgggatggag caggtgctga tgctgatgtg gtcaccagag ggcagcaggg 70860
accagggagc tgacttcctc ttcacacccc ctccacccgg ggcggtgccc ctgacttgtc 70920
aataaacgtc gtggaaacag gtgggatttg gtggaagaac tgacacggtc ccgccaccat 70980
cacaggcaag ttaagcttga gagcagcctg gtgtcacctg gcctccaccc accacgtcat 71040

ctgctgctca ccccaccact gtggcgtgca gagtactgct cttgtgtgcc ttttaccaag 71100
gaggaaattt aggcccagag tgggcaaaca cttttcccat ggtcacacag cttggaattg 71160
gaaaggctgg acctaaccag gcagggtgag cgcagagccc ttgttcctct gagtctctgt 71220
tctgagctgg aaccagggaa gatgatgagc ccatggtata tacagggcgg ttagctagga 71280
ctctggtttg aagcctggct atgccactcg gtaacagcac atcgtgggca agtcccatgc 71340
agcctcatcg gctgccggga ggatccagtg acgtcaggcc tccctcgtgg tgggagctca 71400
aacacttgct gggtttttg tttttcggt gttttgcttt ttgtttttta attaataact 71460
ttcgtgggaa aaataaagtc agaaaggttg ttcaaccgga tgttcacgga gatactacgc 71520
cttcaaaaac aagcgctcat ttcgtgatcg cccacacagt gccagatgct ggggcgggga 71580
tgggatttcc ggtggttttt cttcactttc ctctttatat cttcaggctt tctctgaata 71640
ggaaaaggtc tgcacgtgct gatagagtaa tcagaaaaat gcaataagcc actttttgta 71700
aaaaaaaaaa aaaaattcag atgcactcac tgccttcctc ccccctttcc ccctcccctc 71760
atcccccaca aattctgtgt aaaggcaggg ctgagggagt acccccaaat caacatctct 71820
gaaagctccc ccgcaggggc ccaggccact cagcccactc tggcgctgct acccaggggc 71880
gtcccctcct gccctctggt ccagccccgg aggctgaaac tcccgcagcc ggcatctcac 71940
tgggcctatg accctggact tttcctactt cctgcagggt gggggccagc ctgagttggg 72000
cccacctgat ccaccaggtc ttctcatccc tctgtagctt cccaaagccc agccagttca 72060
gtaatagttg ctgaggccaa caacttggat ttcttttgcc taagatcctt tgttgggctc 72120
ccagtcccat tgcatctcta gcagtcaaat atattttcta tttgctgcct aatttttaaa 72180
ttagatttct ctgagacaat gctcaggcaa agtggattcg agccgacatc tgcgtaggtg 72240
tcaggatgac ataagctgat atatcacggc ggctgggagc tcttaggtca gacccgggtt 72300
caaatcctgg ctccaggatt tcctagctgt gtggttctag gctaatcctc aacctctctg 72360
gacctgtttc cttatctgta aaattgggat aatcattgag cctcccaact agagctgttt 72420
ggagacgtaa atgacacagt ggctggacgg cacttagcat attgcttggc cctaaacaga 72480
taactatttt tatttaaaat tatattttat tataaagagc ttttggaggc aagaagtcca 72540
gaaggctagt tcaggctctg ccattctctg ggtgactttg ggcaagtccc ttttcctctc 72600
taaaactgtt tctccatctg taaactgtgt gatttgggca gctaaaggga ttgattttgg 72660
cttagaacaa ggtttttttt gttttttttt ttttttcctg agacagggtc tcactctgtc 72720
atccaagctg gagtgcaatg gcacaatctc agctcactgc aacctccgcg tcccaggttg 72780
aagcaattct accaactcag cctcccaagt agctgagatt acaggcaccc accaccacac 72840

ccggctaatt ttttattttt tggtagagat agggtttcac catattggcc aggctggtct 72900

tgaactcctc acctcaagtg atacacccgc ctcagcctcc caaattgctg ggattacagc 72960

catgagccat catgcctggc gcaaggaggt gtttctaaca aacatcaggc tgcatgtgag 73020

gtagagagct ccccatcatt tccagtgtag tatgtaggct cctagagcag gaaggaatct 73080

aaaatggcca agcccctcaa gtgtgaaatc agggcagcta ctcagagaag caaaagtgat 73140

acagacatag gagcttcagg ttcccagcct tcccatctaa ggcccacacc atccctctgc 73200

ctgcctcgga tgtgaagcgc agatcactac agagagcctg ggtgatggag acactgtctg 73260

gattccacta tagatctgtt gctaactagc tgtgtgacct tggcagtgta tcatcagcca 73320

ctgttggtgt cctgttcctg tcttcttggt gtgccagagt ctctcaacac ctccaactct 73380

cagttgaaga attttttttt tcctggagta gctggaagtg ctggagaatt aatgctcctg 73440

gaagcagctc tcagccaatg acagatggga gctggtggac aaataccca gctccctctc 73500

ctttgcaggc ataactgggc atgtgccatg catggtcttc cagagatccc cggcaggtaa 73560

gcatcagtta ctcatgctgg taatctgttg aataactcac ccttttgtca gaggtttatt 73620

taaactggag ctttgaagag gggctgggta aaatgaggct gagacttact gggctgcatt 73680

cctgggaggt tagggcattc ttagtcatag aatgagacag gaggtcgtca ccgatacaag 73740

tcataaagac cttgctgata aaacaggttg cagtaaagaa gatggctcta acccaccaaa 73800

accaagatgg caacaagagt gacctctggt tgtcctcact gctacactcc caacagcgcc 73860

atgacagttt acaaatgcca tggcaacatc aggaagttac cctatatggt ctaaaaggga 73920

ggcataataa tccacccctt gtttggaata taattaatca ataaccataa aaataggcag 73980

ccagcaaccc tcagggctgc tctgtctacg gagtagccat tcttttattc ctttactttc 74040

ctcattaact tgctttcact gactctatgg actcgctctg aattctttct tgcatgagat 74100

tcaagaaccc tcttttgggg tctgcatctg gaccccttc aggtaacact ttcttggttt 74160

gtgtccttcc ctgcctcagt tccccactac cttagcaggg tttcccggga ttacttccta 74220

aatcaactat ttgaatcctt gtatggggaa cccaaaccca gacggcaagt ttcttaacct 74280

cttgcatcct ggttcctctg gatgggtgaa ttccaggagt taacagagac acagcactca 74340

cagcagtgtc tggaggtatg tttggttaat acataaaaaa taaagagaca aagaaacttt 74400

atttatacaa aactccaccc cttctgttcc actctcctca gcaaacacag ataacaggtg 74460

atgaaactaa aacacacaga cgagcattac tcaacccaag gttcccgcct tccctagcac 74520

ctgaggtctg ggccaacatg cagggtaact ggtgccttat gcctgctgtc tggattgccc 74580

ggcccacagg gtggctgagc atatttattc tgggggttcc atgcatacga ggagccccca 74640

gccatacagc tgggcatggg tgtttggcag caaattgtcc ctgctttagt cacagcaatt 74700
tttcatgtcc tctgttgctc cccttaactg aacacccctt gacagccggg attctgcctc 74760
cagcatcaac ctaaggaagc tctgttaaat gaatgagcac ctctaggcaa tgaccaccct 74820
ccctgaagtt caatttctca tcgatttccc aaggccgccc aagtcattcc cttgatggct 74880
gtgttagttt ctaggctcgg cttctagttc agtgagacag aggcaggtgg gcctccatgt 74940
gggccatgat ctggtgggca aaacatgcct tgggcagtta caacagggac agtctgctgc 75000
agaagctgtg gagggagcag ccaacaactc gttcacaagt cgaggtgccc aagggcctca 75060
gttttcctgc aggggcctgc cgtggggcac ggcgacccag tgccctctac tctcatggga 75120
tgtggcgagc ccagcccaat gctgggcggg gtccagtctc tgcagcccag tgttaggcca 75180
acgtcagtgg ggccaatcac cttcatacca tggttcttca agtcttttgg aaatctgcca 75240
gcctggctgc cttccaggag gtggcatttc ccaggcatgg ataagcccta gtcctcatct 75300
gcagactcag caacaagagg acatgcacct aagagaccct catgtatgtg ggtcccacgg 75360
agacaaagtt cacgattttg gggtctggc ttgagctctg agcattgcca ggggcaggat 75420
ggaaggatga tgaggattta ctcttctctg agatgcccga gggtgccagg gaggccggac 75480
acagactggc ctccagtgga accccacctg gagaggggtc tggggccctc aggggggttg 75540
taggggcgga ggacctgtct ccacagcagc agccacagca aggactggcc atgacagggg 75600
tctggccacc atcctcctgg ccatgacact gtttcaggtg gccgcacagg tggcaggtaa 75660
gggctgagta gacaatgcca ctgcccaggc tgtccacaag ggggtctgtg gcctgctcct 75720
ggggaagtgg gggctttggc atgtcctcta ccttttcccc cggctccaga cccaggtgct 75780
ctggggagct gcttgggaga tgtgagctct gcggactgcg aggtggctcc ctgtccagtc 75840
caaaggtgaa caaggggaca gggactgggg cagggtcccc agggcagcca ggaatgaggt 75900
cttggaaagg cttatacccc tcttccccac tgctagcccc aaacccacat ttctctgggg 75960
acacagcact gctggcaagc aggcttgaga aggccttgta accagcctct cctgggggac 76020
ccaagcccac caccgcactg gcctgggtgc caccctgctc caccgcatgt acaaactcct 76080
gatagccact ggtgggggcc gagacggggg ctgcagctgc cccatgctgg aggacatttc 76140
ggcggaggat ctgctcccag gtttctggct caggttgggg cacagtggtt ggctcagaga 76200
gctgggggac acagggcatc tcgggttcta cttcctccag gtgtctggcc agcagtgggt 76260
ctggacccag ctctctggga cacggtgact ggctcaggga gttgctgaag ctgcggtaag 76320
cagggttgcc tgcgatgacg aggggcgtct ctgtgcaagt caggttgtct ggactctggg 76380
tcgggctggc aggaggactt ggctccaggt ggagaggctg ctccttgccc cagggaggtg 76440

cctccttggg ccctgcactt gggaactcat cccagggcat gtgagcactc gtacttcccg 76500
aaggtggaag aaggcatgac tcccccatgt cctgctggca aaagccccca ttctcctctc 76560
cgagcaggtc caggaacagg ctctctgtta gccgggccac aatgccctcc cttccctcct 76620
ggaagtcatc cctgctgctc tcaggcgatg cacagaagct cccttttttct tcctctacct 76680
cctcctcctc ctcacactcc accggggcct caaacaactc cacacatcgc accacgctga 76740
tgctctctgg ccagaggact gtcttgctga tctccactgg gcaccatgct gattttccag 76800
agccctggaa aggcatctct ttggcagcct tgtgaggatc ttcatccctt ttcatgttgt 76860
gctccagaaa acagggcaag agcttggtaa gacaattctt ccagtgtctg caaaagcaaa 76920
ggttggtcag ggttcagttt cgacacttga aaaactccta ttcattcttc aaagcccagc 76980
ctgaaatcac catgtcctga tggctgtcta cttcagacat gccttctaac gttataatca 77040
gtaattgatc agtctatgtt gctagaatga gaaatccctg agggtagggt ctgtgtcttt 77100
gggatttcgg gaacccagca catgtcctgg gcaccaaata caagctcaga tctgttgagt 77160
aagaaaaagt ctttatggat gatgataatt aaaagcaaca ccattatccc aagccatgac 77220
caattagaaa ggatatggaa gccgggtgcg gtggctcacg cctgtaatcc cagcacttta 77280
ggaggctgag gagggaggat cgcttgagct caggagtttg agaacagcct gggcaacaga 77340
gcccaagccg tctcaaacat ctctacaaga attaaaaaaa aggaaaatta gctgggcatg 77400
ttggtgtatg actgtagtcc cagctactta gaaggctgag gctagagaat cacttgagcc 77460
cacgagtttg aggctgcagt gaatcactgc actccagcct gagggacaca gcaagaccct 77520
gcgtccaaaa aaaaaaaaaa aaaaaaaaga tgcgggatcc tagggttgaa gcagggtcct 77580
ggaccccctt tagtttctgg agtatggaaa ggtccagcag gccccaggga agaagctaag 77640
gaggccacag tggcggggca acacagcgag agcttgtctt tacaaaaact ttttttggta 77700
ttattttttt tgagacaaga gtctcactct gtcgcccagg ctggagtgca gtggcacaat 77760
ctgcgctcac tgcaacctct gcctcccggg ttcaagtgat tctcatgcct cagcctcctg 77820
agtagctgga attacaggca aaaacctttt aaaatagcca ggcgtggtaa tgcacatctg 77880
tagtatcagc tactcgggag gctaagatgg gaggatcact tgagctcagg agtttgaagg 77940
tgcagtgagc tatgatcgca ccactgcaca ccagcctggg ccacagagtg agatcctgta 78000
tattaaaaaa aaggagggga gtgttcagga tagtggctat ttgccgacaa gtatacaatt 78060
aggtcaagat tttaacaaca ggcacggctg ctctggttgt taactataac agcactccca 78120
aggggtatca ccatgtacct gacacaccag tgaaactcag ggggcttggc cagcagtcca 78180
cgtttccaga acacccctcc tcctgccgtc ccttgaaggc taatgtcaca gggcaattac 78240

cgaatgcagg gaagagaagg cagacatgga gctaggggct tgcctctcca attctatcac 78300
cttcccaatg catgcaggct gtgacctaag ttcagataca tacgggcact tggctggttc 78360
ctggcctcgg gaccgcttct cccactgtga cccctaaaat acagagacag ccaatgaaag 78420
agcagagtgg ccttgcagcc cggcctggag tacggcaatg ctcaggaagg ggtacaagcc 78480
tcgacatcac acagatcaga agtccaacag gtcacttacg ctttctgagc ctcagattcc 78540
tgggaattaa ttacattgtc ctaatattta cagggactgt ggtcgatggc aaaagtggcc 78600
acagaggagt gctctgaatc tcctcccatc ccttgaatct acactggccc tgggacttgc 78660
ttgggcccat agaatgtgat ggaagtgata tcatgccagt gctgcgccta ggcctcaaga 78720
agccttggga cttcccaccc ttgaaaactg agccactctg taaacaagcc tgggccggcc 78780
tgctggagag tgagaggcac atggctcagg cacctgcact gtcccaggta aaagtcagcc 78840
aactaggctg ggtgcagtgg ctcacgcctg taatcccagc actttgggag gctgaggtgg 78900
gtggatcatc tgatattagg agttccagac cagcctgacc aacatgaaga aaccccatct 78960
ctattaaaaa tacaaaatta gcctggcgtg gtggcacatg cctgtaatcc cagctacttg 79020
ggaggctgag gcaagaaaat tgcttgaacc caggaggcag aggttgcagt gagccaagat 79080
catgccattg cactcctggg caataagagc aaaactccgt ctcagaaaaa gaaaaaaaaa 79140
aaaaaagcca actgtcagac acggagggag gctacgggga cgggtcagct caattcagat 79200
ctgcccagcc cagcccagcc cagcctcccc cgccaccctc actgctgagc ccagccccaa 79260
atgccagcct gcagaagcat aagctaaata aagttgttgt aaccctctac atttcggggt 79320
catttgttac acagcaaaca ctaactgata cataaatggc acttggtatt tgcaagaggc 79380
tggaatatgc actttatatg tattgtctca ataaattcca atatctgtcc taccaggtaa 79440
acactgttat aacccacaag aggaaactga ggctcagaga attaagcaac ttgctcaagt 79500
ccacactgcc agtgagtggc agagtcagga ttcaaagcca acactagatt ccagagtccc 79560
tggaaaagca aggaccctca gaccttctct tctagacagg acctataatc ccccttctc 79620
ctacctagag aactctcatc ttgctcaggc aaaccccacc cactcaccca caggaccatc 79680
gccttgggga agctgactcc acccctggct taggggtggg atacatttca ggccaatgag 79740
aacccggaag ggatttgcta gtgcttcggg ggcagtttcc tatgcttaag aagaagacac 79800
agaaagcctg tcaccctctc cttcctccac tccttctggc ctctagccct ggtactggca 79860
cctccccagg gaatgacaca gtgatgatga cccaagggtc attctcccaa ggggagggaa 79920
aaaaacagga aaccaacccc agagaagccc accccaggct gggattgcca aggggaaggg 79980
cgggaaagcc tctggtctgc taatgacctc attcggcttc tgcctctgtc tcccctgcaa 80040

ccctcctgac tgtctatttg ccattccaga agccagtgaa ggtccttaac agcccaggtg 80100
ttctgaacca cacttcttca tgtacacagt cccacatgtc ctcatccacg cctactccta 80160
cctgagcatc ctggattatt atagccacga ggcggctgcg ggctgggttg ggaatctgat 80220
cccaccattc tttcttaatc ctgaaaaaca aaaggaaaag gtgttgatct ctgaattatt 80280
gacaggtgaa cgctcctccc ataaaagtgg tcgttatcac caatacttat ttggcatcag 80340
agatcaggct gattagtttc tggaggtcaa accactgact catcattcta acctggtgac 80400
atggattcta ttgctaaccc tattctacag atgaggagag tgaggcccaa ggaggtggag 80460
tgacagctgg taggtggcag agccaggttc acactcccgc attctgttac ccagcacaga 80520
gttcctgagg aggccaccta tgtcctcagt gggaatgaga ccccagggga ttaggaaaga 80580
ctcaaactgc agagagagga ttctaggttc ccctcaacaa tcccttttc agagcagctg 80640
agggcaaatg accagggaat ggcctgggat ggcctgggaa gctgggggac ctacggagaa 80700
acacaaccct tctctcttcc tggcctgcct ggggccatgg tgcatcatca ctgggaggta 80760
aggtcctcag accgcttctg aattagcctc tgcccatggt ttcaataaat aaacacatat 80820
ttattgagca ccttctatgt ttgactcact gtgcctggcc ctgaggctac aacagtgaac 80880
gagacaaaga tgcctgcctt ggtgaaactg accttcaaga gggaaagatg gatgataaat 80940
ggagacagaa atgcttatat gaggccaggc atggcggctc acgcctgtaa tcccagcact 81000
ttgggaggcc aaggcaggtg tatcacctga ggtcagaagt tcgagaccag cctggccaat 81060
atggtgaaac accgtctcta ctaaaaatgc aaaacttagc caggcatggt ggcaggcgcc 81120
tgcaatccca gctactcagg aggctgaggc aggagaatcg cttgaacccg gaaggcggag 81180
gttgcagtga gctgagatcg cgccactgca ctccagcctg ggggacaaca tgagactcca 81240
tctcaaagaa aaaaaaaaaa aaacgaaaga aatgcttata tgaggtcatt cattcatggt 81300
aataagtgcc atgaataaag ataaaggggt agaagttcaa gatcagcctg ggctacataa 81360
gtgacagcca gtctctacaa aatgtaagaa aattggccag gcacagtggc tcatgcctgt 81420
acccagcacc ttgggagaac gaggtgggtg gatcacttga ggtcaggagt tcaagaccat 81480
cctggccaac atggtgaaac cccatctcta ctaaaaatac aaaaattagc tgggtgtcgt 81540
ggcacacgcc tgtaatccca gctagtcggg aggctaaggc aggagaatcg cttgaacccg 81600
ggaggcagag attgcagtga gtcgagatcg caccactgca ctctagcttg gtttttgaga 81660
ctccactctg tcccaaaaaa taaaataaaa ttaaattaaa ataagaaaat tagccaggtg 81720
tggtggcaag cgcctgttgt cccagctgct ctggaagttg aaatgggagg atctctttag 81780
cccaggaatt ggaggctgca gtgagccgta accgcgccac tgcacttcag cctggctaac 81840

agagtgagac cctgtctaaa aaacagaaca aaacaagaaa ccaaagacaa agaacagcaa 81900
agaggctggc atgctgggat atagtgatgg ggagtgggaa gaggtacagg tgatggggaa 81960
actgaggctc agagcaaggc gatcagtcca aggttgctcg gaggcatcag tgacaagata 82020
tggaggggaa tttgcattcc ctgcctacct agtgtgtgct gggccaagag tgggcacttt 82080
catcaatcat gttaataatc cttagccacc tctaatcctg acttagtgag gttgagcttg 82140
ctagtcccat ttcagagatg ggaggactga ggctctgaga caccatgatt ccaagggctg 82200
gcaagagctc tctggaaacg aggctccttc caccacctcc cagccactcg ccactcatct 82260
cctccctcag cctttactgt gatttggcct cttctggctg atgaaatatg ttctcaccat 82320
cactgtcact ctgcaaagca ggaaccccag gctcaggggt ttcagcaact tgtcggccgc 82380
cagctaccaa gctccaaggc caggattcaa acccaggtct cctgactcca tcagggcaat 82440
cgtcgcaaat tcctctggct cctaccacat gcccccaaat gcccccagtt gcccaggcac 82500
ccagtgttgt aaggggatgt gggatgactc catcttgctt atccctggcc agctcattct 82560
ccccagtcag ggccccccctc aaattttccc tccttggaga gccttatcct ggtccccctt 82620
gcaaaaagca ccccagaaga tctctggcct ccaccctggt ctagtgcatt cagagagtgg 82680
gtcactatgg aaaaccacct gtttgtgatt gatatgtatt ttcagtctcc ccagtaggat 82740
gccggtttta gaaatgtgga gtcctggttg ggtgcagtgg ctcacacctg taatcccagc 82800
actttgggag gctgaggcag gcagatcatg aggtcaggag ttcgagacca gcctgaccaa 82860
catggtgaaa ccccatctct accaaaaata caaaaaatta gccgggcata gtggcatgcg 82920
cctgtaatcc cagctactct ggaggctgag gcagaagaat tgcttgaacc cgggaggcgg 82980
aggttactgt taggcaagat tgtgccacta tactccagcc tgggtgacag agtgagactc 83040
cgtctcaaaa aaaaaaaaaa aagaaaaaaa gaaaaaagtg gggtccttgt cgatatgttc 83100
tcagctctac tctcaaggtg tgtagtagct gctcagtaaa tacgtattat tgactgagag 83160
gactgcaaag gggcagacta gaggggcaag tccctgccac cctgcactcc agagggactg 83220
ctcggcagca ctgggcccag gactcacttg gtgatgctga catagcacaa caggcagacg 83280
gccaggatga caatgcagga aacgctgacg cccagcagga ggtgctgctc gaagggctcc 83340
ctgtaggctg gaagtgggag gggaagccat gagctgaggt cagccatggt acagcctggg 83400
ctccacttcc caggactgga gacccagggt caggaccctc gtccgagacc ctccacgatc 83460
tgaccgccga ccacccctcc acctcatctc cccaccactc tccctcttgc acattgagtc 83520
ctcgtcacac cggccacctc attgcccccct gctcctccaa agctcagtgc cacctccaga 83580
tcttaaactt gctgctcttt ctttccggaa gcacagaagt caaatgcacc aacttaggag 83640

ccggatggct ccttgctgtg tgacacagga caagtccctt atcttctctg tgcctccgca 83700
tcctcatctg tactatgggg ttaatgacag aattgatcgt gcagggctgt tgcgaggatg 83760
acatgatgaa tcaatagata atgcactcag aacagagtgg cctgtccaca tcattagctg 83820
ccatcatctc aacacggttg acttctcctt gttcactttc atcgtcacat tctccaaaaa 83880
gttttccccg acaccctccc taccgttgcc ccctgcccat cgctccatcc ccttcctctg 83940
cttttatttt cttcccagca ctcactgtca tccagatcca tgtgatttct ttctttcttt 84000
atatgtttgc tttttgtctc tccctccaga atgtcagctc cacaagagtg gggatttcta 84060
tctgctttgt tcatcgccga ttccccagtg tctagcacag ggctagccat gtaggaggaa 84120
ctcagcaaat atttgctgaa ataatgaatg agggtcccat acattgttat ctgtttcata 84180
caagctagtt cccttcccca tgccaatggc agatgagaat caattcaaaa ctttcccaag 84240
cacctggttc tgtgctgggt tctggagata cagccatgga gaccctgcct atggagctca 84300
cagcccaggg caggagacac gtggctcaac tgatatccac agtgcaggat gagcaatgct 84360
gagagggctg tgcggtggca gggtcagggt actgccagct gccactgcac ctaccacgtt 84420
gaagaatctt ctttcattga gttttcacga cagcccttac tcctcccatt atacagatgg 84480
ggaaactgaa gcagtcttct tcactcaaca gctttttcct ctctcccttg tcagggagcc 84540
atgcccaccc taaggcttga gccaatcacg attatcctgt tcctttttcc aggcatttga 84600
gttcccagtc ttccttgcaa tgagcaagta ataacagtaa ccatgtaaca tgtaacagtc 84660
ttggccaatg agatgtaagg ggaattttgc tagggaagct tctagaaaga tgtttgcttc 84720
ctgattaaaa aagagatgca caaggtacac tctctcgtag cagcctttcc tttccttcct 84780
gctttatgta agacagtgtg caacggccaa cctgagggca tgggtggcaa gtctaaggat 84840
acaatgccac catgtgaagg acggcagaag ggaagacagc agaagggaag agatatcact 84900
tggctgctgt acaaaaccca gtaggtccat ttgtaagact tcattttatg tgagataatc 84960
aatatttcta ctgcgaaaga cctgtgggat acctcactga aaagagttgt ttctgggatg 85020
taggttccaa gtgagaaagt tcccagcctc ctttgtggct atgtgtagtc atgtgaccat 85080
tttcaccaat agaatttgag aggaagtgat gcggcccact tccgggctgt ctgctttcaa 85140
gaagcagatg tgatctccat aatcacgcct cttctgctgg ctggatacag acaatggtgg 85200
ggccctagag cagggattct tcacttgggg ttgatggacc aacatggctg caggtagaat 85260
tcaggggtct gagaacttgg atgggaaaga aattacatct ttactttccc taacctctca 85320
cacctagcat ctccttccat taagaatgga ggaaggggcc aggaacactg gctcccactt 85380
gtaatcccag cactttggga agccaagatg ggaagatcac tggagcccag gaggtcaaaa 85440

gcagcctggg caacatagtg ataccctgtc tctatttatt ttaaaatttt aaaaataaaa 85500
taaaatacaa ttttaaaaaa agaatggagg gaagagaccc cagtaatgac agcatcagtg 85560
atcctgtccc cagcaaaaca aaactcacag ctattctcat atcacatcac aatttcagta 85620
tcataaagta ttatttatgc ttatctactt tgaaattaca gtatttatgc gagctacccc 85680
tagaacttat tatttataga tcttaataaa gaagcacata tatctctaga ttaaaaatgt 85740
gtttaaatgt tttataactg catttcaata caattagttt cctctgtagt tctatttatt 85800
ttattttatt tcattgtatt tatttatttt tagagatggg ctcttgctct gtcacccagg 85860
tgggagtaaa gtggtacgat cacggctcac tgcagcctca aactcctggg ctcaagtgat 85920
cctcccacct cagccttccc agtacctggg actacaggtg catgccacca tgcctggcta 85980
atttttgtac agatgggggt ttcactatat tgcccaggct gggattttat gtatttaaaa 86040
atattctgaa atgaggtcca taggcttcac caggctgcca aaggaaacca ggacacaaaa 86100
gtaggtaaga atctttgtcc tggagaatga tggcaccaca agatggaaag aacctcactc 86160
cccagatttc tacagggagg gaaaccacct acaaaccagc aacattcacg ttagcttgtt 86220
acatgagcaa gaaatatatt cctatagtgt taagccatgg aaattgtggg cttgtgtgtt 86280
actgcagcct agcctgccta cctggcagat acaccacagc aggtgtatct aatggcagga 86340
ttgccattag aggcatccta tgatataaaa gcccagagac tcagccaggg gaagtgtctg 86400
gccacagggg aagaatggag agtggagatt accattgctt aggcctcttg atactcacag 86460
ttgtgccact tggtgctggg gctccactca ctccaggtgg tgttatagca ctgagcccag 86520
gccctcaccc gtgccctgta ggaaatccca gacttcaggg tgctggctgc gatgcggagg 86580
gagggttcta ggtaggtcac gttatagatt ctgaactggt agagaaaagg gagatgttag 86640
ggcaccagcc gggggcgagg ggcaccagtg cccaccctgg ttgctgtcgt tagctgaccc 86700
caccatgttt aagagccacc agcctgggta tttggttgtt cgtcctgttc aggccattgc 86760
ttgccacctc tgtaactgtg accaggctag agcaacatgc gtcaggctcc tcgactaatt 86820
agctgtatgt tacttaggca agttgtttaa cctttctgag ttttcgtgag ataattcaat 86880
ctcatctgta aaacaagggt aagagtccct actaccactt cctggctgtg tgaccttaag 86940
taagtgacat aacctctctg gacctcaatg tcctcatctg caaaatgaag ataataaaag 87000
tgcctatctc ccttcccgca gggttgtggt gataaggaaa tgggttaata tacgtaagta 87060
tttcaaatag ggtttggtat acaaaaagca ctgggtgatc tttctaaagg actggcaggg 87120
tcgaggcatc tctctctgtc cttcatgggc ttcctagtgt ctccatggtc aagtccaaac 87180
tcactggcct agaatccaat gctctgccca acctagcctc tactgagaag tgacttccaa 87240

gatctattgt taagtaaaca agggaagatg cagatgtgta tgtgttctct gcttcttttt 87300

atgtttttg tttgtttgtt tgtttttaa agagagatgg ggctgggcat ggtggctcat 87360

gcctgtaatc ccagcacttt gggaggccga agtgggtgga tcacttgagg tgaggagttc 87420

aagaccagcc tggccaacat ggtgaaactc tgtctctact aaaaatacaa aaattagctg 87480

ggtgtggtgg cgggcacctg taatcccagc tactcaggag gctgaggcag aagaatcatt 87540

tgaacctggg aggcagagat tgtggtaagc tgagatcatg ccactgcact ccagcctggg 87600

tgacagagtg acgacttcgt ctcggaaaaa aaaaaaaaaa aaagagagag agagagatgg 87660

cacagtgatc actgcagaca aacctaagat ggccccgatg atccccactt tgaatcacat 87720

ctttgtgtca tctggtcttc tcgagtgtgg gcaggacctg ctacttgttt ctaacccaca 87780

gaatatggta aaggtgatgg gatatataga ggatttcatg gatgtgatta tattacagaa 87840

gactgtaata tcctacttgc taggcaagtc tctctgcctt gatggctttg caaagggcac 87900

atgggaagga attcagggtt aactacaacc aacagccagc acgaaactga ggctctcagt 87960

tcaacagcct gccagggact gaatgtcacc aatattgtat gagcttagaa gtgacccttc 88020

cccaattgag cctcagatga aactgcagcc ccatcccaca ccttgattat agcctcacga 88080

gactctaaag cagagaacct ggctaagcta tgtccagact cccaaccaca gaaactatga 88140

gataataaat gggtgttgtt taaagctgtg aagctggtag taccattatt atgcagcaat 88200

agaaaactaa gacagcgatg actgaacaca cacatttgct tgtatagaca taagctctct 88260

ctaaaggatg cacatgccct ctgcctgccc taaccttgcc tccctttccc ttctggcaag 88320

agacagacag cagtggtcag tctctgggaa gaaaaacagg aggctggagt cgggagagaa 88380

acatgtttcc attctaagtc cttttgtacc atttgtattt ttattttgtg taactattac 88440

ttttaccctc caaaaaagct ttaaaaagtg atacatgaac gctatattca tgactgaccc 88500

tgatggcaag tgagcaacct gagttctcat cctgtcaccc ctctccctgc gagatccagg 88560

gctatgcacc tccctctcct cttcctcagc ccccggcagt ttcttcctgg ttactggcag 88620

ctccatttct gcttgtggtt ggaagcacat ttggggaaac ctggtgcaaa gtgagccaag 88680

gggaggtttt ttcacaagaa agaagcaaag ttccaaaaga ttgctggagc aactacagag 88740

agttcagaga ttttcctatt acagagggag ctgagggtgg ggtgtgagtt gtggggagac 88800

tggggaaggg agagaaaatt ctcactctct tttgtctttc tttctaaaca tgagcaaaca 88860

agaagcaaca gctagatgta agttaccgaa gaaatcttac accctggaag ggagctggga 88920

actgggaaat gttttactaa gaaacaaaag agaaatctga gcttccaaag aactacttgc 88980

ggtttgtctg tttttaaca agaaggacag gtgtttggga gaccatctct gagagaaatg 89040

tccatgggag gaaaaagagc acaggcttgg gagttcaaat actgggtgca aatctcagat 89100
ctgggctgga tgcggtggct caagcctgta atcccagcac cttgggaggc tgagccaggg 89160
ggatcacttg aagccaggag atccagacca gcccggacaa catggcgaga ccctgtcgct 89220
actaaaaata caaaaaatta gcctggcgtg gtggcctgtg cctgtagtcc cagctacctg 89280
ggaggcagag gtgggagaat cacctgatcc caggaagtca aggatgcagt gagccatgat 89340
cacgcccctg cattccagcc tgggtgatag agtgagactc tgtctcaaca acaacaacaa 89400
caacaaaaac tcagccctgc catttccagc tgtgtgaccc tgagcaatgt gcctggcaca 89460
tagtaggcat ataaaatatg gaaaccatta tgaggcaggc attcagggct ctaactaaac 89520
ttttggcttt gtttggtgct ttatcgctac acatattccc acatccccca accccataat 89580
cctcaaacat tccatgaatt ttcacaccca taaacctttt atcatgcagt tccctctgcc 89640
tggtctaccg ttcccttctt tccccatcta gaaaactcct atgcatcctt caatgcccaa 89700
gccaaaactc cccctccact atggaacctt gcctagaaat tatctagggg aatgaatgag 89760
tagctctagc cccaaaattc tatagtaagt tcctaatgtt tctttctgga atttatccca 89820
ctcagtatga gccacagttg gtgcctatag tgctgtggca gatgcaggtg gacacccgcc 89880
cagcttttat gccatcgggt ttaggtgccc actgcttagg acagaactcc actgcctgca 89940
actccagggc caaccctcat tagtctaagc caattgtgcc aatcccatca cctggcctat 90000
cattcatcag aggggagcat gtggccaagc tctggccaat cactaaattc tttatgttca 90060
gctctgttct tgggaaggga gagatggggt atctcagttg ccactgttcc acccacaaga 90120
gaagctggac ttcattcttc tgcctcagac ctggtatctg gaggcctgtg gcatccctga 90180
gcaccaacat acccctaagc catagcccag gcatgctgac caatccaatc ccatgactgg 90240
catctggtgc cacgtcttaa acctggctgg cattttagcc agcacagtcc tgcttcctct 90300
tccccagttt cccctgggga agccactcct gccttatttt cagttcatct ggtttggatg 90360
gagctgagcc cacctgcagc cctagaggca ggtatgtgac tcctccctgg acaagcaggg 90420
ccctccacca gcaccctctc tccacccaca gccagtgatt ggttcagagg ctggcacagc 90480
atcgaagcca ggccactgag cagcagcctt aggactctta ttagaaccgt caagaaaaaa 90540
aacccttttc ccactgtggc tttgggatgt aggcctggag caactagtag gcatatctgc 90600
cactacatga gggaagagcc tgcctaaaaa cgaagccaat acagaataaa gcagggcgca 90660
gagctggaca ggttcctaga agctggatcc agccatgcct gaaggcagat ctagaaatat 90720
gaaaatatta aataacatat ctttggactc tgaaggtggt acctttctct tccccccagc 90780
ccatccttgg gactctctgt aggtcttgca caggcctggg gcatgagaag gagatttatt 90840

gaccaaacaa aacttgcccc actctctagc atctgtgcac tccttccccc catgcccccc 90900
gctttctgag cctctgctgt gtgagccaac tgccccattc ctgctctttc cccatacctg 90960
gcccagtgcc aaaggggaaa ccagacctga gagatataaa tcgacacccc tggcttggcc 91020
agattgagag gaagcagtga cagacaccag catgggagct taaggtggcg ggtgcctgct 91080
ccctgagagc caaccaggcc cagcctgggc ccctgggatg cacagagccg gccctcacaa 91140
acctcaagga acaaatgaca gaccagtgtg tgggacagcg ggcagatggg gatctctgtg 91200
gcctggggca ctcaagagaa agggcacatg gcttttagga cacatgtggg ccaaacaggg 91260
gagctcatct gtatcacaca ccttgtactg tcatagccct gtgggcgggt gctcttgtca 91320
ctcctgtgtt acagatgagg aaactgaggc acaaagaggt tatgggatat gcccaacatc 91380
acacagctgg cgagtggcac agcaggcatt ggattgcagc cctgtgggac cccaggtccc 91440
atatgtccag agattgtccc tccaatggga atgtgaggaa ggtggattca gggtggggaa 91500
acatgggtgg caggaaatga cctccctgca caccagcaga aaaatagggg gactcagcca 91560
cccactgtgg acggcccctg ccattactga cctgatagtt tcctgtaaat cctctcgccc 91620
tttttaaaga tgtcaataca tgtacttaag aaggaactcc actggccagg tgcggtggct 91680
cacacctgta atcccagcac tttgggaggc caaggcgagt ggatcatctg aggtcaggag 91740
tttgagacca gcctggccaa cgcagtgaaa ccccatctct actaaatata caaaaattag 91800
ctgggcacgg tggcaggtgc ctgtagtccc ggctgctcag gaggctgagg caggagaatc 91860
gctagaaccc aggaggcgga ggttgcagtg agccaagatc acgccactgt gttccaccct 91920
gggtgacaga gtgagactct gtctcaaaac aaacaacaac aaaaactcca ccaacactac 91980
tgtgaaggga atgtcaggac cagcttccat ggggaacaac caacaaactg atcttgtttt 92040
cttgggaaag tctaagcctg gggtgtggcc tctgctctct tcattaagag gaattagtaa 92100
acgagtcagc tgtatgggga cttcctcctt aacccgttgg cctcaaaagg gaactgacac 92160
gggtcactgt ctcttgagtc agtattattc tcttcaataa tagggttatt caagtgagct 92220
gtcaatgtgc cacctacgat cacttgtgtc ccccaaactg ggaaacacag atgtggccca 92280
cagccctacc tttacagagg aggagcggca tgcagagata actccccttc tctgatgtga 92340
ggggtggggg gccaggaagc aaagggactt gcctagggca gagccaggtg agaagccagg 92400
tctctagctc ctgggatttg ctatccagcc ccagggcttg cctcccaggc tcctggctcc 92460
agggactggg cctctgctgg tcacccaccc tgttccccag aggtcacaga aaaacgtcaa 92520
agcatgccca ctcacatctg ccgggtcgtt ttcactccaa atgttgactg cataggtgag 92580
atgattatac aggtaattgt caggggggata cgggttgctc caggtcagca gcagagtgtc 92640

```
ggagacattg gtgtgaactg tcaggtttcc tggggccctg ggtttcactg cgggagaagc  92700
ggcctctatg tgaggatcta tgcagtgact cccccatcac ccagcccagc cccggcctac  92760
aaaccacagc caccacccag atttttggtc gacaaaacct ccatagcata cttctggagc  92820
accacacaga ttcagagcca tagcaaagaa gattataata acaataatgt tgctaaaatt  92880
ctacatagaa ctttagtgtt tattaattac cctttttttc ttcttttttg agacagggtc  92940
ccactctgtt gcccaggctg gagtgcagtg gcacaatccc agctcactgc agcctcaata  93000
gcccagggct caagtgatct gcctgcgctg ggctcccaaa gtgctgggat tccaggcatg  93060
agccaccgcg cctggtcttt actaaccacc tttctacagt gaaggcaaag tgtatgagcc  93120
aaagtaaccc aaacttcagc cttttaaaaa atctttttat ttttatttat ttttattttt  93180
ttgagacgta atctcactct gtcgcccagg ctggagggca gtagcatgat ctaagctcac  93240
tgcaacctcc ttctcctggg ttcaagccat tctcctacct cagcctccca agtagctggg  93300
attacaggcg cctgccacca tgccagacta atttttttgta tttttagtag agacggggtt  93360
ttaccatgtt gtccaggctg gtctcgaact cctgacctcc ggtgatccgc tgggctcggc  93420
ctcccaaagt gctgggatta taggcatgaa ccaccacacc cggcctcaaa cttcagcttt  93480
tacagctgca tccttcacac ctttggccac atctctgtac tttatgatga tttactgagt  93540
atttttcttt attttgattc atctgtttta aaacttggtc tcctttgaag caatcatatc  93600
catgagacct caggttttta ttcctgtgat gcatgtcata tcatgcatca ctggaataca  93660
acgcatgttt taaactcctg tcaccacagc ccagcaagct caagggggat tcctgcaggg  93720
actccgggta ccagaagaag gtcagccttc cacggtcaag accatccaaa gccgcagcag  93780
ctgaccagtg gacacagaat ggaaggaatg ttcccagttc ggttttcaat cagcagcagg  93840
catgccttcc agcaagcaca cctcatctca atacgccttc ctgggcactt aagttcaggg  93900
gtggaaacag ctgggctgaa gggggcttgg tggtctactg cgaccagctc cgtttcaaag  93960
gtgggaggac tgaggcccag gcaggggcag gaatcccatc agatttggga aaatacaggc  94020
ggcttcctcc tgctgttgct atgaccccac ctcctcccct gcccacccca ccctcagccc  94080
aggcagctgt gggaacacac ccagccaccc ctgccgcact ccgccctgct caccatgctc  94140
gctgggcttg aaggagccct tccacagcag ctgctgccca gcccacaggt ccagtgtata  94200
gttatccgca ctgaccacgt catccatgag caggtggcac acgcaccccg cgcctccgtt  94260
gttctcaggg atacacgtgt gggctctgca gacacagggg ctgggttagg ctggtcacct  94320
gtaggctggc tgggccccac agctgtgtcc gggacatgcc tccaggaacc gcatcagact  94380
cttccagctt cccgatcacg gatgtgagga cagatctggg ggcttgggag cggccagggt  94440
```

gtgctggggc agccgactag ggttgcctct cttgctggcc acagcctctt accacatgca 94500
ccaaggatgc tcctcacacc tgcctagctg ccggccctgg tcctgggact tagcttgttc 94560
ctgaaagccc atgaaaggga agaaccggtg gcactaatta atttagaaca acataagctc 94620
tgcatcgcac agtttggcca tcgtaagata attccaatgt tccaccttaa cagccaaccc 94680
acatctcacg ggctaggcca ggggtctgca aactttttct gtaacaggcc agatagttaa 94740
tattttggac ttcaaaggcc acacaatttc cttttttttt ttttttttg gttatttgta 94800
taaatttagg gggtacgagt tttttgtttt ttgtttttgt ttttttttcc tgagacaaag 94860
tctcactgtc acccaggcta gagtacagtg atgtgatctc ggctcactgc aacctccacc 94920
tcctgggctc aagcaattct cccgcctcag cctccctagt agctgggatt acaggtgtgc 94980
gccactacac ccagttaatt gttgtatttt tagtagagat ggagcttcac catgttggcc 95040
aggccggtct cgaactcctg acctcaagtg atctgcccac ctcggcctcc caaagggctg 95100
ggattacagc cgcgagccac cacagtgccc ggccttacaa gtgcaatttt gatggcaagc 95160
ctcagatctg ttctcactgg atatgctgcc tagtggtgaa gtctggggtt ctggtgggtc 95220
cgtcacccaa atggtgtaca ctatgcccat taagtaactt ctcttccttc acccctctcc 95280
caccctccca agtcttcaat gtctgttatt ccatggtaca tggaataatg cccatgtgta 95340
cacattattt aactcttact tataagtgag aacatgcagt atttgacttt ccatttctga 95400
attatttcaa ttaagataaa ggaggaggcc gggctcagtg gctcatgcct gtaatcccag 95460
cactttggga cgctgaggcg ggtggatcac ctgagttcag gagttcgaga ccagcttggc 95520
caacatgatg aaaaccggtc tctactaaaa atacgaaaaa ttagccgggt gtggtggcag 95580
gcaccattaa tcccaactac tcgggaggct gagacaggag aatcgcttga acccgggagg 95640
cagaggttgc agtgagccga gattgtgcca ctgcactcca gcctgggcaa caagagcgaa 95700
actctgtctc aaagaaaaca aacaaacaaa caaacaaaca aaaaacataa aggagggcca 95760
tgcagtttct gttacaacta cagcagccag tgtgttccaa taaaacttta tttacaaaaa 95820
caagcagtgg gccggatttg gctcacgggc catagtttgc caatccctgg actaagccaa 95880
tggaaacaaa gcacagccca tctaccttag aataatcatg gaataccacc tttactggtg 95940
aacgctgcat gctggtgtgc cgtatatccc cagtctgccc cgtaactgtg tgtcttgtgt 96000
acgtgattat gtcctgggca ccagtgtaac gaagcatcct gtgcaccatg gagtagccca 96060
gggaaccacc tgactcctat gccatgtgtc ctggtccatc caccatcaca gaggactcca 96120
ctctcaacac cgtactcctc aatcaagggt ctagtccagt ggttctcaaa gtgtggtctg 96180
ggcccaccag gtaaaactct tttcatcata atacagggac gttatttgcc ttttcactct 96240

ctctcactgg cgtacagtgg aattttctag aggctacatg acctgtgatc atgtaattga 96300
tctgaagatt tgttgaatgc gggcttgcat gttgttggga cttaaaattt ctccattttg 96360
ggccggatac agcggctcac gcttgtaatc ccagcacttt gggaggcgga ggcaggagga 96420
tcactggagc ccaggagttt gagagcagcc tgggcaacac agtgagacct ccatttcagt 96480
aagaaaaaaa atttttaatt gttttaattt tttaaaaatt acaccatttc ttcattgcaa 96540
gttctaatta ggtaaacata gaatgatgtg ttacctacac caaagccctt tgggtcctca 96600
ctcattttta agtgtgaaaa agggtcctga gaccaaaaag tgtgagaact gctggtctag 96660
tccagtattc cagccgtatc catgtgccac gtggaccaac actcccacca tgcgctccac 96720
tgtatcttac ataacatgtc cctggagatg ggacctcccc tctaacacca tataaacagc 96780
agcaaaccat actcagcatc ctgcactcca gcgcaccacc tggacccagc tcactcttgg 96840
ggcaaaccct ttgggcacaa cctccccaaa cctccagctc cagcgcaggc ttactcggag 96900
agcagaaaaa ccagctggta caacaggcgg agctcggtgc tgcaattggt gggaccattc 96960
atcttccact cgcaagtaga gatgctcatg tagtcggaga cgcaggtggg ctcctgcaag 97020
accttcatgt tccctgggga gacacaggag cagcctgagc ggcccaggaa ctcaatgcgt 97080
gaggggact tgcacccaaa caggaccaag gggaatagca caaaagcaca gtgcaggcaa 97140
agttgagggc cagcgcccac agagctgggg ccaggaccag ggctgctgga gcttcccagc 97200
tagtgtcggg atgactgtgg aagctgaaga aatacccatg ggtgcggggg ccattagaca 97260
agaacgaacc aagtgcacct ttatctcaca caaacttgac aacttgttct tggccaaatt 97320
aaagggaaaa tatgtagcag tttaaggagt gccattccat cacaccaagc ctctgccctc 97380
gagctagggg aagtgccact tccccatttg cccactatct gcgcccctct ttgtctccac 97440
ccctttctgc cctctctgtg ctcaggaggc tgataccaca gtcttcatca ccgggtaccc 97500
ttggccttta gtttccggtt gggtttggcc aatgggagag aggagatgga tgggctggag 97560
gagacatcgg ggtccttctc ctttcagcgc cctcctagct tgtcagtggc catggcagga 97620
ctggaattgg caggatgtac actgagtttg ctaaaccacc tgagcatgag accactcagg 97680
cgcctccatg ctcccatgct cagtgccacg cccatctcag ctgacaacaa cttgcataag 97740
ggcttaccat gttaaggagc tgtcttagat agcaaataaa tattccaagc ttatctcatg 97800
tccaaaacta actcctgatt cccgcccctc cccaaagtac cttctcttct ggcatccttc 97860
tgcatttcag aaatgacaac cgcgtccttc ccagtagttt aggtcagaaa tcttggagtc 97920
atcctggcca cttctctttc tctgtctttc acctcaattc caatctgtcc tcaaatctac 97980
ctcccagagg gatccggagc ctctcatctc ctccactcca gacctccctc cagacctcct 98040

ggcttccacc ctcttccttt ctccacacgg catcctgaag agtcctttgg aacataatgg 98100
atgatatgga tcctctgctc aaacactgca aggtcttttt tgattcagcc actctgtcac 98160
cttgctgacc ttcactgctc cctcctgctg ctcactctcc tccaccacat tccctcctgt 98220
gctcaaccct cccgccaggc tcagtgcagg gcctctctgc caagaatgag ccttccccag 98280
acatctgcac ggttcccgct tcctccaagt cagctcaatg aggccttccc tgacttccct 98340
actttcaact gcaatccccc ccactccacc cagcttcccc aaccccctct tatgctgttc 98400
tgtgttcttt ccccatcacc ttctcttttt ttttgacagg gtctcacttt ctcacgcagg 98460
ctggagtgga gtggcgcgat cacagttcac tgcagccttg acctcctgag ttcaagggat 98520
cctcccacct cggcctcctg agtagctggg gctacaggcg tgcactgcca cacctggcta 98580
atattttatt atttgtagag acaaggtctc cctatgttgc ccaagctgac ctcgaactcc 98640
tgggctcaag cgatcctcct gcctcggcct cctgaagtgc tgggattaca ggtgtgagcc 98700
actgcacccg gctcccatca ctttctaaca atctttagaa tttactgata atcagggcca 98760
cttgtcattg gctgtcccct cccactgcag tgtaatctcc ataaggccag gtgacatact 98820
gcaggaccct ggcacgccat taatagccaa caaatgaatg attgagaagt ggtgacttac 98880
cagagcttgc cacctgcagc aggaccaggc agctcacagg gaacaggagc ccagagcaaa 98940
gccaccccat tgggagatgc caaggcacct gcagagagag aggaagcagg ttagtgctga 99000
cctgtgcttc cagcagagct catcttattc aatatgcaac cctcccctgc cctggccatt 99060
tatttgtttg tttgtttgct tgtttgtttg tttttccgag acagagtctc gctctgtctc 99120
caaggctgga gtgcagtggt gcaatctcag ctcactgcaa cctccacctc ccgggttcaa 99180
gcgattcttc tgcctcagcc tcccgagtag ctgggattac aggtgcccac caccacacct 99240
ggctaatttt tgtattttta gtagagacgg ggtttcacca tgttggccag gctggtcttg 99300
aacttctgac ctcatgatct gcccctctgg tcttctcgaa gtgctgggat tacaggcctg 99360
agccactgcg gctgcccagc catgccctgg ccatttgtgt ctctgccgac aacaggtcct 99420
ccacgttcac agagctctct ctataatcat gaaacaacac ccagacccac aaagtccaga 99480
gggtgagcta cacaacaact gtcctgggag atgcttctgg atgtggcctt ccttggacaa 99540
ccctctacac atgtttctct gattatttcc tgaggtttaa atacctgctc tgtcactgac 99600
tagatgtgaa ccttgagcaa gctgtgcttc catgcctcag tttccttatc tataaagtgg 99660
gactaagaat agccccatct gattgaggtg ttgtggggat taaatgagct aacacagaca 99720
gagccctcca acagtgaccg gaacacttta aagtctcaag taacgttaca agtcagctta 99780
gtccatatag gctgctataa caaaaatacc ttagagcagg taatttacaa agaacagaaa 99840

cgtattgctc tattgggtgc aatggtgtgc atctctagtc ccagctactc gggaaggtgg 99900
ggtggatcac ttggagtcca ggggtttgag accagcctag ggaatatagt gagacctcat 99960
ggtgaatttt tgaaaaattg ttttaaattt tattattcag gccagacaca gtagctcaca 100020
cctgtatttc cagcactttg gggagccaag gtgggtggat cacctgaggt caggagttcg 100080
agatcagcct ggccaacatg gagaaacccc gtctgtacta aaaatacaaa aactagccga 100140
gtgtggtggc gggcacctgt aattccagct actctggagg ctgaggcagg agaatcgctt 100200
gaactggggt aggcagaggt tgcagtgagt cgagatcgcg tcattgtact ccagcctggg 100260
cgacaagagc aagactccat ctcaaaataa ataaataata aataaataaa ttttctaatg 100320
catagttctg gagactggga agtccaagat cacgacacta ggagatccag atctggtgag 100380
agcccattcc tcacggacga tgccatgctg ctgtgtcttc atatgacaga aggggcaaac 100440
acacttcctt cagcccgttc atgagggcac taaccccatt catgaccaac cacagagccc 100500
tcatgactta atcacttctc caaaggcccc atctcttaat atgactacaa tgggatgaag 100560
tttcaacata tgaatttggg ggaacacatt cagcccatag caccagctat tatcattaag 100620
aaaaaaatgt tcagaagcca ggtgtggtgg ctcacacctg taatcccagc actttgggag 100680
gctaaggagg gaggatggct tgagcccagg agttcaagac cagcctgggc aacagagtga 100740
gaccttatct ctacaaaaat taaacaaaat cagccaggca cggtggtgca tgcctgtaat 100800
cctagctact tgggaggttg aggtgggaag atcgtttgag cctgggatgt caaggctgca 100860
gtaagccaag actgtaccac tgcactccag caagaccctg tcataaaaaa aaaagaaaag 100920
aaaagaaaac agaaagtttg ggaggctgag gtgggcggat cacgaggtca ggagtttgag 100980
accagcctgg ccaacatagt gaaaccctgt ctctactaaa aatacaaaaa ttagccaagt 101040
gtggtggcgc acacctgtag tcccagctac acgggaggcc gaggtgggag aattgcttga 101100
acccaggagg cggaggttgc agcgagccga gaccatgcca ttgcactcca gcctgggtga 101160
cagagtgaga ctctgtctca aaaaaaaaaa aaaaggaaaa gaaaaaaatg ctaagaagca 101220
gaatctcacg gagtcaagtg tgtacttttc cccagcactc ctggtacata acgtcaaata 101280
ccctccaaaa agtgtgtact ggggccgggc gtggtggctc acatctgtaa tcccagcact 101340
ttaggagttc acaaccagcc tggccaacat ggtgaaacct gtctctacca aaaatacaaa 101400
aaaaaattag ccgggcggta gtggcgcgtg cctgtagtcc cagctactca ggaggctgag 101460
gcaggaaaat cgcttgagcc taggaggtgg aggttgtggt gagccgagat ggcaccactg 101520
cactctaggc tgggcgacac agtgagaccc tgttgcaaaa agagaaaaaa aaaaaaaaga 101580
aaaagaaaaa aagaaaacag cacgcgctgg gttatattcc ctgcaacatt tcaagtgccc 101640

atccttcact tcccgcttgg gtctctctcc tgggatctta ttcaaaaaag cagctcccag 101700
gctttgttct gctagtgagt agttctttgc cattaacagc atttctcttt tggtgctctg 101760
gctgtgtttc tgtggcacag gaatggttcc aaggctaacc tcacaggctt gcagcaaaca 101820
ggaacggagg tgttgcctct gaagtactgt gtgcagtgtc tggggcatca taagaactta 101880
aggccacctc aggagctcta ggcccctgct ggatgctctc cgagtgactt gggcacactt 101940
gaaggccctc cccccagcag ggctccacct ccctctccac ctgggcccag cacccatgca 102000
tcctacgcct gccacctaca cttcttcttt tttgtttttt aacattttat tttattttat 102060
ttcattttat ttttttgaga cagagtttca ctcttgttgc ccaggctgga gtgcaatggc 102120
gcgatctcgg ctcactgcaa cctccgcctc ccaggttcaa gcgattctcc tgcctcagcc 102180
ttctgagtag ctgggattac aggcatgggc cacacaccca actaattttg tatttttagt 102240
agagacaggg tttctccatg ctggtcaggc tggtcttgaa ctcccgaccc caggtgatcc 102300
gcctgcctca gcctcccaaa gtgctgggat tacaggcgtg agccactgag cccggctgcc 102360
acctgcactt cttaaacaca ccaaactcag tcccacctcc atgccttttc tctctctgtc 102420
ccctccacct ggagggacct tcccttcctc cctttccttt tttttttttt tttttttgag 102480
acagagttta ctctgttacc caagctagag tgtagtggca caacctcgac tccctgcaac 102540
ctccacctcc cgggctcaag caatcttccc acctcagcct cctgagtagc tgggaccaca 102600
gatgtgcacc atgaggcctg gctaagtgtt tgtatttttg atagagatgg ggtctcatta 102660
tgttgcccag gctggtctcg aactcctgag ttcaagcgat ctgcccacct tggtctccca 102720
aagtgctggg attacaggcg tgagccactg tacccggccc ttcctccctt acgcgtgctt 102780
ttcctctctt cccctaattt atgatggtgc aaaatcaatg ggtgttcagt agaagcagta 102840
cttggagcac ccatacgacc attctgtttt tcactctcag tattccataa actacacaag 102900
ctattcaaca ctttataaca aaacaggctt tgtgttagat gactttgccc aactgtaggc 102960
taatgcaagt gttccgagct aagttaagct ataacgttca gtaggttagg agtatgaagt 103020
acatttctga cttaataata ttttcaactt atgatgagtt tatggggaca aaacccccatc 103080
attaagaaac atctgtgtat gagacacata aacacactca catatctcca taatgaataa 103140
attaccccaa tacctcagtg tgcatgtcag aaaaacaagg acattctctg cataatcaca 103200
atacaaccat cgaaatcagg aaattaacag tgatgcatca ccactatctc atccacggcc 103260
cctatttatg ttttgctaat tgcctcaata atgcccttta tacgtccagg atgcaatcca 103320
agatcattca tcacatttga ttatcatgcc ttcctagtga ccaggtcttg gcttctaaat 103380
actattctgt actgaaagga accagagcgc cttagagaaa tagctgattc caggagaagg 103440

aaaagtacca gataggcttg gaattcttgt gccagacaat aaggaagtac tcagcagcta 103500
gcctgacctg gacaaaacag gcacattttg agcatcaaaa tgaatattat ggtatccact 103560
tgtaaccctt ggagttaaag tccaggagtc tgtattgaca tagatcaata aatggaggag 103620
gccgggtgca gtgtggtgtg tgcctgtaat cccaacttct tgggaggctg aggcaggagg 103680
atggcttgag cctgggagtt ggaggctgca gtgagctatg atcgcaccac cgcactccag 103740
cctgggcaac agagtcagac ttcatctcta ataaataaat aaacagggaa gaaggaaaag 103800
ctctatttta ctatgacagc ctcaaagtaa ctccctgcaa aacttgtatt gtatattaac 103860
taatgagtga aaatacatat caatgaacgt tacaatacag aaacctggca gacaccacct 103920
tcaccaagtg atctaaactc accaacaaga atgggacaaa tccccaacat cacctccctg 103980
aaacaattcc tgagaagagc acagcatgtc ttttcctggg ttcctgccaa ggatgcatac 104040
atcatttgga aacgtcagac aaatgtacac caagggccag tctatataca agaactggcc 104100
gctgctcctc acgaagtgcc gggggtgcaa aggacaagcc agcgtgggac atctgtccct 104160
ttttaaattg tctgtcatcc tgacagccag ggtctgtccc attcactgcc gagtccccag 104220
tatctacaac ggtgcctggc acataggacg tgttcagtaa ctgtttgttg attaaattca 104280
ttttggacat gatgtggggc ccctaactgg ctcagcatga ggctgcaaag ctctctctag 104340
gggaaggctc tgtatcggct caaaggttga actcgtcttc cttgctgaga gttacaaaag 104400
taaagtctca agtaaaggag gggcgggaat ggggtggtgg gttcctgaca ctgatttcct 104460
tggcaagtcc tagtaacttt tactctcaca actacacaga tgcccacagt atcctgtggc 104520
agagatggca aaggtgtcac tttagcctca attatgaata aagagaaaca gagaggccaa 104580
gacgcctgtc caaagtcaca cagcagaaga ctttcatggt aaagtcctcc agggaaacag 104640
gggagtcaac aaccctgtca agattcacta aaacatccca gcatggcgtc aggattgata 104700
tccttaatct gttgtggaag gcacgcccag acttacgcat gcccatgtgc acacacacgt 104760
gccgcagcta gcttctcagg gatgtgtggg tgctacattt caggagagat cacagatgca 104820
gtgggagaag aggcttccct gaacatcaga ggcaaaaaga acaaagatgc aggtgagtaa 104880
aagaattaaa aagataagtg ccaggttatc gcgttcaagt acacggcaag aggagatgat 104940
ttcaagttcc cattctaaat caagagactc ctggctgggc gcggtggctc acgcctgtaa 105000
tcccagcact tcgggaggct gaggcaggca gatcatgagg tcaggagatc gagaccatcc 105060
tggctaacat ggtgaaactc agtctctact aaaaatacaa aaaattagct gggcgtggtg 105120
gcgggtgcct gtagtcccag ctactcagga ggctgaggca ggagaatggc ttgaacccgg 105180
gaggcggagc ttgcagtgag ccaagattga gccactgcac tccagcctgg gtgacagagc 105240

aagactgtct cagaaaaaaa aaaaagagat tcctgaagtg gtggagttgg aaggggtctc 105300
agcggtcacc tgccaagctc aactgccaac ctggacaagg atgtcctgcg aacaccccac 105360
ttctgacatg ctgctaggca gtttcagccc atatgccacc acagatggag caatcactct 105420
caggtctcag ttaagcagag ccattggaaa cagcctcctt atgttgagtc aaggctttgt 105480
gtgaacaaaa tccctgcttg cccagcactt accttgtact tgacactttg ctaggtgctt 105540
tagaagtatt acctcaccga atccacacaa gaatcctgaa aggtaggctg ttatcccaac 105600
tgtctagcta aggaaactga ggccagagag gccaaataac ccatccacca ggtcccacag 105660
ctggtaaaag gtcagagcta gaatcagaac ccacgtttgt ttgcttccga gcccaccgtg 105720
gattccagta taccagttcc ttcaatctca tctcctaaaa ctgcaggctg aacccaataa 105780
gatgaaataa aatgagccgg gcagagttgg ccatcctgct tgtcaatttc aaacaacagt 105840
tccgcaagtg taggataaga aaatctgact gagcagcaga aaagtcctga ggattttatc 105900
atctgcaaaa tctccaaaag ccaatggtga agcagcaact taaaaaaaaa aaaagagtca 105960
cccgaggtca tattaacaga ggtatccatt tatagactgt tacatctcac aacagcagaa 106020
tatacattct cctcaggttc atgtggaata atcaccaaga cagaccacat tctgggacat 106080
aaaacaacct taagagattg aaaacattag aaatcacaca acatatgctc ttaggccaca 106140
atggattcaa attagaagtc actagcagga agaaagccag aaaatttcca agtatttgga 106200
gatttaaaaa cacacttccg aataacatat ggatcaaaga agaaatcaca agagaaacta 106260
aaaatatttt taactaaata aaaatgaaaa tatagcctat caaaatcggt gagacgtagc 106320
aaaagcagtg cttagaggga aatttatatt attgaatgta tatcttagaa agaaagttct 106380
aaaatcaata atctgtttcc atctagagta ctagaaacag aaaagcaata tatgtctaaa 106440
acaagcagaa gaaaaaaata caaaattgga gcatacatca atgaaattaa aaatagaaaa 106500
ttaatcgagg aaaatcaatg aaatcaaaaa ttggttcttc gaaaagatca atgaaattga 106560
taaatctcta cctaggcgaa tcaaaaaaat gagagagaag gcactaatta ataataatgt 106620
gatatggcct ggctctgtgt ccccacccaa atctcacctt gaattgtaat atgaattgta 106680
atccccacgt gttgggggaa ggacctcatg ggaggtgatt agattacggg agcagttccc 106740
ccatgctgtt ttcgtgacag tgagtgagtt ctcctgagat ctgatggttt tataaagggc 106800
tttccccccc tttccttggc gtgtctcctt cctgacgtca tgtgaaaaag gatgtctttg 106860
cttccctttc cgccatgata atatgtttcc tgaagcctcc ccagccctgc agaactgtga 106920
gtcaaacctc tttcctttat aaattaccca gtcttgggca tgtccttaga gcagcatgag 106980
aacaaactaa tactattgta aattaaagaa aagttattag aggtggcaca tgcctgtagc 107040

cccagctact gggaaggctg aggcaggagg atcacttgag cccaggaggt taaggctgca 107100
gtgggctgtg ttcatgccac tgaactccag cctgggcaac agagaaagat cctgtctcaa 107160
aaaataaaaa ataaaatttt taaattaaaa aaaagtggca ctatataatg attcgggagt 107220
taattctcca agaagatgta aataacaatc cttaatgtgt aggcaactaa caacaaaggg 107280
tcaaaatatg taaggcaaaa actgagagca gcaaggagaa atagagaaat ctgtgtgatt 107340
attctgaata atactattat tggataataa tagtattatt actattactt caactctttt 107400
ggtaatcgac agatccagca ggcagaaatc agtaaggaca cagttgactt gaacagtgct 107460
gtcaatcaac tggatctaag tgcagtgtgc aaccacaact tggcatccct ttggaaggaa 107520
tactgctgtc atgtatgaca acaaccaaca cagagagggc ctgataatcc caggaggcct 107580
agaaagtctt tacctggaaa gctctgccct cctatactgg gcagcctcac cattcactta 107640
aacacacaga gtttaccaca tgccaagttt tcttttacat cttttacata caagaatgca 107700
tttaataatc tatgaaatag gtaatattat tttctccatt ttcagatgag gaaaccaagg 107760
cacagaaagg ctgagtgatt tgtctgaggt tgcacagctg tgacagtcat gggtcttaaa 107820
tccagcactc aagctccaaa gctttggttc ttaactacta tctgtctaga agtcagagac 107880
cttagtgttt tggggatcaa tgacctatct gagcatctaa tgtaaacccc aaaccctgtt 107940
tctggcaagg tgcatacact taattctgca ttccatttta ggggttcaca gaatccctga 108000
agcccaagtg aggacctcag gtcaaaggcc tgtcctctaa gcagtgggca gcttttgagg 108060
gtctttgagc aggggagtgg caaatttttg agaagtctgg ggggatgtgg gcagttagca 108120
gttgctgcag atgaggctca ccctgcctct tctccacatc acagccagag ccattttaaa 108180
atcagaaatg agaccagggc cagctcacac atccaatgcc ccctggctcc taaagggctt 108240
cttcacaggg gccacagatc cccagggggt tcacagctca ttagagaagc atctctgact 108300
tagaataaaa tccaagccct agagtgaaaa aagccagttc ccagagtaca caccccgtga 108360
ctccatttat gtaacatact tgaaatcaca taaccatgga aatggaaaac acatcagtgg 108420
gtgccagggg ctgagggggt gggtcaggaa gggagttgga tgtggctata aaaggagaat 108480
ttgagccatc cttgtgatga tagaactgtg ctggatcctg gctgcataca caaacctatg 108540
ccgtgataaa attgcatagc actaaaccac acatgcgtac gcaaacacaa aaatgagtac 108600
aagtaaatcg ggaaatccca ataaggtcag tagcttatat cagtgtcagt atcccagctg 108660
tgatactgta ttacagattt ctacaatctt gccctcgggg ggaacagcat agaagcaggg 108720
gtctctctgt gatttctttt tctttttctt ttcttttttt ttttttgag atggagtctc 108780
actctgttgc ccaggctgga gtgcagtggt gtgatctcgg ctcactgcaa cctctacctc 108840

ctaggctcaa gtgattctcc tgcctcagcc tccccagtag ctgggactac aggcaagcca 108900
ccatgcccaa ctaattttg tatttttagt agagatgggg tttcatcata ctgaccaggc 108960
tggtctcgaa ctcctgatct caagtgatct gcccgcctca gcctccaaaa gtgctgggat 109020
tataggcatg agccaccacg cccggcttct ctatgatttc ttccagctgt gtgtaaatct 109080
ttaattatct gaaaataaaa agtttaattt aagaaaatga agaaaagagc caattcccct 109140
gcttatccct ctaacattcc cctcctatcc cctctccctc ttctgcaatc ctccattggc 109200
ctcagccctg ggatacactc ccagctctcc cttttccaag catttgcacc agaaacactt 109260
cagaagcctt ctctgacccc cctcaactaa tgcgggccct ccctgtgact tcttatcaca 109320
gcaccctgtt tatgtctgtc ccagtactta tcactatctc taatcagatt aatgaggtta 109380
catgtatgaa tgtttatttt atttcaagat gcagatagca aagatagctt tgactgcaaa 109440
atgttttcaa cttccctaga tttttttttt tttttttgag acggaggttt gctctgttcc 109500
ccaagctgga gagcagtggc gtgatgtcgg ctcactgcaa cctccacctc ccgggttcaa 109560
gcgattcttg tgtctcagcc tctcgagtag ctggaattac aggtgtgtgc caccacaccc 109620
aactaatttt ttgtgttttt agtagagaca gggtttcccc atgttggcca ggctggtctt 109680
aaactcctga cctcaagtga tccacccgcc ttggcctcca aagtgctggg attacaggca 109740
tgagccactg tgtctggcct agattaaaat atgccataag aaaaagacag aactcaagtg 109800
tgttagtcca tttttgcatt gctataaaga aatatctgag actgagtaat ttacaaagaa 109860
aagaggttta attggcttat ggctctgcag gctgtacagg aagcatagca cccgtatcta 109920
cttggcttct ggggaggcct cagggagctt ttattcacgg ggaaggcaag aggggagcag 109980
gcgtacatgg tgacagccag ggcaagagag aggagggagg tgccgcacac ttctaaacaa 110040
ccagatcttg tgagaactca ctcactatgg caaggacaga accaaaccat gagggacccg 110100
tccccatgac ccaacatctc ccaccaggac ccacctccaa cactggggat cacatttcaa 110160
caagagattt agagaggatg acatcccaac tatatcagca agcaaaagac tagaaaaagg 110220
tagcaatcta cattatgaac ggttaatggc cttaatatat gaagagttct tacaaatcaa 110280
taagacaaac atccctttaa gaaaaatagg caaagaacat aaatggttca caaaagacat 110340
attcctccat cagatgtgaa atagccacct caatatactg ctagtttttc aagttgggag 110400
tataataggt aacctcaaat ttaaattatg tatttgtcta taaatgcatt gtttgcctcc 110460
ctctgagaga ccacaagccc catgagggca gagctttatg tctgctgtgc tcactgcttt 110520
atccccagtg cctgaaacag tgcctgacac aggcatagca tgtgtacaat aaacatatat 110580
taaatgaatg aatggtttaa aagtcagcaa taataggctg ggcacagtgg ctcacgcctg 110640

taatcccagc actttgggag gccgaggcag gtggatcacc tgaggtcagg agttcaccaa 110700
catggtgaaa ccacatctct acaaaaatac aaaaattagc cgggcatgat ggtgggtgcc 110760
tgtattccca gctactcggg aggctgagag gctgaggcag gagaatcgct tgaacccaag 110820
aggcagaggc tgcaatgagt tgagatcaca ccattgcact ccagcccggg caatagagca 110880
aagttctgtc tcaaaaaaaa aaaaattcag caataataaa ttcaacaatc tttttttttt 110940
tttttttga gatggagcct agctttgtca ccagtctgga gtgcagtggc acgatatcag 111000
ctcactgcaa cctccgcgtg ggttcaagtg attctcctgc ctcagcctcc tgagtagctg 111060
ggactacagg agcacgccat cacacctggc taatttttgt atttttagta gaaacgggat 111120
ttcaccatgt tggccaggat ggtctcgatc tcttgacctc ctgatccgcc tgcctcagcc 111180
tcccatagtg ctgggattac aggcatgagc taccgcacct ggcctcaata atcattttta 111240
aacatgaacc aggtcccaag cttttgtcca tagttaactg tatatgcagt acttattttg 111300
tgcctggccc tgttctaagt acttacaata tattaatcaa tttggttctc ccaacgaccc 111360
tatgaggtag gtgccattaa cattcccatt ttactgatga gaagactgag gcacaaaaag 111420
gctagagcac ttgctggtgg tcacagaacc ataacctagg cattgtcact ccaaaatcca 111480
cgtgtttaag gactgtggtt gctgaccctt gaaatgcagt ccccaaaacc ctggaacttt 111540
gtccggggag ccccactggc ctgagaccca ttggcctgag acccagagac agcaagtgac 111600
aaggccaagg tcacagagtt aggaagcagc aaagtcagtg aaggccgcag ccaggaaatg 111660
agcagaagca tgggcctggg aaaacacgcc cgccaccagg agaacacact tgactcactt 111720
tttttaaaaa actcggggga gattaagtga ttaagccttg aaatgtcaga agccagcagc 111780
agcctggaag cgacccagca ggactcttcc cgaacccaca gcgggtggca gggggcagg 111840
gggcggagga ctgggggcgc acagggggcg gtacaggcct tctgctcttc ttccctctgg 111900
gcaggaaacc tggcttggag gaggggctgg ttctgcagac aggatgcaag cacctctgtg 111960
acaggtggca tgtggagagg aatccaggga cagcaggggc ctcgggtggg ggctggggct 112020
ttggagccgc tgacccctga aactccacga cagaaactgg cctgaaacct cagctcctgt 112080
ggcgcaatca gttagtcatg ctacttataa gaaaccagcc cagagcttcc tctggcttct 112140
gggacagctg ggctgggctg gcaggaagct aggcatgcac ttcttcctgg gacagtaacc 112200
tacctagaac tggggtttcc agaaatggca gccacgtcgg tggccccact gggatagtaa 112260
ggcaggccag gccagggccc cagaatggcc cgggatctgc ctggttggct ccgtccccag 112320
cccagccagt gggagggagg ggaggcaaca gactggctct gcagccacca ggaacacaaa 112380
tcggccccct ccaccaacag cacccaccag cctccctgtg ggcctgctgt ggctcaacca 112440

tcctcaccca cttgctcaag tacctcactt attctccttc tctttttgc tttagagaca 112500
gggtctcatt ctgtggctag aatgcagtcg caccgtcacg gctcagtgca gccttgaact 112560
cctgggctca agtgatcctc ctgccttagc ctcccaagta gttgtgacta caggcacaca 112620
ccaccatgct cgaataattt ttaaattttt gtagagatgg ggatctctct ctgtggccca 112680
agctggagtg ctatggcaca atcatagctc actgcaacct caaactcctg gcctcaagca 112740
aacctcctgc ctcggccttc caacatgcta caggcatgag ctgccgtgtc cagccatctc 112800
ctcatttatt cttgctgtgg gtgttttcca agtactgcct atgtgctggc tattacagat 112860
gcaaacaggc cattcattca ttcattcctt tcttctttta acaaatattt gagcaaggaa 112920
gaagttgcca gagcgttttg agcaaagaag tgacatgatg agactttaaa aaagacctct 112980
cttgcttggg tggtgggggc acgggtagaa gtgggtagca gtgaggaggc taccacactg 113040
gccccagaga gaggtgccag ggctcacacc aggctgcgga gatggggagg ttgagaaggg 113100
gctgaattct agatctgtgt tgaagacaga gcccacagga tttgctgaca ggatggatgc 113160
tgtggggcgg gagagagaac aggaaggcta ataggagtca agaatgattc cacagccatt 113220
ggcctttcc aagatggcgg cctgtggggg tagcccgtgt gacaagccag cctgctgggc 113280
aagctgcagg acttcagatt agcagaggag gctgttagca ggcgctcagg gaagaccctc 113340
cctcatccta gagggtcagg tttctttatc ggggatgcac cagagttctc tcggggaggg 113400
gcccgacata cagcaggcac tcaataagtg agcatgtgta ttttatttta ttcttatttt 113460
aacaggtata cagtgtttcc ttggtgccag acactggcct aggtgcttta agccctccct 113520
atgagataag tataattatt aaacccattt tatgattctg aaggttcttt tttttttttt 113580
gagacagagt ctcactgtgt agtccaggct ggagtgcaat ggtgggatct cagttcactg 113640
caacctctgt ctcccaggtt caagtagttc tcctgcctca gtctcccgag tagctgggac 113700
tacaggtgcc cacaacaacg cccagctaat ttttttgtat ttttagcaga cacagggttt 113760
tgccatgttg gccaagctgg tctcaaactc ctgacctcag gtgatccacc cacctcagcc 113820
tcccaaagtg ctgggattac aggcgtgagc cactgcgtct ggcagattct gaaggttctt 113880
atgagagggc ggggtgaggg agggaatgag ggaagaccca gcgaataggc aggcaggagg 113940
gagggcacgg cccacttccc accctacaag cggggccctt ggctggagca agggggagaa 114000
ggactggctg ggatggcagc tggaaggttg gcaggccagg gacaacatcg tctgccaagc 114060
catggcagta gactcaaact ctgtctctag ggcagtgggg agccagtgaa ggctcttaag 114120
caggagagga gcgggctgag ctgagagctg gcgaagaggg ggcaggtttg agcctgtttg 114180
ggaggaggag gcagcaggga cttggtggcg cttggtgggg aaagcaggct ggggagagcg 114240

gggacagagg gaaagtggcc tcaggctggg gtggtgggga ggatcccagg gacccttccc 114300
acgatggaga gccgagaaga gaagcagatt tgctggggaa gaccacgcgc tccggcgcac 114360
tgtggacact ttgagtccac acagtcaaca ggcaactcca tccgggatcc tggaagccag 114420
gcctgtcttc agaagattcc agaggcccaa aggaggaaac agagcctgag gaaactgtgt 114480
ccagaagcca cctacccagg aacagcagcc agcctggcct ccatctttct gcaacctgtt 114540
atcttgcttt atttctctgc aatctttgtg gcatattgca cactcgggcc ttatgtgttt 114600
ggtgctcata tcccctactg caaggccagc tcacacaagg ccagtgtcga tcccgctcac 114660
agccatctcc ccagagccta gcacggtgcc tggcacacag caggtgctcc atgaatattt 114720
gctgaatgga agaacgacag gatgaattcc cctggctgtg agcagggagg cctggttggt 114780
catccccaat tttccacctc tccctgctgg gcaaacacta gcaaatcctg gcttggagtc 114840
cccttaaatg tcctaatgca ggcacagatc tcagcccacc tcggggccct gacctccaga 114900
gagctggtta tcgactatca ctcactgtcc tgagcagcgg acaatggcct cttggcgcca 114960
gggtgctgat tagattttga ctgtcttagt gacagctatg gctcctagct ggcctccttc 115020
ttgaggtctg cagaggtcac tgtgactggc cacgtgtccc ctcttccttc agccatgaga 115080
ggccatgccc tgggcccttc tgcccttggt ttcgcccctc cccaactggg tgcaactttg 115140
ctcaccagcc ttggcattgg ttgtaccagt ctcactaaca aaaaaaagca cttgaaggct 115200
gggcgcggtg gctcctacct gtaatcacag ccatttggga ggctgaggca ggtagatcac 115260
ttgagtccag gagttcaaga ccagcctggg taacatggca agaccccgtc tctaccaaaa 115320
atacaaaaaa aaattgccag gcatggtggc acatgcctgt agtcccagct actaagggag 115380
atgaggcagg aggatggctt gagcccacga ggtcgcagct gcagtgaact gtgatcatgc 115440
cactgcactc cagcctgggc aacagagtga gaccctatct ctaaaaaaaa aaaaagaagc 115500
ccttgagcct cactcactca cttaaccatg gacccaatcc aaggagcatt gcagatacaa 115560
ttaatatcat ctccattgca gagatggaga agccgaggcc cagagagggt caggaacctg 115620
cctaaggatg cacagctagt gagtgggctg ggatttgaac ccaggccttt ggctctggag 115680
tgcacagcac agtgcctggc acagaagcag tcataaactt gctttttat acaccactct 115740
tttttgcttc tcctggaaag ctcactatgg aagatacagt aaaatattct ccctgcaagc 115800
acagatggag gaatgaggct gagggaggtt cagaatttgc ccgaggtgat ggtgacggag 115860
caggaagtaa ccaggtagga atttagaatc tggcctttgt caccatttgc tcggccctca 115920
gcagcagtga acttgtctca acaatgacca ggactttgcc ccctgggcaa acccaaagcc 115980
aggacaacac atgggcacag gtaagagctt cccgttcatt ttctgcttgc agactggcat 116040

atggaaggca gtgggtgggg actacccatg gagctcttcc tgttgtctca ccccatacca 116100
ttcctggatg ggtggggaca cacagacatg ggggccacct ttgtgggcag ggacccccaa 116160
gcatctgaga cacagaagca taactctggg cctaagtgca ggctccaacc tgactcggtg 116220
ggagaccgtg gcaggctgga gtccactcag agcctcagtc tcctcatctg tgaaatgaga 116280
ataagatcac ttgcttcatg gctggttgca aggctgctgt tgtttttaac tcatctggct 116340
tattagtaca ggagtactga catcaataaa atagaattca tgacaatgat gtcttgagta 116400
agtattagaa gagagaaggt ggatggtttt tttgtttttg ttttttgag aatgtctcac 116460
tctgtcacct agactggagt gcagtggagc aatcatagct cactgcggcc ttgaattcct 116520
ggtctcaagt gattctccca cctcgccctc ccaagtagct ggaactacag gtgcacgcca 116580
ccacacccag ctaattttta gtttttggta gagatggggt ctcactatgc tgcccaggct 116640
ggaagttggc tcttttatac tgattaaagg aacaattcag ccagctgcgg tggctcatgc 116700
ctgtaatccc agcactttgg gaggctgagg tgggcagact gcttgaacct aggagtttga 116760
gactggcccg ggaaacatag cgaaatccca tctctacaaa aacacaaac atcagctagg 116820
cgtggtagtg cacatctgta gtcccagcta ctcgggaaaa tgcaacaatt caccaaaaag 116880
gtataaaaat cctgaaccct tattctactc atatggctat gcacttaaca acatagcttc 116940
aaaaataaag caaaaaactg acagactcaa aaggagaact taacaaagcc tctattatgg 117000
tgaattgtaa ttgtattacg gtgggaggta aatcaggtaa aaaaaaaaaa aaccacatag 117060
tgacataaca aatatagata atataatagc tagcttgatc aaatagatac atttctagaa 117120
ccttataccc aacagaaaat atacattctt ttcaaacaca agaggaaaaa aatgaccaga 117180
aaatcctata aagatctcag caaattctct accactgata ttataggaac cacatatatt 117240
gaccacaaca accatgatga ccaaatttag aaatcaacaa taaaaagata acttaaaaaa 117300
ttctatctgt ctcaaaaatt taaaaagcat ataattaaat aagtcttaaa atgggaacca 117360
aaagggaaat tctaaactgg gcagtgaata acattgtgta acaagttgtg gaaagaaacc 117420
aaagcagtac tcaaagaaaa aatgatagca tgttagcctg aagtagattt attagagagc 117480
atgaaaaact ggaacctctt agctttagac aaggactctc agaatagaag aaacttggat 117540
taacagcagc tcatatagaa gagaaacatg gagattttag ctgacaagcc caatatgcac 117600
tccaagtggg atatggctgc tacacagtat gattccaggt tgcatcaaca gaagtataag 117660
ctctagatcc aaggaggtaa tagtctcctt cctctttgac caaacaaaca gtctttgcgt 117720
gggactccaa ccagttgact catgtgccaa ggcaggccaa acttataatt tcctgagtca 117780
gggagaatac ctgaaataac tacagttgtg tagtctgaat aagagaagta tccgagtttg 117840

gagggagatg gtcactacct ccaagaggca gaaacagact cagaaagtgg aagctcctag 117900
aaagctaatt tgggctgggc acggtggctc acacctgtaa taccagcact ttgggaggct 117960
gaggtgggcg gatcacccta ggtcaggagt ttgagaccag tctggccagc atggtgaaac 118020
cccattgcta ataaaaatac aaaaattagc caggcgtggt ggtgtgtgcc tgaaatccca 118080
gctactcggg aggttgaggc aggagaatca cttgaacccg ggaggcagag attgcagtga 118140
gccgagattg cgccactgca ctccagcctg ggcagcagag tgagactcca tctcaaaaaa 118200
aaaaaaaaga aagaaagaaa gctaatttgg ctaaatctca ggaagatttt ctcccagtta 118260
gagctgccca cctgtggaaa tgtggcttct ggaagaaata tgctagaggc aagaccgtcg 118320
ggaggctgtg gccctgccag cttggagacc tgcccagccc agagagctgg tgcctctcct 118380
ccctagaatt cagtcttccc ttttaaaaag gcagaatact tccctggccg cttcaggaaa 118440
acataggcct tctctgagtt tttcaaaaat ggttctgggc gactatgctc actgatttcc 118500
aaccaagaaa tgtaatcctt ttaaactctc ctctttgggc tggatagctc agaaccgtgg 118560
ttaaatgcac aggcttctgg gtccgtagcc ctgggtttga atctggactc tgccacttag 118620
gagccgtgtc acccaggtaa gtctctagcc tgagcctcag gttcctcctc agggaaatga 118680
gcaggacacc cactcatctc cattcctgca cccagcacag agcgtgatgt ggactccagc 118740
ctccactgcc agaggccgtg gttggggcca tggcttcggg atgattcaag cacattacat 118800
gtattacgta ctttatttct attattatta tatggtaaca tataatgaaa taattataca 118860
actcaccata atgcagaatc agtgggagcc ctgagcttat tttcctgcaa ttagatggtc 118920
ccatccaagg gcgatgggag acagtgacag atcatcaggc attagagtct cataagaagc 118980
acgcacccag ggccgcgtgc agtggctcat gcctgtaatc ccagcacttt gggagactga 119040
ggcaggtgga tcacctgagg ccaggagttc gacaccagcc tggccaacac agtgaaaccc 119100
tgtctctact aaaaatacaa aaattagctg ggtgtgatag cacaggcctg taatcccagc 119160
caccaggctg aggcaggaga atcacttgaa catggggagg tggagattgc agtgagccaa 119220
gatcgcacca ttgcactcca gtctgggcga caagagcgaa actccgtctt aaaaaaaaaa 119280
gaaaaagtac gcaacccaga tccctcacat gtgcagttca caaaagggtt tgtgctcctg 119340
tgagaatcat ctaatgcctc agctgacctg acaggtggcc gagcttgggt ggtaatgcga 119400
gcgatgggga gcagctgtaa atacagataa aacttcgctc actcacctgc cactcacctc 119460
ctgctgtgtg gccctgctgt gtggggaccc ctggtctgag acactgagga aggtgctcat 119520
tcataaaata atatgtaaaa tgattggact gtctgtaaaa tggggcttgg aggaaccagt 119580
cctgcgctgc ccacattcac agtatttctg ggcagatgga tggcagggag tacaccgaca 119640

atgtaaaaaa gtggatcgat ctaaatggca ggtgtgtctt cagcactcag gcatagattt 119700
tattgtgttt aaacaccatg gtgaggactg aggactctgg agcttctagg ttcacagccc 119760
tgctctgcca ccttcaggtg tgtgaccctg gacaagtcac tgaacctctc tgtgcctctg 119820
ttacttcatc tgtaacattt gcatgagagt agctcctatc tggtagggtt attacacaag 119880
ttaaatgaga caatgcaggt atctggcccc agctccctcc ctgacctctg ctcctagacc 119940
tctttcttgt ttggttccat ccaaccacac cagcttcctt gctgtgggtc aaacacacca 120000
agctcattca tgcctctggg cctttgcact ctctgttccc actgccagga tcactctttc 120060
cccagatatc cacatgcctt ggtatttcag gtctcagttc aaaagccact tcctcaaagt 120120
caccaagccc agaatgcttt cagctccatg agggccctca tttctgggtc actcattccc 120180
gtgtccccag cccctggcac tttggctaag caagcgttaa gcacctagcc ccgggctggc 120240
actcgagata tgtgagcgcg tgttacgttc aactttgctt tgcaggcgag aggctctaag 120300
acccagaaca cagcccctcc ccaccaggaa ctcaattcca ccaaaccctt ggaaggcccc 120360
acggctcctc ttcccctccc gggacccact tcctccaact ccagatgcaa gcccagctgg 120420
gcaccatcct cggccgagag gttacagcac ccaggctggc tttagcgcag acctcagatt 120480
tcttaagcaa acgagaagag gtgaggaggc ctgtgggggc acctaaagag agaatgagtc 120540
agtcagagct agacttgaaa cgtaagagac atcagcactt cttggaaagg caagaacgga 120600
cgcatttaga cagaactgtg catgcagaat gaggggtaag cacaggccag tcaggaagtg 120660
gagtcttccg tcctctgtga gtccctccac ctggccctct cttcctcatt tccctctcaa 120720
atgtcacctc ctcagggaag ccctccctga ttccacttgc acccagcttt gctttccttc 120780
ctagcacagt ctccacctga taggatattg caggtccatg ttctctcctg taaaatcctg 120840
ggggccagac atagttcaga attcaggatg actcaggctg tagaaaggtc accttgtata 120900
aaatacccac cgacatgccc atcatgcagg gcgggaaagc acccaaagat caattgcact 120960
aatatctctg cagaaagaca tctgagcatc acaccgaaga ggatgaagac tctaaaaagc 121020
ctcaagttta gcttcaaaat gagttatgaa aaactttcgg ttttcagaaa tcgtggattt 121080
cagaataaca gataaagctg ccgatctgaa catatgtatt ggttgtacat tgcctgtatg 121140
ctcaactaga atctaaaccc caggagagca aaggacttgg tccgatgctc tggcacatat 121200
aggtgctcaa taaattcctg aggaagaaat gcaggaggaa gccaggtgct ggatacgcag 121260
aagggactcg gtccaaaatg cagagaaaaa cagcaatcac tgagcgcctt ccaagggcaa 121320
aatgtctata caaactatct catgaatgct ctccctgcaa atacccatga caaaacaaag 121380
ggaggctcag agaggttaaa caacttcctc cagattacac agtggagagc tgggaagtag 121440

ccagctccga tttaaacctg ggatcccctc ttcaagctca ggcccttctc tctgccttaa 121500
gctcagagac gcagagcaat ttgctggata tcataaaaca gtggtgatgc acccggtctg 121560
acagtggatc ccccagcctc tgtagcacct caagcctctg ccggggcgct ccgatccaca 121620
ttcaccaagg gcaggtctgt gcaacctcca gacaagctcc tggcctctct tctagcccag 121680
ccatggtaag tctagctaaa cgtggctggg catgtctcca ctgtgccagc caggaggaaa 121740
actcagaaat gtctttcttg gtcaccccag tgaactggta caagcaggaa agaatacatg 121800
ggaagaaaag taggatggag tctgtccctc agatgtggac ctgaggcaag gctactgcat 121860
tcatctccgg gtctcagttc tcacctgcaa aatgagtgga ctaaatccca accaataaag 121920
ctcaaaggag gcaacagtaa agatttattc atttaggcca ggcacgtttg ctcgcatcta 121980
taaatccaac actttaggag gctgaggcag gaggactgct tgagaccagc agttcaagac 122040
tagcctaggc aacatagcaa gatcctgtct ctaaaaatat aaaaacaatt agccagccgt 122100
ggtggcacac acctgtagtc ccagctactc aggaggctga ggtggaagga ttacttgaac 122160
ccaggtattg gatgctacag tgagctatga ttgtgccact gtgctccagc ctgggaaata 122220
gagagagacc atggctctaa aaaaaaaaaa aaaaggtatt catttaacaa atacatagtt 122280
tgtactacat accaggtact gttttgtatg cttgacatat attaactcat ttagtcatca 122340
ctacaatggc atagtcacta taatctcata aatgcagaaa ctgaggctct gggaagtcag 122400
ttacacagta agcagcaaag ccagcatttg cactgggtga tctggctcaa gtccatactg 122460
tcactcccca gctatacgtt ttgacaggta acaacagaca tatacattga ttgtggaccc 122520
actgggtgtt ggacattgtg tgaagtagct tataaaacat gtcatcaacc ctcaagaaag 122580
ttcactttta caaatgtgag gaagaacagt acttaggcgg cttctgtcct ggcctcactc 122640
ccaggtacca gtgagggcag tcgagaggtg gggcatctaa tcccaaacgc tctcctccat 122700
cgtcccagat ggggtgggtg gaagagggca gggagatggg ggaggtagaa aaggttctct 122760
cttctcatgt atttatgtat ttgtttattt tgagacagag tctcgctctg tcacccaggc 122820
tacagtgcag tggcacaatc atagttcact gcagccttga ccacctgggc tcaagccatc 122880
ctcccacctc agcctcccaa atagctgggg ctagaggcac ctgctaccac gcctggctaa 122940
gtttttaaat ttttttgta ggaacaggat ctcactatgt tgcccaggct ggtctcaaac 123000
tcctgggttc aagcgatcct ctctcagcct cccaaagtgc tgggattaca ggtgtgagtc 123060
actgcgccca gcttgtcact tctttgacaa ttcatttcag ctcctttttg aactgggagc 123120
attagaaaac tgaacacccc caccccttgcc ctgggcattt tgcaaccggg attgtattgt 123180
attaaacata tcagtcattg agtcacttcc tagcatttgg aaacccttag gttatctcgg 123240

acctgccggt aactgcatgt gttgtaactg gggtgagtga ctgcaggttt tcctccctgc 123300
tgcctcccca gcgtctccct ccagattcag atccagaggc ccagggtggg gcccaggaat 123360
ctgcactgtt gcttttacaa ctcaagccaa ggtgaataca ctggcccaag ttccttggca 123420
tggcattcaa ggctttccac aatgcagccc agcccaactt tccatccatc tctggcttct 123480
gcttactccc taggttctgt acacagtgcc ctttcacact tcccagcctc cgcccactct 123540
gtgcctccct ttacctccca tcctctacct gccagactcc cctgctctac tcacaaggct 123600
agctcagggt ccctgggtgc tgggaagcct ggcccagtgc cctttgaaga gctgatcccc 123660
tctggcttgt ggtcctgcag ccgctggtgc atccgcaccc tcccagccct cacgtctgtc 123720
ttcctgcaga gggggtgctc cttgaggcaa gcaccagtgc ccagaacacc acccagggcc 123780
tcgcaggagc tgaatgagtg ctagtgagtg aatgagtggc ttagcaggtc acctcccatt 123840
catgggctgg gctagccgag ttctaagaaa cccctgcagg accctggaat gccctaatgt 123900
gcaggacacc ctggccaaac ctacacctga gcctgtttcc ccattggact ttgggatttt 123960
gataaaggca ctgaggtccc agcaaatgcc ttgggctcct cataggctac tgggggcagg 124020
gaagtgcagt gggtcatgcc tcccagaggg ctggggaagg ggagagacta gaactggagc 124080
agggttagcg ctggcctggg ggcattcctt gaaccccaga agcagacaaa ctagtggtcc 124140
cctatcttgc ttaagtctaa gaaatccaga ttttgtttaa aaaaaaaatc agggctcagt 124200
ggctcacacc tgtaatccca gcattttggg aggccgaggt gggaggatca cttgaggcca 124260
gcctggacaa catagtgaga caccatcttt ataaaataaa tttttaaaaa taaaataaag 124320
atcagattcc tgggtttact gaatctgaat ctcagggcaa ggacctggga atctgcattt 124380
taagcaaatc tcccggagat tctggttatt gtgggggctc ctcaaacgtg attgagagat 124440
ggagagcagc tggctctggc gtgggattgc ctgggctcta gtcctgctgc cccacggact 124500
cccaagtggg cggcacctta gggcaagtta ctaaagcggc tggggtcttg gtgacctcat 124560
ctgtacaatg tccatgacgc acagcaccgg ccacagagaa gttagtggga ggtttcactt 124620
ggcatccttt cagtaaaaca agtcccagtc aatgatcgcg attattgggt ccatctgata 124680
ttcggaggcg gagaccgccc cgattcactg acctactact caaatttaca gttctcagaa 124740
ctttccccac cctttctctc tccgaagtcc tcactgactt cctgagcgca gggctgggcg 124800
ccactgcccg gagaagaggg gctgcgtggc ctccgacagc ctaaccccaa gccccccacc 124860
cccgcaggcc gtcccggcgg aggtctcact gccagcaccg gctttgggga gcgctgggtg 124920
tccacgtacc ccgggcctcg cgcctccgct gccgttcggc ggcaaggacg gaaccgggcg 124980
ctgctccgcg cgcaccgaga gtcgcgggct cgggccccgc tcggagtcgg ggtggtcccc 125040

gtggtccccg ccggcggccg agcccttccc gaacgccctc agccggcgtg cccgggcgat 125100
accttcgcaa cccgatccgc gcgcgggccc cggatcctac ctgcgcgccc ctgctccatt 125160
cgccgtccgg gcgcccaagc gggaagtggg ggacccctgg gtggcccttc cctggcccgc 125220
gccgcgcggg gctttcctgg gcgcggagac ccagcccgcc ccggcttcct gctccccgcc 125280
ccggcgcccg gcagatttgc atgcgccgcc ccgcccggcg cgcccggccc gggtcccggc 125340
cacctcgcgc ccctgtcgct gtccccgccg ccgccgccgg acagtccgag ccgccgtcct 125400
ctcctggggg cgaccccagc acctcgctgg gtgtcttgcc gcgcgcttcc tcccacgctg 125460
cagcccgagg tcacgcccag cggagaactc tgcagtggat ccccttggtc tcggaataaa 125520
acgcccacgc cgaccaggcc tccaggacct gctcctgcct ggcctccgcg gcctcttttg 125580
cgccggggca gttgcgtttg tggggggagg gctgggcgac tggaatcctc ctccccttcc 125640
taacagcccg gcttccaagt gagagtggtt gcacattctt caataaatgt ttacccaact 125700
cctactccag ccacatttgg atagtaaaac agcccttcaa tcagtaacta gtacaatctg 125760
tcgggcactg caaatgttaa ctcgttcaat cctggcaact accctataag ggagtgatac 125820
tatcaccggt tttacaaatc aggaaactga ggcacagagt ccttaagaaa ttcaaaatgg 125880
gttgggcaaa gtggctcatg cctgtaatcc cagcactttg ggaagctgag gcgggaggat 125940
cacttgaacc ccatagttcg agaccagcct gggcaaccct gttagcgaga cccctatcta 126000
caaaaattga catttataaa attttaaaaa agaaaagaaa gaaactcaga atcactctgc 126060
aggtaagcag gagcaggggt ggagctggga ttcgaacaca ggccccatag accattaacg 126120
tctggacacc ataagaaccc ggatgcagcc agatgactgg ggggaaaaca ctcaacaaag 126180
tatttcaggt caggagggcc caagaagaaa gtagggacct cttagaccag tggggcaggt 126240
ggtctggaag aggctttttg agtagaggcc atttgagctg agactctgaa gagcctatgg 126300
gggaagactt gtgtagggag agggaagagc tagtgcaaag gccctgtggg aaccagctca 126360
gcctgcagga ggaattgctg aaggccggca tggctgtgga gccctgagtg aggaaagagc 126420
ggagacaggg gaggtgggag atatttggac tcacactggg cagtgctgag aacccagaga 126480
gtaatttgac cccctgctgc cctcaaggag ctctcagttt gagggtggaa gccaaggact 126540
gcccaatggg atcaagtgct gtggtggagg gtgagggttg gcttcccagg gcaggggcca 126600
ctgcacgtca atcattcttt tttttttttt gaagatgggg tctcattcca ttgcctaggc 126660
tggagtgcag tgggtgatct cagctcactg caacctccac ttcctgagtt caagcaattc 126720
tcctgcctca gcctcctgag tagctggaat tacaagcgtg caccaccacg tccggctcat 126780
ttttgtattt ttagtagaca tgaggtttca ctatgttggc caggctggta tcaaactcct 126840

gacctcaggt gatctgcctg cctctgcctc ccgaagtgct gggattacag gtgtgagcca 126900
ccacgcctgg cccatatgtc aatcattctt tcatgtactt ggtctgtctt ggtcctactg 126960
tgtgtctaac cttgtgacaa gcgctgatga cgcagacctg aagacatgaa acagacacaa 127020
tccctgccct ctgagacctt acaaccaagt gagcaagaca gccaccaacc cacccccaga 127080
agtgacagta aagataagtc agttccctgg ggtcagggat ggcttcctgg aggaggtgac 127140
ctctgaccag tgcctgaatc aatgcagaaa aacatgcagg tggaagggac agcatgagca 127200
aatacctgga ggtggcgtgg gagtgtggaa aaactcccat ccttcggcat ctgtttgtgg 127260
atgcctccat atgccaagtc gtgagctggg ggctagggag agcaagaaat agatccagcc 127320
cctgcttcct agccctgagg acctggttgg ggcactgaca agtgagcaga cgtaagcaat 127380
tgaagtgaac agatgtaagc aattgagtgt aataaacccc gtactggggg cagcccaggc 127440
agacgctaag aggactaggg agagaaggcc acaggaaggg ctttcctgga agcttgggtt 127500
tcaggaggaa taggaatcct ctggattagg tgtgacttcc actcccgtcc acactcatac 127560
tccctcacac tcattactca cacatccgca cacccacacg gttgatgctt catcccaggc 127620
agttcagccc tgcaagctgc ctttgtcctg tctgtgtttc tgagtttgtg ccccatgtca 127680
tcgggagacg acagcaaaag tctacctttg aagtcctttg ggaatgtatg gcttgggcta 127740
tatcccctcc tggactccca aactgggctc ccatcctgtt ttcccagtca agtcatcctt 127800
cgtcggtttc ctcgcttgcc tctagactgc cggcattggc ataccaactc tgctacgtat 127860
tgactatgtg actttgggca aagtagatca tcctccctgt gcttcagttt ccccatctgt 127920
aaaatgggga taataataga cccacctcat agggctatga aatggtgaaa tggcaaggta 127980
cgtatcgcag tgcctagaat agtgcctggc gtggattagg cattcaaaat gtcaattgct 128040
gttcttatca ggatgaaaat gagaaagaag caaagtcgaa gcctgttgcc ttggtctgtt 128100
gcttggttgt gaaatggaga tgaacgtggc attgagaaga caggcaaaga aagcacagct 128160
gtggtcagag gctaagacat tcaggaaatc tgggggaaga gacttcggga attctttgta 128220
ctcttctccc aactttcccg ccagtctgaa attatgtcaa attacaaagt taaagaaaac 128280
agggcattgt tctcgggtgc aagagagata ataaatgtct tcttgcatac agtcccactg 128340
ttcaagttcc tgcccaagat gcccagactt tatctgtgac tgctccgtca gtcctgggag 128400
caggtccaaa ggcaccaaga tagtaaaacc tctgttcccc catctagggg ttcccaagtc 128460
ttgcctcagt ataagctcaa tcctcattat atcctaacac gcttacttgt ttccaagcct 128520
ttcggtcttt aaaactaata gagtttattt tttagaacca ttgtagatta tggaaaaatt 128580
gagccgatag tactgagagt tcccatacat ccctctcccc catgcaaaat ttcctctttt 128640

atcaacatct tgcattagtg tgtagtgcct tccttataat gctgatacat tattataaac 128700
taaagtccat agcatacatt gtttcactgt ttctcggggt tttgttttgt tttgttttgt 128760
tttgtgtttg tttgcttgtt tggaaacaag gtctcactct gttgcccagg ctggagcagc 128820
ctggaactcc tggactcagg ggatcctcct gcttctgcct tctgagtagc ttgggctaca 128880
agaacatgcc accacactag gctttttttt ttttatactt ttagagacag tcttgctaca 128940
ttgcccaagc tggtctcaga ctcctaagct gaagttatcc ttccatctca gcctcctaaa 129000
gtgctgggat tacaagcatg agccactgtg cctaacctct gcagcacagt tctgagtctt 129060
tttttttct ttgagacaga gtcttgttct gtcacccagg ctggggtaca gtggcgtgat 129120
ctcaactcac tgcaacctgt gcctcccagg ttcaagcgat tctcactcct cagccttcca 129180
agtagctggg actacaggtg cacgcctggc taatttttgt atttttagta gagacggagt 129240
ttccccatac tggccaagct ggttttgaac tcctgacctc aagtgatcca ccagcctcgg 129300
cctcccaaag tgttgggatt acaggcatga gccaatgcac ccagccaagt tctgggtctt 129360
aacaatgtat aatgccatgg atccacacat gtagcaacaa aaagaatagt ttcccagcct 129420
gggcaacata gcaagacccg gtctctacca aaaaacacaa acaaacaaca acaacaaaaa 129480
acaggccagg catggtggtg cactgtagtc ccagctactc agaggctgag gcaggaggat 129540
cccttgagcc caggagttcg aggctgcagt gaactatgat catgccactg cactccagcc 129600
tgggcaacag aatgagaccc tgtctctaaa aaaaaaaaga aaaaaataaa gaatagtttt 129660
acttccctaa aaatccccaa ttctctacct attcttcctg gattctggaa accactgatt 129720
tttttactgt ctccatagtt ttgccttttt cagaatgtca cgtaattgaa atcagaaagt 129780
atgtagtctc ttaatactga cttcttttac ttagcaatat gcttttcagg ttcctcaatg 129840
tcttttccag gcttcacacc tcatttcctt ttgtggctga atagtgccct attgtatgga 129900
tgtaccacag tttatccatt ctacctattg aaagacatct ttgttgcttc caatttttgg 129960
caattatgaa taaagctgct atcaacactc atgtgcaagt ttttgggtgg acataagctt 130020

<210> 89
<211> 9900
<212> DNA
<213> Homo sapiens

<400> 89

gaattcaata aaaaacaagc agggcgcgtg gtggggcact gactaggagg gctgatttgt 60

aagttggtaa gactgtagct ctttttccta attagctgag gatgtgttta ggttccattc 120

aaaaagtggg cattcctggc caggcatggt ggctcacacc tgtaatctca gagctttggg 180

agactgaggt aggaggatca cttgagccca ggaatttgag atgagcctag gcaacatagt 240
gagactctta tctctatcaa aaaataaaaa taaaaatgag ccaggcatgg tgcggtggac 300
cacgcaccta ctgctagggg ggctgaggtg ggaggatcat tgagcctggg aggttgaggc 360
tgcagtgatc cctgatcaaa cattgcattt cagcctgggt gacagagtga gaccctgtct 420
cagaaaaaaa aaaaaaaagt cattcctgaa acctcagaat agacctacct tgccaagggc 480
ttccttatgg gtaaggacct tatggacctg ctgggaccca aactaggcct cacctgatac 540
gacctgtcct tctcaaaaca ctaaacttgg gagaacattg tcccccagtg ctggggtagg 600
agagtctgcc tgttattctg cctctatgca gagaaggagc cccagatcat cttttccatg 660
acaggacagt ttccaagatg ccacctgtac ttggaagaag ccaggttaaa atacttttca 720
agtaaaactt tcttgatatt actctatctt tccccaggag gactgcatta caacaaattc 780
ggacacctgt ggcctctccc ttctatgcaa agcaaaaagc cagcagcagc cccaagctga 840
taagattaat ctaaagagca aattatggtg taatttccta tgctgaaact ttgtagttaa 900
ttttttaaaa aggtttcatt ttcctattgg tctgatttca caggaacatt ttacctgttt 960
gtgaggcatt ttttctcctg gaagagaggt gctgattggc cccaagtgac tgacaatctg 1020
gtgtaacgaa aatttccaat gtaaactcat tttccctcgg tttcagcaat tttaaatcta 1080
tatatagaga tatctttgtc agcattgcat cgttagcttc tcctgataaa ctaattgcct 1140
cacattgtca ctgcaaatcg acacctatta atgggtctca cctcccaact gcttccccct 1200
ctgttcttcc tgctagcatg tgccggcaac tttgtccacg gacacaagtg cgatatcacc 1260
ttacaggaga tcatcaaaac tttgaacagc ctcacagagc agaaggtgag tacctatctg 1320
gcaccatctc tccagatgtt ctggtgatgc tctcagtatt tctaggcatg aaaacgttaa 1380
cagctgctag agaagttgga actggtggtt ggtggcagtc cagggcacac agcgaggctt 1440
ctccctgcca ctctttttc tgagggtttg taggaagttt cctcagttgg agggagtgag 1500
agctgctcat caaggacttc tctgtccggt tggaggttaa ctctgtctct tgctctctca 1560
tttctgcctg gaccaagact ctgtgcaccg agttgaccgt aacagacatc tttgctgcct 1620
ccaaggtaag aagccgtccc acggtctgtt ttagcaaatg gggagatcca tccccaaatg 1680
tctgaacaag aaacttgtct aatggaaaac gagcgggccc aaattaactc taaggtgtta 1740
gatgttttca aagaacgaga agtctgatct ttactcttaa gcatgttttg gtctttctgg 1800
tttcacttga tttagaagac atgtaataga aagcttacat gctgtagtcc tgactcagat 1860
cctggtcaaa gaaaagccct cttgggtttt acttagcttt ggcatagtgc ctggaacgta 1920
ggaggcactc aataaatgcc tgttgaatga gagaattttt ctggcccata catttctgaa 1980

aaaccaaata ctctcacaga aacagatatt gagatgacag gttgagggag ctttcatttt 2040
gtctaagaga cttcctatgg caacagaaaa ggtatcgcca gagcccctcc tcttccacag 2100
cctggccacc taacagccct ctggttccgg ggctgccgtc cagagctctc agcttgctct 2160
ggccggccga actcccctcc agctcggtct ggaaccatcc tgctgggcag cgtccagcac 2220
atccctgctt cgggctgcct gggcacctcg cctctctgcc tcctgtgctg cctcaccccc 2280
acccctctat ctgtagtggg agggagatag atttgacagc tgatagtgca ttttctctga 2340
caaacacatg actacagccg tatcaatagt tttgtgcatt tcagttcctg ttttcatgga 2400
aacacacggc tgagaatgaa agccccaaag cctcaatttc acagtggtct cctaactacc 2460
tgctttccat gcaaactagg gagatgatat ggccaggagt gaagccctgt gtgttgggca 2520
gggtcacact ccagcaccca gaccatagaa cagggcccat cctgcttcat gagggaaact 2580
gctcttcggg cctttagctg gactatctca tttcattagt tatcccggga gtccgataca 2640
ggatgagatt ctgaagggca aatacacact tttttttttt ttttgagata gggtcttgtt 2700
ctgtcaccca ggctggagtg cagtggtgcg atttcagctc atagcagcct ccacctccca 2760
ggctcaagct atcttcctac ctcagcctcc caagtagccg ggacgacagg tgtgcaccac 2820
cacgcctggc taatttttgt atttttttgt agagatggag tcttgccatt ttgcccaggc 2880
ttgtctcgaa cttctgggct caagcaatcc gtccacctcg gcctctcaaa gtgctgggat 2940
tagccactgc acctgggcaa cagtttatgt gtgtgtgtgt gtgtgtgtgt atatatgtgt 3000
gtgtgtgtat atatatgtgt gtatgtatat atgtgtatgt atatgtgtgt gtgtgtgtgt 3060
gtgtgtgtgt gtgtataaaa tctccaagtc catccaaccg agatggctcc tactagaagc 3120
caagagtcca ccgggttgag cactgggtct ctggaggcct gtcggactgc tgagaaggct 3180
ctaacaaagc caagggaagg gccacctcac tagaagccag gcctggagga agggtgaggg 3240
ctgagggctg gaggtaagac tgcctgtggt tttagaccca ggctctgcca ctgactagct 3300
gtgtggctgg ccttcagaca tcttcacagc tctctgcacc tcagtttcca catgtgaaga 3360
tatgaaagtg attctgaagg tgattgcaag gttgattgga atccagctct tgagttagtg 3420
caaagtgtta ttgtgagatg atataaccac gattaaaagc aagaacaggt gcagagaagc 3480
gatgattcta agaaggaggg gaccgggttg gaaaggatca aaccatccag gatgccgagt 3540
ctggggcaat ccatctgggc tgtttctgga agacccccgg gtgcaggcca ggacactgct 3600
gccctcccgt ccttaactcc cctcttcact cagtcctcac tcacctccct ctcacacaca 3660
caaacatctc ctagaataat ccccactgcc tgccttcact cttacccgtc tcatttgcct 3720
cccctgaact tcatcctcct ggagttcacg atctcactct tcactctttt cttcccctcg 3780

aagattcagc actgcttact tacatgttaa gatatttcag aacagtgaaa tgttgctatt 3840

ttcaaaaacc tacaaaggtg gtatgcagag gaaaaggtac ttctttgtgt tcccaaagaa 3900

aacatctttc caaaatccag cctattgatt ttatttcttc gggggaacaa gaattttagt 3960

atctctaagt tgggtagcat tctactcttg gcagttgctg gaaagaaggc actggtctag 4020

gtcctgggct tcacaggtaa cacctgtcag ggtgtctatg aagtcaaggc tgtctgagga 4080

acagcaaagt gggaagaagc aagctggctg gctgatgaag ggtttcttgg gtggacaagt 4140

agttggagcg atttcctatt taccaaagag agctaaagtt cataattcta cagagagttc 4200

cataatgaac ctcaaatacc tctgtttttt gaaggagttt ctcatataca gcactagctg 4260

actatcctgg gcaggatggg agataatgaa tgcagtgcca atcgggctgg atttatatgg 4320

tcctagtgag gctggtcaag aaccgagtta gaactctcac agagtcactg cccacagaag 4380

aaatctccca agtggctgtt tcctgacatt cccgggaggc aggcctcctt ctgagtcact 4440

ccctaagcag ttctgaactg tgaggtcagc caggctgtcc aagtgcactc cctgagccac 4500

tggcagacac actcagcagc cagagctaga caggcaggtg gtaggagtcc agggccacgg 4560

cagggatgga gtgtcgcccc ctcgctgcga taccagagca agtaaaacgt taaggccttg 4620

cactaaagct gcccttagga tgcattcttt taaagttttt ccatttaatg cagactcttt 4680

tcaattctta ttttatcctt gtttccttta gaaagtcctt tgaaaaatat ctttagaggg 4740

tttttttccta tactatgtgg ccatatacgg gtcaaaatta agtttaattt ccaggctcca 4800

agccagcgtt tcagaaaaat ctcaccaagg tttgtggtaa aagaagcaaa gggctgactt 4860

tttggttttc ttgaatctca ctgttccctc tgcagcagca tgcatgtctg cccacctcca 4920

gacacacagg caccatctgc cgcccccat cagcccgtgt cccttccacc tcgactcgcc 4980

tacaaagccc agagaggtct gtttcttggc ccccagagcc caaagatact gacacactct 5040

tacatttcca actagaatca ggaacgagga gtgactctca gtcagttcat taagtaaatg 5100

tctttctaac cgctctgccc atgggacatc acgccccaca ggggaaaggg gaagcttctg 5160

tagcctggga ttctggtgcc tcagtctggg tctagacttt cctgaaaaaa cgttaaaata 5220

tgaactgcat tcctagaatt tagcctacat aaataagaga tgaacacaaa gatttctata 5280

gtttactcac tgccgcttat ttacagaagc aaaaatctgc cacgataggg gcctgacaaa 5340

tgacagtacc actgtgcaat gcgtttctac gcagctctca atcccatgtt ctctaatacc 5400

accgaaggct taggaaatgc ttatggtata tgtaaagagt aaagaagtta caaacagtat 5460

caacagttga cccctatttt aaaaagtatt tttgaaaagt gtgacgatat ttaccaaaat 5520

attaacgagc aatagttacc tctggctggt gggatgagtg aatgtatttt tgttgaatat 5580

atgttacctt tatagtaaat atatgttatc ttgatcatca gaaaaaaaaa tatgtaagaa 5640
cttgaaagct gcttggacag cgctgctgat agaaacccct gagcatcttg tcactgttct 5700
tctgattcag agggtctggg tggggcaggg gtggtctgag attctgtatt tctaagaagc 5760
tcccagtgat gtccatgctg ctgtccatgg accacacttt gagtatcaag ggaccagagc 5820
atgtcggggg agaggctggg gatagctttc tttatctgaa ctggataaag gaactgggct 5880
caagctaaga accctctcca ggttctgcat ctttgttctt cagtgaaaaa tgagaggaca 5940
caccaggcca ggttcagact gagacacaat ccctctcctg ggttcccaat gacttgtctc 6000
ttgtccattc ccttctctaa ggctaagggc cccccaggaa gagccatgtg gccagaccct 6060
cacagttgct ggcattccaa ggagattctc actccgcatc attggtccaa aaggcccctt 6120
acagaagctc tgcccaaggc tcagatcaat ggcacctgct cccagagctc ctctgatctc 6180
ccaggacacc tttccctgat ctgtgcactt atctcttgct gcctggcaaa atgtcttagc 6240
tcctcacttg ggccatgtgc tgctctcctc tcccatgggg agagccacac ggagagtgct 6300
ggccaaagca gcagagttca ggccaaagga tgtgcactca tttattcaac aggcatgcag 6360
gatttccagg gaaagctgga ttttaaaacc tctgggaaca agagcagaac ctgactgaga 6420
gctcatgtgg gcacttttca tagcagaata gctcatgagg tatagagaca cggacgcaga 6480
acgtgggctg tagcgacaga tggtcctgca ttctagtccc cactgtgcct tttcctcatg 6540
ggatgacttt attcaggtac cctttcggca aaatcctcca agagaaagga aactgggagg 6600
ttctggggag aaggctgctg cgtttgcaat tgggagaggt tgttgacaga ggtttatgtc 6660
tgtggcaagc agccttcctt cagtggaata cttgaagaca ggtctgtagt tgagcaaact 6720
cacctccatt tgtcctcctg gaaagaagaa atcaagagga aaaatctctc tcccatcctc 6780
caaatggagc tggcacattg ctatctgtgg catttgtctt tccagaacac aactgagaag 6840
gaaaccttct gcagggctgc gactgtgctc cggcagttct acagccacca tgagaaggac 6900
actcgctgcc tgggtgcgac tgcacagcag ttccacaggc acaagcagct gatccgattc 6960
ctgaaacggc tcgacaggaa cctctggggc ctggcgggct tggtaagctg cactgtattc 7020
ctggcaagcc ggccgcgtgg ctcctggtgg acagcagcct cacttctaaa cactccttag 7080
gagctgcagc acccttggtc aacccattca ttcattcact cattcaataa gtatttgctg 7140
aagttccaca agtgctgggt gtggttctag gtgctgagga cgtgtcacta aagacagcag 7200
gccgagtccc tgttctcatg gaatgttcta atgggagagt tagaaaaaca aacatgtaaa 7260
atgatggcca gcagtgatac gtgctacaaa gaaaaacata gaaataaaga acataagagt 7320
catgggggag gggctgact taggagctgg tgacattatc tgagcagata tttgaattga 7380

gggagcaggc cacatgacta actagggaga ccattccagg gagaaggagg aggtatgcaa 7440
aggccttagg atggaaatga actaacttcc tgtatttaaa gaccagtagg aaggccagtg 7500
tggctggatc agagtgagtg aggggtagtt tccaggacag cagatcacac aaggccttta 7560
gattccacca cgagtatgga gggaacacct gcagagcttt gggcaggaca aagactgtac 7620
aatctgattt acgtgattta aaagggtcag tctggctact gtgtggtaaa taggctgaaa 7680
gggggaaagc atagaagcaa gatggcctgt tgggaggcta ccacagtaaa ccaggctaga 7740
gatgatggtg gcgtggacag aatgaagcaa gatggcctgt tgggaggcta ccacagtaaa 7800
ccaggctaga gatgatggtg gcgtggacag aatgaagcaa gatggcctgt tgggaggcta 7860
ccacagtaaa ccaggctaga gatgatggtg gcgtggacaa atggagcagt tgaggtgaac 7920
agatttggga tatgactaaa aataaaacca gaagatttgc tgacagatcg gttgtagggg 7980
gtaagataca ggggaggaaa agatgacctc tttgttcctg cccaaacccc tctggcgatg 8040
gtcagtactg tttacagaga gatgaaagac tggcggcaag gcagggctgg aggttcagca 8100
gaagatcaag agttcaattt tgtacatcgt acatgtaagg tggctcttgg atagccaagt 8160
gaaggtgttg agaagatggt tagaaaagtc tggaacttag gggagaggtc agaacttgca 8220
atacaaaaag gagagtcctt agatagatac tgctgaaaat ctgaatgaca gaaagggaga 8280
gatcaaagga ctgagcctga gatcaacaca tggaggtcag gagaggagga tccagccaag 8340
gggcctgagg aggagtgacc agtgaggcag gagaacatgg agagtgggcg gtaccccagg 8400
aagccggtga ggacactcaa ggagggaggg ttgactgtgt caaatgtact gaaaggacag 8460
gtcaggtgag gaccaagaaa ggcccctggg tttggctgat ggaggccatg ggtgaggctg 8520
atgtaaatgg agaggcagga aggaaagccc agctggagtg ggctcaccga ggatagggtg 8580
gcgagaggag acaaagaagg aacagtgagg gcagacaact ctttgaagat gtttagctat 8640
aaggctgcag agaaactgag cccacagctg cagggtggtt atggagtgag ggaagctctt 8700
ttaaggttgg gggtataccc agcatgttaa tgcacctggg ggaatggtcc agtggagcag 8760
gaagaactga agagagcaga aagaggaaga atcattaggg ggcagaagtc cttgtagccc 8820
agagtggatg ttatctaata tcgagtggag gaattaattg gctttagagg agaacaagga 8880
catgtatccc ctctctgggc ctatcacctt gtagacaatg ggataggtca tgggatagga 8940
acttggcaca acacatgttc tctcttttaa ttctctccat tatcttatga agcaggcaag 9000
taggcaaaca attgtcccaa ctttacaaaa gaaactgaag cttttataaa ttaagtagta 9060
catcctaagc aatacaatta ataaatggta gagctgagat tcaaactgaa gcagtggcct 9120
gggggtagca tctggaatcc ttcccacctt tagggctgct gtgctgcggt gctgctgttt 9180

aatggcacag agggccagat gactgaatct ctctcagcag tccaggcagt catgcagaag 9240
gcccagtaga gcaccgggca ggtctgagcc agcatcttca agttccaccc tgtgagcaag 9300
cacttagctg tgacacactt ctcgagagac tggactcccc cccgcgcaac ccacccaaaa 9360
gcagataggt aatggtatac agtaaccatt tctagaagtg taagtagtat gcacccaaaa 9420
taggcaaaac ctgctggcct agtgatagag acaactccca gtcaggctag actggaggcc 9480
ttggttttat aagtgttcag gtgacaagtg ccacagtagg cttgatcaag tagacaggca 9540
ggcaagacaa atgcttacca atgcaagcta atgaaatgtt tcttttgcag aattcctgtc 9600
ctgtgaagga agccaaccag agtacgttgg aaaacttctt ggaaaggcta aagacgatca 9660
tgagagagaa atattcaaag tgttcgagct gaatatttta atttatgagt tttgatagc 9720
tttattttt aagtatttat atatttataa ctcatcataa aataaagtat atatagaatc 9780
taacagcaat ggcatttaat gtattggcta tgtttacttg acaaatgaaa ttatggtttg 9840
caacttttag ggaaatcaat ttagtttacc aagagactat aaatgctatg gagccaaaac 9900

<210> 90
<211> 4740
<212> DNA
<213> Homo sapiens

<400> 90

```
ggatccccgc tgacaatcta gaaacaagca acaggaccct ctgatgtagc catctgtgcc    60
gcgcctctcc gcaccgcccg ccacgccttg gtccctggag accaccctcc agggcagggg   120
ctgccgctcg gccgggcccg cggggtccct cggcctgaca tggccggtgc tggagcggca   180
cgtgcgcgcc tcggcccctc ggccgctccc gcccctcgcc ggtgcgcacc ggcgctcggg   240
gagccgctgg cccgggtgtc cagccggccc ttgccctgcc tggcgctcgg accgccacct   300
ttgccgcccc ctcgccagcc tccgcagctt ccagactggc cggtctgcgc gcccacccct   360
gcctcccgga ccggccaccg ccgaggcgcg gaggagggcc cggccgcgca gatcccgctt   420
atcggcccat ctcccgttac ataaggccac ccccctatct ccgcgggcca tcgccgccgc   480
aaccgccgcg ccagcgcctt ctcccacgcg cgggggcgcc cctgcccacc gctcccggca   540
gggcttttgg tggccatggg ggataagggg cgttgactca cccgggcggg gctccgggag   600
ttgcacagac caaggtagtt ccccgctcct tcccccatca cggagaccct gtgggagatg   660
ccgtgggccc tctactacag attaggaaac aggcccgtag aggggtcaca cggccaagta   720
gcggcactcc aggcactggg ggccctcgag gggaaggggc agacttctgg gagtcagagc   780
cagcagctgg gctgggaagc ttcgagtgtg gacagagagg gtgggaatga cgttccctgt   840
gggaagagag ggtggcaagc ctgggatgcc tctgagcggg aatccagcat gccttgtgag   900
```

gagggtcaca agcacaccct tgtgaggagg ttgagcccca tcgaggacag gacggaggga 960
gcctgagcag gcagagaggg ggcctgggga ggcgctggtt cggggaggaa gtgggtaggg 1020
gagaaatctt gacatcaaca cccaacaggc aaatgccgtg gcctctgctg tgggggtttc 1080
tggaggactt ctaggaaaac gagggaagag caggaaaagg cgacatgctg cagagactgg 1140
tgagcaaagg ggatcacccc aagccccagt ggcactagga acacttacaa tctctgacct 1200
ggactaaggc tgccagctgg cccagttaag agtttcccag aaggatggcc catacacttt 1260
aaattaaagg ggccagacac gtgcacacta cttccagcca ctctggaagc tgaggtgggg 1320
ggatcgcttg agtctgggag ttggaggcca gcctaggcag gcaacatagt gagaccccat 1380
ctccaaaaaa acaaaacaaa acaaaacaaa aaaacaccaa aaaagctccc agaaagacct 1440
ctgaatcttt ctggatctct cagtggagac cttggaaatc tgaactttga caatccctct 1500
cacagtgggg ccaaggagga attaggcaag ccaaaagaag tgaactttac tcttctattg 1560
cctgtttgaa ttttgtatcc aagcaagtgt tacttaagta atttaagaga ctggttcatc 1620
gaaaaaataa aactccccaa attcccatag ctggtagact gtggtcacag ccacagtgca 1680
ctaagactat ctgctcagca cttctggtga cccaaaaggg tctgaggaca ggagctcaga 1740
gttgggtcag ctgtccaggt actcagggtt gtcacaggca aaactgctgg aactcagggc 1800
agcattgcaa atgccacgcc gctctcaggg ccccttgcct gccgctggaa ttaaacccac 1860
ccagatcttg gaaactctgc cctggaccct tctcaataag tccatgagaa atcaaactct 1920
ttcctttatg cgacactgga ttttccacaa agtaaaatca agatgagtaa agatgtggtt 1980
tctagatagt gcctgaaaaa gcagagacca tggtgtcagg cgtcaccact tgggcctata 2040
aaagctgcca caagacgcca aggccacaag ccacccagcc tatgcatccg ctcctcaatc 2100
ctctcctgtt ggcactgggc ctcatggcgc ttttgttgac cacggtcatt gctctcactt 2160
gccttggcgg ctttgcctcc ccaggccctg tgcctccctc tacagccctc agggagctca 2220
ttgaggagct ggtcaacatc acccagaacc agaaggtgag tgtcggctag ccagggtcct 2280
agctatgagg gctccagggt gggtgattcc caagatgagg tcatgagcag gctgggcctg 2340
gtcctaagat gcctgtaggt caggaaaaat ctccatggac caaggcccgg cccagccatg 2400
agggagagag gagctgggct gggggctca gcactgtgga tggacctatg gaggtgtctg 2460
gcagactccc cagggactac ctgctctcct ggcctggcct tgtctgccac tgccagctcc 2520
tactcagcca ttcctgaaca gaggacagca gagaagggtc cagcaccctc ccagaaccat 2580
gtggcatttg ccaactggat tttgaccata acaatgcagc cattctcccc agcaccatca 2640
taggcccgcc cttacaggag gattcgttag tagagtcgct ccttgcccca ctagtaacag 2700

ctcacatgtc ttgagcactg cttacaccag gcctggtgca cgtgctttat gtgtcatttc 2760
atcactgcca gccacctcaa gaggcaggta cgatgaaccc attctgctaa ggttcgtgag 2820
gttaagtgac agaggctgga ttcaagccag gcctggccaa caccagagtg tccatgctcc 2880
taactgcagt gttccctcac catcagaagg cagggcattt aatacaccag atccccaccg 2940
cctcccatct gatttgtctt ggtcaacatg gcccaggcca ctcctacttc actcgtcccc 3000
accctgaccc ttcccgcagg cccctgtcct cctgccctga ctatggcaag ccttgcatgc 3060
agcttgtccc ttactagtgg tgtcaatttt tttctctcag ctccaagacc ctaaacagtg 3120
ggacctcacc cctatgcctg ctgttcaaag cagaaaacga agctcaggaa tgctgagggg 3180
ctgccaggcc tgcctctgtg ccacaccagg gatgcttgtg gggcctgtgc tggggcagac 3240
ctggcctggg ctgccagggc aggcccacaa cccctgccag cactctgctc actgtcactt 3300
tgctcccaca gcgtccgctc tgcaatggca gcatggtatg gagcatcaac ctgacagctg 3360
gcatggtaag gacctttggg tgcagggagg atggggcaga ggctccaggc cttgggctta 3420
tcttctctga gcctcccttc catggctggg gttccaagca agcttcaagt gctctcctcc 3480
ctcccgccat aatctggccc cttcccgccc accacccaga ctcacctgcg ccaggcatct 3540
cagccccatc ttcctgcaga ctcacaaaag gcagctgccc aagcagggcc tgacccctcg 3600
gtgtcccctc cccacagtac tgtgcagccc tggaatccct gatcaacgtg tcaggctgca 3660
gtgccatcga gaagacccag aggatgctga gcggattctg cccgcacaag gtctcagctg 3720
gggtaaggca tcccccaccc tctcacaccc accctgcacc ccctcctgcc aaccctgggc 3780
tcgctgaagg gaagctggct gaatatccat ggtgtgtgtc cacccagggg tggggccatt 3840
gtggcagcag ggacgtggcc ttcgggattt acaggatctg ggctcaaggg ctcctaactc 3900
ctacctgggc ctcaatttcc acatctgtac agtagaggta ctaacagtac ccacctcatg 3960
gggacttccg tgaggactga atgagacagt ccctggaaag cccctggttt gtgcgagtcg 4020
tcccggcctc tggcgttcta ctcacgtgct gacctctttg tcctgcagca gttttccagc 4080
ttgcatgtcc gagacaccaa aatcgaggtg gcccagtttg taaaggacct gctcttacat 4140
ttaaagaaac tttttcgcga gggacggttc aactgaaact tcgaaagcat cattatttgc 4200
agagacagga cctgactatt gaagttgcag attcattttt ctttctgatg tcaaaaatgt 4260
cttgggtagg cgggaaggag ggttagggag ggggtaaaatt ccttagctta gacctcagcc 4320
tgtgctgccc gtcttcagcc tagccgacct cagccttccc cttgcccagg gctcagcctg 4380
gtgggcctcc tctgtccagg gccctgagct cggtggaccc agggatgaca tgtccctaca 4440
cccctcccct gccctagagc acactgtagc attacagtgg gtgccccccct tgccagacat 4500

```
gtggtgggac agggacccac ttcacacaca ggcaactgag gcagacagca gctcaggcac   4560
acttcttctt ggtcttattt attattgtgt gttatttaaa tgagtgtgtt tgtcaccgtt   4620
ggggattggg gaagactgtg gctgctggca cttggagcca agggttcaga gactcagggc   4680
cccagcacta aagcagtgga ccccaggagt ccctggtaat aagtactgtg tacagaattc   4740
```

**Description**

## FIELD OF THE INVENTION

The present invention relates to the fields of immunology and molecular biology. In particular, it relates to methods and reagents for detecting an individual's risk for autoimmune diseases. More specifically, it relates to methods and reagents for detecting an individual's increased or decreased risk for type 1 diabetes.

## DESCRIPTION OF RELATED ART

The immunological response to an antigen is mediated through the selective differentiation of CD4+ T helper precursor cells (Th0) to T helper type 1 (Th1) or T helper type 2 (Th2) effector cells, with functionally distinct patterns of cytokine (also described as lymphokine) secretion. Th1 cells secrete interleukin 2 (IL-2), IL-12, tumor necrosis factor (TNF), lymphotoxin (LT), and interferon gamma (IFN-γ) upon activation, and are primarily responsible for cell-mediated immunity such as delayed-type hypersensitivity. Th2 cells secrete IL-4, IL-5, IL-6, IL-9, and IL-13 upon activation, and are primarily responsible for extracellular defense mechanisms. The role of Th1 and Th2 cells is reviewed in Peltz, 1991, *Immunological Reviews,* **123:** 23-35.

IL4 and IL13 play a central role in IgE-dependent inflammatory reactions.

IL4 induces IgE antibody production by B Cells and further provides a regulatory function in the differentiation of Th0 to Th1 or Th2 effector cells by both promoting differentiation into Th2 cells and inhibiting differentiation into Th1 cells. IL13 also induces IgE antibody production by B Cells.

An SNP in the promotor region of IL4 is associated with type 1 diabetes (Ohkubo et al., Diabetologia, vol 44, suppl 1, page 80a, 2001).

IL4 and IL13 operate through the IL4 receptor ("IL4R"), found on both B and T cells, and the IL13R, found on B cells, respectively. The human IL4 receptor (IL4R) is a heterodimer comprising the IL4R α chain and the IL2 receptor γ chain. The α-chain of the IL4 receptor also serves as the α-chain of the IL13 receptor. IL4 binds to both IL4R and IL 13R through the IL4R α-chain and can activate both B and T cells, whereas IL 13 binds only to IL13R through the IL13R α1 chain and activates only T cells. Some IL4R SNPs are associated with type 1 diabetes (Bugawan et al., American Journal of Human Genetics, Vol 69, no 4, supplement, page 403, 2001).

## SUMMARY OF INVENTION

The present invention provides methods for detecting an individual's increased or decreased risk for an autoimmune disease such as type 1 diabetes, also known as insulin-dependent diabetes mellitus ("IDDM"). The present invention also provides kits, reagents and arrays useful for detecting an individual's risk for autoimmune diseases such as type 1 diabetes.

The present invention provides a method for detecting an individual's increased or decreased risk for an autoimmune disease such as type 1 diabetes by detecting the presence of a type 1 diabetes-associated polymorphism in the IL13 loci in a nucleic acid sample of the individual, wherein the presence of said polymorphism indicates the individual's increased risk for type 1 diabetes.

In one embodiment, an IL4R polymorphism and an IL13 polymorphism are detected. In another embodiment, an IL4 polymorphism and an IL13 polymorphism are detected. In another embodiment, an IL4R polymorphism, an IL4 polymorphism and an IL13 polymorphism are detected.

The IL13 polymorphism is selected from the IL13 polymorphisms listed in Table 21. The individual can belong to any race or population. In one embodiment, the individual is an Asian, preferably a Filipino, or a Caucasian.

The nucleic acid sample can be obtained from any part of the individual's body, including, but not limited to hair, skin, nails, tissues or bodily fluids such as saliva, blood, etc. The nucleic acid sample can, but need not, be amplified by any amplification method including, but not limited to, polymerase chain reaction ("PCR").

In one embodiment, the polymorphism can be a single nucleotide polymorphism ("SNP") in the IL13 loci. In another embodiment, specific haplotypes in the IL4R, IL4 and IL 13 loci as well as specific combinations of, and interactions between, SNPs at these loci can be indicative of an increased or a decreased risk to an autoimmune disease such as type 1 diabetes.

The polymorphism can be detected by any method known in the art for detecting the presence of a specific polymorphism in a nucleic acid sample. These methods include, but are not limited to, contacting the nucleic acid sample with one or more nucleic acid molecules that hybridize under stringent hybridization conditions to at least one type 1 diabetes-associated IL4R, IL4 or IL13 polymorphism and detecting the hybridization, detection by amplification of the nucleic acid sample by, for example, PCR, and by direct sequencing of the nucleic acid sample.

Another aspect of the invention relates to a kit useful for detecting the presence of a predisposing or a protective polymorphism in the IL4R, IL4 or IL 13 loci in a nucleic acid sample of an individual whose risk for type 1 diabetes is being assessed. The kit can comprise one or more oligonucleotides capable of detecting a predisposing or protective polymorphism in the IL4R, IL4 or IL13 loci as well as instructions for using the kit to detect susceptibility for an autoimmune disease such as type 1 diabetes. In preferred embodiments, the oligonucleotide or oligonucleotides each individually comprise a sequence that hybridizes under stringent hybridization conditions to at least one type 1 diabetes-associated IL4R, IL4 or IL 13 polymorphism. In some embodiments, the oligonucleotide or oligonucleotides each individually comprise a sequence that is fully complementary to a nucleic acid sequence comprising a type 1 diabetes-associated IL4R, IL4 or IL13 polymorphism.

The kit of the present invention comprises:

(a) one or more sequence-specific oligonucleotides each individually comprising a sequence that hybridizes under stringent conditions to the variant allele of type 1 diabetes-associated IL 13 polymorphism A(-1512)C or C(-1112) T as listed in Table 2; and

(b) instructions to use the kit to determine the individual's risk for type 1 diabetes.

In some embodiments, the oligonucleotide can be used to detect the presence of a type 1 diabetes-associated IL4R, IL4 or IL13 polymorphism by hybridizing to the polymorphism under stringent hybridizing conditions. In some embodiments, the oligonucleotide can be used as an extension primer in either an amplification reaction such as PCR or a sequencing reaction, wherein the type 1 diabetes-associated IL4R, IL4 or IL13 polymorphism is detected either by amplification or sequencing.

In certain embodiments, the kit can further comprise amplification or sequencing primers which can, but need not, be sequence-specific. The kit can also comprise reagents for labeling one or more of the oligonucleotides, or comprise labeled oligonucleotides. Optionally, the kit can comprise reagents to detect the label.

In some embodiments, the kit can comprise one or more oligonucleotides that can be used to detect the presence of two or more predisposing or protective IL4R, IL4 or IL13 polymorphisms or combinations of predisposing polymorphisms, protective polymorphisms or both.

In another aspect, the invention provides an array useful for detecting the presence of a predisposing or a protective IL4R, IL4 or IL13 polymorphism in a nucleic acid sample of an individual whose risk for type 1 diabetes is being assessed. The array can comprise one or more oligonucleotides capable of detecting a predisposing or protective IL4R, IL4 or IL 13 polymorphism. The oligonucleotides can be immobilized on a substrate, e.g., a membrane or glass. In preferred embodiments, the oligonucleotide or oligonucleotides each individually comprise a sequence that can hybridize under stringent hybridization conditions to a nucleic acid sequence comprising a type 1 diabetes-associated IL4R, IL4 or IL 13 polymorphism. In some embodiments, the oligonucleotide or oligonucleotides each individually comprise a sequence that is fully complementary to a nucleic acid sequence comprising a type 1 diabetes-associated IL4R, IL4 or IL13 polymorphism. The oligonucleotide or oligonucleotides can, but need not, be labeled. In some embodiments, the array can be a microarray.

In some embodiments, the array can comprise one or more oligonucleotides used to detect the presence of two or more predisposing or protective IL4R, IL4 or IL 13 polymorphisms or combinations of predisposing polymorphisms, protective polymorphisms or both.

In certain embodiments, an individual's risk for particular Th1-mediated diseases is diagnosed from the individual's IL4R, IL4 or IL 13 genotype. In a preferred embodiment, the Th1-mediated disease is type 1 diabetes. An individual who has at least one polymorphism statistically associated with type 1 diabetes possesses a factor contributing to either an increased or a decreased risk of a type 1 diabetes as compared to an individual without the polymorphism. The statistical association of IL4R, IL4 or IL13 polymorphisms (sequence variants) is shown in the examples.

The genotype can be determined using any method capable of identifying nucleotide variation consisting of single nucleotide polymorphic sites. The particular method used is not a critical aspect of the invention. A number of suitable methods are described below.

In one embodiment of the invention, genotyping is carried out using oligonucleotide probes specific to variant IL4R, IL4 or IL13 sequences. Preferably, a region of the IL4R, IL4 or IL13 genes which encompasses one or several polymorphic sites of interest is amplified prior to, or concurrent with, the hybridization of probes directed to such sites. Probe-based assays for the detection of sequence variants are well known in the art.

Alternatively, genotyping is carried out using allele-specific amplification or extension reactions, wherein allele-specific primers are used which support primer extension only if the targeted allele is present. Typically, an allele-specific primer hybridizes to the IL4R, IL4 or IL13 genes such that the 3' terminal nucleotide aligns with a polymorphic position. Allele-specific amplification reactions and allele-specific extension reactions are well known in the art.

## BRIEF DESCRIPTION OF THE FIGURES

FIG. 1 provides a schematic of a molecular haplotyping method;
FIG. 2 provides an illustration of epistasis between the IL4R SNPs and IL4 and IL13 SNPs.

## BRIEF DESCRIPTION OF THE TABLES

Table 1 provides the nucleotide sequence of the coding region of an IL4R (SEQ ID NO: 2);
Table 2 provides IL4R, IL4 and IL13 SNPs useful in the methods of the invention;
Table 3 provides probes used to identify IL4R polymorphisms (SEQ ID NO: 3-19);
Table 4 provides computationally estimated haplotype frequencies compared between Filipino controls and diabetics (SEQ ID NO: 20-24);
Table 5 provides genotypes of affected and nonaffected individuals;
Table 6 provides single nucleotide polymorphisms detected;
Table 7 provides amplicon primers and lengths (SEQ ID NO: 25-36);
Table 8 provides hybridization probes and titers (SEQ ID NO: 37-53);
Table 9 provides allele frequency of wildtype allele in HBDI founders;
Table 10 provides D' and $\Delta$ values for pairs of IL4R SNPs;
Table 11 A provides results of single locus TDT analysis;
Table 11B provides results of single locus TDT analysis;
Table 12 provides allele-specific PCR primers (SEQ ID NO: 54-62);
Table 13 provides IBD distributions for IL4R haplotypes;
Table 14A provides haplotype transmissions;
Table 14B provides haplotype transmissions;
Table 14C provides haplotype transmissions;
Table 15A provides SNP by SNP allele transmissions;
Table 15B provides SNP by SNP allele transmissions;
Table 16A provides a TDT analysis;
Table 16B provides a TDT analysis;
Table 16C provides a TDT analysis;
Table 17A provides a TDT analysis;
Table 17B provides a TDT analysis;
Table 18 provides allele frequencies in Filipino controls and diabetics;
Table 19 provides estimated haplotype frequencies;
Table 20 provides observed haplotype frequencies;
Table 21 provides allele frequencies in diabetics and controls;
Table 22 provides pairwise linkage disequilibrium values for IL4R SNPs;
Table 23 provides pairwise linkage disequilibrium values for IL4 and IL13 SNPs;
Table 24 provides genotype frequencies in patients and controls;
Table 25A provides IL-4R 7-SNP Haplotypes in Filipino diabetics and controls;
Table 25B provides estimated IL4R 10-SNP haplotype frequencies in diabetics and controls;
Table 26 provides estimated IL4 and IL13 5-SNP haplotype frequencies in diabetics and controls;
Table 27 provides correlation between genotype frequencies at IL4R SNPs and five IL4 and IL13 SNPs;
Table 28 provides epistatic interaction between IL4R SNPs and five IL4 and IL13 SNPs;
Table 29 provides probes used to identify IL4 and IL13 polymorphisms (SEQ ID NO: 63-68);
Table 30 provides amplicon primers and lengths for an IL4 promoter and IL13 SNPs (SEQ ID NO: 69-74);
Table 31 provides amplicon primers and lengths for IL4R promoter SNPs (SEQ ID NO: 75-80);
Table 32 provides amplicon primers and lengths for IL13 promoter SNPs (SEQ ID NO: 81-86)

## DETAILED DESCRIPTION OF THE INVENTION

The present invention provides methods, reagents and kits for detecting an individual's increased or decreased risk for the autoimmune disease is type 1 diabetes.

**Abbreviations and Terminology:**

The term "IL4R gene" or "IL4R locus" refers to the genomic nucleic acid sequence that encodes the alpha subunit of the interleukin 4 receptor protein. The nucleotide sequence of a gene, as used herein, encompasses coding regions, referred to as exons, intervening, non-coding regions, referred to as introns, and upstream or downstream regions. Upstream or downstream regions can include regions of the gene that are transcribed but not part of an intron or exon, or regions of the gene that comprise, for example, binding sites for factors that modulate gene transcription. The gene sequence of a Human mRNA for IL4R is provided at GenBank accession number X52425.1 (SEQ ID NO: 1). The coding region is provided as SEQ ID NO: 2. The genomic sequence for the IL4R gene is included in GenBank accession number AC004525.1 (SEQ ID NO: 88).

The term "IL4 gene" or "IL4 locus" refers to the genomic nucleic acid sequence that encodes the interleukin 4 protein. The nucleotide sequence of a gene, as used herein, encompasses coding regions, referred to as exons, intervening, non-coding regions, referred to as introns, and upstream or downstream regions. Upstream or downstream regions can include regions of the gene that are transcribed but not part of an intron or exon, or regions of the gene that comprise, for example, binding sites for factors that modulate gene transcription. The genomic sequence for the IL4 gene is provided at GenBank accession number M23442.1 (SEQ ID NO: 89).

The term "IL13 gene" or "IL13 locus" refers to the genomic nucleic acid sequence that encodes the interleukin 13 protein. The nucleotide sequence of a gene, as used herein, encompasses coding regions, referred to as exons, intervening, non-coding regions, referred to as introns, and upstream or downstream regions. Upstream or downstream regions can include regions of the gene that are transcribed but not part of an intron or exon, or regions of the gene that comprise, for example, binding sites for factors that modulate gene transcription. The genomic sequence for the IL13 gene is provided at GenBank accession number U10307.1 (SEQ ID NO: 90).

The term "allele", as used herein, refers to a sequence variant of the gene. Alleles are identified with respect to one or more polymorphic positions, with the rest of the gene sequence unspecified. For example, an IL4R allele may be defined by the nucleotide present at a single SNP, or by the nucleotides present at a plurality of SNPs. In certain embodiments of the invention, an IL4R is defined by the genotypes of 6, 7, 8 or 10 IL4R SNPs. Examples of such IL4R SNPs are provided in Table 2, below.

For convenience, the allele present at the higher or highest frequency in the population will be referred to as the wild-type allele; less frequent allele(s) will be referred to as mutant-allele(s). This designation of an allele as a mutant is meant solely to distinguish the allele from the wild-type allele and is not meant to indicate a change or loss of function.

The term "predisposing polymorphism" refers to a polymorphism that is positively associated with an autoimmune disease such as type 1 diabetes. The presence of a predisposing polymorphism in an individual could be indicative that the individual has an increased risk for the disease relative to an individual without the polymorphism.

The term "protective polymorphism" refers to a polymorphism that is negatively associated with an autoimmune disease such as type 1 diabetes. The presence of a protective polymorphism in an individual could be indicative that the individual has a decreased risk for the disease relative to an individual without the polymorphism.

The terms "polymorphic" and "polymorphism", as used herein, refer to the condition in which two or more variants of a specific genomic sequence, or the encoded amino acid sequence, can be found in a population. The terms refer either to the nucleic acid sequence or the encoded amino acid sequence; the use will be clear from the context. The polymorphic region or polymorphic site refers to a region of the nucleic acid where the nucleotide difference that distinguishes the variants occurs, or, for amino acid sequences, a region of the amino acid where the amino acid difference that distinguishes the protein variants occurs. As used herein, a "single nucleotide polymorphism", or SNP, refers to a polymorphic site consisting of a single nucleotide position.

"Odds Ratio" ("OR") refers to the ratio of the odds of the disease for individuals with the marker (allele or polymorphism) relative to the odds of the disease in individuals without the marker (allele or polymorphism).

"Linkage Disequilibrium" ("LD") refers to alleles at different loci that are not associated at random, i.e., not associated in proportion to their frequencies. If the alleles are in positive linkage disequilibrium, then the alleles occur together more often than expected assuming statistical independence. Conversely, if the alleles are in negative linkage disequilibrium, then the alleles occur together less often than expected assuming statistical independence.

The term "genotype" refers to a description of the alleles of a gene or genes contained in an individual or a sample. As used herein, no distinction is made between the genotype of an individual and the genotype of a sample originating from the individual. Although, typically, a genotype is determined from samples of diploid cells, a genotype can be determined from a sample of haploid cells, such as a sperm cell.

The term "haplotype" refers to a description of the variants of a gene or genes contained on a single chromosome, i.e., the genotype of a single chromosome. A haplotype is a set of maternally inherited alleles, or a set of paternally inherited alleles, at any locus.

The term "target region" refers to a region of a nucleic acid which is to be analyzed and usually includes at least one polymorphic region.

Individual amino acids in a sequence are represented herein as AN or NA, wherein A is the amino acid in the sequence and N is the position in the sequence. In the case that position N is polymorphic, it is convenient to designate the more frequent variant as $A_1N$ and the less frequent variant as $NA_2$. Alternatively, the polymorphic site, N, is represented as $A_1NA_2$, wherein $A_1$ is the amino acid in the more common variant and $A_2$ is the amino acid in the less common variant. Either the one-letter or three-letter codes are used for designating amino acids (see Lehninger, *BioChemistry 2nd ed.,* 1975,

Worth Publishers, Inc. New York, NY: pages 73-75). For example, 150V represents a single-amino-acid polymorphism at amino acid position 50, wherein isoleucine is the present in the more frequent protein variant in the population and valine is present in the less frequent variant. The amino acid positions are numbered based on the sequence of the mature IL4R protein, as described below.

"Stringent" as used herein refers to hybridization and wash conditions at 50 °C or higher. Other stringent hybridization conditions may also be selected. Generally, stringent conditions are selected to be about 5 °C lower than the thermal melting point (Tm) for the specific sequence at a defined ionic strength and pH. The Tm is the temperature (under defined ionic strength and pH) at which 50% of the target sequence hybridizes to a perfectly matched probe. Typically, stringent conditions will be those in which the salt concentration is at least about 0.02 molar at pH 7 and the temperature is at least about 50°C. As other factors may significantly affect the stringency of hybridization, including, among others, base composition, length of the nucleic acid strands, the presence of organic solvents, the extent of base mismatching, the combination of parameters is more important than the absolute measure of any one.

Representations of nucleotides and single nucleotide changes in DNA sequences are analogous. For example, A398G represents a single nucleotide polymorphism at nucleotide position 398, wherein adenine is the present in the more frequent (wild-type) allele in the population and guanine is present in the less frequent (mutant) allele. The nucleotide positions are numbered based on the IL4R coding region sequence provided as SEQ ID NO:2, shown below. It will be clear that in a double stranded form, the complementary strand of each allele will contain the complementary base at the polymorphic position.

Conventional techniques of molecular biology and nucleic acid chemistry, which are within the skill of the art, are fully explained in the literature. See, for example, Sambrook *et al*., 1989, Molecular Cloning - A Laboratory Manual, Cold Spring Harbor Laboratory, Cold Spring Harbor, New York; Oligonucleotide Synthesis (M.J. Gait, ed., 1984); Nucleic Acid Hybridization (B.D. Hames and S.J. Higgins. eds., 1984); the series, Methods in Enzymology (Academic Press, Inc.); and the series, Current Protocols in Human Genetics (Dracopoli *et al*., eds., 1984 with quarterly updates, John Wiley & Sons, Inc.).

**Association With Tyne 1 Diabetes**

As IL4R, IL4 or IL13 are a small component of the complex system of genes involved in an immune response, the effect of the IL4R, IL4 or ILI3 loci is expected to be small. Other factors, such as an individual's HLA genotype, may exert dominating effects which, in some cases, may mask the effect of the IL4R, IL4 or IL13 genotypes. For example, particular HLA genotypes are known to have a major effect on the likelihood of type 1 diabetes (*see* Noble *et al*., 1996, *Am. J. Hum. Genet*, 59:1134-1148). The IL4R, IL4 or IL13 genotypes are likely to be more informative as an indicator of predisposition towards type 1 diabetes among individuals who have HLA genotypes that confer neither increased nor decreased risk. Furthermore, because allele frequencies at other loci relevant to immune system-related diseases differ between populations and, thus, populations exhibit different risks for immune system-related diseases, it is expected that the effect of the IL4R, IL4 or IL13 genotypes may be of different magnitude in some populations. Although the contribution of the IL4R, IL4 or IL 13 genotypes may be relatively minor by itself, genotyping at the IL4R, IL4 or IL13 loci will contribute information that is, nevertheless, useful for a characterization of an individual's predisposition towards type 1 diabetes. The IL4R, IL4 or IL13 genotype information may be particularly useful when combined with genotype information from other loci.

**Methods for Detecting Risk for Autoimmune Diseases**

The present invention provides methods of determining an individual's risk for any autoimmune disease or condition or any Th-1 mediated disease. Such diseases or conditions include, but are not limited to, multiple sclerosis, myasthenia gravis, Crohn's disease, ulcerative colitis, primary biliary cirrhosis, type 1 diabetes mellitus (insulin dependent diabetes mellitus or IDDM), Grave's disease, autoimmune hemolytic anemia, pernicious anemia, autoimmune thrombocytopenia, vasculitides such as Wegener's granulomatosis, Behcet's disease, rheumatoid arthritis, systemic lupus erythematosus (lupus), scleroderma, systemic sclerosis, Gullian-Barre syndromes, Hashimoto's thyroiditis spondyloarthropathies such as ankylosing spondylitis, psoriasis, dermatitis herpetiformis, inflammatory bowel diseases, pemphigus vulgaris and vitiligo. In certain embodiments of the invention, the methods are used to determine an individual's risk for type 1 diabetes. Preferably, the individual is a human.

**Nucleic Acids**

Accordingly, one embodiment of the invention is an isolated nucleic acid molecule comprising a portion of the IL4R, IL4 or IL13 genes, their complements, or variants thereof. Preferably said variant comprises at least one of the polymorphisms identified herein. Even more preferably, said variant comprises at least one of the polymorphisms identified herein to be associated with type 1 diabetes. Thus, in one embodiment, the nucleic acid molecule comprises at least one of the IL4R, IL4, and/or IL13 polymorphisms provided in Table 2. In a further embodiment, the nucleic acid molecule comprises or consists of primers and probes specific to polymorphisms identified in the IL4R, IL4, or IL13 gene, including but not limited to SEQ ID NOS: 3-19, 25-36, 37-53, 54-62, 69-74, 75-80, and 81-86.

The isolated nucleic acid molecules may be RNA, mRNA, DNA, cDNA, and may be double- or single-stranded. They may encode the sense strand, the non-coding regions, or the antisense strand. The nucleic acid molecule can include all or a portion of the coding sequence of the gene and can further comprise additional non-coding regions such as introns and non-coding 3' and 5' sequences (including regulatory sequences for example). Additionally, the nucleic acid molecule can be fused to a marker sequence, for example, a sequence that encodes a polypeptide to assist in isolation or purification of the polypeptide.

An "isolated" nucleic acid molecule, as used herein, is one that is separated from nucleotide sequences which normally flank the nucleic acid molecule and/or has been completely or partially purified from other biological material (e.g., protein) normally associated with the nucleic acid.

The nucleic acid molecule can be fused to other coding or regulatory sequences and still be considered isolated. Thus, recombinant DNA contained in a vector is included in the definition of "isolated" as used herein. Also, isolated nucleic acid molecules include recombinant DNA molecules in heterologous host cells, as well as partially or substantially purified DNA molecules in solution. "Isolated" nucleic acid molecules also encompass *in vivo* and *in vitro* RNA transcripts of the DNA molecules of the present invention. An isolated nucleic acid molecule or nucleotide sequence can include a nucleic acid molecule or nucleotide sequence that is synthesized chemically or by recombinant means. Also, isolated polynucleotides include recombinant DNA molecules in heterologous organisms, as well as partially or substantially purified DNA molecules in solution. *In vivo* and *in vitro* RNA transcripts of the DNA molecules of the present invention are also encompassed by "isolated" nucleotide sequences. Such polynucleotides are useful in the manufacture of the encoded polypeptide, as probes for isolating homologous sequences (e.g., from other mammalian species), for gene mapping (e.g., by *in situ* hybridization with chromosomes), or for detecting expression of the gene in tissue (e.g., human tissue), such as by Northern blot analysis.

The nucleic acid molecules of the invention can comprise one or more modified nucleotide residues. The modification may be at the base, sugar and/or phosphate moiety and include, for example, halogenation, hydroxylation, alkylation, an attached linker and/or label. The modifications can further comprise, for example, labeling, methylation, internucleotide modifications such as uncharged linkages (*e.g.*, methyl phosphonates, phosphotriesters, phosphoamidates, carbamates), charged linkages (e.g., phosphorothioates, phosphorodithioates), pendent moieties (*e.g.*, polypeptides), intercalators (*e.g.*, acridine, psoralen), chelators, alkylators, and modified linkages (*e.g.,* alpha anomeric nucleic acids). Also included are synthetic molecules that mimic nucleic acid molecules in the ability to bind to a designated sequence via hydrogen bonding and other chemical interactions. Such molecules include, for example, those in which peptide linkages substitute for phosphate linkages in the backbone of the molecule.

In certain embodiments, nucleic acid molecules of the invention include, but are not limited to, IL4R, IL4 and/or IL13 mRNA, cDNA and/or genomic DNA molecules. The nucleotide sequence of the coding region of a IL4R mRNA is available from GenBank under accession number X52425.1, nucleotides 176-2653 are provided as SEQ ID NO: 2, shown in a 5' to 3' orientation in Table 1, below. The IL4R mRNA is provided as SEQ ID NO: 1. Although only one strand of the nucleic acid is shown in Table 1, those of skill in the art will recognize that SEQ ID NO: 1 and SEQ ID NO: 2 identify regions of double-stranded genomic nucleic acid, and that the sequences of both strands are fully specified by the sequence information provided. The genomic sequence for the IL4R gene is included in GenBank accession number AC004525.1 (SEQ ID NO: 88). The nucleotide sequence of the coding region of a IL4 mRNA is available from GenBank under accession number M23442.1 (SEQ ID NO: 89) and the nucleotide sequence of the coding region of a IL13 mRNA is available from GenBank under accession number U10307.1 (SEQ ID NO: 90).

Primers And Probes

By "oligonucleotide" is meant a single-stranded nucleotide polymer made of more than 2 nucleotide subunits covalently joined together. In one embodiment said oligonucleotides are between about 10 and 1000 nucleotide units, in a further embodiment, said oligonucleotides are between about 12 and 100 nucleotides units. The sugar groups of the nucleotide subunits may be ribose, deoxyribose or modified derivatives thereof such as o-methyl ribose. The nucleotide subunits of an oligonucleotide may be joined by phosphodiester linkages, phosphorothioate linkages, methyl phosphonate linkages or by other linkages, including but not limited to rare or non-naturally-occurring linkages, that do not prevent hybridization of the oligonucleotide. Furthermore, an oligonucleotide may have uncommon nucleotides or non-nucleotide moieties. An oligonucleotide as defined herein is a nucleic acid, preferably DNA, but may be RNA or have a combination of ribo- and deoxyribonucleotides covalently linked. Oligonucleotide probes and amplification oligonucleotides of a defined sequence may be produced by techniques known to those of ordinary skill in the art, such as by chemical or biochemical synthesis, and by in vitro or in vivo expression from recombinant nucleic acid molecules, e.g., bacterial or retroviral vectors. As used herein, an oligonucleotide does not consist of wild-type chromosomal DNA or the in vivo transcription products thereof.

Primer and probe sequences may comprise DNA, RNA (oligonucleotides - see above) or nucleic acid analogs such as uncharged nucleic acid analogs including but not limited to peptide nucleic acids (PNAs) which are disclosed in International Patent Application WO 92/20702 or morpholino analogs which are described in U.S. Pat. Nos. 5,185,444, 5,034,506, and 5, 142,047. Such sequences can routinely be synthesized using a variety of techniques currently available. For example, a sequence of DNA can be synthesized using conventional nucleotide phosphoramidite chemistry and the instruments available from Applied Biosystems, Inc, (Foster City, Calif.); DuPont, (Wilmington, Del.); or Milligen, (Bedford, Mass.). Similarly, and when desirable, the sequences can be labeled using methodologies well known in the art such as described in U.S. patent application numbers 5,464,746; 5,424,414; and 4,948,882.

Primers and Probes may be used in a variety of ways and may be defined by the specific use. For example, a "capture probe" is immobilized or can be immobilized on a solid support by any appropriate means, including, but not limited to: by covalent bonding, by adsorption, by hydrophobic and/or electrostatic interaction, or by direct synthesis on a solid support (see in particular patent application WO 92 10092). A "detection probe" may be labeled by means of a marker chosen, for example, from radioactive isotopes, enzymes, in particular enzymes capable of acting on a chromogenic, fluorigenic or luminescent substrate (in particular a peroxidase or an alkaline phosphatase), chromophoric chemical compounds, chromogenic, fluorigenic or luminescent compounds, analogues of nucleotide bases, and ligands such as biotin. A "primer" is a probe comprising, for example, from 10 to 100 nucleotide units and having a hybridization specificity under determined conditions for the initiation of an enzymatic polymerization, for example in an amplification technique such as PCR (Polymerase Chain Reaction), in a process of sequencing, in a method of reverse transcription and the like. One use of a probe is as a hybridization assay probe; probes may also be used as in vivo or in vitro therapeutic amplification oligomers or antisense agents to block or inhibit gene transcription, or translation in diseased, infected, or pathogenic cells.

All of the oligonucleotides, primers and probes of the present invention, whether hybridization assay probes, amplification oligonucleotides, or helper oligonucleotides, may be modified with chemical groups to enhance their performance or to facilitate the characterization of amplification products. For example, backbone-modified oligonucleotides such as those having phosphorothioate or methylphosphonate groups which render the oligonucleotides resistant to the nucleolytic activity of certain polymerases or to nuclease enzymes may allow the use of such enzymes in an amplification or other reaction. Another example of modification involves using non-nucleotide linkers (*e.g.*, Arnold, *et al.,* "Non-Nucleotide Linking Reagents for Nucleotide Probes", EP 0 313 219) incorporated between nucleotides in the nucleic acid chain which do not interfere with hybridization or the elongation of the primer. Amplification oligonucleotides may also contain mixtures of the desired modified and natural nucleotides.

The 3' end of an amplification oligonucleotide may be blocked to prevent initiation of DNA synthesis as described by McDonough, *et al.,* entitled "Nucleic Acid Sequence Amplification", WO94/03472 which enjoys common ownership with the present invention. A mixture of different 3' blocked amplification oligonucleotides, or of 3' blocked and unblocked oligonucleotides may increase the efficiency of nucleic acid amplification, as described therein.

The 5' end of the oligonucleotides may be modified to be resistant to the 5'-exonuclease activity present in some nucleic acid polymerases. Such modifications can be carried out by adding a non-nucleotide group to the terminal 5' nucleotide of the primer using techniques such as those described by Arnold, et al., supra, entitled "Non-Nucleotide Linking Reagents for Nucleotide Probes".

Once synthesized, selected oligonucleotide probes may be labeled by any of several well-known methods (*e.g.,* J. Sambrook, *supra*). Useful labels include radioisotopes as well as non-radioactive reporting groups. Isotopic labels include $^{3}H$, $^{35}S$, $^{32}P$, $^{125}I$, $^{57}Co$ and $^{14}C$. Isotopic labels can be introduced into the oligonucleotide by techniques known in the art such as nick translation, end labeling, second strand synthesis, the use of reverse transcription, and by chemical methods. When using radiolabeled probes hybridization can be detected by autoradiography, scintillation counting, or gamma counting. The detection method selected will depend upon the particular radioisotope used for labeling.

Non-isotopic materials can also be used for labeling and may be introduced internally into the nucleic acid sequence or at the end of the nucleic acid sequence. Modified nucleotides may be incorporated enzymatically or chemically. Chemical modifications of the probe may be performed during or after synthesis of the probe, for example, through the use of non-nucleotide linker groups as described by Arnold, *et al., supra* "Non-Nucleotide Linking Reagents for Nucleotide Probes,". Non-isotopic labels include fluorescent molecules, chemiluminescent molecules, enzymes, cofactors, enzyme substrates, haptens or other ligands.

In one embodiment, the probes are labeled with an acridinium ester. Acridinium ester labeling may be performed as described by Arnold et al., U.S. Pat. No. 5,185,439, entitled "Acridinium Ester Labeling and Purification of Nucleotide Probes," issued Feb. 9, 1993.

## Table 1

## SEQ ID NO: 2

```
1    atggggtggc tttgctctgg gctcctgttc cctgtgagct gcctggtcct gctgcaggtg
61   gcaagctctg ggaacatgaa ggtcttgcag gagcccacct gcgtctccga ctacatgagc
121  atctctactt gcgagtggaa gatgaatggt cccaccaatt gcagcaccga gctccgcctg
181  ttgtaccagc tggtttttct gctctccgaa gcccacacgt gtatccctga gaacaacgga
241  ggcgcggggt gcgtgtgcca cctgctcatg gatgacgtgg tcagtgcgga taactataca
301  ctggacctgt gggctgggca gcagctgctg tggaagggct ccttcaagcc cagcgagcat
361  gtgaaaccca gggccccagg aaacctgaca gttcacacca atgtctccga cactctgctg
421  ctgacctgga gcaacccgta tccccctgac aattacctgt ataatcatct cacctatgca
481  gtcaacattt ggagtgaaaa cgacccggca gatttcagaa tctataacgt gacctaccta
541  gaaccctccc tccgcatcgc agccagcacc ctgaagtctg ggatttccta cagggcacgg
601  gtgagggcct gggctcagtg ctataacacc acctggagtg agtggagccc cagcaccaag
661  tggcacaact cctacaggga gcccttcgag cagcacctcc tgctgggcgt cagcgtttcc
721  tgcattgtca tcctggccgt ctgcctgttg tgctatgtca gcatcaccaa gattaagaaa
781  gaatggtggg atcagattcc caacccagcc cgcagccgcc tcgtggctat aataatccag
841  gatgctcagg ggtcacagtg ggagaagcgg tcccgaggcc aggaaccagc caagtgccca
901  cactggaaga attgtcttac caagctcttg ccctgttttc tggagcacaa catgaaaagg
961  gatgaagatc ctcacaaggc tgccaaagag atgcctttcc agggctctgg aaaatcagca
1021 tggtgcccag tggagatcag caagacagtc ctctggccag agagcatcag cgtggtgcga
1081 tgtgtggagt tgtttgaggc ccccggtgag tgtgaggagg aggaggaggt agaggaagaa
1141 aaagggagct tctgtgcatc gcctgagagc agcagggatg acttccagga gggaagggag
1201 ggcattgtgg cccggctaac agagagcctg ttcctggacc tgctcggaga ggagaatggg
```

```
1261 ggcttttgcc agcaggacat gggggagtca tgccttcttc caccttcggg aagtacgagt
1321 gctcacatgc cctgggatga gttcccaagt gcagggccca aggaggcacc tccctggggc
1381 aaggagcagc ctctccacct ggagccaagt cctcctgcca gcccgaccca gagtccagac
1441 aacctgactt gcacagagac gcccctcgtc atcgcaggca accctgctta ccgcagcttc
1501 agcaactccc tgagccagtc accgtgtccc agagagctgg gtccagaccc actgctggcc
1561 agacacctgg aggaagtaga acccgagatg ccctgtgtcc cccagctctc tgagccaacc
1621 actgtgcccc aacctgagcc agaaacctgg gagcagatcc tccgccgaaa tgtcctccag
1681 catggggcag ctgcagcccc cgtctcggcc cccaccagtg gctatcagga gtttgtacat
1741 gcggtggagc agggtggcac ccaggccagt gcggtggtgg gcttgggtcc cccaggagag
1801 gctggttaca aggccttctc aagcctgctt gccagcagtg ctgtgtcccc agagaaatgt
1861 gggtttgggg ctagcagtgg ggaagagggg tataagcctt tccaagacct cattcctggc
1921 tgccctgggg accctgcccc agtccctgtc cccttgttca cctttggact ggacagggag
1981 ccacctcgca gtccgcagag ctcacatctc ccaagcagct ccccagagca cctgggtctg
2041 gagccggggg aaaaggtaga ggacatgcca aagcccccac ttccccagga gcaggccaca
2101 gacccccttg tggacagcct gggcagtggc attgtctact cagcccttac ctgccacctg
2161 tgcggccacc tgaaacagtg tcatggccag gaggatggtg gccagacccc tgtcatggcc
2221 agtccttgct gtggctgctg ctgtggagac aggtcctcgc cccctacaac cccccctgagg
2281 gccccagacc cctctccagg tggggttcca ctggaggcca gtctgtgtcc ggcctccctg
2341 gcaccctcgg gcatctcaga gaagagtaaa tcctcatcat ccttccatcc tgcccctggc
2401 aatgctcaga gctcaagcca gacccccaaa atcgtgaact ttgtctccgt gggacccaca
2461 tacatgaggg tctcttag
```

**SNPs**

In one aspect, a method for detecting an individual's increased or decreased risk for an autoimmune disease such as type 1 diabetes by detecting the presence of one or more IL4R, IL4 or IL13 SNPs in a nucleic acid sample of the individual, wherein the presence of said SNP(s) indicates the individual's increased or decreased risk for type 1 diabetes is described. The SNPs can be any SNPs in the IL4R, IL4 or IL13 loci including SNPs in exons, introns or upstream or downstream regions. Examples of such SNPs include, but are not limited to those provided in Table 2, below, and discussed in detail in the Examples. In one embodiment, the SNPs present in the IL4R, IL4 or IL13 loci are identified by genotyping the IL4R, IL4 or IL13 SNPs.

In certain embodiments, the genotype of one IL4R, IL4 or IL13 SNP can be used to determine an individual's risk for an autoimmune disease. In other embodiments, the genotypes of a plurality of IL4R, IL4 or IL13 SNPs can be used. For example, in certain embodiments, the genotypes of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 of the SNPs in Table 2 can be used to determine an individual's risk for an autoimmune disease. In other embodiments, certain combinations of SNPs at either the same or different loci can be used, as described in the Examples, below.

**Table 2 IL4R, IL4 and IL13 SNPs**

| **IL4R SNPs** | | | | | | | |
|---|---|---|---|---|---|---|---|
| dbSNP ID rs# | Exon* | Variation | **WT allele** | **Var allele** | Acc X52425.1 (cDNA) | AC004525.1 (genomic) | Formal SNP name |
| | P | C(-3223)T | **G** | **A** | NA | 128387 | G128387A |
| | P | T(-1914)C | **A** | **G** | NA | 127078 | A127078G |
| | 3 | 150V | **A** | **G** | 398 | 94272 | A398G |

(continued)

| IL4R SNPs | | | | | | | |
|---|---|---|---|---|---|---|---|
| dbSNP ID rs# | Exon* | Variation | **WT allele** | **Var allele** | Acc X52425.1 (cDNA) | AC004525.1 (genomic) | Formal SNP name |
| | 4 | N142N | **C** | **T** | 676 | 92548 | C676T |
| | 4 | C92516T | **C** | **T** | NA | 92516 | C92516T |
| | 4 | A92417T | **A** | **T** | NA | 92417 | A92417T |
| 2234896 | 7 | P249P | **C** | **G** | 997 | 80189 | C997G |
| 2234897 | 9 | F288F | **T** | **C** | 1114 | 76868 | T1114C |
| 1805011 | 9 | E375A | **A** | **C** | 1374 | 76608 | A1374C |
| | 9 | E375E | **G** | **A** | 1375 | 76607 | G1375A |
| 2234898 | 9 | L389L | **G** | **T** | 1417 | 76565 | G1417T |
| 1805012 | 9 | C406R | **T** | **C** | 1466 | 76516 | T1466C |
| 2234899 | 9 | C406C | **C** | **T** | 1468 | 76514 | C1468T |
| 2234900 | 9 | L408L | **T** | **C** | 1474 | 76508 | T1474C |
| 1805013 | 9 | S411L | **C** | **T** | 1482 | 76500 | C1482T |
| 1805015 | 9 | S478P | **T** | **C** | 1682 | 76300 | T1682C |
| 1801275 | 9 | Q551R | **A** | **G** | 1902 | 76080 | A1902G |
| | 9 | V554I | **G** | **A** | 1910 | 76072 | G1910A |
| | 9 | P650S | **C** | **T** | 2198 | 75784 | C2198T |
| 1805016 | 9 | S727A | **T** | **G** | 2429 | 75553 | T2429G |
| | 9 | G759G | **C** | **T** | 2567 | 75455 | C2567T |
| 1805014 | 9 | S761P | **T** | **C** | 2531 | 75451 | T2531C |
| | 9 | P774P | **T** | **C** | 2572 | 75410 | T2572C |
| 1049631 | 9 | 3'UTR | **G** | **A** | 3044 | 74938 | G3044A |
| 8832 | 9 | 3'UTR | **A** | **G** | 3289 | 74693 | A3289G |
| 8674 | 9 | 3'UTR | **C** | **T** | 3391 | 74581 | C3391T |
| **IL4 SNPs** | | | | | | | |
| SNP Description | Exon | Variation | **WT allele** | **Var allele** | Genbank Access # | Chromosomal Position | Formal SNP name |
| 5' (-524) | P | (-524) | **C** | **T** | M23442.1 | 5q31 | C582T |
| **IL13 SNPs** | | | | | | | |
| SNP Description | Exon | Variation | **WT allele** | **Var allele** | Genbank Access # | Chromosomal Position | Formal SNP name |
| (-1512) | P | (-1512) | **A** | **C** | U10307.1 | 5q31 | A(-1512)C |
| (-1112) | P | (-1112) | **C** | **T** | U10307.1 | 5q31 | C(-1112)T |
| Intron 3 | - | Intron 3 | **C** | **T** | U10307.1 | 5q31 | C4045T |

(continued)

| IL13 SNPs | | | | | | | |
|---|---|---|---|---|---|---|---|
| SNP Description | Exon | Variation | **WT allele** | **Var allele** | Genbank Access # | Chromosomal Position | Formal SNP name |
| R110Q | 4 | R110Q | **G** | **A** | U10307.1 | 5q31 | G4166A |
| P: Promoter region<br>* The exon numbering is based on the scheme disclosed by Ober *et al.*, 2000, *Am J Hum Genet* **66**:517-526. Other numbering schemes are available and one of skill in the art will be able to identify the SNP and the scheme is used. | | | | | | | |

## Genotyping Methods

In the described methods, the alleles present in a sample are identified by identifying the nucleotide present at one or more of the polymorphic sites. Any type of tissue containing IL4R, IL4 or IL 13 nucleic acid may be used for determining the IL4R, IL4 or IL13 genotypes of an individual. A number of methods are known in the art for identifying the nucleotide present at polymorphic sites. The particular method used to identify the genotype is not a critical aspect of the invention. Although considerations of performance, cost, and convenience will make particular methods more desirable than others, it will be clear that any method that can identify the nucleotide present will provide the information needed to identify the genotype. Preferred genotyping methods involve DNA sequencing, allele-specific amplification, or probe-based detection of amplified nucleic acid.

IL4R, IL4 or IL13 alleles can be identified by DNA sequencing methods, such as the chain termination method (Sanger *et al.*, 1977, *Proc. Natl. Acad Sci,.* **74**:5463-5467), which are well known in the art. In one embodiment, a subsequence of the gene encompassing the polymorphic site is amplified and either cloned into a suitable plasmid and then sequenced, or sequenced directly. PCR-based sequencing is described in U.S. Patent No. 5,075,216; Brow, in PCR Protocols, 1990, (Innis *et al.*, eds., Academic Press, San Diego), chapter 24; and Gyllensten, in PCR Technology, 1989 (Erlich, ed., Stockton Press, New York), chapter 5; each incorporated herein by reference. Typically, sequencing is carried out using one of the automated DNA sequencers which are commercially available from, for example, PE Biosystems (Foster City, CA), Pharmacia (Piscataway, NJ), Genomyx Corp. (Foster City, CA), LI-COR Biotech (Lincloln, NE), GeneSys technologies (Sauk City, WI), and Visible Genetics, Inc. (Toronto, Canada).

IL4R, IL4 or IL13 alleles can also be identified using amplification-based genotyping methods. Various nucleic acid amplification methods known in the art can be used in to detect nucleotide changes in a target nucleic acid. A preferred method is the polymerase chain reaction (PCR), which is now well known in the art, and described in U.S. Patent Nos. 4,683,195; 4,683,202; and 4,965,188. Examples of the numerous articles published describing methods and applications of PCR are found in PCR Applications, 1999, (Innis *et al.*, eds., Academic Press, San Diego), PCR Strategies, 1995, (Innis *et al.*, eds., Academic Press, San Diego); PCR Protocols, 1990, (Innis *et al.*, eds., Academic Press, San Diego); and PCR Technology, 1989, (Erlich, ed., Stockton Press, New York); Commercial vendors, such as PE Biosystems (Foster City, CA) market PCR reagents and publish PCR protocols.

Other suitable amplification methods include the ligase chain reaction (Wu and Wallace, 1988, *Genomics* **4**: 560-569); the strand displacement assay (Walker *et al.,* 1992, *Proc. Natl. Acad. Sci. USA* **89**:392-396, Walker *et al.* 1992, *Nucleic Acids Res.* **20**:1691-1696, and U.S. Patent No. 5,455,166); and several transcription-based amplification systems, including the methods described in U.S. Patent Nos. 5,437,990; 5,409,818; and 5,399,491; the transcription amplification system (TAS ) (Kwoh *et al.*, 1989, *Proc. Natl. Acad Sci. USA,* **86**:1173-1177); and self-sustained sequence replication (3SR) (Guatelli *et al.*, 1990, *Proc. Natl. Acad. Sci. USA,* **87**:1874-1878 and WO 92/08800); Alternatively, methods that amplify the probe to detectable levels can be used, such as Qβ-replicase amplification (Kramer *et al.*, 1989, *Nature,* **339**:401-402, and Lomeli *et al.,* 1989, *Clin. Chem.*, **35**:1826-1831). A review of known amplification methods is provided in Abramson *et al.,* 1993, *Current Opinion in Biotechnology,* **4**:41-47, incorporated herein by reference.

Genotyping also can also be carried out by detecting and analyzing IL4R, IL4 or IL13 mRNA under conditions when both, maternal and paternal, chromosomes are transcribed. Amplification of RNA can be carried out by first reverse-transcribing the target RNA using, for example, a viral reverse transcriptase, and then amplifying the resulting cDNA, or using a combined high-temperature reverse-transcription-polymerase chain reaction (RT-PCR), as described in U.S. Patent Nos. 5,310,652; 5,322,770; 5,561,058; 5,641,864; and 5,693,517 (see also Myers and Sigua, 1995, in PCR Strategies, *supra,* chapter 5).

IL4R, IL4 or IL13 alleles can also be identified using allele-specific amplification or primer extension methods, which are based on the inhibitory effect of a terminal primer mismatch on the ability of a DNA polymerase to extend the primer. To detect an allele sequence using an allele-specific amplification or extension-based method, a primer complementary to the IL4R, IL4 or IL13 genes is chosen such that the 3' terminal nucleotide hybridizes at the polymorphic position. In the presence of the allele to be identified, the primer matches the target sequence at the 3' terminus and primer is extended. In the presence of only the other allele, the primer has a 3' mismatch relative to the target sequence and primer extension is either eliminated or significantly reduced. Allele-specific amplification- or extension-based methods are described in, for example, U.S. Patent Nos. 5,137,806; 5,595,890; 5,639,611; and U.S. Patent No. 4,851,331.

Using allele-specific amplification-based genotyping, identification of the alleles requires only detection of the presence or absence of amplified target sequences. Methods for the detection of amplified target sequences are well known in the art. For example, gel electrophoresis (see Sambrook *et al.,* 1989, supra.) and the probe hybridization assays described above have been used widely to detect the presence of nucleic acids.

Allele-specific amplification-based methods of genotyping can facilitate the identification of haplotypes, as described in the examples. Essentially, the allele-specific amplification is used to amplify a region encompassing multiple polymorphic sites from only one of the two alleles in a heterozygous sample. The SNP variants present within the amplified sequence are then identified, such as by probe hybridization or sequencing.

An alternative probe-less method, referred to herein as a kinetic-PCR method, in which the generation of amplified nucleic acid is detected by monitoring the increase in the total amount of double-stranded DNA in the reaction mixture, is described in Higuchi *et al*., 1992, *Bio/Technology*, **10**:413-417; Higuchi *et al.,* 1993, *Bio/Technology*, **11**: 1026-1030; Higuchi and Watson, in PCR Applications, supra, Chapter 16; U.S. Patent Nos. 5,994,056 and 6,171,785; and European Patent Publication Nos. 487,218 and 512,334. The detection of double-stranded target DNA relies on the increased fluorescence that DNA-binding dyes, such as ethidium bromide, exhibit when bound to double-stranded DNA. The increase of double-stranded DNA resulting from the synthesis of target sequences results in an increase in the amount of dye bound to double-stranded DNA and a concomitant detectable increase in fluorescence. For genotyping using the kinetic-PCR methods, amplification reactions are carried out using a pair of primers specific for one of the alleles, such that each amplification can indicate the presence of a particular allele. By carrying out two amplifications, one using primers specific for the wild-type allele and one using primers specific for the mutant allele, the genotype of the sample with respect to that SNP can be determined. Similarly, by carrying out four amplifications, each with one of the possible pairs possible using allele specific primers for both the upstream and downstream primers, the genotype of the sample with respect to two SNPs can be determined. This gives haplotype information for a pair of SNPs.

Alleles can be also identified using probe-based methods, which rely on the difference in stability of hybridization duplexes formed between a probe and its corresponding target sequence comprising an IL4R, IL4 or IL13 allele. Under sufficiently stringent hybridization conditions, stable duplexes are formed only between a probe and its target allele sequence and not other allele sequences. The presence of stable hybridization duplexes can be detected by any of a number of well known methods. In general, it is preferable to amplify a nucleic acid encompassing a polymorphic site of interest prior to hybridization in order to facilitate detection. However, this is not necessary if sufficient nucleic acid can be obtained without amplification.

A probe suitable for use in the probe-based methods of the present invention, which contains a hybridizing region either substantially complementary or exactly complementary to a target region of SEQ ID NOS: 2, 88, 89 or 90 or the complement of SEQ ID NOS: 2, 88, 89 or 90, wherein the target region encompasses the polymorphic site, and exactly complementary to one of the two allele sequences at the polymorphic site, can be selected using the guidance provided herein and well known in the art. Similarly, suitable hybridization conditions, which depend on the exact size and sequence of the probe, can be selected empirically using the guidance provided herein and well known in the art. The use of oligonucleotide probes to detect nucleotide variations including single base pair differences in sequence is described in, for example, Conner *et al.*, 1983, *Proc. Natl. Acad. Sci. USA,* **80**:278-282, and U.S. Patent Nos. 5,468,613 and 5,604,099.

In preferred embodiments of the probe-based methods for determining the IL4R, IL4 or IL13 genotypes, multiple nucleic acid sequences from the IL4R, IL4 or IL13 genes which encompass the polymorphic sites are amplified and hybridized to a set of probes under sufficiently stringent hybridization conditions. The alleles present are inferred from the pattern of binding of the probes to the amplified target sequences. In this embodiment, amplification is carried out in order to provide sufficient nucleic acid for analysis by probe hybridization. Thus, primers are designed such that regions of the IL4R, IL4 or IL13 genes encompassing the polymorphic sites are amplified regardless of the allele present in the sample. Allele-independent amplification is achieved using primers which hybridize to conserved regions of the IL4R, IL4 or IL13 genes. The IL4R, IL4 or IL13 genes contain many invariant or monomorphic regions and suitable allele-independent primers can be selected routinely from SEQ ID NOS: 1, 88, 89 or 90. One of skill will recognize that, typically, experimental optimization of an amplification system is helpful.

Suitable assay formats for detecting hybrids formed between probes and target nucleic acid sequences in a sample are known in the art and include the immobilized target (dot-blot) format and immobilized probe (reverse dot-blot or line-blot) assay formats. Dot blot and reverse dot blot assay formats are described in U.S. Patent Nos. 5,310,893; 5,451,512; 5,468,613; and 5,604,099.

In a dot-blot format, amplified target DNA is immobilized on a solid support, such as a nylon membrane. The membrane-target complex is incubated with labeled probe under suitable hybridization conditions, unhybridized probe is removed by washing under suitably stringent conditions, and the membrane is monitored for the presence of bound probe. A preferred dot-blot detection assay is described in the examples.

In the reverse dot-blot (or line-blot) format, the probes are immobilized on a solid support, such as a nylon membrane or a microtiter plate. The target DNA is labeled, typically during amplification by the incorporation of labeled primers. One or both of the primers can be labeled. The membrane-probe complex is incubated with the labeled amplified target DNA under suitable hybridization conditions, unhybridized target DNA is removed by washing under suitably stringent conditions, and the membrane is monitored for the presence of bound target DNA. A preferred reverse line-blot detection assay is described in the examples.

Probe-based genotyping can be carried out using a "TaqMan" or "5'-nuclease assay," as described in U.S. Patent Nos. 5,210,015; 5,487,972; and 5,804,375; and Holland *et al.*, 1988, *Proc. Natl. Acad Sci. USA,* **88**:7276-7280. In the TaqMan assay, labeled detection probes that hybridize within the amplified region are added during the amplification reaction mixture. The probes are modified so as to prevent the probes from acting as primers for DNA synthesis. The amplification is carried out using a DNA polymerase that possesses 5' to 3' exonuclease activity, *e.g., Tth* DNA polymerase. During each synthesis step of the amplification, any probe which hybridizes to the target nucleic acid downstream from the primer being extended is degraded by the 5' to 3' exonuclease activity of the DNA polymerase. Thus, the synthesis of a new target strand also results in the degradation of a probe, and the accumulation of degradation product provides a measure of the synthesis of target sequences.

Any method suitable for detecting degradation product can be used in the TaqMan assay. In a preferred method, the detection probes are labeled with two fluorescent dyes, one of which is capable of quenching the fluorescence of the other dye. The dyes are attached to the probe, preferably one attached to the 5' terminus and the other is attached to an internal site, such that quenching occurs when the probe is in an unhybridized state and such that cleavage of the probe by the 5' to 3' exonuclease activity of the DNA polymerase occurs in between the two dyes. Amplification results in cleavage of the probe between the dyes with a concomitant elimination of quenching and an increase in the fluorescence observable from the initially quenched dye. The accumulation of degradation product is monitored by measuring the increase in reaction fluorescence. U.S. Patent Nos. 5,491,063 and 5,571,673 describe alternative methods for detecting the degradation of probe which occurs concomitant with amplification.

The TaqMan assay can be used with allele-specific amplification primers such that the probe is used only to detect the presence of amplified product. Such an assay is carried out as described for the kinetic-PCR-based methods described above. Alternatively, the TaqMan assay can be used with a target-specific probe.

Examples of other techniques that can be used for probe-based genotyping include, but are not limited to, Amplifluor™, Dye Binding-Intercalation, Fluorescence Resonance Energy Transfer (FRET), Hybridization Signal Amplification Method (HSAM), HYB Probes™, Invader/Cleavase Technology (Invader/CFLP™), Molecular Beacons™, Origen™, DNA-Based Ramification Amplification (RAM™), Rolling circle amplification (RCA™), Scorpions™, Strand displacement amplification (SDA).

The assay formats described above typically utilize labeled oligonucleotides to facilitate detection of the hybrid duplexes. Oligonucleotides can be labeled by incorporating a label detectable by spectroscopic, photochemical, biochemical, immunochemical, radiological, radiochemical or chemical means. Useful labels include $^{32}P$, fluorescent dyes, electron-dense reagents, enzymes (as commonly used in ELISAs), biotin, or haptens and proteins for which antisera or monoclonal antibodies are available. Labeled oligonucleotides of the invention can be synthesized and labeled using the techniques described above for synthesizing oligonucleotides. For example, a dot-blot assay can be carried out using probes labeled with biotin, as described in Levenson *et al.*, 1989, in *PCR Protocols: A Guide to Methods and Applications* (Innis *et al.*, eds., Academic Press. San Diego), pages 99-112. Following hybridization of the immobilized target DNA with the biotinylated probes under sequence-specific conditions, probes which remain bound are detected by first binding the biotin to avidin-horseradish peroxidase (A-HRP) or streptavidin-horseradish peroxidase (SA-HRP), which is then detected by carrying out a reaction in which the HRP catalyzes a color change of a chromogen.

Whatever the method for determining which oligonucleotides of the invention selectively hybridize to IL4R, IL4 or IL13 allelic sequences in a sample, the central feature of the typing method involves the identification of the IL4R, IL4 or IL13 alleles present in the sample by detecting the variant sequences present.

The present invention also related to methods for determining an individual's risk for type 1 diabetes comprising, detecting 2 or more, especially 6, 7, 8 or all 10 IL4R polymorphisms listed in Table 21 in a nucleic acid sample of the individual, wherein the presence of said polymorphism(s) indicates the individual's risk for type 1 diabetes.

The present invention also relates to a kit, a container unit comprising useful components for practicing the present method. A useful kit can contain oligonucleotide probes specific for IL4R, IL4 or IL13 alleles as well as instructions for their use to determine risk for an autoimmune disease such as type 1 diabetes. In some cases, detection probes may be fixed to an appropriate support membrane. The kit can also contain amplification primers for amplifying regions of the IL4R, IL4 or IL13 loci encompassing the polymorphic sites, as such primers are useful in the preferred embodiment of the invention. Alternatively, useful kits can contain a set of primers comprising an allele-specific primer for the specific amplification of IL4R, IL4 or IL13 alleles. Other optional components of the kits include additional reagents used in the genotyping methods as described herein. For example, a kit additionally can contain an agent to catalyze the synthesis of primer extension products, substrate nucleoside triphosphates, reagents for labeling and/or detecting nucleic acid (for example, an avidin-enzyme conjugate and enzyme substrate and chromogen if the label is biotin) and appropriate buffers for amplification or hybridization reactions.

The present invention also relates to an array, a support with immobilized oligonucleotides useful for practicing the present method. A useful array can contain oligonucleotide probes specific for IL4R, IL4, IL13 alleles or certain combinations of IL4R, IL4 and/or IL13 alleles. The oligonucleotides can be immobilized on a substrate, e.g., a membrane or glass. The oligonucleotides can, but need not, be labeled. In some embodiments, the array can be a micro-array. In some embodiments, the array can comprise one or more oligonucleotides used to detect the presence of two or more IL4R, IL4, IL13 alleles or certain combinations of IL4R, IL4 and/or IL13 alleles.

The examples of the present invention presented below are provided only for illustrative purposes and not to limit the scope of the invention. Numerous embodiments of the invention within the scope of the claims that follow the examples will be apparent to those of ordinary skill in the art from reading the foregoing text and following examples.

## EXAMPLES

### Example 1:

### Genotyping Protocol: Probe-Based Identification of IL4R, IL4 and IL13 Alleles

This example describes a method of genotyping SNPs in the IL4R, IL4 and IL13 loci that are associated with type 1 diabetes. Two different genotyping methods, line blot assays and kinetic thermocycling, were used, depending on the region and gene genotyped.

### Line Blot Assay for Identifying 8 IL4R SNPs, 1 IL4 SNP and 2 IL13 SNPs

Eight exemplary SNPs in the human IL4R gene (listed in Table 6), one exemplary SNP in the human IL4 gene (Table 2) and two exemplary SNPs in the human IL4R gene (Table 2) were genotyped using this method. Each SNP is described by its position in the reference GenBank accession sequence. For example, SNP 1 of Table 6 is found at position 398 of X52425.1 (SEQ ID NO: 1), where an "A" nucleotide is present. The variant allele at this position has a "G" nucleotide. The SNPs will be referred to by the SNP # in the subsequent text.

The regions of the IL4R, IL4 and IL13 genes that encompass the SNPs were amplified and the nucleotide present identified by probe hybridization. The probe detection was carried out using an immobilized probe (line blot) format.

*Amplicons and primers*

The pairs of primers used to amplify the regions encompassing the eight IL4R SNPs are listed in Table 7 (SEQ ID NO: 25-36) and those used to amplify the regions encompassing the IL4 SNP and two IL13 SNPs are listed in Table 30 (SEQ ID NO: 69-74). IL4R SNP numbers 3, 4, and 5 (Table 6) were co-amplified on the same 228 basepair fragment. The primers were modified at the 5' phosphate by conjugation with biotin. Reagents for synthesizing oligonucleotides with a biotin label attached to the 5' phosphate are commercially available from Clontech (Palo Alto, CA) and Glenn Research (Sterling, VA). A preferred reagent is Biotin-ON from Clontech.

*Amplification Primers*

Amplification of six regions of the IL4R gene, which encompass eight polymorphic sites, the one region of the IL4 gene, which encompass one polymorphic site, and the two regions of the IL13 gene, which encompass two polymorphic sites, was carried out using the primer pairs shown below. All primers are shown in the 5' to 3' orientation.

The following primers amplify a 114 base-pair region encompassing nucleotide position 398 the IL4R gene.

| | | |
|---|---|---|
| RR192B | (SEQ ID NO: 25) | CAGCCCCTGTGTCTGCAGA |
| RR193B | (SEQ ID NO: 31) | GTCCAGTGTATAGTTATCCGCACTGA |

The following primers amplify a 163 base-pair region encompassing nucleotide position 676.

| | | |
|---|---|---|
| DBM0177B | (SEQ ID NO: 26) | CTGACCTGGAGCAACCCGTA |
| DBM0178B | (SEQ ID NO: 32) | ACTGGGCCTCTGCTGGTCA |

The following primers amplify a 228 base-pair region encompassing nucleotide positions 1374, 1417, and 1466 of the IL4R gene.

| | | |
|---|---|---|
| DBM0023B | (SEQ ID NO: 27) | ATTGTGTGAGGAGGAGGAGGAGGTA |
| DBM0022B | (SEQ ID NO: 33) | GTTGGGCATGTGAGCACTCGTA |

The following primers amplify a 129 base-pair region encompassing nucleotide position 1682 of the IL4R gene.

| | | |
|---|---|---|
| DBM0097B | (SEQ ID NO: 28) | CTCGTCATCGCAGGCAA |
| DBM0098B | (SEQ ID NO: 34) | AGGGCATCTCGGGTTCTA |

The following primers amplify a 198 base-pair region encompassing nucleotide position 1902 of the IL4R gene.

| | | |
|---|---|---|
| RR200B | (SEQ ID NO: 29) | GCCGAAATGTCCTCCAGCA |
| RR178B | (SEQ ID NO: 35) | CCACATTTCTCTGGGGACACA |

The following primers amplify a 177 base-pair region encompassing nucleotide position 2531 of the IL4R gene.

| | | |
|---|---|---|
| DBM0112B | (SEQ ID NO: 30) | CCGGCCTCCCTGGCA |
| DBM0071B | (SEQ ID NO: 36) | GCAGACTCAGCAACAAGAGG |

The following primers amplify a 107 base-pair region encompassing nucleotide position 582 in the promoter region of the IL4 gene.

| | | |
|---|---|---|
| RR169B | (SEQ ID NO:69) | ACTAGGCCTCACCTGATACGA |
| RR170B | (SEQ ID NO: 72) | CATAGAGGCAGAATAACAGGCAGA |

The following primers amplify a 118 base-pair region encompassing nucleotide position 4045 in intron 3 of the IL13 gene.

| | | |
|---|---|---|
| DBM0165B | (SEQ ID NO: 70) | CTCGGACATGCAAGCTGGAA |
| DBM0166B | (SEQ ID NO: 73) | ACTGAATGAGACAGTCCCTGGA |

The following primers amplify a 187 base-pair region encompassing codon 4166 in exon 4 of the IL13 gene.

| | | |
|---|---|---|
| DBM0167B | (SEQIDNO:71) | AATCGAGGTGGCCCAGTTTGTA |
| DBM0168B | (SEQ ID NO: 74) | CCTAACCCTCCTTCCCGCCTA |

*Amplification*

The PCR amplification was carried out in a total reaction volume of 25-100 µl containing the following reagents:

0.2 ng/µl purified human genomic DNA
0.2 mM each primer
800 mM total dNTP (200 mM each dATP, dTTP, dCTP, dGTP)
70 mM KCl
12 mM Tris-HCl, pH 8.3
3 mM MgCl2,
0.25 units/µl AmpliTaq Gold™ DNA polymerase*

* developed and manufactured by Hoffmann-La Roche and commercially available from Applera (Foster City, CA).

Amplification was carried out in a GeneAmp™ PCR System 9600 thermal cycler (Applera, Foster City, CA), using the specific temperature cycling profile shown below.

| | | |
|---|---|---|
| Pre-reaction incubation: | | 94°C for 12.5 minutes |
| 33 cycles: | denature: | 95°C for 45 seconds |
| | anneal: | 61°C for 30 seconds |
| | extend: | 72°C for 45 seconds |
| Final extension: | | 72°C for 7 minutes |
| Hold: | | 10°C - 15°C |

*Detection Probes*

Preferred probes used to identify the nucleotides present at the 8 SNPs present in the amplified IL4R nucleic acids are described in Table 3. Two probes are shown for the detection of T1466; a mixture of the two probes was used. Preferred probes used to identify the nucleotides present at the one SNP present in the amplified IL4 nucleic acids and the two SNPs present in the amplified IL13 nucleic acids are described in Table 29. All probes are shown in the 5' to 3' orientation.

*Probe Hybridization Assay, Immobilized Probe Format*

In the immobilized probe format, the probes were immobilized to a solid support prior to being used in the hybridization. The probe-support complex was immersed in a solution containing denatured amplified nucleic acid (biotin labeled) to allow hybridization to occur. Unbound nucleic acid was removed by washing under stringent hybridization conditions, and nucleic acid remaining bound to the immobilized probes was detected using a chromogenic reaction. The details of the assay are described below.

For use in the immobilized probe detection format, described below, a moiety was attached to the 5' phosphate of the probe to facilitate immobilization on a solid support. See *Cheng et al.,* 1999, Genome Res **9**:936-949, incorporated herein by reference. Preferably, Bovine Serum Albumin (BSA) is attached to the 5' phosphate essentially as described by Tung *et al.*, 1991, *Bioconjugate Chem.,* **2**:464-465. Alternatively, a poly-T tail is added to the 5' end as described in U.S. Patent No 5,451,512.

The probes were applied in a linear format to sheets of nylon membrane (e.g., BioDyne™ B nylon filters, Pall Corp., Glen Cove, NY) using a Linear Striper and Multispense2000™ controller (IVEK, N. Springfield, VT). The detection of the wildtype allele of SNP #5 (table 6) was carried out using a mixture of two probes as listed; this mixture enables the detection of SNP #5 indiscriminately of another nearby SNP. Probe titers were chosen to achieve signal balance between the allelic variants; the titers used are provided in the table of probes, above. Each sheet was cut to strips between 0.35 and 0.5 cm in width. To denature the amplification products, 20 μl of amplification product (based on a 50 μl reaction) were added to 20 μl of denaturation solution (1.6% NaOH) and incubated at room temperature to complete denaturation.

The denatured amplification product (40 μl) was added to the well of a typing tray containing 3 ml of hybridization buffer (4X SSPE, 0.5% SDS) and the membrane strip.

Hybridizations were allowed to proceed for 15 minutes at 55°C in a rotating water bath. Following hybridization, the hybridization solution was aspirated, the strip was rinsed in 3 ml warm wash buffer (2X SSPE, 0.5% SDS) by gently rocking strips back and forth, and the wash buffer was aspirated. Following rinsing, the strips were incubated in 3 ml enzyme conjugate solution (3.3 ml hybridization buffer and 12 μl of strepavidin-horseradish peroxidase (SA-HRP)) in the rotating water bath for 5 minutes at 55°C. Then the strips were rinsed with wash buffer, as above, incubated in wash buffer at 55° for 12 minutes (stringent wash), and finally rinsed with wash buffer again.

Target nucleic acid, now HRP-labeled, which remains bound to the immobilized amplification product was visualized as follows. A color development solution was prepared by mixing 100 ml of citrate buffer (0.1 M Sodium Citrate, pH 5.0), 5 ml 3,3',5,5'-tetramethylbenzidine (TMB) solution (2 mg/ml TMB powder from Fluka, Milwaukee, WI, dissolved in 100% EtOH), and 100 μl of 3% hydrogen peroxide. The strips were first rinsed in 0.1 M sodium citrate (pH 5.0) for 5 minutes, then incubated in the color development solution with gentle agitation for 8 to 10 minutes at room temperature in the dark. The TMB, initially colorless, is converted by the target-bound HRP, in the presence of hydrogen peroxide, into a colored precipitate. The developed strips were rinsed in water for several minutes and immediately photographed.

**Kinetic Thermocycling to Identify 2 IL4R Promoter and 2 IL13 Promoter SNPs**

The two IL4R promoter and the two IL 13 promoter SNPs were genotyped using allele-specific PCR on a PE9700 thermal cycler (ABI) measuring SyBr Green (Molecular Probes) fluorescence (Higuchi, Fockler, Dollinger, Watson. Biotechnology 11:1026-30 (1993)). For each DNA, two ampifications were set up in parallel. One contained the common primer and one allele-specific primer; the other contained the common primer and the other allele-specific primer. The primers used to genotype the two IL4R promoter SNPs are provided in Table 31 and the primers used to genotype the two IL13 promoter SNPs are provided in Table 32. The amplification of the DNA with a particular allele-specific primer indicated the presence of the corresponding allele. An increase in the fluorescence of SyBr Green was indicative of the accumulation of amplification product. One of skill in the art will be able to correlate the change in fluorescence with the presence or absence of amplification product, and thus, the presence or absence of the corresponding allele.

The PCR amplification was carried out in a total reaction volume of 100 µl containing the following reagents:

| Volume (µl) | Component |
|---|---|
| 1 | 1 M Tris pH 8.0 |
| 12 | 25 mM MgCl2 |
| 1 | 100X dNTPs (50 mM each dA, dC, dG; 25 mM dT; 75 mM dU) |
| 1 | 20X SYBR Green |
| 1 | 200 µM ROX |
| 2 | 1 U/µl UNG |
| 2.5 | 80 % Glycerol |
| 4 | 100 % DMSO |
| 1 | 20 µM primer 1 |
| 1 | 20 µM primer 2 |
| 1 | 12 U/µl CEA2 Gold DNA Polymerase* |
| 2 | 10 ng/µl genomic DNA |
| to 100 µl | sterile water |

* Developed and manufactured by Roche Molecular Systems. Alternatively, 1µl of AmpliTaq Gold DNA Polymerase, also developed and manufactured by Roche Molecular Systems and sold commercially by Applied Biosystems, Inc, (Foster City, Calif.), can be used.

Amplification was carried out in a GeneAmp™ PCR System 9600 thermal cycler (Applera, Foster City, CA), using the specific temperature cycling profile shown below.

| | | |
|---|---|---|
| | | 2 minutes at 50 °C |
| Pre-reaction incubation: | | 12 minutes at 94 °C |
| 50 cycles: | denature: | 20 seconds at 95 °C |
| | anneal: | 20 seconds at 58 °C |
| Final extension: | | 5 minutes at 72 °C |

**Example 2: Association of IL4R SNPs with Type 1 Diabetes in HBDI Families**

This example demonstrates the association of IL4R SNPs with type 1 diabetes in HBDI families.

IL4R genotyping was carried out on individuals from 282 Caucasian families ascertained because they contained two offspring affected with type 1 diabetes. The IL4R genotypes of all individuals were determined. IL4R genotyping was carried out using a genotyping method essentially as described in Example 1. In addition to the 564 offspring (2 siblings in each of 282 families) in the affected sibling pairs on which ascertainment was based, there were 26 other affected children. There were 270 unaffected offspring among these families.

The family-based samples were provided as purified genomic DNA from the Human Biological Data Interchange (HBDI), which is a repository for cell lines from families affected with type 1 diabetes. All of the HBDI families used in this study are nuclear families with unaffected parents (genetically unrelated) and at least two affected siblings. These samples are described further in Noble *et al.*, 1996, *Am. J. Hum. Genet.* **59**:1134-1148.

It is known that the HLA genotype can have a significant effect, either increased or decreased depending on the genotype, on the risk for type 1 diabetes. In particular, individuals with the HLA DR genotype DR3-DQB1 *0201/DR4-DQB1*0302 (referred to as DR3/DR4 below) appear to be at the highest risk for type 1 diabetes (see Noble *et al.*, 1996, *Am. J. Hum. Genet.,* **59**:1134-1148). These high-risk individuals have about a 1 in 15 chance of being affected with type 1 diabetes. Because of the strong effect of this genotype on the likelihood of type 1 diabetes, the presence of the DR3-DQB1*0201/DR4-DQB1*0302 genotype could mask the contribution from the IL4R allelic variants.

Individuals within these families also were genotyped at the HLA DRB1 and DQB1 loci. Of the affected sibling pairs, both siblings have the DR3/DR4 genotype in 90 families. Neither affected sibling has the DR 3/4 genotype in 144 families. Exactly one of the affected pair has the DR 3/4 genotype in the remaining 48 families.

*Statistical analysis, methods and algorithms*

Since the eight SNPs in IL4R are both physically and genetically very closely linked to each other, the presence of a particular allele at a particular SNP is correlated with the presence of another particular allele at a nearby SNP. This non-random association of two or more SNPs' alleles is known as linkage disequilibrium (LD).

Linkage disequilibrium among the eight IL4R SNPs was assessed using the genotypes of the 282 pairs of parents. These 564 individuals are not related to each other except by marriage. A summary of the calculated frequency of the WT allele for each SNP in this group of 564 individuals (the "HBDI founders") is shown in Table 9.

The calculation of LD can be performed in several ways. Two complementary methods to assess LD between all pairs of IL4R SNP loci were used. In the first method, the values of two distinct but related metrics for LD, namely D' and Δ (Devlin and Risch 1995, *Genomics,* **29**(2): 311-22), using the Maximum Likelihood Estimation algorithm of Hill (Hill, 1974, *Heredity*, **33**(2): 229-39) were calculated. The values for D' and Δ for all pairs of IL4R SNPs are shown in Table 10, in the lower left triangular portion. Both D' and Δ can have values that range between -1 and +1. Values near +1 or -1 suggest strong linkage disequilibrium; values near zero indicate the absence of LD.

A second measure of LD uses a permutation test method implemented in the Arlequin program (Excoffier *et al.,* 1995, *Mol Biol Evol*, **12**:921-7, University of Geneva, CH) (Slatkin *et al.,* 1996, *Heredity,* **76**:377-83). This method maximizes the likelihood ratio statistic (S= -2log ($L_{H^*}/L_H$)) by permuting alleles and recalculating S over a large number of iterations until S is maximized. These iterations allow the determination of the null distribution of S, and thus the maximum S obtained can be converted into an exact P-value (significance level). These P-values are listed in the upper right triangular portion of Table 10.

Table 10 of pairwise LD shows that there is significant evidence for LD between SNPs 1 and 2, and among (all combinations of) SNPs 3, 4, 5, 6, 7 and 8. SNPs 3 through 8 are known to exist within 1200 basepairs of each other in a single exon (exon 9) of the IL4R gene, and the LD between these SNPs is evidence for very small genetic distances as well.

The Transmission Disequilibrium Test (TDT) of Spielman (Spielman and Ewens, 1996, *Am J Hum Genet*, **59** (5): 983-9; Spielman and Ewens, 1998, *Am J Hum Genet*, **62**(2): 450-8) was performed on the IL4R genotype data for the 282 affected sib pairs (namely, a family structure consisting of the two parents and the two affected children). The TDT was used to test for the association of the individual alleles of the eight IL4R SNPs to type 1 diabetes. The TDT assesses whether an allele is transmitted from heterozygous parents to their affected children at a frequency that is significantly different than expected by chance. Under the null hypothesis of no association of an allele with disease, a heterozygous parent will transmit or will not transmit an allele with equal frequency to an affected child. The significance of deviation from the null hypothesis can be assessed using the McNemar chi-squared test statistic (= (T-NT)^2/(T+NT), where T is the observed number of transmissions and NT is the observed number of non-transmissions). The significance (P-value) of the McNemar chi-squared test statistic is equal to the Pearson chi-squared statistic with one degree of freedom (Glantz *et al*., *Primer of biostatistics*., New York, McGraw-Hill Health Professions Division, 1997).

The results of the single SNP locus TDT results are shown in tables 11A and 11B. The TDT / S-TDT program (version 1.1) of Spielman was used to perform the counting of transmitted and non-transmitted alleles (Spielman, McGinnis *et al*., 1993, *Am J Hum Genet,* **52**(3): 506-16; Spielman and Ewens, 1998, *supra*). The table lists the observed transmissions of the wildtype allele at each SNP locus. Since these are biallelic polymorphisms, the transmission counts of the variant allele are equal to the non-transmissions of the wildtype allele.

The counts of transmissions and non-transmissions of alleles to the probands only shown in Table 11A do not quite reach statistical significance, at $\alpha$= 0.05. However, it is valid to count transmission events to all affected children. However, when the TDT is used in this way (or, for that matter, with more than one child per family), then a significant test statistic is evidence of linkage only, not of association and linkage. Table 11B shows the TDT analysis when 26 additional affected children are included. The results presented in Table 11B below show that there is a significant deviation from the expected transmission frequencies for alleles of SNPs 3, 4, 5 and 6. Inspection of the "% transmission" values for these SNPs indicates that the wildtype allele is transmitted to affected children at frequencies greater than the expectation of 50%.

The evidence for strong LD among the eight IL4R SNPs suggested that the transmission of the ordered set of alleles from each parent to each affected child in the HBDI cohort could be detected. This ordered set of alleles corresponds physically to one of the two parental chromosomes, and is called a haplotype. By inferring the parental haplotypes and their transmission or non-transmission to affected children, more statistical information is expected to be obtained than that from alleles alone.

Haplotypes were inferred using a combination of two methods. As the first step, the GeneHunter program (Falling Rain Genomics, Palo Alto, CA) (Kruglyak, *et al.*, 1996, *Am J Hum Genet,* **58**(6): 1347-63) was used as it very rapidly calculates haplotypes from genotype data from pedigrees. Each HBDI family pedigree was then inspected individually using the Cyrillic program (Cherwell Scientific Publishing, Palo Alto, CA), to resolve any ambiguous or unsupported haplotype assignments. Unambiguous and non-recombinant haplotypes could be confidently assigned in all but six of the 282 families. The haplotype data for these 276 families were used in subsequent data analysis.

The IL4R gene has the property that many of the SNPs reside within the 3'-most exon (exon 9), whose coding region is approximately 1.5 kb long. A method was developed for directly haplotyping up to five of these exon 9 alleles (namely, SNPs #3-7) without needing parental genotypes. As many of these SNPs direct changes to the amino acid sequence of the IL4R protein, different haplotypes encode different proteins with likely different functions.

Haplotypes, in an individual for which no parental genotypic information is known, can be inferred unambiguously only when at most one of the SNP sites of those is heterozygous. In other cases, the ambiguity must be resolved experimentally.

Two allele-specific primers with one common primer to perform PCR reactions (using Stoffel Gold™ polymerase) to separately amplify the DNA from each chromosome, as shown in Figure 1 below were used. The alleles on each amplicon were then detected by the same strip hybridization procedure, and the linked alleles called directly. The choice of allele-specific (colored or shaded arrows) and common (black arrows) primers depends on which SNP loci are heterozygous. The primers were modified at the 5' phosphate by conjugation with biotin, and are shown in Table 12 (SEQ ID NO: 54-62).

For each haplotyping assay, two PCR reactions were set up for each DNA to be tested. One reaction contained the common primer and the wildtype allele-specific primer, the other contained the common primer and the variant allele-specific primer. Each PCR reaction was made in a total reaction volume of 50-100 μl containing the following reagents:

0.2 ng/ml purified human genomic DNA
0.2 mM each primer
800 mM total dNTP (200 mM each dATP, dTTP, dCTP, dGTP)
10 mM KCl
10 mM Tris-HCl, pH 8.0
2.5 mM $MgCl_2$
0.12 units/ml Stoffel Gold™ DNA polymerase*
*developed and manufactured by Roche Molecular Systems.

Amplification was carried out in a GeneAmp™ PCR System 9600 thermal cycler (PE Biosystems, Foster City, CA), using the specific temperature cycling profile shown below:

| | | | |
|---|---|---|---|
| Pre-reaction incubation: | | 94°C for 12.5 minutes | |
| 33 cycles: | Denature: | 95°C, 45 seconds | |
| | Anneal: | 64°C, 30 seconds | |
| | Extend: | 72°C, 45 seconds | |
| | Final Extension: | | 72°C, 7 minutes. |
| | Hold: | | 10°C-15°C |

Following amplification, each PCR product reaction was denatured and separately used for hybridization to the membrane-bound probes as described above.

*Haplotype sharing in affected sibs*

Evidence for linkage of IL4R to type 1 diabetes (as opposed to association) can be assessed by the haplotype sharing method. This method assesses the distribution over all families of the number of chromosomes that are identical-by-descent (IBD) between the two affected siblings in each family. For example, if in a family, the father transmits the same one of his two IL4R haplotypes to both children, and the mother transmits the same one of her two IL4R haplotypes to both children, then the children are said to share two chromosomes IBD (or, to be IBD=2). If both parents transmit different IL4R haplotypes to their two children, the children are said to be IBD=0.

Under the null hypothesis of no linkage of IL4R to type 1 diabetes, the proportion of families IBD=0 is 25%, IBD=1 is 50% and IBD=2 is 25%, as expected by random assortment (see Table 13). Evidence for a statistically significant difference from this expectation can be assessed using the chi-square statistic.

Identity-by-descent (IBD) values of parental IL4R haplotypes in the affected sibs could be determined unambiguously in 256 families. In the rest of the families, one or both parents were homozygous and/or the parental source of the child's chromosomes could not be determined. The distribution of IBD is shown in Table 13.

It is known that the HLA genotype can have a significant effect, either increased or decreased depending on the genotype, on the risk for type 1 diabetes. In particular, individuals with the HLA DR genotype DR3-DQB1*0201/DR4-DQB1*0302 (referred to as DR3/4 below) appear to be at the highest risk for type 1 diabetes (see Noble, Valdes *et al.*, 1996), incorporated herein by reference). These high-risk individuals have about a 1 in 15 chance of being affected with type 1 diabetes. Because of the strong effect of this genotype on the likelihood of type 1 diabetes, the presence of the DR3/4 genotype could mask the contribution of IL4R alleles or haplotypes.

The distribution of IBD in families was stratified into two groups based on the DR3/4 genotype of the children. The first group contains the families in which one or both of the sibs are DR3/4 ("Either/both sib DR3/4", n=119). The second group contains the families where neither child is DR3/4 ("Neither sib DR3/4", n=137). The IBD distribution in these subgroups is shown in Table 13. There was no statistically significant departure from the expected distribution of IBD sharing in the "either/both sib DR3/4" subgroup of families. There is a statistically significant departure from the expected distribution of IBD sharing in the "neither sib DR3/4" subgroup of families (Table 13). This indicates that there is evidence for linkage of the IL4R loci to IDDM in the "neither sib DR3/4" families.

*Association by AFBAC*

Association of IL4R haplotypes with type 1 diabetes was assessed using the AFBAC (Affected Family Based Control) method (Thomson, G., 1995, *Am J Hum Genet* **57:**487-98). In essence, two groups of haplotypes, and the haplotype frequencies in the groups, are compared with each other as in a case/control scheme of sampling. These two groups are the case (transmitted) and the control (AFBAC) haplotypes.

The case haplotypes, namely those transmitted to the affected children, were collected and counted as follows. For every pair of siblings, regardless of the status of the parents (homozygote or heterozygote) all four transmitted chromosomes were counted. However, the haplotypes in the two siblings in a pair are not independent of each other. The way to make a statistically conservative and valid enumeration is to divide all counts by two.

The control (AFBAC) haplotypes are those that are never transmitted to the affected pair of children (Thomson, 1995). The AFBAC haplotypes permit an unbiased estimate of control haplotype frequencies. AFBACs can only be determined from heterozygous parents, and furthermore, only when the parent transmits one haplotype to both children; the other, never-transmitted haplotype is counted in the AFBAC population. The AFBAC population serves as a well-matched set of control haplotypes for the study.

Table 14A shows the comparison of transmitted and AFBAC frequencies for all HBDI haplotypes that were observed at least five times in the complete sample set. Each row represents data on an individual haplotype. However, in all 16 distinct haplotypes were observed in the HBDI data set, although some very rarely. The seven rarest haplotypes are grouped together in the "others" row. Each haplotype is listed by the ordered set of alleles (namely, from SNPs 1-2-3-4-5-6-7-8) present at each of the eight IL4R SNPs as described in Table 6. A "1" denotes the presence of the reference allele, a "2" the presence of the variant allele for each SNP. The "reference" allele for each SNP is that present in GenBank Accession X52425.1 as described in Table 6.

Tables 14B and 14C show the comparison of transmitted and AFBAC frequencies for all HBDI haplotypes seen in the "either/both sib DR3/4" and the "neither sib DR3/4" subgroups of families, respectively. These tables show that stratifying the families based on the DR3/4 genotype of the children permits the identification of haplotypes that are associated with IDDM. In particular, in the "neither sib DR3/4" subgroup one haplotype (labeled "2 1 2 2 2 2 2 1 ") is significantly underrepresented in the pool of transmitted chromosomes ($P < 0.005$).

From the transmitted and AFBAC haplotype frequency information in Tables 14B and 14C, one can derive by counting the frequencies of transmitted and AFBAC alleles. The locus-by-locus AFBAC analyses are shown in Tables 15A and 15B.

The data present in Tables 15A and 15B show that there statistically significant evidence, in the "neither sib DR3/4" subgroup of families, that alleles of SNPs numbers 3, 4 5, 6, and 7 are associated with IDDM. The evidence for association is especially strong for SNP #6. In the "either/both sib DR3/4" subgroup, there is the same trend of allelic association, although the trend does not quite reach statistical significance.

*Association by Haplotype-based TDT*

The TDT analysis can be utilized for determining the transmission (or non-transmission) of 8-locus haplotypes from parents to affected children, once the haplotypes have been inferred or assigned by molecular means. Tables 16A, B, and C summarize the TDT results for the HBDI families. Each haplotype is listed by the ordered set of alleles (namely, from SNPs 1-2-3-4-5-6-7-8) present at each of the eight IL4R SNPs as described in Table 6. A "1" denotes the presence of the reference allele, a "2" the presence of the variant allele for each SNP. The "reference" allele for each SNP is that present in GenBank Accession X52425.1 as described in Table 6. Table 16A counts informative transmission events only to one child (the proband) per family, Table 16B counts informative transmissions to the two primary affected children per family, and Table 16C counts informative transmissions to all affected children. The 8-locus haplotype TDT results reach statistical significance when all affected children (2 or more per family) are included.

The TDT analyses can be performed on families after stratifying for the DR3/4 genotype of the children. The summary of counts of informative transmissions to the two primary affected children per family, in the "either/both sib DR3/4" and the "neither sib DR3/4" subgroups of families, are shown in tables 17A and 17B respectively. Each haplotype is listed by the ordered set of alleles (namely, from SNPs 1-2-3-4-5-6-7-8) present at each of the eight IL4R SNPs as described in Table 6. A "1" denotes the presence of the reference allele, a "2" the presence of the variant allele for each SNP. The "reference" allele for each SNP is that present in GenBank Accession X52425.1 as described in Table 6.

As presented above, there is significant evidence of linkage of IDDM to IL4R in the "neither sib DR3/4" subgroup. The data in Table 17B indicate that there is significant evidence of association of IL4R haplotypes to IDDM, in the presence of this linkage. In particular, in the "neither sib DR3/4" subgroup one haplotype (labeled "2 1 2 2 2 2 2 1") is significantly under-transmitted to affected children.

**Example 3: Association of IL4R With Type 1 Diabetes in Filipino Samples**

This example demonstrates the association of IL4R SNPs with type 1 diabetes in a Filipino population.

As used in this section, "patients" refers to individuals with the disease, namely individuals with type 1 diabetes and "controls" refers normal individuals, those without the disease.

Ninety patients (n=90) were selected for this study from amongst the Filipino population. The patients included in the study were affected by type 1 diabetes as defined by the recent ADA classification (the Expert Committee on the Diagnosis and Classification of Diabetes Mellitus 1997). The patients were born in the Philippines and all had two Filipino parents. These patients had been characterized for C-peptide levels below 0.3mmol/l and for autoantibodies to islet cell autoantigens (Medici *et al.*, 1999, *Diabetes Care,* **9**:1458-62). Samples were also collected from ninety-four Filipino normal subjects without a family history for diabetes. This was the control group. All patients and controls were from the southern region of Luzon, Philippines. The study was approved by the local Ethics Committee and informed consent was given by patients. In addition, independent samples from a previous study of HLA class II loci in Filipinos (Bugawan *et al.,* 1994, Genetics, **54**:331-340) originating from the same region were used, following a statistical test of heterogeneity, to supplement the control samples. These comprised a total of 194 chromosomes taken from family and individual samples.

The individuals were genotyped as described above. The genotypes of the affected and unaffected individuals are shown in Table 4 (SEQ ID NO: 20-24). Both the actual numbers and the frequencies are provided for each genotype. The data (Table 5) confirm the presence of an association of IL4R SNP variants with type 1 diabetes.

*Statistical methods & algorithms*

Allele and haplotype frequencies between groups were compared using the z-test. Haplotype compositions and frequencies were estimated from the genotype data using the EM algorithm in the Arlequin program (L. Excoffier, University of Geneva, CH) (Excoffier *et al.,* 1995, *Mol Biol Evol,* **12**:921-7; Slatkin *et al.*, 1996, *Heredity*, **76:**377-83).

*Results*

The wildtype allele frequencies for each of the eight IL4R SNPs in the Filipino control and diabetic groups are shown in Table 18. Table 18 provides evidence that the allele frequencies for SNPs #3 and 4 are significantly different between the two groups, and suggests an association to IDDM.

It is also possible to infer and construct the multi-locus IL4R haplotypes in the Filipino subjects, either computationally by Maximum-likelihood estimation (MLE), or by using molecular haplotyping methods described previously. Table 19 lists the five most frequent computationally estimated haplotypes and their frequencies in the Filipino diabetics and controls, and presents the significance of the differences in frequencies. Each haplotype is listed by the ordered set of alleles (namely, from SNPs 1-2-3-4-5-6-7-8) present at each of the eight IL4R SNPs as described in Table 6. A "1" denotes the presence of the reference allele, a "2" the presence of the variant allele for each SNP. The "reference" allele for each SNP is that present in GenBank Accession X52425.1 as described in Table 6.

Table 20 lists the observed haplotypes as derived and inferred by molecular haplotyping; the unambiguous seven-locus haplotypes (SNP#1 allele not shown, as indicated by the "x") are compiled. Each haplotype is listed by the ordered set of alleles (namely, from SNPs 1-2-3-4-5-6-7-8) present at each of the eight IL4R SNPs as described in Table 6. A "1" denotes the presence of the reference allele, a "2" the presence of the variant allele for each SNP. The "reference" allele for each SNP is that present in GenBank Accession X52425.1 as described in Table 6. Tables 18 and 19 both provide evidence of a statistically significant difference in the frequency of one or more haplotypes between the Filipino control and diabetic populations, and support the presence of an association of IL4R to IDDM. In particular, the haplotype (labeled "x 1 2 2 2 2 2 1") is significantly underrepresented in the Filipino diabetics group.

**Example 4:**

**Association of IL4R, IL4 and IL13 SNPs With Type 1 Diabetes in Filipino Samples**

This example demonstrates the association of IL4R, IL4 and IL13 SNPs with type 1 diabetes in the same Filipino population as described above, in Example 3.

As used in this section, "patients" refers to individuals with the disease, namely individuals with type 1 diabetes and "controls" refers normal individuals, those without the disease.

The individuals were genotyped as described above.

**Individual SNPs**

The distributions of alleles at the individual SNPs in the IL4R locus (n = 10), the IL4 locus (n = 1) and the IL13 locus (n = 4) among patients and controls are shown in Table 21. Linkage disequilibrium patterns were estimated using maximum likelihood approaches from individual genotype data from unrelated individuals (Slatkin and Excoffier, 1996 *Heredity* **76**:377:383). The patterns of pairwise linkage disequilibrium (LD) for these SNPs inferred among the control population are shown in Tables 22 and 23. Among the individual IL4R SNPs, three (E375A, L389L, and C406R) showed a nominally significant association with type 1 diabetes while the variant allele at two additional SNPs (I50V, p = 0.062 and S478G, p = 0.064) was decreased among patients (Table 21).

The two promoter SNPs were not significantly associated with type 1 diabetes, although the variant allele of the -3223 SNP was slightly increased among patients (OR =1.45, p = 0.10). With the exception of this promoter SNP and the 150V SNP, with which it is in strong LD, the variant allele at each SNP was underrepresented among patients. Some of the polymorphic amino acid residues in this chain appear to be biologically important and affect IL-4 receptor signaling (Kruse *et al.,* 1999, *Immunology,* **96**:365-71).

Of the 10 IL4R SNPs typed, the L389L SNP showed the strongest association with type 1 diabetes in this population, with significantly lower frequencies among patients than controls (OR = 0.34; p = 0.001). Without being bound by theory, it is believed that because this is a silent (synonymous) polymorphism, it is unlikely that this SNP is responsible for the observed protective effect for type 1 diabetes. This SNP is in very strong LD (Table 22) with the nonsynonymous flanking SNPs (E375A, C406R, S478P and Q551R) and that these SNPs all show a trend toward protection (negative association). The L389L SNP is also in strong negative LD with the -3223 promoter SNP (Table 22).

In the comparison of genotypes at the individual IL4R SNPs (Table 24), the protective effect is dominant in that the heterozygote for IL4R 389 has an OR = 0.29. Among the individual SNPs on chromosome 5q31, only the variant alleles at the two IL13 promoter SNPs were increased among patients (OR = 1.58 and p = 0.05 for -1512 and OR = 1.49 and p = 0.12 for -1112) (Table 21) When genotype frequencies are compared, however, the IL13 R110Q showed a nominally significant association in this population (p = 0.03; Table 24). These data suggest that the variant homozygote, but not the heterozygote, may be at increased risk for type 1 diabetes. In Table 24, each haplotype is listed by the ordered set of alleles (namely, from SNPs 1-2-3-4-5-6-7-8) present at each of the eight IL4R SNPs as described in Table 6. The letters refer to the actual allele (nucleotide) present, as described in Table 6.

**Haplotypes**

**IL4R**

IL4R haplotypes were estimated based on an expectation-maximization (EM) method (Excoffier *et al.,* 1995, *Mol. Biol. Evol.,* **12**:921-927.) and were directly determined by molecular haplotyping methods, described in Example 4. The molecular haplotyping method allowed the unambiguous assignment of phase for 7 IL4R SNPs (C676T, A1374C, G1417T, T1466C, T1682C, A1902G and T2531C). Using molecular haplotyping of these 7 SNPs, 7 different IL4R haplotypes were determined in this population and their frequencies among patients and controls compared (Table 25A). In Table 25A each haplotype is listed by the ordered set of alleles (namely, from SNPs 2-3-4-5-6-7-8) present at seven of the eight IL4R SNPs as described in Table 6. The letters refer to the actual allele (nucleotide) present, as described in Table 6.

One specific haplotype (CCTCCGT)) was significantly underrepresented among patients (OR = 0.4, p = 0.013). This same haplotype was also found to be protective (significant negative association), by the TDT methods, in the HBDI families, as described in Example 2. This protective effect has thus been observed in two different populations and in two different study designs, namely case/control (Filipino) and TDT, in addition to the biological plausibility (i.e., functional consequences) of these SNPs. This strongly suggests that variants of the IL4R molecule influence the susceptibility to type 1 diabetes. In particular, this specific haplotype of IL4R appears to confer a dominant protective effect.

In the HBDI families, stratification based on the highest risk HLA genotype (HLA-DRB1*0301-DQB1*0201/HLA-DRB1*04-DQB1*0302) was necessary to demonstrate the protective effect of the IL4R haplotype. A significant negative association was found only among those families in which neither affected sib was DR3/4, presumably because the effect of the IL4R polymorphism was relatively modest compared to the risk conferred by this high risk HLA genotype, which confers a disease risk higher than DR3/3 or DR4/4 homozygotes. Among Filipinos, a significant protective effect of a specific IL4R haplotype was observed without stratification (Table 25). Without being bound by theory, this may reflect the absence among Filipinos of a higher risk associated with DR3/4 than with DR3/3 or DR4/4 genotypes. The lack of the "DR3/4 effect," well-established by many studies of Caucasian type 1 diabetes, can be attributed among Filipinos to the differing patterns of linkage disequilibrium of DQB1 alleles with DRB 1*04 alleles between Asians and Caucasians.

The molecular haplotyping approach used in this example did not assign phase for the two promoter SNPs and the 150V SNP in the IL4R locus. Consequently, the EM approach was used to estimate frequencies for 10-SNP haplotypes for these 3 individual SNPs and the 7-SNP haplotype previously determined by molecular methods (Table 25B). In Table 25B, each haplotype is listed by the ordered set of alleles (namely, from SNPs 1-2-3-4-5-6-7-8-9-10) present at each of the ten IL4R SNPs described in Table 21 (SNPs in order: C(-3223)T - C(-1914)T - I50V - N142N - E375A - L389L - C406R - S478P - Q551R - S761P). The letters refer to the actual allele (nucleotide) present, as described in Table 21.

Of the 17 10-SNP haplotypes with an estimated frequency > 1%, in either group, only one 10-SNP haplotype containing the protective 7-SNP haplotype (H5A or CCACCTCCGT) appeared strongly negatively associated (OR=0.0; 95%CI [0 - 0.5]; p = 0.001) with disease. Interestingly, the other haplotype which contained the same 7-SNP haplotype (H5B or CTA-CCTCCGT) was not significantly associated with disease (OR=0.66 p=0.33). This suggests that a specific combination of IL4R promoter SNPs with a particular coding sequence variant contributes to the risk for type 1 diabetes.

**IL4 and IL13**

The IL4 and the IL13 SNPs are in strong LD (Table 23). The estimated frequencies for these 5-SNP haplotypes were compared among patients and controls (Table 26). In Table 26, each haplotype is listed by the ordered set of alleles present at the one IL4 SNP and the four IL13 SNPs as described in Table 21. The order is IL4 C(-524)T - IL13 A(-1512)C - IL13 C(-1112)T - IL13 intron3 - IL13 R110Q. The letters refer to the actual allele (nucleotide) present, as described in Table 21.

The overall distributions were different (p = 0.005) and one haplotype, TCTTA, was strongly associated with type 1 diabetes (OR = 3.47, p = 0.004). Two other haplotypes showed a nominally significant association (p = 0.02 and 0.03). One surprising observation was that the IL13 haplotype CTTA appeared to be associated with disease only in combination with the T allele at the IL4 -524 promoter SNP because the CCTTA haplotype showed no disease association. These data could reflect LD between the associated 5-SNP haplotype with some nearby causal gene or suggest that a particular combination of a promoter variant at IL4 and promoter and coding variants at IL13 are responsible for an elevated type 1 diabetes risk (gene-gene interaction).

**Example 7:**

**Dependence of Risk for Type 1 Diabetes Conferred by IL4R SNPs on Genotype at IL4 and IL13 in Filipino Samples: Evidence for Epistasis**

This example demonstrates epistasis, the interaction between SNPs on the IL4R locus on chromosome 16 and those on the IL4 and IL13 loci on chromosome 5.

Because IL4 and IL13 both serve as ligands for a receptor composed, in part, of the IL4R alpha chain, there is a likelihood of gene-gene interactions between polymorphisms in the IL4R locus on chromosome 16p11 and the five SNPs in the IL4 and IL13 loci on chromosome 5q31. In one approach, the statistical independence for genotypes at the 10 IL4R SNPs and the genotypes at each of the IL4 and IL13 SNPs (Table 27) was examined. Gene by gene interactions at SNPs in different genes were evaluated by assessing whether the genotype frequencies at unlinked loci were independent (*i.e.,* the IL13 and IL4 SNPs on chromosome 5 and the IL4R SNPs on chromosome 16) among patients. These analyses were done for each pair of unlinked SNPs carrying out a chi-square test in contingency tables with marginals defined by genotype counts either in patients or controls. The chi-square values and the corresponding degrees of freedom for each IL4R SNP comparisons were summed and p-value of the sum of chi-squares computed.

No deviation from independence was found for these SNPs among controls but a significant deviation was found for the IL4 -524 promoter SNP (p = 0.001) and the IL13 intron 3 SNP (p = 0.019) among patients.

To assess whether the effect on type 1 diabetes susceptibility due to IL4R SNPs was modified by the IL4 or the IL 13 SNPs, epistasis was modeled using a logistic regression model (see below). For each of the five IL4 and IL13 SNPs, we tested whether the effect of the combined IL4R SNP genotypes on type 1 diabetes susceptibility differed depending on the IL4 and IL 13 SNPs. The results (Table 28) indicate that there is indeed an epistatic interaction between the IL4R genotypes and IL4 and IL13 genotypes. To address the issue of multiple comparisons, we carried out permutation analysis on this test. In 22/200 permuations one or more of the 5-SNP tests showed a p<0.035, in 13/200 one or more of the SNP tests had p<0.035 and another one had p<0.075. In 9/200 one or more of the 5-SNP tests showed a p<0.035, another had a p<0.075 and another had a p<0.135. Thus, the pattern observed in Table 28 has a probability of p<0.045. The conclusion from this is that the epistatic interaction observed between the IL4 and IL13 SNPs and the IL4R genotypes is statistically significant indicating that, in this data set, the genotypes in the IL4, IL13 region affect the genetic susceptibility to type 1 diabetes conferred by IL4R.

To illustrate this interaction, the odds ratios for individual IL4R SNPs as a function of the IL4 and IL 13 SNP genotype were also calculated. The differences among the Odds Ratios were greatest for the IL4R -3223 SNP and the four IL13 SNPs. The odds ratios with the 95% confidence intervals and the p values from the stratified contingency table analyses are shown in Figure 2. Figure 2 shows the Odds ratios, patient and control counts of specific IL4R and IL4, IL13 genotypes. Figure 2A shows the interaction of IL4R Ala375Glu with and IL4 -524 (promoter). Figure 2B shows the interaction of IL4R Gln551Arg with IL13 A-1512C. Figure 2C shows the interaction between IL4R C-3223T and IL13 A-1512C and Figure 2D shows the interaction between IL4R C-3223T and IL13 C-1112T. The p-value for the association of each genotype combination is shown above each odds ratios bar.

The most striking observation was that the IL4R -3223 SNP CT genotype had an OR of 8.55 (95%CI= 1.05, 69.8) when present with the TT IL13 -1112 genotype and an OR = 0.53 (95%CI= 0.29, 0.98) when present with the CC genotype. Without being bound by theory, the observation of an interaction between polymorphisms in the IL13 and IL4 genes and polymorphism in the gene encoding the receptor for the products of these two genes represents an interesting and biologically plausible hypothesis that, given the multiple comparisons, requires further testing. A recently published study of asthma patients reported a gene-gene interaction between IL4R and IL 13 in the determination of serum IgE levels (Howard *et al.,* 2002, *Am JHum Genet,* **70**:230-6).

As described above, the IL4R association data obtained in this Filipino case control study indicate that the 7-SNP haplotype, composed primarily of variant alleles at these SNPs, confers dominant protection to type 1 diabetes (Table 25A), consistent with our recent observations, based on TDT and AFBAC analysis in a set of multiplex Caucasian families (the HBDI registry). The replication of this observation in two different populations and in two different study designs strengthens this inference. The analysis of 10-SNP IL4R haplotypes among Filipinos suggests that a specific promoter variant in combination with specific coding sequence variants may be responsible for the observed protection (Table 25B). Several recent studies have shown that the reference or wild-type allele at several of these IL4R SNPs is associated with atopic asthma and increased IgE levels (Howard *et al.,* 2002, *Am J Hum Genet,* **70**:230-6, Sandford *et al.,* 2000, *J Allergy Clin Immunol,* **106**:135-40). Thus, it appears that the <u>same</u> alleles at IL4R SNPs confer an increased risk to a canonical Th1 (type 1 diabetes) and a Th2 disease (atopic asthma). Without being bound by theory, these associations argue against an effect on Th1/Th2 balance mediated by polymorphism in the IL4R gene and suggest instead that this polymorphism may influence some aspect of immune regulation and homeostasis in Th1 and Th2 pathways and possibly, B cell activation. Conceivably, the observed patterns of disease association reflect the effect of IL4R polymorphisms on the balance between the activation of Th1 and Th2 cells and that of T regulatory cells. In conclusion, the extent of risk for type 1 diabetes may be determined by specific combinations of variants at the IL4R locus and at the genes encoding its two ligands, IL4 and IL13.

<u>Calculations Performed to Achieve the Results of Table 28</u>

For each IL4R SNP, the homozygote genotype with the highest odds ratio was given a value of 2, the heterozygote was given a value of 1, the other homozygote was 0. A logistic regression was carried out on nine IL4R polymorphisms (S761P did not show the variant) in this way and a new numerical variable "il4r" was derived given by:

$$il4r = \alpha_1 G_{-3223} + \alpha_2 G_{-1914} + \alpha_3 G_{50} + \alpha_4 G_{142} + \alpha_5 G_{375} + \alpha_6 G_{389} + \alpha_7 G_{406} + \alpha_8 G_{478} + \alpha_9 G_{551}$$

where Gi denotes the genotype (0, 1 or 2) at the ith position and $\alpha j$ denotes the coefficient fitted by logistic regression. The coefficients fitted by the regression were $\alpha 1 = 0.368$; $\alpha 2 = 0.053$; $\alpha 3 = 0.37$; $\alpha 4 = 0.061$; $\alpha 5 = 0.66$; $\alpha 6 = 1.08$; $\alpha 7 = 0.57$; $\alpha 8 = 0.54$ and $\alpha 9 = 0.22$. Epistasis was then tested independently for each of the five chromosome 5 SNPs by fitting the following logistic regression model:

$$P(T1DM) = \exp(X) / (1 + \exp(X)) \text{ Where } X = C + \beta_1 il4r + \beta_2 G_{chr5i} + \beta_3 (il4r \cdot G_{chr5i})$$

and $G_{chr5i}$ is the genotype of one of the chromosome 5 SNPs (values 0, 1 or 2).

Permutation Analysis.

Because five different SNPs were compared, it was important to correct for multiple tests. However, a Bonferroni or a Dunn-Sidak correction was not appropriate since the IL4 and IL13 SNPs are not independent (see Table 23). Therefore permutation analysis was carried out, keeping constant the patient and control genotype frequencies, but permutating the IL4 andIL13 genotypes and the IL4R genotypes within the patient and within the control groups separately. In this way, only the epistatic interaction between the two genetic regions was tested and not the individual IL4 or IL13 and IL4R genetic associations. 200 permutations were carried testing for epistasis at all five chromosome 5 SNPs each time. Analyses were carried out using S-Plus version 6.0 Professional (Insightful Corporation).

Various embodiments of the invention have been described. The descriptions and examples are intended to be illustrative of the invention and not limiting.

**TABLE 3**

| Variant | Probe | Probe Sequence | Seq ID No: | titer ($\mu$l) |
|---|---|---|---|---|
| **A398** | DBM0081P | CCACACGTGTATCCCTGAGAA | 3 | 1.0 |
| **398G** | DBM0082P | TCTCAGGGACACACGTGTG | 4 | 4.0 |
| **C676** | DBM0172P | TGGAGTGAAAACGACCCGGCAG | 5 | 1.0 |
| **676T** | DBM0073P | CTGCCGGGTCATTTTCGCTCC | 6 | 1.0 |
| **A1374** | DBM0043RC | GAGGGAAGGGAGGGCATTGTG | 7 | 1.0 |
| **1374C** | DBM0139P | AGGGAAGGGCGGGCATTGT | 8 | 4.0 |
| **G1417** | DBM0124P | CTCTCCGAGCAGGTCCAGG | 9 | 1.0 |
| **1417T** | DBM0046RC | TCCTGGACCTTCTCGGAGAGG | 10 | 1.0 |
| **T1466** | DBM0076P<br>DBM0132P | AAGGTGGAAGAAGGCATGACTCC<br>AAGGTGGGAGAAGACATGACTCC | 11<br>12 | 1.0<br>1.0 |
| **1466C** | DBM0171P | GGAGTCACGTCTTCTCCTACCTT | 13 | 2.0 |
| **T1682** | DBM0157P | TGGCTCAGAGAGTTGCTGAAGC | 14 | 2.0 |
| **1682C** | DBM0094P | TTCAGCAACCCCCTGAGCC | 15 | 2.0 |
| **A1902** | KW86 | AGTGGCTATCAGGAGTTTGT | 16 | 1.6 |
| **1902G** | KW85 | AGTGGCTATCGGGAGTTTGT | 17 | 0.8 |
| **T2531** | DBM0080P. | CTCTTCTCTGAGATGCCCGAG | 18 | 0.5 |
| **2531C** | DBM0048RC | CTCGGGCATCCCAGAGAAGAG | 19 | 0.5 |

| haplotype | Seq ID No: | label | MLE frequency | MLE frequency | O.R | COUNT: control | COUNT: diabetics | Total count | exp control | exp diabetic | chi-square | P-value |
|---|---|---|---|---|---|---|---|---|---|---|---|---|
| ACATTTAT | 20 | H-1 | 0.318 | 0.329 | 1.1 | 59.7 | 58.5 | 118.2 | 60.7 | 57.5 | 0.033 | 0.855 |
| ACAGTTGT | 21 | H-6 | 0.074 | 0.074 | 1.0 | 14.0 | 13.2 | 27.2 | 14.0 | 13.2 | 0.000 | 0.987 |
| ACCTCCGT | 22 | H-3 | 0.145 | 0.058 | 0.4 | 27.3 | 10.3 | 37.6 | 19.3 | 18.3 | 6.727 | 0.009 |
| GCAGTTAT | 23 | H-2 | 0.390 | 0.441 | 1.2 | 73.3 | 78.5 | 151.8 | 78.0 | 73.8 | 0.582 | 0.445 |
| GCCGCCGT | 24 | H-10 | 0.032 | 0.051 | 1.6 | 6.0 | 9.0 | 15.0 | 7.7 | 7.3 | 0.776 | 0.378 |
| | | **n=** | **188** | **178** | | | | **366** | | | | |
| | | | | | | | | | | overall | 8.118 | 0.0987 |

Table 4: Computationally estimated haplotype frequencies compared between Filipino controls and diabetics.

**TABLE 5**

| SNP | Affected Genotypes (n=89) | | | Control Genotypes (n=94) | | |
|---|---|---|---|---|---|---|
| **A398G (50 I/V)** | AA=21 **(0.236)** | AG=40 **(0.449)** | GG=28 **(0.315)** | AA=32 **(0.340)** | AG=41 **(0.436)** | GG=21 **(0.223)** |
| **C676G (142 N/N)** | CC=89 **(1)** | | | CC=92 **(0.979)** | CG=2 **(0.021)** | |
| **A1374C (375 E/A)** | AA=70 **(0.787)** | AC=17 **(0.191)** | CC=2 **(0.023)** | AA=55 **(0.630)** | AC=30 **(0.341)** | CC=3 **(0.034)** |
| **G1417T (389 L/L)** | GG=78 **(0.876)** | GT=10 **(0.112)** | TT=1 **(0.011)** | GG=63 **(0.670)** | GT=29 **(0.309)** | TT=2 **(0.022)** |
| **T1466C (406 C/R)** | TT=70 **(0.787)** | TC=17 **(0.191)** | CC=2 **(0.023)** | TT=60 **(0.638)** | TC=32 **(0.340)** | CC=2 **(0.022)** |
| **T1682C (478 S/P)** | TT=70 **(0.787)** | TC=17 **(0.191)** | CC=2 **(0.023)** | TT=61 **(0.649)** | TC=31 **(0.330)** | CC=2 **(0.022)** |
| **A1902G (551 Q/R)** | AA=50 **(0.562)** | AG=35 **(0.393)** | GG=3 **(0.034)** | AA=50 **(0.532)** | AG=36 **(0.383)** | GG=8 **(0.085)** |
| **T2531C (761 S/P)** | TT=89 **(1)** | | | TT=94 **(1)** | | |

| SNP # | LOCUS | SNP | Variation | Genbank Access # |
|---|---|---|---|---|
| 1 | IL4R | A398G | I50V | X52425 |
| 2 | IL4R | C676T | N142N | X52425 |
| 3 | IL4R | A1374C | E375A | X52425 |
| 4 | IL4R | G1417T | L389L | X52425 |
| 5 | IL4R | T1466C | C406R | X52425 |
| 6 | IL4R | T1682C | S478P | X52425 |
| 7 | IL4R | A1902G | Q551R | X52425 |
| 8 | IL4R | T2531C | S761P | X52425 |
| **Table 6: SNPs detected** | | | | |

| SNP # | Amplicon size, bp | Amplicon Left Primer Name | Left primer sequence | Seq ID No: | Amplicon Right Primer Name | Right primer sequence | Seq ID No: |
|---|---|---|---|---|---|---|---|
| 1 | 114 | RR192B | CAGCCCCTGTGTCTGCAGA | 25 | RR193B | GTCCAGTGTATAGTTATCCGCACTGA | 31 |
| 2 | 163 | DBM0177B | CTGACCTGGAGCAACCCGTA | 26 | DBM0178B | ACTGGGCCTCTGCTGGTCA | 32 |
| 3,4,5 | 228 | DBM0023B | ATTGTGTGAGGAGGAGGAGGAGGTA | 27 | DBM0022B | GTTGGGCATGTGAGCACTCGTA | 33 |
| 6 | 129 | DBM0097B | CTCGTCATCGCAGGCAA | 28 | DBM0098B | AGGGCATCTCGGGTTCTA | 34 |
| 7 | 198 | RR200B | GCCGAAATGTCCTCCAGCA | 29 | RR178B | CCACATTTCTCTGGGGACACA | 35 |
| 8 | 177 | DBM0112B | CCGGCCTCCCTGGCA | 30 | DBM0071B | GCAGACTCAGCAACAAGAGG | 36 |
| Table 7: Amplicon primers and lengths | | | | | | | |

| SNP # | WT allele probe name | WT allele probe sequence | Seq ID No: | WT allele probe titer (μM) | Variant allele probe name | Variant allele probe sequence | Seq ID No: | Variant allele probe titer (μM) |
|---|---|---|---|---|---|---|---|---|
| 1 | **DBM0081P** | CCACACGTGTATCCCTGAGAA | 37 | 1.0 | **DBM0082P** | TCTCAGGGACACACGTGTG | 46 | 4.0 |
| 2 | **DBM0172P** | TGGAGTGAAAACGACCCGGCAG | 38 | 1.0 | **DBM0073P** | CTGCCGGGTCATTTTCGCTCC | 47 | 1.0 |
| 3 | **DBM0043RC** | GAGGGAAGGGAGGGCATTGTG | 39 | 1.0 | **DBM0139P** | AGGGAAGGGCGGGCATTGT | 48 | 4.0 |
| 4 | **DBM0124P** | CTCTCCGAGCAGGTCCAGG | 40 | 1.0 | **DBM0046RC** | TCCTGGACCTTCTCGGAGAGG | 49 | 1.0 |
| 5 | **DBM0076P +**<br>**DBM0132P** | AAGGTGGAAGAAGGCATGACTCC<br>AAGGTGGGAGAAGACATGACTCC | 41<br>42 | 1.0<br>1.0 | **DBM0171P** | GGAGTCACGTCTTCTCCTACCTT | 50 | 2.0 |
| 6 | **DBM0157P** | TGGCTCAGAGAGTTGCTGAAGC | 43 | 2.0 | **DBM0094P** | TTCAGCAACCCCCTGAGCC | 51 | 2.0 |
| 7 | **KW86** | AGTGGCTATCAGGAGTTTGT | 44 | 1.6 | **KW85** | AGTGGCTATCGGGAGTTTGT | 52 | 0.8 |
| 8 | **DBM0080P.** | CTCTTCTCTGAGATGCCCGAG | 45 | 0.5 | **DBM0048RC** | CTCGGGCATCCCAGAGAAGAG | 53 | 0.5 |
| Table 8: Hybridization probes and titers | | | | | | | | |

| SNP # | WT Allele Frequency |
|---|---|
| 1 | 0.53 |
| 2 | 0.90 |
| 3 | 0.89 |
| 4 | 0.89 |
| 5 | 0.89 |
| 6 | 0.83 |
| 7 | 0.79 |
| 8 | 0.99 |
| Table 9: Allele Frequency Of wildtype allele in HBDI founders | |

| SNP | 1 | 2 | 3 | 4 | 5 | 6 | 7 | 8 |
|---|---|---|---|---|---|---|---|---|
| 1 | | 0.000 | 0.067 | 0.068 | 0.069 | 0.000 | 0.000 | 0.003 |
| 2 | 1.00 (0.35) | | 0.000 | 0.000 | 0.000 | 0.000 | 0.000 | 0.000 |
| 3 | 0.23 (0.09) | -1.00 (-0.12) | | 0.000 | 0.000 | 0.000 | 0.000 | 0.779 |
| 4 | 0.23 (0.09) | -1.00 (-0.12) | 1.00 (1.00) | | 0.000 | 0.000 | 0.000 | 0.782 |
| 5 | 0.22 (0.08) | -1.00 (-0.11) | 1.00 (0.97) | 1.00 (0.97) | | 0.000 | 0.000 | 0.807 |
| 6 | 0.50 (0.24) | 0.39 (0.29) | 0.97 (0.78) | 0.97 (0.78) | 1.00 (0.78) | | 0.000 | 0.000 |
| 7 | 0.30 (0.16) | 0.36 (0.23) | 0.99 (0.70) | 0.99 (0.70) | 1.00 (0.68) | 0.99 (0.87) | | 0.000 |
| 8 | 1.00 (0.11) | 0.90 (0.29) | -0.58 (-0.02) | -0.58 (-0.02) | -0.49 (-0.02) | 1.00 (0.23) | | |

**Above diagonal:**
**Exact P-value by permu.test**

**Below diagonal:**
**D' (and Δ)**

**Table 10: Pairwise Linkage Disequilibrium Measures**

| SNP # | Transmissions of WT allele to affected (T) | Non-transmissions of WT allele to affected (NT) | Total observed Transmissions (T + NT) | McNemar chl-squared statistic | Significance |
|---|---|---|---|---|---|
| 1 | 144 | 138 | 282 | 0.128 | N.S. |
| 2 | 55 | 39 | 94 | 2.723 | N.S. |
| 3 | 82 | 46 | 108 | 2.370 | N.S. |
| 4 | 62 | 46 | 108 | 2.370 | N.S. |
| 5 | 61 | 46 | 107 | 2.103 | N.S. |
| 6 | 90 | 67 | 157 | 3.369 | N.S. |
| 7 | 103 | 81 | 184 | 2.630 | N.S. |

(continued)

| SNP # | Transmissions of WT allele to affected (T) | Non-transmissions of WT allele to affected (NT) | Total observed Transmissions (T + NT) | McNemar chl-squared statistic | Significance |
|---|---|---|---|---|---|
| 8 | 5 | 7 | 12 | 0.333 | N.S. |

Table 11A: Results of single locus TDT analysis, to the 282 probands only

| SNP # | Transmissions of WT allele to affected (T) | Non-transmissions of WT allele to affected (NT) | Total observed Transmissions (T + NT) | McNemar chi-squared statistic | Significance | % Transmission T/(T+NT) |
|---|---|---|---|---|---|---|
| 1 | 311 | 280 | 591 | 1.626 | N.S. | |
| 2 | 107 | 90 | 197 | 1.467 | N.S. | |
| 3 | 130 | 98 | 228 | 4.491 | P<0.05 | 57.0% |
| 4 | 130 | 98 | 228 | 4.491 | P<0.05 | 57.0% |
| 5 | 128 | 98 | 226 | 3.982 | P<0.05 | 56.6% |
| 6 | 189 | 1.49 | 338 | 4.734 | P<0.05 | 55.9% |
| 7 | 212 | 180 | 392 | 2.612 | N.S. | |
| 8 | 12 | 13 | 25 | 0.040 | N.S. | |

Table 11B: Results of single locus TDT analysis, to 564 primary affected and 26 additional children

| SNP detected allele-specifically | Amplicon Size, bp | Other SNPs spanned by amplicon | Common Primer Name | Common Primer Sequences | Seq ID No: | Wildtype Allele-Specific Primer Sequence | Wildtype Allele-specific Primr Sequence | Seq ID No: | Variant Allele-Specific Primer Name | Variant Allele-specific Primer Sequence | Seq ID No: |
|---|---|---|---|---|---|---|---|---|---|---|---|
| [illegible] | 678 | 4,5,6,7 | RR178B | CCACATTTCTCTGGGGACACA | 54 | DBM0149B | TTCCAGGAGGGAAGGGA | 57 | DBM0150B | TTCCAGGAGGGAAGGGC | 60 |
| 7 | 639 | 3,4,5,6 | DBM0023B | ATTGTGTGAGGAGGAGGAGGAGGTA | 55 | DBN9155B | CACCGCATGTACAAACTCCT | 58 | DBM0156B | CACCGCATGTACAAACTCCC | 61 |
| 6 | 413 | 3,4,5 | DBM0023B | ATTGTGTGAGGAGGAGGAGGAGGTA | 56 | DBM0194B | GGTGACTGGCTCAGGGA | 59 | DBM0195B | GGTGACTGGCTCAGGGG | 62 |

Table 12: Allele-specific PCR primers and amplicon lengths

| Group (n) | IBD=2 | IBD=1 | IBD=0 | P-value |
|---|---|---|---|---|
| All families (256) | 30.7% | 47.9% | 21.5% | N.S. |
| Either/both sib DR3/4 (119) | 26.1% | 50.4% | 23.5% | N.S. |
| Neither sib DR3/4 (137) | 34.7% | 45.6% | 19.7% | 0.03 |
| *Expectation* | *25%* | *50%* | *25%* | |

Table 13: IBD Distributions for IL4R haplotypes

| | Counts | | | Observed Frequency | | Chi-squared Statistic | p-value |
|---|---|---|---|---|---|---|---|
| IL4R 8-locus Haplotype | Transmitted | AFBAC | Total | Transmitted | AFBAC | | |
| 1 1 1 1 1 1 1 1 | 259.5 | 109.5 | 369 | 46.3% | 41.8% | 0.82 | 0.36 |
| 2 1 1 1 1 1 1 1 | 170.5 | 71.5 | 242 | 30.4% | 27.3% | 0.60 | 0.44 |
| 2 1 2 2 2 2 2 1 | 29.5 | 23.5 | 53 | 5.3% | 9.0% | 3.79 | 0.05 |

(continued)

| IL4R 8-locus Haplotype | Counts | | | Observed Frequency | | Chi-squared Statistic | p-value |
|---|---|---|---|---|---|---|---|
| | Transmitted | AFBAC | Total | Transmitted | AFBAC | | |
| 2 2 1 1 1 1 1 1 | 23.5 | 13 | 36.5 | 4.2% | 5.0% | 0.24 | 0.63 |
| 1 1 2 2 2 2 2 1 | 23 | 12.5 | 35.5 | 4.1% | 4.8% | 0.18 | 0.67 |
| 2 2 1 1 1 2 2 1 | 20.5 | 14 | 34.5 | 3.7% | 5.3% | 1.20 | 0.27 |
| 1 1 1 1 1 1 2 1 | 19 | 9 | 28 | 3.4% | 3.4% | 0.00 | 0.98 |
| 2 1 1 1 1 1 2 1 | 3.5 | 3 | 6.5 | 0.6% | 1.1% | 0.61 | 0.43 |
| 2 2 1 1 1 2 2 2 | 4.5 | 2 | 6.5 | 0.8% | 0.8% | 0.00 | 0.95 |
| Others | 6.5 | 4 | 10.5 | 1.2% | 1.5% | 0.19 | 0.67 |
| TOTAL | 560 | 262 | 822 | | | 7.64 | 0.94 |

Table 14A: Haplotype transmissions by AFBAC method: all families

| IL4R 8-locus Haplotype | Counts | | | Observed Frequency | | Chi-squared Statistic | p-value |
|---|---|---|---|---|---|---|---|
| | Transmitted | AFBAC | Total | Transmitted | AFBAC | | |
| 1 1 1 1 1 1 1 1 | 254 | 50.5 | 304.5 | 46.7% | 41.6% | 0.57 | 0.45 |
| 2 1 1 1 1 1 1 1 | 156 | 30 | 186 | 28.7% | 24.7% | 0.56 | 0.45 |
| 2 1 2 2 2 2 2 1 | 35 | 11 | 46 | 6.4% | 9.1% | 0.99 | 0.32 |
| 2 2 1 1 1 1 1 1 | 24 | 7 | 31 | 4.4% | 5.8% | 0.39 | 0.53 |
| 1 1 1 1 1 1 2 1 | 20 | 6 | 26 | 3.7% | 4.9% | 0.40 | 0.52 |
| 1 1 2 2 2 2 2 1 | 18 | 7 | 25 | 3.3% | 5.8% | 1.59 | 0.21 |
| 2 2 1 1 1 2 2 1 | 18 | 4 | 22 | 3.3% | 3.3% | 0.00 | 0.99 |
| 2 2 1 1 1 2 2 2 | 8 | 2 | 10 | 1.5% | 1.6% | 0.02 | 0.89 |
| 2 2 1 1 1 2 2 2 | 8 | 1 | 9 | 1.5% | 0.8% | 0.31 | 0.58 |
| 2 1 1 1 1 1 2 1 | 1 | 2 | 3 | 0.2% | 1.6% | 4.71 | 0.03 |
| 2 1 1 1 1 2 2 1 | 2 | 1 | 3 | 0.4% | 0.8% | 0.46 | 0.50 |
| TOTAL | 544 | 121.5 | 665.5 | | | 10.00 | 0.44 |

Table 14B: Haplotype transmissions by AFBAC method: "Either/both sib DR3/4" subgroup of familes

| IL4R 8-locus Haplotype | Counts | | | Observed Frequency | | Chi-squared Statistic | p-value |
|---|---|---|---|---|---|---|---|
| | Transmitted | AFBAC | Total | Transmitted | AFBAC | | |
| 1 1 1 1 1 1 1 1 | 267 | 62.5 | 329.5 | 45.7% | 40.7% | 0.68 | 0.409 |
| 2 1 1 1 1 1 1 1 | 185 | 43 | 228 | 31.7% | 28.0% | 0.53 | 0.467 |
| 2 1 2 2 2 2 2 1 | 24 | 15.5 | 39.5 | 4.1% | 10.1% | 8.14 | 0.004 |
| 1 1 2 2 2 2 2 1 | 30 | 6 | 36 | 5.1% | 3.9% | 0.38 | 0.540 |
| 2 2 1 1 1 2 2 1 | 23 | 10 | 33 | 3.9% | 6.5% | 1.80 | 0.179 |

(continued)

| | Counts | | | Observed Frequency | | Chi-squared Statistic | p-value |
|---|---|---|---|---|---|---|---|
| IL4R 8-locus Haplotype | Transmitted | AFBAC | Total | Transmitted | AFBAC | | |
| 2 2 1 1 1 1 11 1 | 23 | 7.5 | 30.5 | 3.9% | 4.9% | 0.26 | 0.607 |
| 1 1 1 1 1 1 2 1 | 17 | 5 | 22 | 2.9% | 3.3% | 0.05 | 0.825 |
| 2 1 1 1 1 1 2 1 | 8 | 1 | 9 | 1.4% | 0.7% | 0.51 | 0.473 |
| 2 2 1 1 1 2 2 2 | 3 | 1 | 4 | 0.5% | 0.7% | 0.04 | 0.837 |
| 1 1 1 1 1 2 2 1 | 2 | 1 | 3 | 0.3% | 0.7% | 0.29 | 0.593 |
| 2 1 2 2 1 1 2 1 | 2 | 1 | 3 | 0.3% | 0.7% | 0.29 | 0.593 |
| TOTAL | 584 | 153.5 | 737.5 | | | 12.97 | 0.226 |

Table 14C: Haplotype transmissions by AFBAC method: "neither sib DR3/4" subgroup of families

| | Transmitted (n=540) | | | AFBAC (n=121.5) | | | Chi-squared Statistic | p-value (df = 1) |
|---|---|---|---|---|---|---|---|---|
| SNP # | allele counts | | variant allele frequency | allele counts | | variant allele frequency | | |
| | wt | variant | | wt allele | variants | | | |
| 1 | 296 | 244 | 45.2% | 64.5 | 57 | 46.9% | 0.12 | 0.730 |
| 2 | 490 | 50 | 9.3% | 107.5 | 14 | 11.5% | 0.58 | 0.446 |
| 3 | 485 | 55 | 10.2% | 103.5 | 18 | 14.8% | 2.17 | 0.141 |
| 4 | 485 | 55 | 10.2% | 103.5 | 18 | 14.8% | 2.17 | 0.141 |
| 5 | 485 | 55 | 10.2% | 103.5 | 18 | 14.8% | 2.17 | 0.141 |
| 6 | 457 | 83 | 15.4% | 96.5 | 25 | 20.6% | 1.97 | 0.161 |
| 7 | 436 | 104 | 19.35 | 88.5 | 33 | 27.2% | 3.77 | 0.052 |
| 8 | 532 | 8 | 1.5% | 119.5 | 2 | 1.6% | 0.02 | 0.893 |
| | | | | | | | | |
| TOTAL | | | | | | | | |

Table 15A. SNP by SNP allele transmissions by AFBAC method: "either/both sib DR3/4" subgroup of familes

| | Transmitted (n=588) | | | AFBAC (n=158.5) | | | Chi-squared Statistic | p-value (df = 1) |
|---|---|---|---|---|---|---|---|---|
| SNP # | allele counts | | variant allele frequency | allele counts | | variant allele frequency | | |
| | wt | variant | | wt allele | variant | | | |
| 1 | 320 | 268 | 45.6% | 77.5 | 81 | 51.1% | 1.53 | 0.216 |
| 2 | 539 | 49 | 8.3% | 139 | 19.5 | 12.3% | 2.36 | 0.124 |
| 3 | 529 | 59 | 10.0% | 132 | 26.5 | 16.7% | 5.50 | 0.019 |
| 4 | 529 | 59 | 10.0% | 132 | 26.5 | 16.7% | 5.50 | 0.019 |
| 5 | 534 | 54 | 9.2% | 136 | 22.5 | 14.2% | 3.41 | 0.065 |
| 6 | 502 | 86 | 14.6% | 121 | 37.5 | 23.7% | 7.38 | 0.007 |
| 7 | 475 | 113 | 19.2% | 115 | 43.5 | 27.4% | 5.10 | 0.024 |

(continued)

| SNP # | Transmitted (n=588) | | | AFBAC (n=158.5) | | | Chi-squared Statistic | p-value (df = 1) |
|---|---|---|---|---|---|---|---|---|
| | allele counts | | variant allele frequency | allele counts | | variant allele frequency | | |
| | wt | variant | | wt allele | variant | | | |
| 8 | 584 | 4 | 0.7% | 157.5 | | 0.6% | 0.00 | 0.946 |
| Table 15B. SNP by SNP allele transmissions by AFBAC method: "neither sib DR3/4" subgroup of familes | | | | | | | | |

| haplotype | transmitted to affected | NOT transmitted to affected | TOTAL TRANSMISSIONS OBSERVED | McNemar chi-squared statistic | P-value (1 df) | % TRANSMISSION |
|---|---|---|---|---|---|---|
| 11111111 | 140 | 131 | 271 | 0.299 | 0.585 | 51.7% |
| 21111111 | 130 | 105 | 235 | 2.660 | 0.103 | 55.3% |
| 21222221 | 27 | 39 | 66 | 2.182 | 0.140 | 40.9% |
| 22111111 | 19 | 28 | 47 | 1.723 | 0.189 | 40.4% |
| 11222221 | 19 | 24 | 43 | 0.581 | 0.446 | 44.2% |
| 22111221 | 17 | 23 | 40 | 0.900 | 0.343 | 42.5% |
| 11111121 | 18 | 15 | 33 | 0.273 | 0.602 | 54.5% |
| 22111222 | 6 | 5 | 11 | 0.091 | 0.763 | 54.5% |
| 21111121 | 1 | 5 | 6 | 2.667 | 0.102 | 16.7% |
| 11111221 | 1 | 3 | 4 | 1.000 | 0.317 | 25.0% |
| 11221221 | 2 | 0 | 2 | 2.000 | 0.157 | 100.0% |
| 21221121 | 1 | 1 | 2 | 0.000 | 1.000 | 50.0% |
| 21221221 | 0 | 2 | 2 | 2.000 | 0.157 | 0.0% |
| 11221111 | 0 | 1 | 1 | 1.000 | 0.317 | 0.0% |
| 11111222 | 1 | 0 | 1 | 1.000 | 0.317 | 100.0% |
| total | 382 | 382 | 764 | | | |
| Table 16A: TDT analysis of 8-loocus haplotypes to probands only. | | | | | | |

| haplotype | transmitted to affected | NOT transmitted to affected | TOTAL TRANSMISSIONS OBSERVED | McNemar chi-squared statistic | P-value (1 df) | % TRANSMISSION |
|---|---|---|---|---|---|---|
| 21222221 | 52 | 80 | 132 | 5.939 | 0.015 | 39.4% |
| 21111111 | 245 | 225 | 470 | 0.851 | 0.356 | 52.1 % |
| 11111111 | 283 | 259 | 542 | 1.063 | 0.303 | 52.2% |
| 22111111 | 43 | 51 | 94 | 0.681 | 0.409 | 45.7% |
| 11221221 | 3 | 1 | 4 | 1.000 | 0.317 | 75.0% |
| 11222221 | 44 | 42 | 86 | 0.047 | 0.829 | 51.2% |
| 22111221 | 37 | 43 | 80 | 0.450 | 0.502 | 46.3% |

(continued)

| haplotype | transmitted to affected | NOT transmitted to affected | TOTAL TRANSMISSIONS OBSERVED | McNemar chi-squared statistic | P-value (1 df) | % TRANSMISSION |
|---|---|---|---|---|---|---|
| 11111121 | 36 | 30 | 66 | 0.545 | 0.460 | 54.5% |
| 22111222 | 1 | 11 | 22 | 0.000 | 1.000 | 50.0% |
| 21111121 | 3 | 9 | 12 | 3.000 | 0.083 | 25.0% |
| 21221121 | 2 | 2 | 4 | 0.000 | 1.000 | 50.0% |
| 21221221 | 0 | 4 | 4 | 4.000 | 0.046 | 0.0% |
| 11111221 | 4 | 4 | 8 | 0.000 | 1.000 | 50.0% |
| 11221111 | 0 | 2 | 2 | 2.000 | 0.157 | 0.0% |
| 11111222 | 1 | 1 | 2 | 0.000 | 1.000 | 50.0% |
| Table 16B: TDT analysis of 8-loocus haplotypes to two primary affected children per family. | | | | | | |

| haplotype | transmitted to affected | NOT transmitted to affected | TOTAL TRANSMISSIONS OBSERVED | McNemar chi-squared statistic | P-value (1 df) | % TRANSMISSION |
|---|---|---|---|---|---|---|
| 11111111 | 295 | 272 | 567 | 0.933 | 0.334 | 52.0% |
| 21111111 | 259 | 236 | 495 | 1.069 | 0.301 | 52.3% |
| 21222221 | 55 | 88 | 143 | 7.615 | 0.006 | 38.5% |
| 22111111 | 45 | 53 | 98 | 0.653 | 0.419 | 45.9% |
| 11222221 | 44 | 43 | 87 | 0.011 | 0.915 | 50.6% |
| 22111221 | 40 | 46 | 86 | 0.419 | 0.518 | 46.5% |
| 11111121 | 38 | 30 | 68 | 0.941 | 0.332 | 55.9% |
| 22111222 | 12 | 11 | 23 | 0.043 | 0.835 | 52.2% |
| 21111121 | 3 | 10 | 13 | 3.769 | 0.052 | 23.1% |
| 11111221 | 6 | 4 | 10 | 0.400 | 0.527 | 60.0% |
| 21221221 | 1 | 5 | 6 | 2.667 | 0.102 | 16.7% |
| 11221221 | 3 | 2 | 5 | 0.200 | 0.655 | 60.0% |
| 21221121 | 3 | 2 | 5 | 0.200 | 0.655 | 60.0% |
| 11221111 | 0 | 2 | 2 | 2.000 | 0.157 | 0.0% |
| 11111222 | 1 | 1 | 2 | 0.000 | 1.000 | 50.0% |
| TOTAL | 805 | 805 | 1610 | | | |
| Table 16c: TDT analysis of 8-loocus haplotypes to all affected children. | | | | | | |

| haplotype | transmitted to affected | TOTAL NOT transmitted to affected | TRANSMISSIONS OBSERVED | McNemar chi-squared statistic | P-value (1 df) | % TRANSMISSION |
|---|---|---|---|---|---|---|
| 11111111 | 133 | 121 | 254 | 0.567 | 0.451 | 52.45 |
| 21111111 | 105 | 101 | 206 | 0.078 | 0.780 | 51.0% |
| 21222221 | 30 | 36 | 66 | 0.545 | 0.460 | 45.5% |
| 22111111 | 22 | 26 | 48 | 0.333 | 0.564 | 45.8% |
| 11222221 | 19 | 19 | 38 | 0.000 | 1.000 | 50.0% |
| 22111221 | 16 | 22 | 38 | 0.947 | 0.330 | 42.1% |
| 22111222 | 14 | 14 | 28 | 0.000 | 1.000 | 50.0% |
| 22111222 | 8 | 6 | 14 | 0.286 | 0.593 | 57.1% |
| 21111121 | 1 | 5 | 6 | 2.667 | 0.102 | 16.7% |
| 11111221 | 2 | 0 | 2 | 2.000 | 0.157 | 100.0% |
| TOTAL | 350 | 350 | 700 | | | |

Table 17A: analysis of 8-loocus haplotypes to two primary affected children per family: "either/both sib DR3/4" subgroup.

| haplotype | transmitted to affected | NOT transmitted to affected | TOTAL TRANSMISSIONS OBSERVED | McNemar chi-squared statistic | P-value (1 df) | % TRANSMISSION |
|---|---|---|---|---|---|---|
| 11111111 | 138 | 126 | 264 | 0.545 | 0.460 | 52.3% |
| 21111111 | 128 | 112 | 240 | 1.067 | 0.302 | 53.3% |
| 21222221 | 22 | 44 | 66 | 7.333 | 0.007 | 33.3% |
| 22111111 | 23 | 29 | 52 | 0.692 | 0.405 | 44.2% |
| 11222221 | 28 | 20 | 48 | 1.333 | 0.248 | 58.3% |
| 22111111 | 21 | 25 | 46 | 0.348 | 0.555 | 45.7% |
| 11111121 | 17 | 11 | 28 | 1.286 | 0.257 | 60.7% |
| 22111222 | 3 | 5 | 8 | 0.500 | 0.480 | 37.5% |
| 11111221 | 2 | 4 | 6 | 0.667 | 0.414 | 33.3% |
| 21111121 | 2 | 4 | 6 | 0.667 | 0.414 | 33.3% |
| 11221221 | 3 | 1 | 4 | 1.000 | 0.317 | 75.0% |
| 21221121 | 2 | 2 | 4 | 0.000 | 1.000 | 50.0% |
| 21221221 | 0 | 4 | 4 | 4.000 | 0.046 | 0.0% |
| 11221111 | 0 | 2 | 2 | 2.000 | 0.157 | 0.0% |
| 11111222 | 1 | 1 | 2 | 0.000 | 1.000 | 50.0% |
| TOTAL | 390 | 390 | 780 | | | |

Table 17B: TDT analysis of 8-loocus haplotypes to two primary affected children per family: "neither sib DR3/4" subgroup..

| SNP # | WT Allele Frequency in controls | WT allele frequency in diabetics | P-value by z- test |
|---|---|---|---|
| 1 | 0.559 | 0.461 | 0.077 |
| 2 | 0.989 | 1.000 | 0.480 |
| 3 | 0.793 | 0.882 | 0.031 |
| 4 | 0.824 | 0.933 | 0.003 |
| 5 | 0.809 | 0.882 | 0.075 |
| 6 | 0.814 | 0.882 | 0.097 |
| 7 | 0.723 | 0.770 | 0.362 |
| 8 | 1.000 | 1.000 | 1.000 |
| n = | 188 | 178 | chromosomes |
| Table 18: Allele frequencies compared between Filipino controls and diabetics. | | | |

| | MLE frequency: controls | MLS frequency: Diabetics | O.R. | COUNT: control | COUNT: diabetics | Total count | exp control | exp diabetic | chi-square | P-value |
|---|---|---|---|---|---|---|---|---|---|---|
| 1L4R | | | | | | | | | | |
| 11111111 | 0.318 | 0.329 | 1.1 | 59.7 | 58.5 | 118.2 | 60.7 | 57.5 | 0.033 | 0.855 |
| 11111121 | 0.074 | 0.074 | 1.0 | 14.0 | 13.2 | 27.2 | 14.0 | 13.2 | 0.000 | 0.987 |
| 11222221 | 0.145 | 0.058 | 0.4 | 27.3 | 10.3 | 37.6 | 19.3 | 18.3 | 6.727 | 0.009 |
| 21111111 | 0.390 | 0.441 | 1.2 | 73.3 | 78.5 | 151.8 | 78.0 | 73.8 | 0.582 | 0.445 |
| 21212221 | 0.032 | 0.051 | 1.6 | 6.0 | 9.0 | 15.0 | 7.7 | 7.3 | 0.776 | 0.378 |
| **n =** | **188** | **178** | | | | **366** | | | | |

Table 19: Computationally estimated haplotype frequencies compared between Filipino controls and diabetics.

| haplotype | calc. frequency: controls | calc. frequency: Diabetics | O.R. | COUNT: control | COUNT: diabetics | Total count | exp control | exp diabetic | chi-square | P-value |
|---|---|---|---|---|---|---|---|---|---|---|
| IL4R | | | | | | | | | | |
| x1111111 | 0.707 | 0.770 | 1.4 | 133.0 | 137.0 | 270.0 | 138.7 | 131.3 | 0.480 | 0.489 |
| x1111121 | 0.074 | 0.112 | 1.6 | 14.0 | 20.0 | 34.0 | 17.5 | 16.5 | 1.413 | 0.235 |
| x1222221 | 0.154 | 0.067 | 0.4 | 29.0 | 12.0 | 41.0 | 21.1 | 19.9 | 6.155 | 0.013 |
| x1212221 | 0.032 | 0.051 | 1.6 | 6.0 | 9.0 | 15.0 | 7.7 | 7.3 | 0.776 | 0.378 |
| x1222111 | 0.005 | 0.000 | 0.0 | 1.0 | 0.0 | 1.0 | 0.5 | 0.5 | 0.947 | 0.331 |
| x2111111 | 0.011 | 0.000 | 0.0 | 2.0 | 0.0 | 2.0 | 1.0 | 1.0 | 1.894 | 0.169 |
| x1221121 | 0.016 | 0.000 | 0.0 | 3.0 | 0.0 | 3.0 | 1.5 | 1.5 | 2.840 | 0.092 |
| | | | **n=** | **188.0** | **178.0** | **366** | | **overall** | **14.504** | **0.024** |

Table 20: Observed haplotypes and frequencies compared between Filipino controls and diabetics..

**TABLE 21**

**Allele Frequencies in Patients and Controls**

| Gene | SNP Description | Major allele | Minor allele | Freq in Controls | Freq in T1D patients | p-value[a] | O.R.[b] | 95% CI |
|---|---|---|---|---|---|---|---|---|
| IL4R | 5' (-3223)[c] | C | T | 41.9% | 51.1% | 0.10 | 1.45 | 0.96, 2.19 |
| | 5' (-1914)[c] | C | T | 44.1% | 42.7% | 0.86 | 1.06 | 0.70, 1.60 |
| | I50V[d] | A | G | 44.1% | 53.9% | 0.06 | 1.48 | 0.98, 2.23 |
| | N142N[d] | C | G | 1.1% | 0.0% | 0.17 | 0.00 | |
| | E375A[d] | A | C | 20.7% | 11.7% | 0.02 | 0.50 | 0.28, 0.90 |
| | L389L[d] | G | T | 17.6% | 6.7% | 0.001 | 0.34 | 0.17, 0.67 |
| | C406R[d] | T | C | 19.1% | 11.7% | 0.05 | 0.56 | 0.31,0.99 |
| | S478P[d] | T | C | 18.6% | 11.7% | 0.06 | 0.58 | 0.32, 1.04 |
| | Q551R[d] | A | G | 27.7% | 23.3% | 0.34 | 0.80 | 0.50, 1.27 |
| | S761P[d] | T | | 100.0% | 100.0% | | n.a. | n.a. |
| IL4 | 5' (-524)[e] | C | T | 30.9% | 34.4% | 0.46 | 1.18 | 0.76, 1.82 |
| IL13 | 5'(-1512)[f] | A | C | 30.6% | 41.0% | 0.05 | 1.58 | 1.03,2.42 |
| | 5'(-1112)[f] | C | T | 23.1% | 30.9% | 0.1.17 | 1.49 | 0.94,2.37 |
| | Intron 3 [f] | C | T | 40.4% | 45.6% | 0.32 | 1.23 | 0.82, 1.86 |
| | R110Q [f] | G | A | 39.9% | 46.1% | 0.23 | 1.29 | 0.85, 1.95 |

[a] Differences in allele frequencies between cases and controls were tested using a chi-square test.
[b] Odds ratios refer to the minority allele.
[c] Accession sequence AC004525.1;
[d] Accession sequence X52425.1;
[e] Accession sequence M23442.1;
[f] Accession sequence U10307.1

TABLE 22

**Pairwise Linkage Disequilibrium Between IL4R SNPs**

| | -3223 (T) | -1914 (T) | 50 (G) | 142 (G) | 375(C) | 389(T) | 406(C) | 478 (C) | 551 (G) |
|---|---|---|---|---|---|---|---|---|---|
| **-3223 (T)** | - | -0.183*** | 0.176*** | 0.004 | -0.063*** | -0.068*** | -0.061*** | -0.059*** | -0.068*** |
| **-1914 (T)** | -1.0 | - | -0.068*** | -0.005 | 0.105 | 0.029* | 0.014 | 0.011 | 0.027 |
| **50 (G)** | 0.75 | -0.81 | - | 0.006 | -0.049** | -0.067*** | -0.043** | -0.039** | -0.079*** |
| **142 (G)** | 1.0 | -1.0 | 1.0 | - | -0.002 | -0.002 | -0.002 | -0.002 | -0.003 |
| **375 (C)** | -0.70 | 0.70 | -0.53 | -1.0 | - | 0.139*** | 0.152*** | 0.147*** | 0.145*** |
| **389 (T)** | -0.93 | 0.29 | -0.86 | -1.0 | 1.0 | - | 0.126*** | 0.122*** | 0.122*** |
| **406 (C)** | -0.74 | 0.13 | -0.49 | -1.0 | 1.0 | 0.89 | - | 0.150*** | 0.133*** |
| **478 (C)** | -0.74 | 0.62 | -0.48 | -1.0 | 1.0 | 0.85 | 1.0 | - | 0.135*** |

(continued)

**Pairwise Linkage Disequilibrium Between IL4R SNPs**

| | -3223 (T) | -1914 (T) | 50 (G) | 142 (G) | 375(C) | 389(T) | 406(C) | 478 (C) | 551 (G) |
|---|---|---|---|---|---|---|---|---|---|
| **551 (G)** | -0.56 | 0.17 | -0.65 | -1.0 | 0.96 | 0.96 | 0.96 | 1.0 | - |

Top diagonal (D= $P_{ab}$ - $P_a \cdot P_b$). Bottom diagonal D' (normalized linkage disequilibrium, D'=D/Dmax). All values refer to the minority allele indicated in the table.
Statistically significant linkage disequilibrium values are indicated as follows:
*p<0.05;** p<0.01; ***p<0.0001

**TABLE 23**

**Pairwise Linkage Disequilibrium Between IL4 and IL13 SNPs**

| | IL4-524 (C) | IL13 - 1512(C) | IL13 - 1112(T) | IL13 intr (T) | IL13 110(A) |
|---|---|---|---|---|---|
| **IL4-524 (C)** | - | 0.062*** | 0.069*** | 0.024 | 0.063 *** |
| **IL13 -1512(C)** | 0.29 | - | 0.163*** | 0.057*** | 0.058** |
| **IL13 -1112(T)** | 0.41 | 1.0 | - | 0.077*** | 0.078*** |
| **IL13 intr (T)** | 0.13 | 0.31 | 0.54 | - | 0.201 *** |
| **IL13 110(A)** | 0.34 | 0.31 | 0.55 | 0.84 | - |

Top diagonal (D= $P_{ab}$ - $P_a \cdot P_b$). Bottom diagonal D' (normalized linkage disequilibrium, D'=D/Dmax). Statistically significant linkage disequilibrium values are indicated as follows: p<0.05; **p<0.01; ***p<0.0001

**TABLE 24**

**Genotype Frequencies in Diabetics and Controls.**

| | genotype | controls | T1DM | Fisher's exact test | OR | 95% CI |
|---|---|---|---|---|---|---|
| | CC | 29.0% | 24.1 % | | 0.78 | (0.40, 1.51) |
| IL4R_3223 | C T | 58.1% | 51.7% | 0.1584 | 0.77 | (0.43, 1.38) |
| | T T | 12.9% | 24.1% | | 2.15 | (0.99, 4.68) |
| | CC | 31.2% | 36.0% | | 1.24 | (0.67, 229) |
| IL4R_1914 | T C | 49.5% | 42.7% | | 0.76 | (0.43, 1.36) |
| | T T | 19.4% | 21.3% | 0.6636 | 1.13 | (0.55, 2.33) |
| | AA | 34.7% | 23.6% | | 0.58 | (0.30, 1.11) |
| IL4R 50 | AG | 43.2% | 44.9% | | 1.08 | (0.60, 1.93) |
| | GG | 22.1% | 31.5% | 0.1846 | 1.62 | (0.84, 3.13) |
| | CC | 97.9% | 100.0% | | | |
| IL4R 142 | CG | 2.1% | 0.0% | 0.4978 | | |
| | AA | 62.1% | 78.7% | | 2.25 | **( 1.17, 4.33 )** |
| IL4R 375 | AC | 34.7% | 19.1% | | 0.44 | ( 0.23, 0.87 ) |
| | CC | 3.2% | 2.2% | **0.038** | 0.70 | ( 0.12, 4.32 ) |
| | GG | 67.4% | 87.6% | | 3.43 | **( 1.60, 7.37 )** |
| IL4R 389 | GT | 30.5% | 11.2% | | 0.29 | **( 0.13, 0.63 )** |
| | TT | 2.1% | 1.1% | **0.002** | 0.53 | ( 0.05, 5.93 ) |
| | CC | 2.1% | 2.2% | | 1.07 | ( 0.15, 7.76 ) |
| IL4R 406 | TC | 33.7% | 19.1% | | 0.46 | **( 0.24, 0.92 )** |
| | TT | 64.2% | 78.7% | 0.0585 | 2.05 | **( 1.06, 3.97 )** |
| | CC | 2.1% | 2.2% | | 1.07 | (0.15,7.76) |
| IL4R 478 | TC | 32.6% | 19.1% | | 0.49 | **(0.25,0.96)** |
| | TT | 65.3% | 78.7% | 0.0904 | 1.96 | **( 1.01, 3.79 )** |

(continued)

**Genotype Frequencies in Diabetics and Controls.**

| | genotype | controls | T1DM | Fisher's exact test | OR | 95% CI |
|---|---|---|---|---|---|---|
| IL4R 551 | AA | 52.6% | 57.3% | | 1.21 | ( 0.68, 2.16 ) |
| | AG | 38.9% | 38.2% | | 0.97 | ( 0.53, 1.76 ) |
| | GG | 8.4% | 4.5% | 0.5613 | 0.51 | ( 0.15, 1.76 ) |
| IL4 -590 | CC | 10.5% | 12.4% | | 1.20 | ( 0.48, 2.98 ) |
| | CT | 40.0% | 42.7% | | 1.12 | ( 0.62, 2.01 ) |
| | TT | 49.5% | 44.9% | 0.8143 | 0.83 | ( 0.47, 1.49 ) |
| IL13INT3 C | CC | 33.7% | 31.5% | | 0.90 | ( 0.49, 1.68 ) |
| | CT | 50.5% | 46.1% | | 0.84 | ( 0.47, 1.49 ) |
| | TT | 15.8% | 22.5% | 0.5413 | 1.55 | ( 0.74, 3.25 ) |
| IL13 110 | AA | 12.6% | 27.0% | | 2.55 | **( 1.19, 5.49 )** |
| | GA | 54.7% | 38.2% | | 0.51 | **( 0.28, 0.92 )** |
| | GG | 32.6% | 34.8% | **0.0252** | 1.10 | ( 0.60, 2.03 ) |
| IL13_1512 | A A | 48.4% | 34.8% | | 0.57 | ( 0.31, 1.03 ) |
| | AC | 41.9% | 48.3% | | 1.29 | ( 0.72, 2.32 ) |
| | C C | 9.7% | 16.9% | 0.1363 | 1.89 | ( 0.78, 4.57 ) |
| IL13_1112 | C C | 60.2% | 51.7% | | 0.71 | ( 0.39, 1.27 ) |
| | C T | 33.3% | 34.8% | | 1.07 | ( 0.58, 1.97 ) |
| | TT | 6.5% | 13.5% | 0.2429 | 2.26 | ( 0.81, 6.31 ) |

**TABLE 25A**

**Molecular IL-4 R Haplotypes in Filipino Diabetics and Controls**

| 7 SNP Haplotype | Frequency in Controls | Frequency in Diabetics | chi -square p-value | O.R. | 95% CI |
|---|---|---|---|---|---|
| CAGTTAT | 70.7% | 77.0% | 0.49 | 1.38 | (0.9,2.2) |
| CCTCCGT | 15.4% | 6.7% | 0.013 | 0.40 | (0.2, 0.8) |
| GAGTTAT | 1.1% | 0.0% | 0.50 | 0.0 | (0.0, 5.8) |
| CAGTTGT | 7.4% | 11.2% | 0.23 | 1.57 | (0.8, 3.2) |
| CCGCCGT | 3.2% | 5.1% | 0.38 | 1.62 | (0.6,4.6) |
| CCTTTGT | 1.6% | 0.0% | 0.27 | 0.0 | (0.0, 3.5) |
| CCTGTAT | 0.5% | 0.0% | 0.98 | 0.0 | (0.0, 15.8) |
| total (6 d.f.) | | | 0.076 | | |
| n= | 188 | 178 | | | |

**TABLE 25B**

**Estimated IL4R 10-SNP Haplotype Frequencies in Diabetics and Controls**

| 8 locus H-ID | IL4R 10 Locus Haplotype | Controls[a] N=188 | std dev | T1DM N=180 | std dev | O.R | 95% CI | P-value[b] |
|---|---|---|---|---|---|---|---|---|
| H-5A | CCACCTCCGT | 5.8% | (1.7%) | 0.0% | | 0.00 | (0.0, 0.8) | 0.001 |
| H-5B | CTACCTCCGT | 8.5% | (2.3%) | 5.8% | (2.2%) | 0.66 | (0.3, 1.6) | 0.33 |
| H-3A | CTGCCTCCGT | 0.9% | (0.6%) | 0.0% | | 0.00 | (0.0, 6.0) | 0.21 |
| H-3B | TCGCCTCCGT | 0.0% | | 0.9% | (1.1%) | n.a. | | 0.19 |

(continued)

Estimated IL4R 10-SNP Haplotype Frequencies in Diabetics and Controls

| 8 locus H-ID | IL4R 10 Locus Haplotype | Controls[a] N=188 | std dev | T1DM N=180 | std dev | O.R | 95% CI | P-value[b] |
|---|---|---|---|---|---|---|---|---|
| | Total (23 d.f.) | | | | | | | 0.04 |

[a] Maximum likelihood haplotype frequencies were computed using an Expectation-Maximization (EM) algorithm (see Excoffier and Slatkin 1995) as implemented by the Arlequin software program L. Excoffier, University of Geneva, CH). The standard deviation was computed carrying out 100 boostrap replicates. 24 of the possible 512 haplotypes were observed. The four shown here all include the disease associated seven SNP haplotype. The two 8 SNP haplotypes containing the seven SNP haplotype were designated H-3 and H-5 in reference Mirel et al, (in press) The standard deviation was computed carrying out 100 boostrap replicates.

[b] Differences in allele frequencies between cases and controls were tested using a chi-square test

TABLE 26

Estimated IL4 and IL13 5-SNP Haplotype Frequencies in Diabetics and Controls

| 5 locus haploytpe | Controls N=188 | *Sd* | T1DM N=176* | *Sd* | Odds Ratio | P-value |
|---|---|---|---|---|---|---|
| CACCA | 4.1% | *(1.3%)* | 3.5% | *(1.7%)* | 0.84 | |
| CACCG | 8.9% | *(2.2%)* | 10.6% | *(2.9%)* | 1.21 | |
| CACTA | 1.3% | *(1.1%)* | 3.0% | *(1.8%)* | 2.26 | |
| CATTA | 0.0% | | 0.6% | *(0.5%)* | n.a. | |
| CCCCG | 1.5% | *(1.0%)* | 0.9% | *(0.9%)* | 0.61 | |
| CCTCG | 1.1% | *(0.9%)* | 5.5% | *(2.3%)* | 5.19 | 0.02 |
| CCTTA | 12.6% | *(2.4%)* | 9.3% | *(2. 0%)* | 0.71 | |
| CCTTG | 0.0% | | 0.4% | *(0.7%)* | n.a. | |
| TACCG | 33.6% | *(3.8%)* | 21.9% | *(3.9%)* | 0.55 | 0.03 |
| TACTA | 16.4% | *(3.3%)* | 16.3% | *(3.0%)* | 0.99 | |
| TACTG | 5.0% | *(1.7%)* | 3.2% | *(1.4%)* | 0.62 | |
| TCCCG | 4.6% | *(1.8%)* | 9.8% | *(2.9%)* | 2.23 | 0.06 |
| TCCTA | 1.3% | *(1.1%)* | 0.0% | | 0.00 | |
| TCCTG | 0.2% | *(0.5%)* | 0.0% | | 0.00 | |
| TCTCG | 5.3% | *(2.4%* | 1.7% | *(1.2%)* | 0.31 | 0.07 |
| TCTTA | 4.1% | *(2.1%)* | 12.8% | *(2.2%)* | 3.47 | 0.004 |
| TCTCA | 0.0% | | 0.6% | *(0.7%)* | n.a. | |
| Total (16 d.f.) | | | | | | 0.005 |

* due to missing genotypes.
(order of the SNPs: IL4-524, IL13-1512, IL13-1112, IL13INT3, IL13R110)

TABLE 27

Test For Independence Between Genotype Frequencies at IL4R SNPs and Genotype Frequencies at Five IL4 and IL13 SNPs.

| Chromsome 5 SNP | controls | T1D |
|---|---|---|
| **IL4 -524** | 0.15 | **0.001** |
| **IL13 -1512** | 0.77 | 0.78 |
| **IL13 -1112** | 0.93 | 0.73 |
| **IL13 110** | 0.99 | 0.91 |
| **IL3 intron 3** | 0.99 | **0.019** |

**TABLE 28**

| Epistasis Between IL4R and Five IL4 and IL13 SNPs | | | | | |
|---|---|---|---|---|---|
| | $\beta_3$. | Std.Error | Odds ratio | Walds Chi-sq | Nominal p-value |
| IL4 -524:il4r | -0.22 | 0.10 | 0.81 | 4.55 | 0.033 |
| IL13-1512:il4r | 0.04 | 0.15 | 1.04 | 0.06 | 0.811 |
| IL13-1112:il4r | 0.10 | 0.16 | 1.10 | 0.37 | 0.545 |
| IL13 110:il4r | 0.14 | 0.09 | 1.15 | 2.24 | 0.135 |
| IL13INT3:il4r | 0.17 | 0.09 | 1.18 | 3.19 | **0.074** |

The Overall p-Value For All Five Tests By Permutation Analysis Was p<0.045 (see text).

**TABLE 29**

| Probes For IL4 and IL13 | | | | | |
|---|---|---|---|---|---|
| **SNP** | **Allele** | **Probe** | **Probe Sequence** | **Seq ID No:** | **Conc. (μM)** |
| IL4P C582T | C | KW66 | AACATTGTCCCCCAGTGC | 63 | 1.2 |
| IL4P C582T | T | KW89 | AGCACTGGGGAACAATGTTC | 64 | 0.9 |
| IL13 Int 3 | C | DBM0161P | TTCTACTCACGTGCTGACCT | 65 | 1.0 |
| IL13 Int 3 | T | DBM0164P | GGTCAGCACATGAGTAGAACG | 66 | 0.3 |
| IL13 Ex 4 R110Q | | DBM0136P | TCAGTTGAACCGTCCCTCG | 67 | 1.0 |
| IL13 Ex 4 R110Q | | DBM0181P | GAGGGACAGTTCAACTGAAAC | 68 | 1.0 |

**TABLE 30**

| Amplicon Primers and Lengths | | | | | | | |
|---|---|---|---|---|---|---|---|
| **Exon** | **Amplicon Size** | **Forward Primer** | **Seq ID No** | **Sequence** | **Reverse Primer** | **Seq ID No** | **Sequence** |
| IL4P | 107 | RR169B | 69 | ACTAGGCCTCACCTGATACGA | RR170B | 72 | CATAGAGGCAGAATAACAGGCAGA |
| IL 13 in 3 | 118 | DBM0165B | 70 | CTCGGACATGCAAGCTGGAA | DBM0166B | 73 | ACTGAATGAGACAGTCCCTGGA |
| IL 13 ex 4 | 187 | DBM0167B | 71 | AATCGAGGTGGCCCAGTTTGTA | DBM0168B | 74 | CCTAACCCTCCTTCCCGCCTA |

TABLE 31

| Amplicon Primers and Lengths for IL4R | | | | | |
|---|---|---|---|---|---|
| Exon | Amplicon Size | Allele | Primer | Seq ID No | Sequence |
| T(-1914)C | 49 | T | DBM0659<br>DBM0661 | 75<br>76 | ACTGACTTATCTTTACTGTCACTTCT<br>GCAAGACAGCCACCAACCC |
| T(-1914)C | 49 | C | DBM0660<br>DBM0661 | 77<br>76 | TGACTTATCTTTACTGTCACTTCC<br>GCAAGACAGCCACCAACCC |
| C(-3223)T | 42 | C | DBM0672<br>DBM0667 | 79<br>80 | CCTGCTCCCAGGACTGAC<br>CCCAGACTTTATCTGTGACTGCTC |
| C(-3223)T | 42 | T | DBM0671<br>DBM0667 | 81<br>80 | CCTGCTCCCAGGACTGAT<br>CCCAGACTTTATCTGTGACTGCTC |

TABLE 32

| Amplicon Primers and Lengths for IL13 | | | | | |
|---|---|---|---|---|---|
| Exon | Amplicon Size | Allele | Primer | Seq ID No | Sequence |
| A(-1512)C | 44 | A | DBM0650<br>DBM0652 | 82<br>83 | GGAAACAGGCCCGTAGA<br>GAGTGCCGCTACTTGGCC |
| A(-1512)C | 43 | C | DBM0651<br>DBM0652 | 84<br>83 | GAAACAGGCCCGTAGC<br>GAGTGCCGCTACTTGGCC |
| C(-1112)T | 55 | C | DBM0656<br>DBM0658 | 85<br>86 | TCTGGAGGACTTCTAGGAAAAC<br>TGCAGCCATGTCGCCTTT |
| C(-1112)T | 55 | T | DBM0657<br>DBM0658 | 87<br>86 | TCTGGAGGACTTCTAGGAAAAT<br>TGCAGCCATGTCGCCTTT |

## Claims

**1.** A method for determining an individuals risk for type 1 diabetes comprising, detecting the presence of at least one of the type 1 diabetes-associated polymorphisms A(-1512)C, C(-1112)T, C4045T and G4166A in the IL13 loci as listed in Table 2 in a nucleic acid sample of the individual, wherein the presence of said polymorphism indicates the individual's risk for type 1 diabetes.

**2.** The method of claim 1, wherein the nucleic acid sample comprises DNA.

**3.** The method of claim 1, wherein the nucleic acid sample comprises RNA.

**4.** The method of claim 1, wherein the nucleic acid sample is amplified.

**5.** The method of claim 4, wherein the nucleic acid sample is amplified by a polymerase chain reaction.

**6.** The method of claim 1, wherein the polymorphism is detected by amplification.

**7.** The method of claim 6, wherein the polymorphism is detected by a polymerase chain reaction.

**8.** The method of claim 1, wherein the polymorphism is detected by sequencing.

**9.** The method of claim 1, wherein the polymorphism is detected by contacting the nucleic acid sample, under conditions that allow binding of the oligonucleotide to the nucleic acid sample, with at least one sequence-specific oligonucleotides that hybridizes under stringent conditions to a type 1 diabetes-associated polymorphism and detecting the hybridization.

**10.** The method of claim 1, further comprising detecting IL4 polymorphism (-524) listed in Table 21.

**11.** The method of claim 1 or 10, further comprising detecting an IL4R polymorphism listed in Table 2.

**12.** The method of claim 11, wherein the IL4R polymorphism is IL4R 5'(-3223).

**13.** The method of claim 1, comprising detecting IL 13 polymorphism 5'(-1112).

**14.** A kit for determining an individual's risk for type 1 diabetes comprising,

(a) one or more sequence-specific oligonucleotides each individually comprising a sequence that hybridizes under stringent conditions to the variant allele of type 1 diabetes-associated IL13 polymorphism A(-1512)C or C(-1112)T as listed in Table 2; and
(b) instructions to use the kit to determine the individuals risk for type 1 diabetes.

**15.** The kit of claim 14, wherein the oligonucleotides are fully complementary to a nucleic acid sequence comprising to type 1 diabetes-associated IL13 polymorphism A(-1512)C or C(-1112)T as listed in Table 2.

**16.** The kit of claim 14, which contains additional sequencing primers.

**17.** The kit of claim 14, wherein one or more sequence-specific oligonucleotides are labeled.

**18.** The kit of claim 17 that includes a reagent to detect the label.

**19.** The use of detecting one or more of IL 13 polymorphisms A(-1512)C, C(-1112)T, C4045T and G4166A as listed in Table 21 in a nucleic acid sample of an individual for determination of the individual's risk for type 1 diabetes.

**Patentansprüche**

**1.** Verfahren zum Bestimmen des Erkrankungsrisikos eines Individuums für Diabetes Typ I, umfassend, das Nachweisen des Vorhandenseins von mindestens einem der mit Diabetes Typ I einhergehenden Polymorphismen A(-1512)C, C(-1112)T, C4045T und G4166A in den IL13-Loci, wie sie in der Tabelle 2 aufgeführt sind, in einer Nukleinsäureprobe des Individuums, wobei die Anwesenheit des Polymorphismus das Erkrankungsrisiko eines Individuums für Diabetes Typ I anzeigt.

**2.** Verfahren nach Anspruch 1, wobei die Nukleinsäureprobe DNA umfasst.

**3.** Verfahren nach Anspruch 1, wobei die Nukleinsäureprobe RNA umfasst.

**4.** Verfahren nach Anspruch 1, wobei die Nukleinsäureprobe amplifiziert ist.

**5.** Verfahren nach Anspruch 4, wobei die Nukleinsäureprobe durch eine Polymerasekettenreaktion amplifiziert wird.

**6.** Verfahren nach Anspruch 1, wobei der Polymorphismus durch Amplifikation nachgewiesen wird.

**7.** Verfahren nach Anspruch 6, wobei der Polymorphismus durch eine Polymerasekettenreaktion nachgewiesen wird.

**8.** Verfahren nach Anspruch 1, wobei der Polymorphismus durch Sequenzieren nachgewiesen wird.

**9.** Verfahren nach Anspruch 1, wobei der Polymorphismus nachgewiesen wird durch Zusammenbringen der Nukleinsäureprobe, unter Bedingungen, die die Bindung des Oligonukleotids an die Nukleinsäureprobe ermöglichen, mit mindestens einem sequenzspezifischen Oligonukleotid, das unter stringenten Bedingungen an einen mit Diabetes Typ 1 einhergehenden Polymorphismus hybridisiert, und Nachweisen der Hybridisierung.

**10.** Verfahren nach Anspruch 1, zudem umfassend das Nachweisen des in Tabelle 21 aufgeführten IL4-Polymorphismus (-524).

**11.** Verfahren nach Anspruch 1 oder 10, zudem umfassend das Nachweisen eines in Tabelle 2 aufgeführten IL4R-Polymorphismus.

**12.** Verfahren nach Anspruch 11, wobei der IL4R-Polymorphismus IL4R 5'(-3223) ist.

**13.** Verfahren nach Anspruch 1, umfassend das Nachweisen des IL13-Polymorphismus 5'(-1112).

**14.** Kit zum Bestimmen des Erkrankungsrisikos eines Individuums für Diabetes Typ 1, enthaltend:

(a) ein oder mehrere sequenzspezifische Oligonukleotide, die jeweils eine Sequenz umfassen, die unter stringenten Bedingungen an die Allelvariante des mit Diabetes Typ 1 einhergehenden IL13-Polymorphismus A(-1512)C oder C(-1112)T, wie in Tabelle 2 aufgeführt, hybridisiert; und
(b) Gebrauchsanweisungen zur Verwendung des Kits zur Bestimmung des Erkrankungsrisikos eines Individuums für Diabetes Typ 1.

**15.** Kit nach Anspruch 14, wobei die Oligonukleotide vollständig komplementär zu einer Nukleinsäuresequenz sind, umfassend einen mit Diabetes Typ 1 einhergehenden IL13-Polymorphismus A(-1512)C oder C(-1112)T, wie in Tabelle 2 aufgeführt.

**16.** Kit nach Anspruch 14, das zusätzliche Sequenzierungs-Primer enthält.

**17.** Kit nach Anspruch 14, wobei eine oder mehrere sequenzspezifische Oligonukleotide markiert sind.

**18.** Kit nach Anspruch 17, das ein Reagenz zum Nachweisen der Markierung enthält.

**19.** Verwendung des Nachweises von einem oder mehreren IL13-Polymorphismen A(-1512)C, C(-1112)T, C4045T und G4166A, wie in der Tabelle 21 aufgeführt, in einer Nukleinsäureprobe eines Individuums zur Bestimmung des Erkrankungsrisikos eines Individuums für Diabetes Typ 1.

## Revendications

**1.** Procédé de détermination d'un risque d'un individu à développer un diabète de type I, comprenant la détection de la présence d'au moins un des polymorphismes associés à un diabète de type I A(-1512)C, C(-1112)T, C4045T et G4166A dans les locus IL13 comme enregistré dans le tableau 2 d'un échantillon d'acide nucléique de l'individu, dans lequel la présence dudit polymorphisme indique le risque d'un individu à développer un diabète de type I.

**2.** Procédé selon la revendication 1, dans lequel l'échantillon d'acide nucléique comprend de l'ADN.

**3.** Procédé selon la revendication 1, dans lequel l'échantillon d'acide nucléique comprend de l'ARN.

**4.** Procédé selon la revendication 1, dans lequel l'échantillon d'acide nucléique est amplifié.

**5.** Procédé selon la revendication 4, dans lequel l'échantillon d'acide nucléique est amplifié par une réaction en chaîne par polymérase.

**6.** Procédé selon la revendication 1, dans lequel le polymorphisme est détecté par amplification.

**7.** Procédé selon la revendication 6, dans lequel le polymorphisme est détecté par une réaction en chaîne par polymérase.

**8.** Procédé selon la revendication 1, dans lequel le polymorphisme est détecté par séquençage.

**9.** Procédé selon la revendication 1, dans lequel le polymorphisme est détecté par mise en contact de l'échantillon d'acide nucléique, dans des conditions qui permettent la liaison de l'oligonucléotide à l'échantillon d'acide nucléique, avec au moins un oligonucléotide avec une séquence spécifique qui s'hybride dans des conditions rigoureuses en un polymorphisme associé à un diabète de type I et par détection de l'hybridation.

**10.** Procédé selon la revendication 1, comprenant en outre la détection du polymorphisme IL-4 (-524) enregistré dans le tableau 21.

**11.** Procédé selon la revendication 1 ou 10, comprenant en outre la détection du polymorphisme IL-4R enregistré dans le tableau 2.

**12.** Procédé selon la revendication 11, dans lequel le polymorphisme IL-4R est IL4R 5'(-3223).

**13.** Procédé selon la revendication 1, comprenant la détection du polymorphisme IL13 5'(-1112).

**14.** Kit de détermination d'un risque d'un individu à développer un diabète de type I comprenant,

(a) un ou plusieurs oligonucléotides avec une séquence spécifique comprenant chacun individuellement une séquence qui s'hybride dans des conditions rigoureuses en l'allèle différent du polymorphisme IL13 associé à un diabète de type I A(-1512)C ou C(-1112)T, comme représenté dans le tableau 2 ; et
(b) les instructions d'utilisation du kit pour déterminer le risque d'un individu à développer un diabète de type I.

**15.** Kit selon la revendication 14, dans lequel les oligonucléotides sont totalement complémentaires d'une séquence d'acide nucléique comprenant le polymorphisme IL13 associé à un diabète de type I A(-1512)C ou C(-1112)T, comme représenté dans le tableau 2.

**16.** Kit selon la revendication 14, qui contient d'autres amorces de séquençage.

**17.** Kit selon la revendication 14, dans lequel un ou plusieurs oligonucléotides avec une séquence spécifique sont marqués.

**18.** Kit selon la revendication 17, qui comprend un réactif pour détecter le marqueur.

**19.** Utilisation de la détection d'un ou plusieurs polymorphismes IL13 A(-1512)C, C(-1112)T, C4045T et G4166A comme enregistré dans le tableau 21 dans un échantillon d'acide nucléique d'un individu pour déterminer le risque d'un individu à développer un diabète de type I.

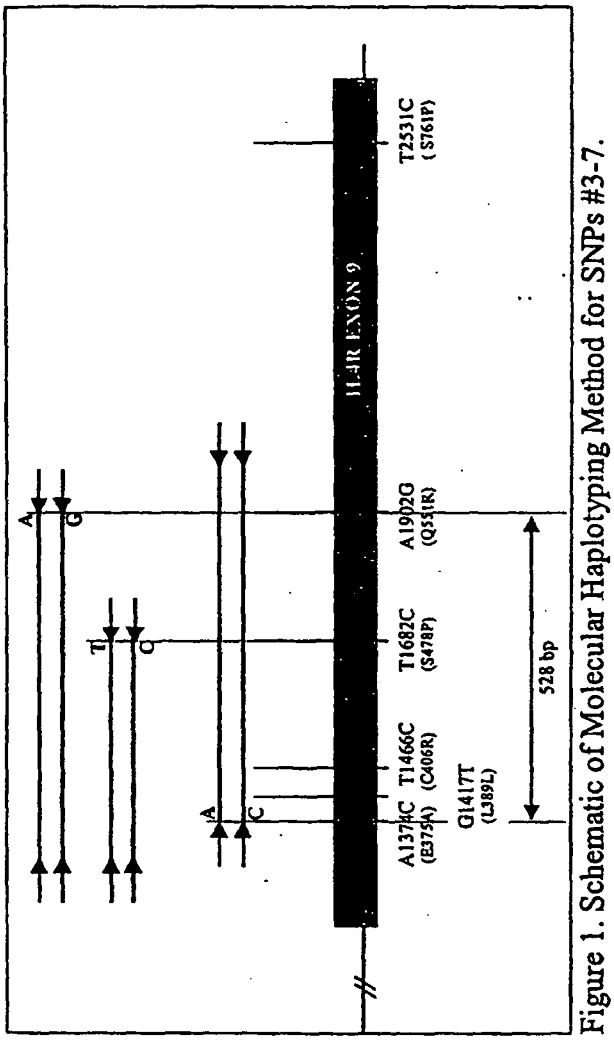


Figure 1. Schematic of Molecular Haplotyping Method for SNPs #3-7.

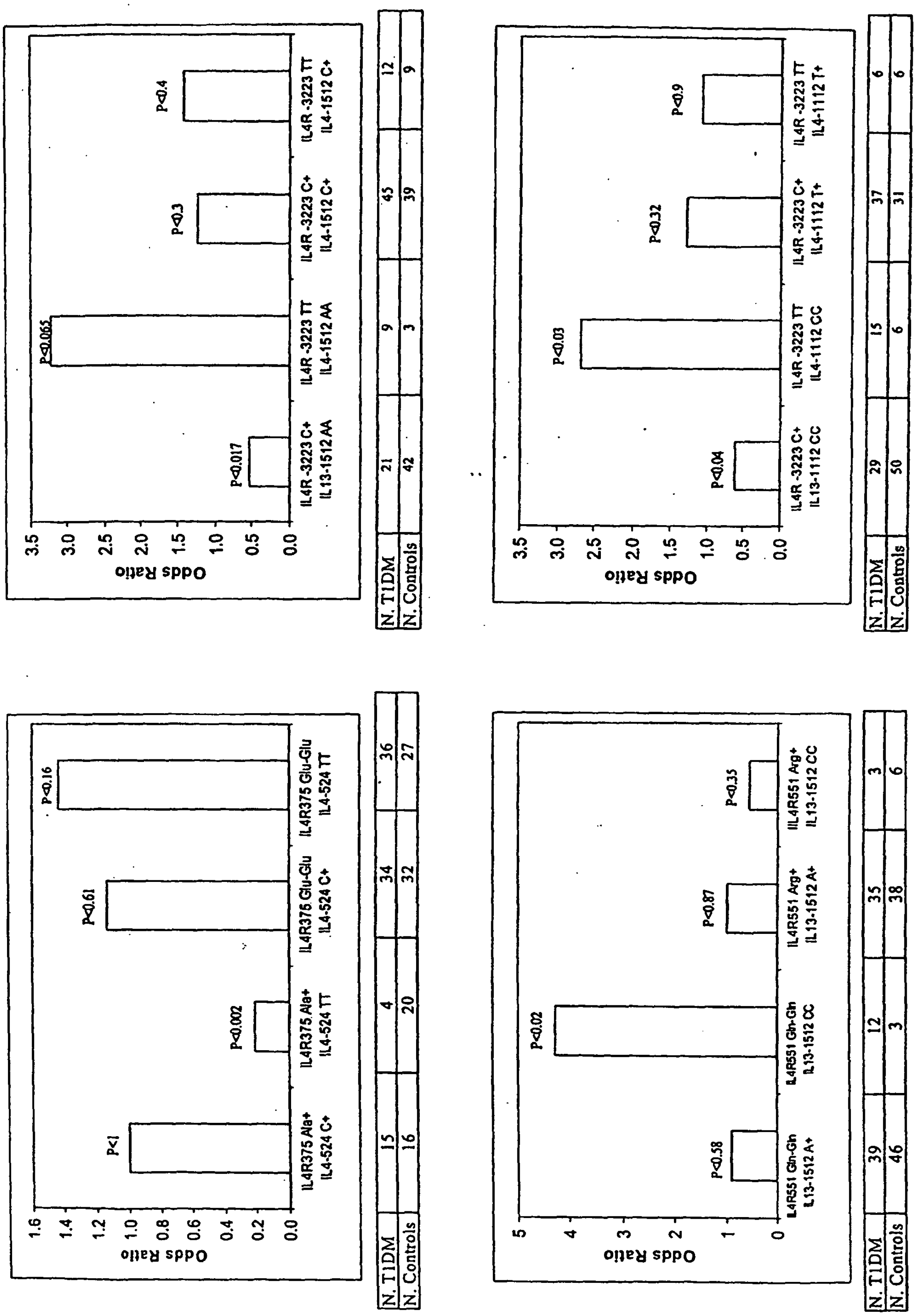
Odds Ratio
P<1
P<0.002
P<0.61
P<0.16
IL4R375 Ala+ IL4-524 C+
IL4R375 Ala+ IL4-524 TT
IL4R375 Glu-Glu IL4-524 C+
IL4R375 Glu-Glu IL4-524 TT
N. T1DM 15 4 34 36
N. Controls 16 20 32 27
P<0.017
P<0.065
P<0.3
P<0.4
IL4R -3223 C+ IL13-1512 AA
IL4R -3223 TT IL4-1512 AA
IL4R -3223 C+ IL4-1512 C+
IL4R -3223 TT IL4-1512 C+
N. T1DM 21 9 45 12
N. Controls 42 3 39 9
P<0.58
P<0.02
P<0.87
P<0.35
IL4R551 Gln-Gln IL13-1512 A+
IL4R551 Gln-Gln IL13-1512 CC
IL4R551 Arg+ IL13-1512 A+
IL4R551 Arg+ IL13-1512 CC
N. T1DM 39 12 35 3
N. Controls 46 3 38 6
P<0.04
P<0.03
P<0.32
P<0.9
IL4R -3223 C+ IL13-1112 CC
IL4R -3223 TT IL4-1112 CC
IL4R -3223 C+ IL4-1112 T+
IL4R -3223 TT IL4-1112 T+
N. T1DM 29 15 37 6
N. Controls 50 6 31 6

FIGURE 2